# EUROPEAN PATENT APPLICATION

(11) **EP 4 595 978 A1**
(43) Date of publication of application: **06.08.2025**
(21) Application number: 23870708.7
(22) Date of filing: 25.09.2023
(51) Int. Cl.: A61K 47/68, A61K 47/65, A61K 31/4745, A61P 35/00, A61P 35/02

(54) **ANTI-CD33 ANTIBODY AND ANTI-CD33 ANTIBODY-DRUG CONJUGATE AND USE THEREOF**

(30) Priority: 26.09.2022 CN 202211174484; 21.09.2023 CN 202311228281
(71) Applicant: SystImmune, Inc., Redmond WA 98052 (US)
(72) Inventor: ZHU, Yi, Chengdu, Sichuan 611130 (CN); WAN, Weili, Chengdu, Sichuan 611130 (CN); ZHUO, Shi, Chengdu, Sichuan 611130 (CN); ZHANG, Yong, Chengdu, Sichuan 611130 (CN); YU, Tianzi, Chengdu, Sichuan 611130 (CN)
(74) Representative: Mathys & Squire
(86) International application number: PCT/CN2023/121083
(87) International publication number: WO 2024/067477

(57) **Abstract**

The application discloses, among others, anti-CD33 antibodies and anti-CD33 antibody-drug conjugates and uses thereof, specifically anti-CD33 antibodies and/or antigen-binding fragments thereof, pharmaceutical compositions comprising antibody-drug conjugates and/or linker-payloads or the stereoisomers, pharmaceutically acceptable salts or solvents thereof, as well as use in the treatment of tumors or cancers.

## Description

### CROSS-REFERENCE TO RELATED APPLICATIONS

This application claims the benefit of the filing date of CN202211174484.2, filed September 26, 2022, and CN202311228281.1, filed September 21, 2023, the entire disclosures of which are incorporated by reference herein.

### TECHNICAL FIELD

The present invention relates to the field of biopharmaceuticals, relates to anti-CD33 antibodies and anti-CD33 antibody-drug conjugates and uses thereof, and specifically relates to an antibody-drug conjugate formed by an anti-human CD33 antibody and a drug having a cytotoxicity effect, as well as a method for making and use of the antibody-drug conjugate.

### BACKGROUND TECHNOLOGY

CD33 is a myeloid cell differentiation antigen consisting of 364 amino acids, a type I transmembrane glycoprotein with a molecular weight of 67 KD, which is predominantly distributed in myeloid cells during the initiation of differentiation. CD33 is a member of the immunoglobulin superfamily and contains two immunoglobulin-like extracellular structural domains, IgV and IgC2, as well as two intracellular structures of tyrosine-dependent signaling motifs, ITIMs, which can act as inhibitory receptors to regulate inhibitory signaling processes. For example, in the immune response, CD33 acts as an inhibitory receptor recruiting cytoplasmic phosphatases and inducing dephosphorylation of cytoplasmic phosphatases, thus hindering the transduction of signaling molecules. CD33 is also a member of the sialic acid-binding Ig-related lectin (SIGLEC) family, which recognizes cell surface glycans by binding to sialic acid, thereby participating in cell-cell interactions and mediating leukocyte-endothelial cell adhesion, etc. CD33 has been characterized as three selective shear types, namely the CD33m isoform with a smaller disulfide bond linking the V and C structural domains, the CD33M variant with a higher molecular weight, and CD33M with a deletion of the IgV structural domain.

CD33 is found to be expressed in the primitive granulocytes of more than 90% of patients with acute myeloid leukemia (AML) and is a specific leukemia antigen in myeloid cells. AML is a malignancy present in the blood system that is refractory, recurrent and treatment lethal. This fatal disease causes the bone marrow to produce abnormal myeloid cells, resulting in a dramatic decrease in red blood cell, platelet and white blood cell counts. Studies have shown that after targeted elimination of CD33-positive cells, their hematopoietic function can be restored after culture. Moreover, CD33 is not found to be expressed in hematopoietic stem cells, mature granulocytes, and other tissues, making CD33 an excellent target for specific immunotherapy of acute myeloid leukemia in the clinic.

Gentuzumab ozogamicin (Mylotarg, GO), an ADC drug targeting CD33, was approved for marketing by the U.S. Food and Drug Administration (FDA) in 2000. Mylotarg is made by conjugation anti-CD33 humanized IgG4 monoclonal antibody to calicheamicin and is mainly used for the treatment of patients with refractory or relapsed acute myeloid leukemia. When Mylotarg binds to CD33 antigen, it releases calicheamicin in cancer cells and induces apoptosis of tumor cells. However, the drug was voluntarily withdrawn from the market in 2010 due to poor clinical efficacy and side effects such as severe fatal liver injury. After withdrawal and adjustments to the dosing regimen, Mylotarg was re-approved by the FDA in 2017 for the first-line treatment of acute myeloid leukemia.

Among therapeutic agents targeting CD33, the humanized IgG1 monoclonal antibody lintuzumab did not perform better than cytarabine alone when used in combination with cytarabine in clinical trials, so the development of lintuzumab was terminated in 2010. SGN-CD33A, a CD33-targeting ADC clinical candidate developed by Seattle Genetics, Inc., had fatal infections and other side effects in the Phase III trial, and high patient mortality was observed. Therefore, the trial was terminated in 2017. The clinical study of AVE9633, an ADC drug conjugated from anti-CD33 IgG1 monoclonal antibody and DM-4, was terminated in 2009 due to poor clinical activity. There are also studies that conjugation vincristine, an alkaloid extracted from *Catharanthus Roseus,* with CD33 antibody peptides through carbodiimidation to promote the tumor targeting of vincristine and improve the efficacy of the drug. CD33 is an AML target verified by research, with sufficient average antigen density. In addition to ADC, it is also an antigen of choice for other new therapies. At present, immunotherapies targeting CD33 have been deployed by companies, including monoclonal antibodies, bispecific antibodies, trispecific antibodies, and ADCs in clinical evaluation or preclinical development.

Antibody-drug conjugate (ADC) is a new type of biological drugs that is made of monoclonal antibody (mAb) targeting specific antigens and small molecule drug (payload) with potent cytotoxicity conjugated by bioactive linker. ADC drugs recognize specifically by antibodyand guide small molecule drugs to cancer cell targets, are endocytosed into cancer cells and release cytotoxic drugs, thereby specifically kill cancer cells. ADC has been continuously innovated and optimized since its proposal and has achieved success in cancer treatment. Clinical studies have shown that ADC drugs have high efficacy and can effectively reduce the toxicity of small molecule cytotoxic drugs to healthy tissues, which is currently a hot spot in the research and development of anti-tumor drugs and have become one of the important means in the field of cancer treatment. CD33 is an excellent target for specific immunotherapy of AML, and ADC drugs have been approved for marketing, which shows that CD33 has the potential to be a target for ADC drug development.

### SUMMARY of THE INVENTION

The present disclosure provides an anti-human CD33 antibody-drug conjugate, which is obtained by conjugation a humanized anti-human CD33 antibody with a drug having a cytotoxic effect, as compared with existing drugs of the same type, has good molecular stability and good preclinical efficacy, and is expected to have an excellent clinical therapeutic effect.

To this end, a first aspect of the present disclosure provides an antibody-drug conjugate, or a stereoisomer, pharmaceutically acceptable salt or solvate thereof, as shown in general formula I. 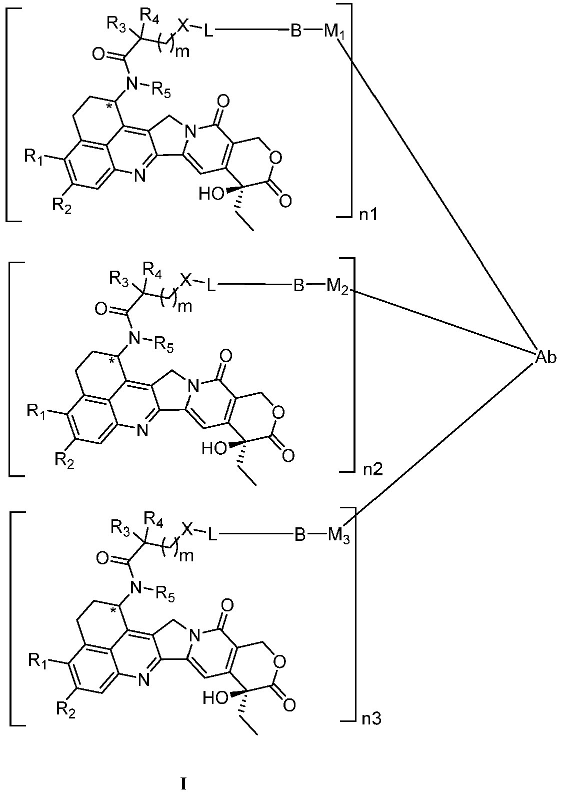 wherein:
The drug in said antibody-drug conjugate is a camptothecin antitumor drug or its stereoisomer thereof, as shown in formula II below 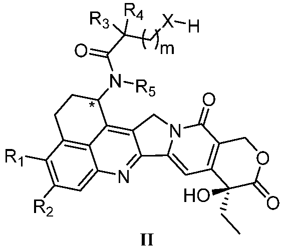
Ab is selected from anti-CD33 antibodies or antigen-binding fragments thereof;
M₁, M₂, and M₃ are each independently selected from 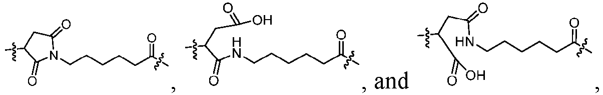
and M₁, M₂, and M₃ are not identical to each other; alternatively, M₁, M₂, and M₃ are each independently selected from the structure shown in formula A₁, formula A₂, and formula A₃, and M₁, M₂, and M₃ are not identical to each other; 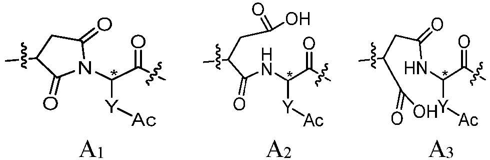
wherein Y is a scaffold selected from C1-C6 alkyl, substituted C1-C6 alkyl, or C3-C8 cycloalkyl; preferably Y is C1-C6 alkyl; Ac is a hydrophilic structural unit; and the position shown by the wavy line on the left is connected to Ab, and the position shown by the wavy line on the right is connected to B or connected to L;
B is present or absent, and when B is present it is selected from a modifier unit;
L is selected from linker units, preferably peptide-containing linker units;
X is selected from -NH-, -O- and -S-, preferably -O-;
R₁ and R₂ are the same or different and are each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, 5-10 heteroaryl, and substituted 5-10 heteroaryl;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, C6- C10 aryl C1-C6 alkyl, 5-10-membered heteroaryl, and substituted 5-10-membered heteroaryl; preferably R₃, R₄ are each independently selected from hydrogen, C1-C6 alkyl, substituted C1-C6 alkyl, C3-C8 cycloalkyl, C3-C8 cycloalkyl C1-C6 alkyl and C6-C10 aryl C1-C6 alkyl;
Alternatively, R₃, R₄ and the carbon atoms to which they are attached constitute a C3-C8 cycloalkyl group, a 3-7-membered heterocyclic group, a substituted 3-7-membered heterocyclic group; preferably R₃, R₄ and the carbon atoms to which they are attached constitute a C3-C8 cycloalkyl group;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl, R₅ is preferably selected from hydrogen, and C1-C6 alkyl;
m is chosen from the integers 0-5;
n1, n2, and n3 are each independently chosen from any integer or any decimal from 0 to 10. n1, n2, and n3 are not simultaneously 0 and 1 ≤ n1+n2+n3≤ 10 (e.g., n1+n2+n3 are 1, 1.5, 2, 2.5, 3, 3.5, 4, 4.5, 5, 5.5, 6, 6.5, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10; or for example, n1 + n2 + n3 is 8.01, 8.32, 8.56, 8.63, 8.66 or 9.40; or for example n1+n2+n3 is selected from any integer or any decimal from 7-7.5, 7.5-8, 8-8.5, 8.5-9, 9-9.5 or 9.5-10; preferably for example n1+n2+n3 is selected from any integer or any decimal from 7-10, i.e., 7≤ n1+n2+n3≤ 10, and more preferably for example n1+n2+n3 is selected from any integer or any decimal from 8-10, i.e. 8≤ n1+n2+n3≤ 10);
The chiral carbon atom shown at position * has a R or S absolute configuration;
and the following conditions are met: when M₁, M₂, and M₃ are each independently selected from 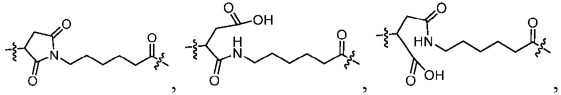 and M₁, M₂, and M₃ are not identical to each other, B exists and is selected from the modifier unit.

In some embodiments, said M₁, M₂, M₃ are each independently selected from 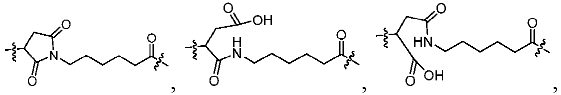 and M₁, M₂, M₃ are not identical to each other; alternatively, M₁, M₂, M₃ are each independently selected from the structure shown in formula A₁, formula A₂, formula A₃, and M₁, M₂, M₃ are not identical to each other;
Preferably, M₁, M₂, M₃ are each independently selected from the structures shown in formula A₁, formula A₂, formula A₃, and M₁, M₂, M₃ are not identical to each other; 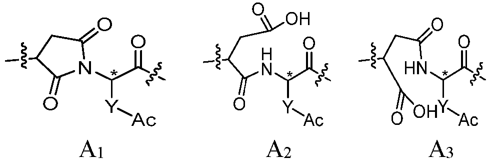
wherein Y is a scaffold selected from C1-C6 alkyl, substituted C1-C6 alkyl, and C3-C8 cycloalkyl; preferably Y is C1-C6 alkyl, more preferably C1-C3 alkyl, and most preferably methylene;
Position shown by * has two chiralities, either R or S absolute configuration;
The position shown by the left wavy line is connected to Ab and the position shown by the right wavy line is connected to B or L;

The hydrophilic structural unit Ac is selected, without limitation, from natural or non-natural amino acids, 1-20 polyethylene glycols, phosphate groups, carboxylic acid groups, sulfonic acid groups, sulfinic acid groups, or the following structures. 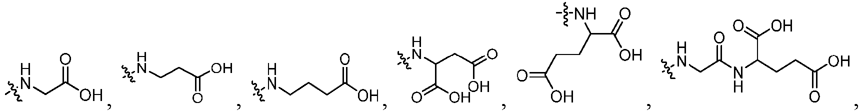 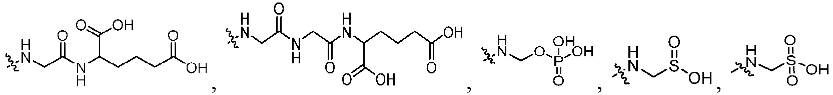 or 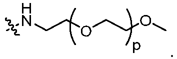 wherein p is selected as an integer from 0 to 10; the position shown by the wavy line is connected to the bracket Y;

Preferably, the hydrophilic structural unit Ac is selected from 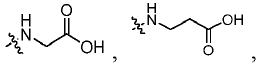 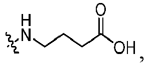 and is connected to the bracket Y at the position shown by the wavy line;
More preferably, the hydrophilic structural unit Ac is 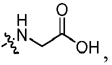 connected to the bracket Y at the position shown by the wavy line.

In some embodiments, M₁, M₂, M₃ are selected from any combination of (1)-(7) below, and M₁, M₂, M₃ are not identical to each other.
(1) 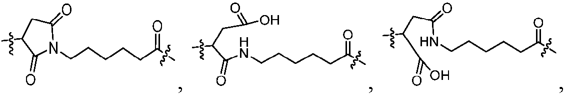
(2) 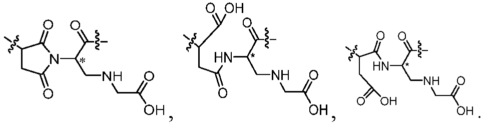
(3) 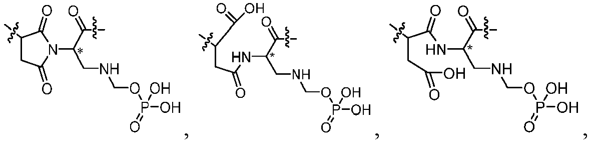
(4) 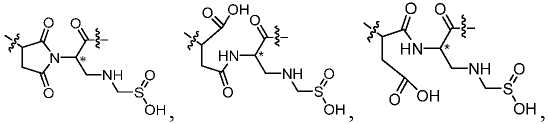
(5) 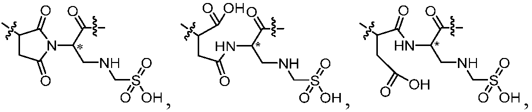
(6) 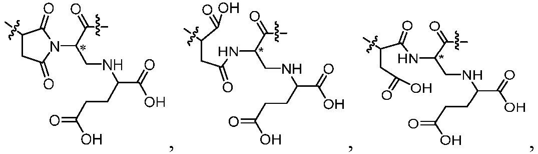
(7)

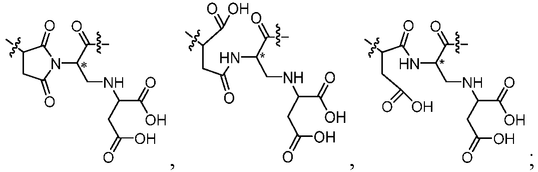

Preferably, M₁, M₂, M₃ are selected from any of the following combinations (1)-(2), and M₁, M₂, M₃ are not identical to each other, the
(1) 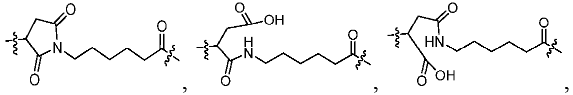
(2)

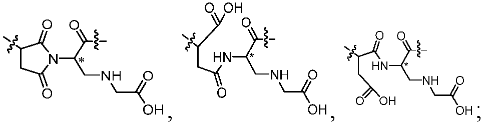

More preferably, M₁, M₂, M₃ are each independently selected from 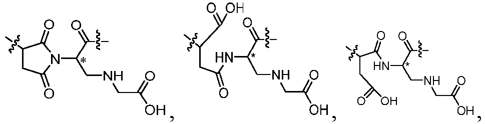 and
M₁, M₂, M₃ are not identical to each other;

Wherein:
Position * has either the R- or S-absolute configuration;
The position shown by the left wavy line is connected to Ab; the position shown by the right wavy line is connected to modification unit B or connection unit L.

In some embodiments, modifying unit B is present or absent, and when said modifying unit B is present, it is selected, without limitation, from a structure that enhances hydrophilicity or a stereoisomer thereof.

In some embodiments, modifier unit B is present or absent, and when said modifier unit B is present, said modifier unit B is selected, without limitation, from a structure that enhances hydrophilicity or a stereoisomer thereof as shown in the following formula.

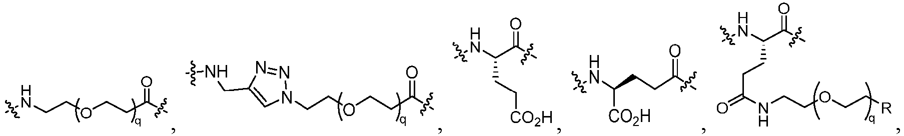

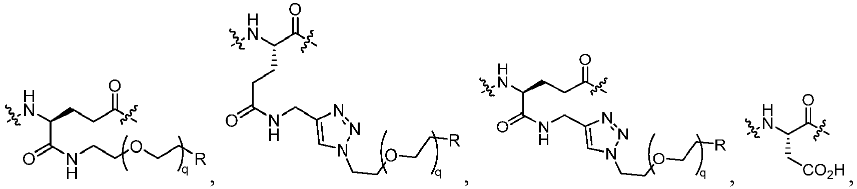

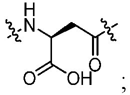

Preferably, modifier unit B is present or absent, and when said modifier unit B is present, said modifying unit B is non-limitingly selected from a structure that enhances hydrophilicity or a stereoisomer thereof as shown in the following formula. 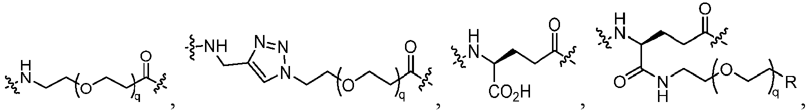 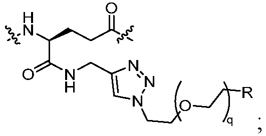 wherein:
R is selected, without limitation, from one or a combination of hydroxyl, amino, polyethylene glycol, carboxylic acid group, sulfonic acid group, sulfinic acid group, phosphoric acid group, C1-C6 alkyl, C1-C6 alkoxy, natural or non-natural amino acid residues, sugar or derivatives thereof;
Preferably, R is selected from C1-C6 alkoxy, carboxylic acid group, and amino group;
More preferably, R is selected from methoxy, carboxylic acid group, and amino group;
The position shown by the wavy line on the left is connected to M₁, M₂, or M₃;
The position shown by the wavy line on the right is connected to the connection unit L;
q is selected from integers 1-10 (preferably from integers 2-8), preferably from 2, 7, 8;
Most preferably, said modifier unit B is selected from a structure that enhances hydrophilicity or a stereoisomer thereof as shown in the following formula:

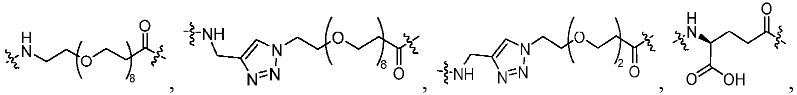

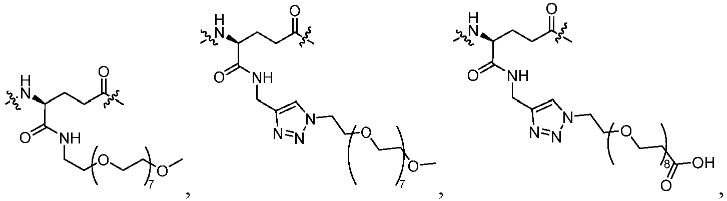

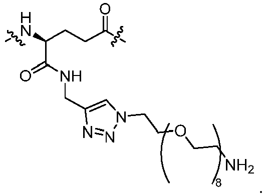

The position shown by the left wavy line is connected to M₁, M₂, or M₃, and the position shown by the right wavy line is connected to the linker unit L.

In some embodiments, said linker unit L is L₁-L₂,
L₁ is a peptide residue comprising 2-10 amino acid residues, said peptide residue having an amino terminal connected to the modifier unit B or to M₁, M₂, M₃ and a carbonyl terminal connected to L₂;
Preferably, L₁ is a peptide residue comprising 2-4 amino acid residues, said peptide residue having the amino terminal connected to modifier unit B or connected to M₁, M₂, M₃ and the carbonyl terminal connected to L₂; preferably, said amino acid is selected from valine, alanine, phenylalanine, glycine, lysine, citrulline, serine, glutamic acid, and aspartic acid; more preferably, said amino acid is selected from valine, alanine, phenylalanine, glycine, lysine, and citrulline; most preferably, said amino acid is selected from valine, phenylalanine, glycine, lysine, and citrulline;
More preferably, L₁ is selected from the following peptide residues: glycine-glycine-phenylalanine-glycine, valine-citrulline, phenylalanine-lysine, valine-alanine, and alanine-alanine-alanine (preferably selected from glycine-glycine-phenylalanine-glycine, valine-citrulline, and phenylalanine-lysine), the amino terminal of said peptide residues is connected to modifier unit B or to M₁, M₂, M₃ and the carbonyl terminal is connected to L₂;
Most preferably, L₁ is selected from the following peptide residues: 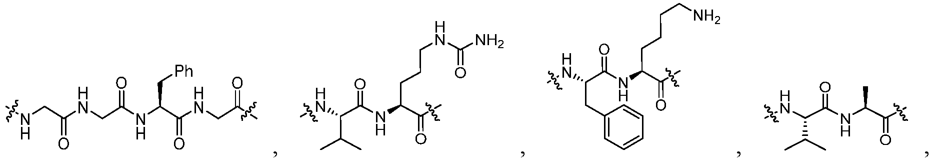 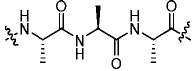
(Preferably selected from 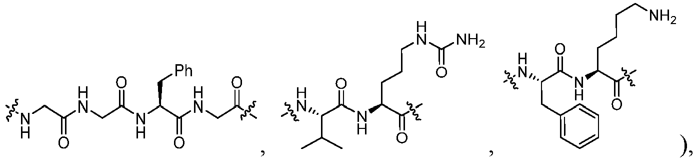
said peptide residues having an amino terminal connected to the modifier unit B or to M₁, M₂, M₃ and a carbonyl terminal connected to L₂;
L₂ is selected from 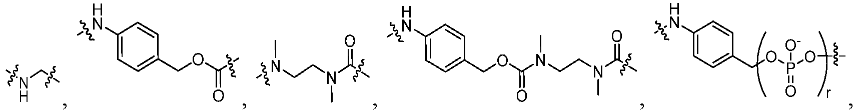
and is connected to L₁ at the position shown by the left wavy line, and to X at the position shown by the right wavy line;
Preferably, L₂ is selected from 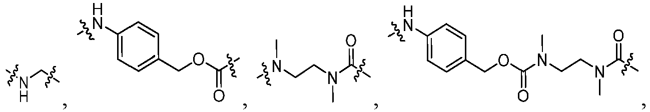
and is connected to L₁ at the position shown by the left wavy line, and is connected to X at the position shown by the right wavy line;
More preferably, L₂ is selected from 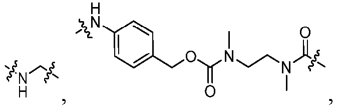 which is connected to L₁ at the position shown by the left wavy line, and is connected to X at the position shown by the right wavy line;

Preferably, said linker unit L is selected, without limitation, from the following structures or stereoisomers thereof. 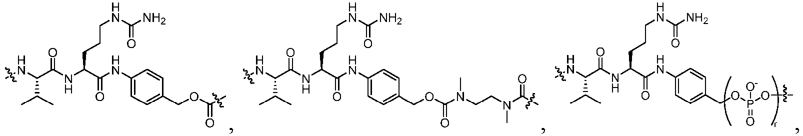 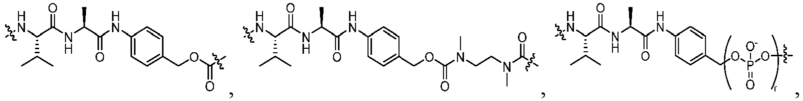 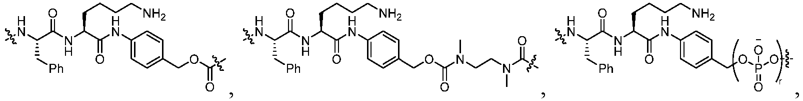 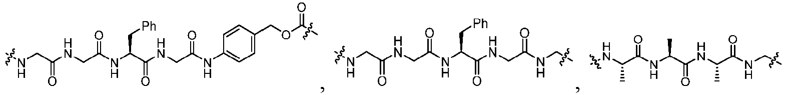 or 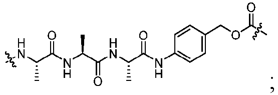 wherein:
r is chosen from the integers 1-10;
More preferably, said linker unit L is selected from the following structures or stereoisomers thereof: 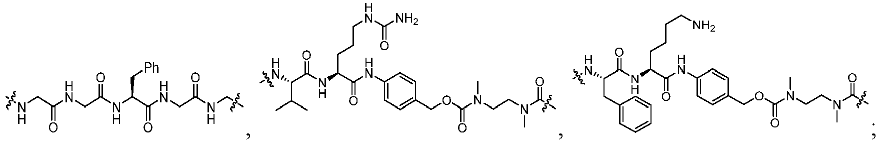
Said linker unit L is connected to the modifier unit B or to M₁, M₂, M₃ at the position shown by the wavy line on the left side;
Said linker unit L is connected to X at the position shown by the wavy line on the right side.

In some embodiments, R₁ and R₂ are the same or different, and each is independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclic, substituted 3-7-membered heterocyclic, C6-C10 aryl, substituted C6 -C10 aryl, 5-10 heteroaryl, and substituted 5-10 heteroaryl;
Preferably, R₁ and R₂ are the same or different and are each independently selected from halogen, and C1-C6 alkyl;
More preferably, R₁ is selected from C1-C6 alkyl and R₂ is selected from halogen;
Most preferably, R₁ is methyl and R₂ is fluorine;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclic, substituted 3-7-membered heterocyclic, C6-C10 aryl, substituted C6-C10 aryl, C6- C10 aryl C1-C6 alkyl, 5-10-membered heteroaryl, and substituted 5-10-membered heteroaryl;
Preferably, R₃, R₄ are the same or different, each independently selected from hydrogen, C1-C6 alkyl, substituted C1-C6 alkyl, C3-C8 cycloalkyl, C3-C8 cycloalkyl C1-C6 alkyl and C6-C10 aryl C1-C6 alkyl;
Further preferably, R₃, R₄ are the same or different, each independently selected from hydrogen, C1-C6 alkyl, halo-C1-C6 alkyl, C3-C6 cycloalkyl, C3-C6 cycloalkyl C1-C6 alkyl, and phenyl C1-C6 alkyl;
More preferably, R₃, R₄ are the same or different, each independently selected from hydrogen, methyl, ethyl, trifluoromethyl, cyclopropyl, cyclopropylmethyl, and benzyl;
Most preferably, of R₃, R₄, any one is selected from hydrogen, methyl, and the other is selected from hydrogen, methyl, ethyl, trifluoromethyl, cyclopropyl, cyclopropylmethyl, benzyl;
Alternatively, R₃, R₄ and the carbon atoms to which they are attached constitute a C3-C8 cycloalkyl group, a 3-7-membered heterocyclic group, a substituted 3-7-membered heterocyclic group;
Preferably, R₃, R₄ and the carbon atoms to which they are attached form a C3-C8 cycloalkyl group;
More preferably, R₃, R₄ and the carbon atoms to which they are attached constitute a C3-C6 cycloalkyl group;
Most preferably, R₃, R₄ and the carbon atom to which it is attached constitute a cyclopropyl, cyclobutyl or cyclopentyl group;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl, R₅ is preferably selected from hydrogen, and C1-C6 alkyl, R₅ is more preferably selected from hydrogen, and methyl;
X is selected from -NH-, -O- and -S-, preferably -O-;
m is selected from an integer from 0 to 5, preferably selected from 0, 1.

In some embodiments, said camptothecin antitumor drug is selected, without limitation, from the following compounds or stereoisomers thereof.

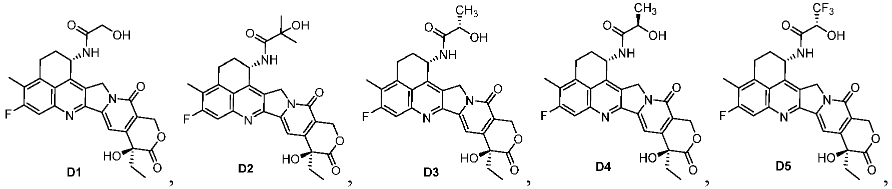

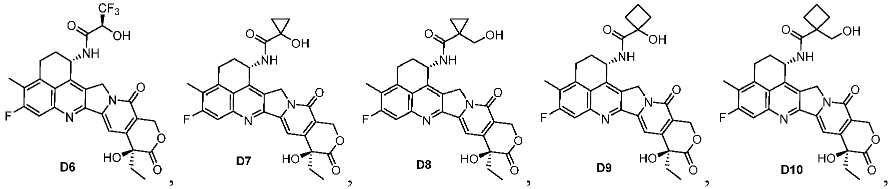

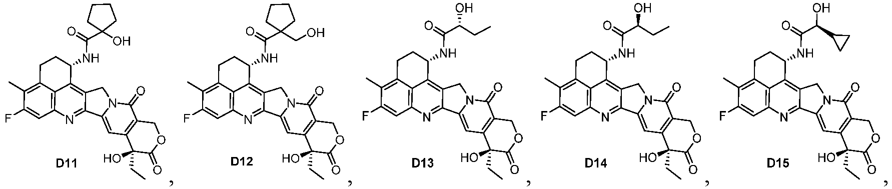

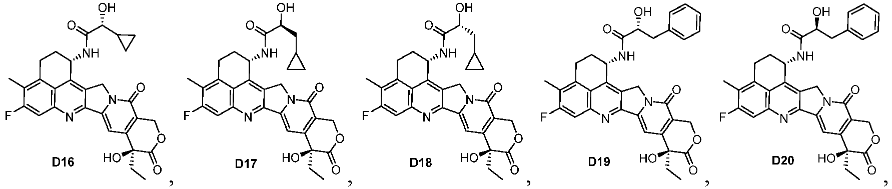

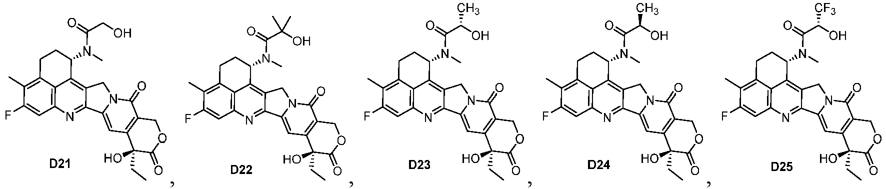

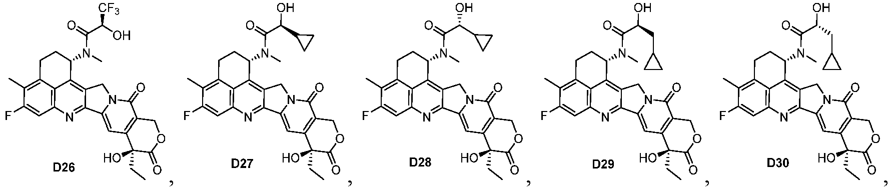

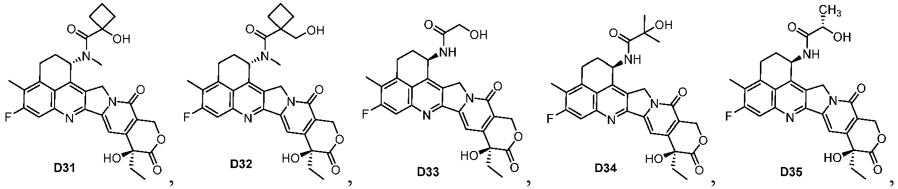

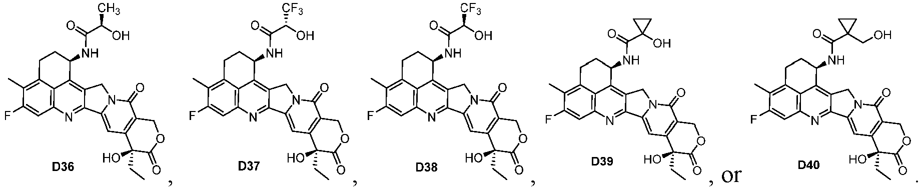

In some embodiments, said antibody-drug conjugate is selected, without limitation, from the following structures: 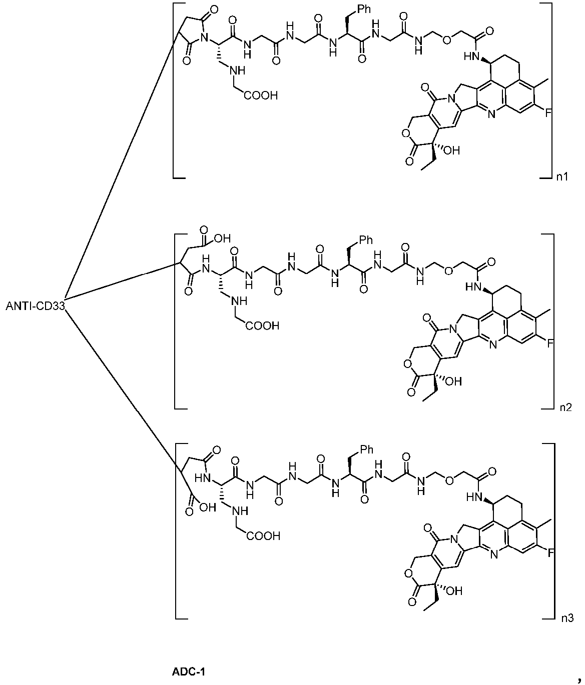 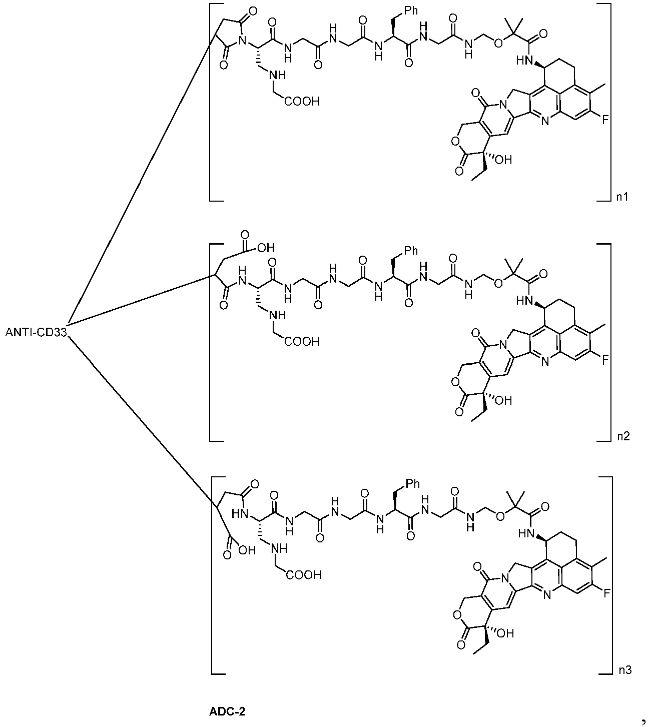 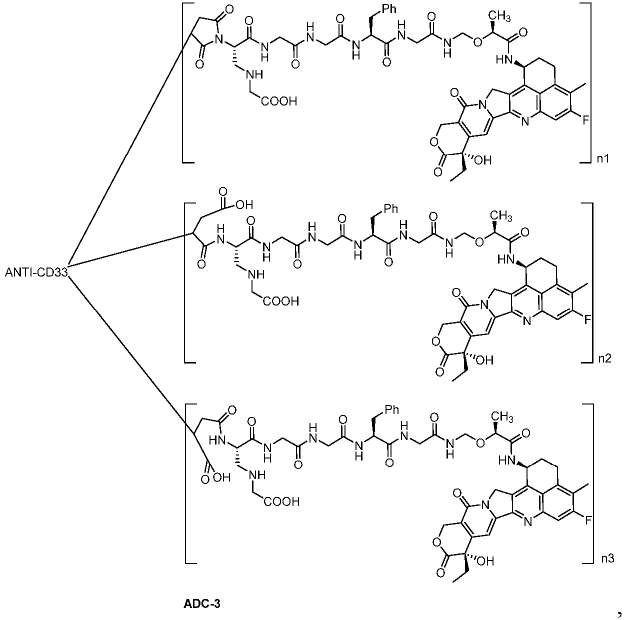 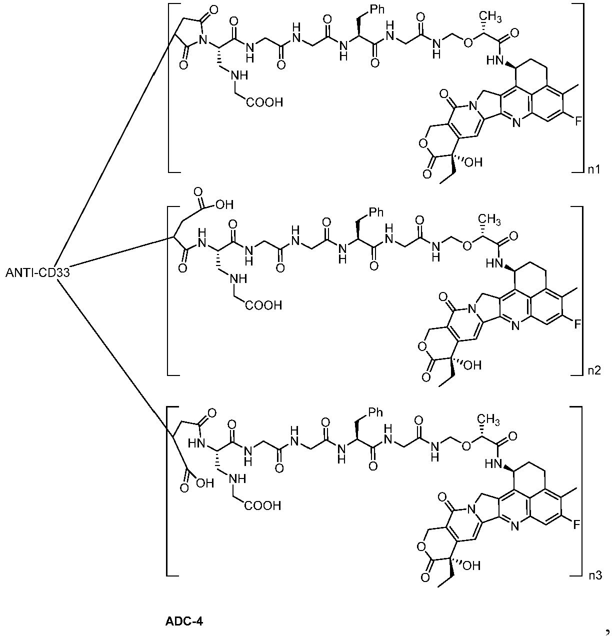 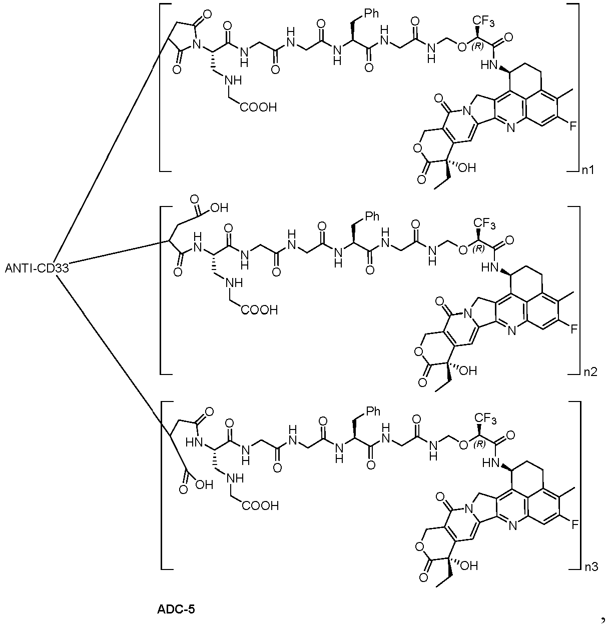 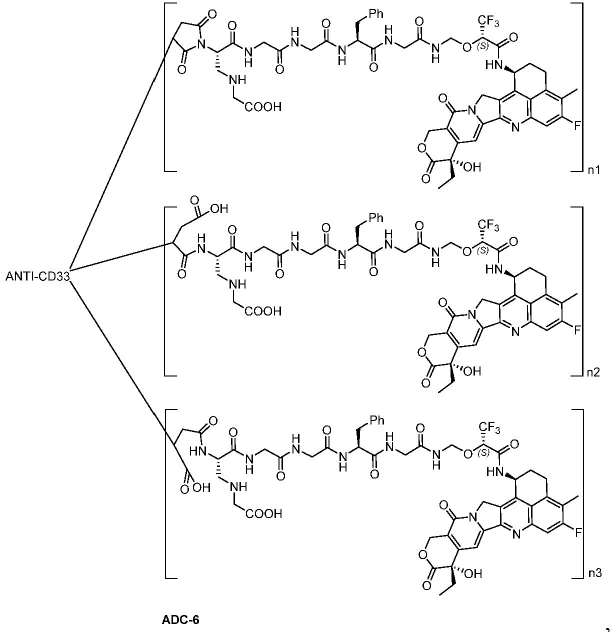 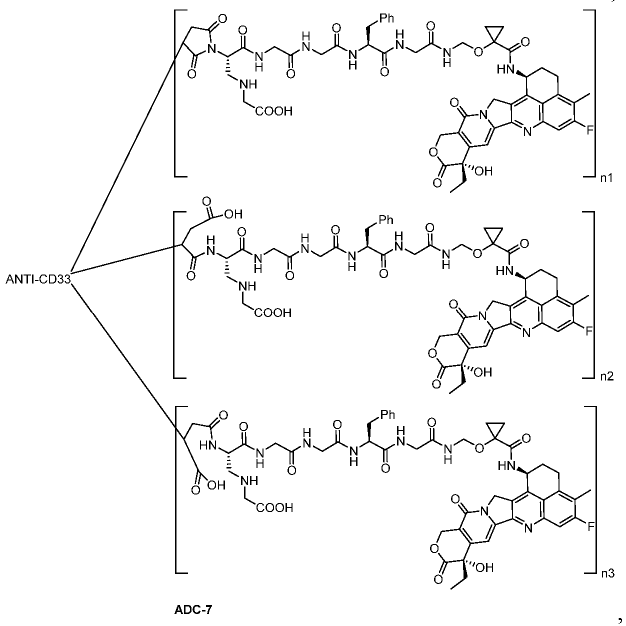 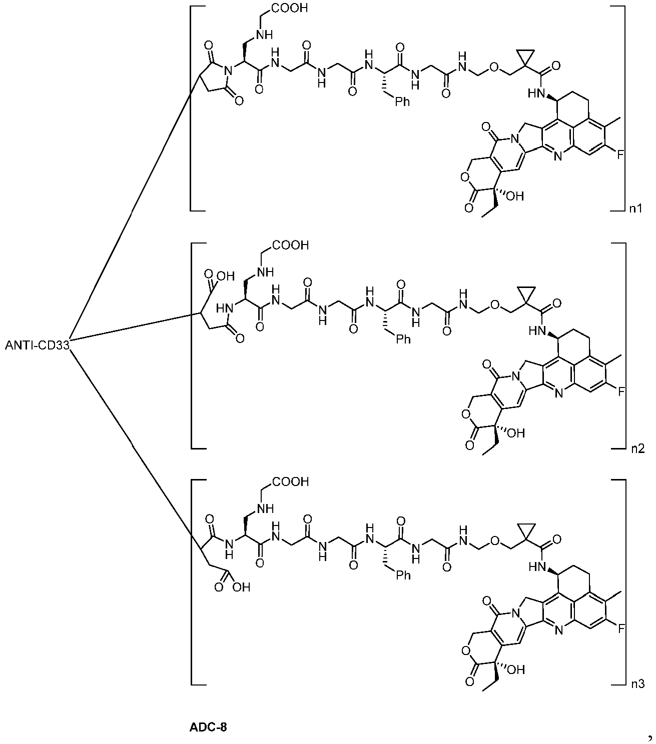 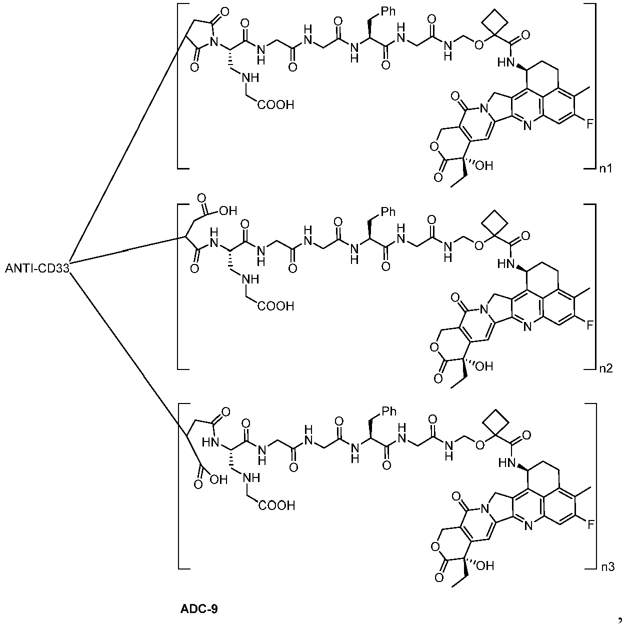 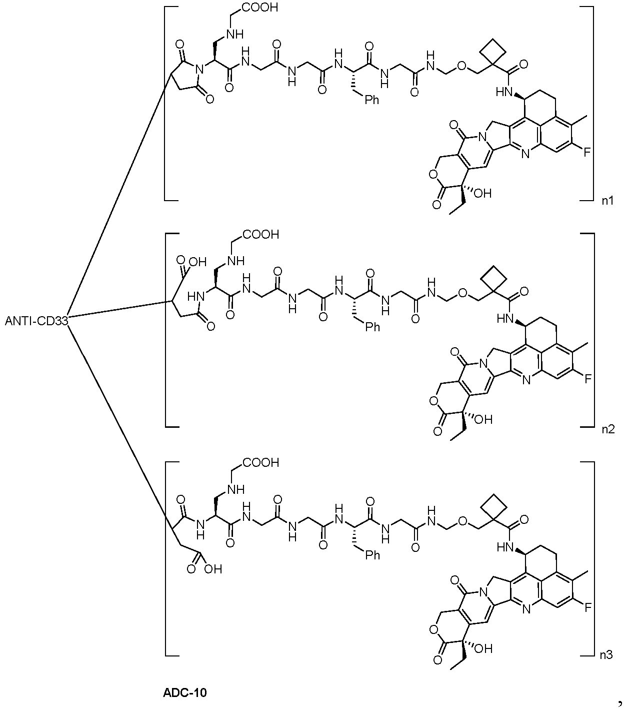 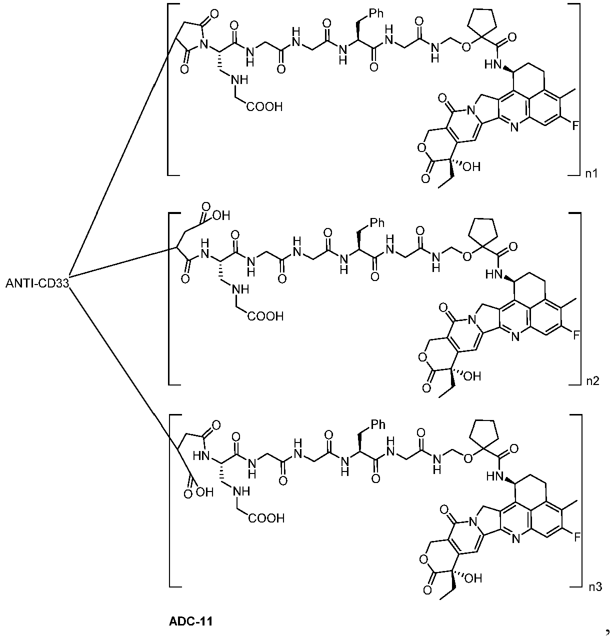 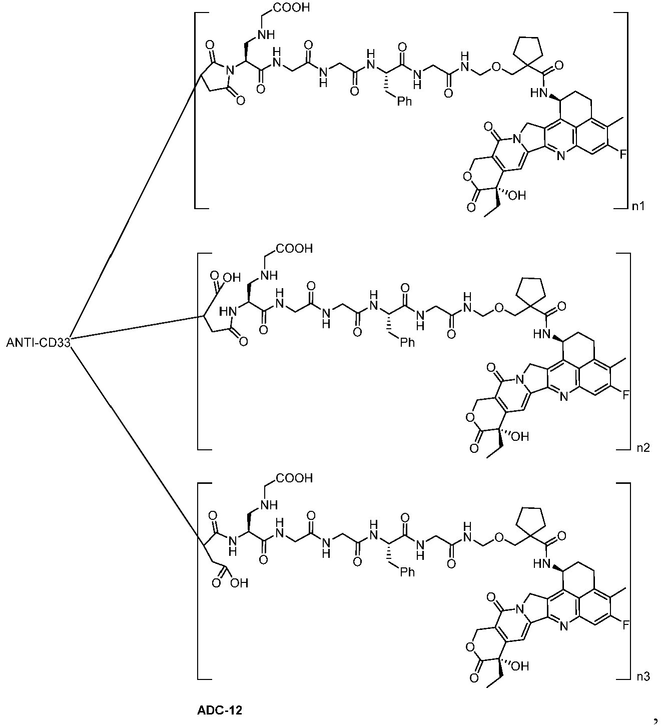 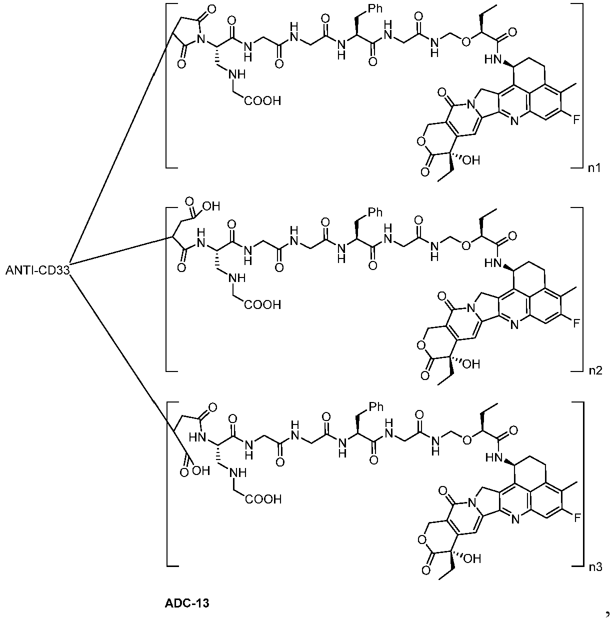 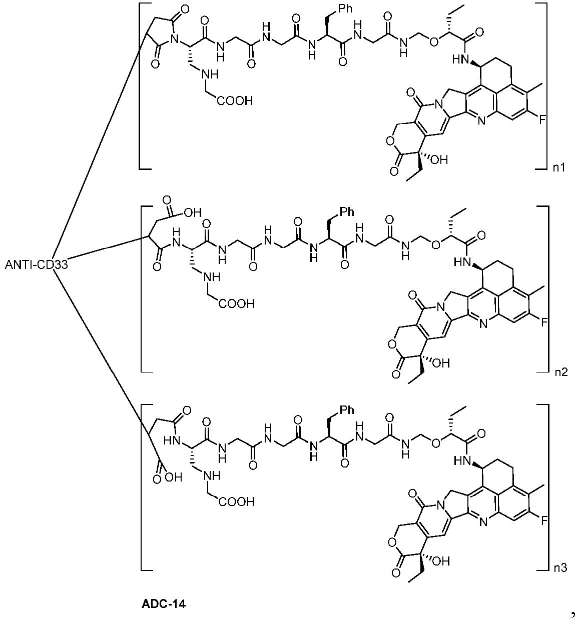 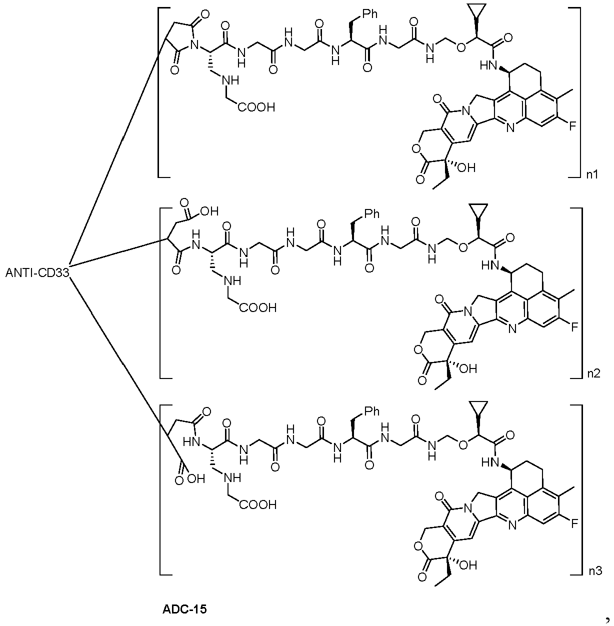 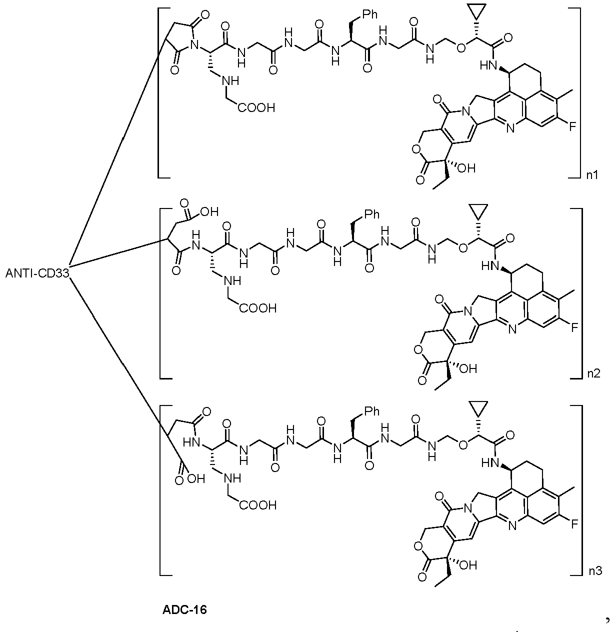 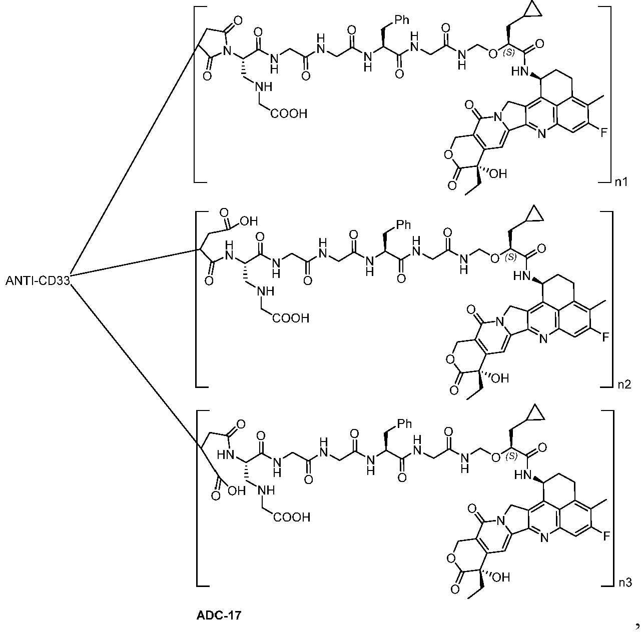 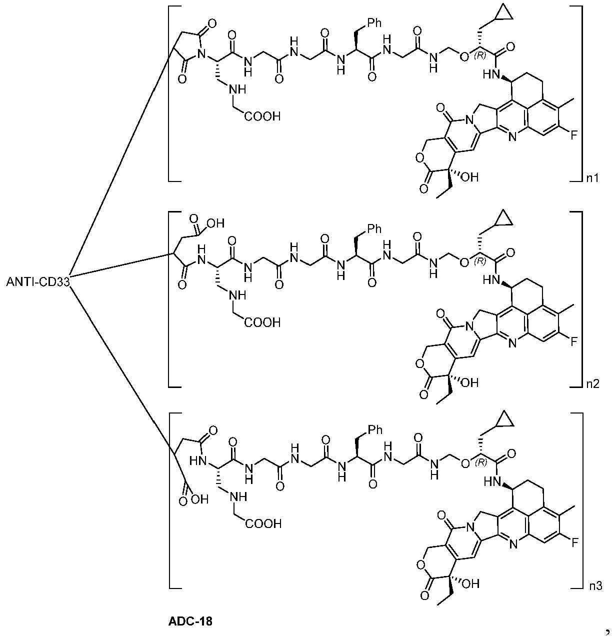 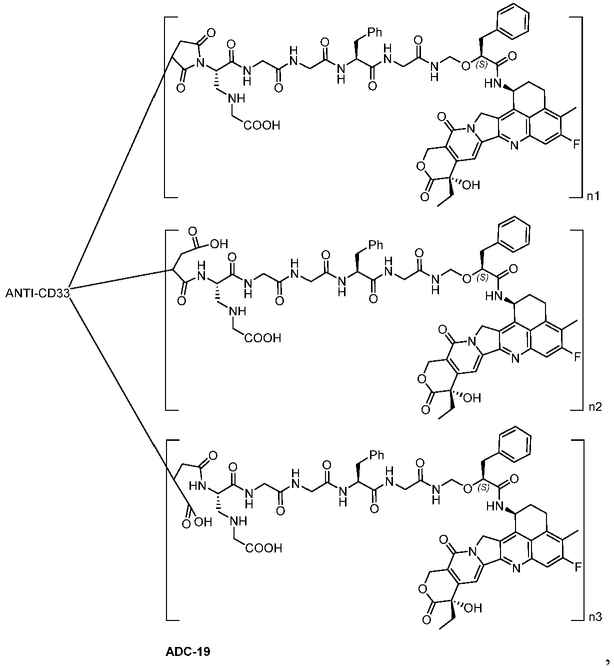 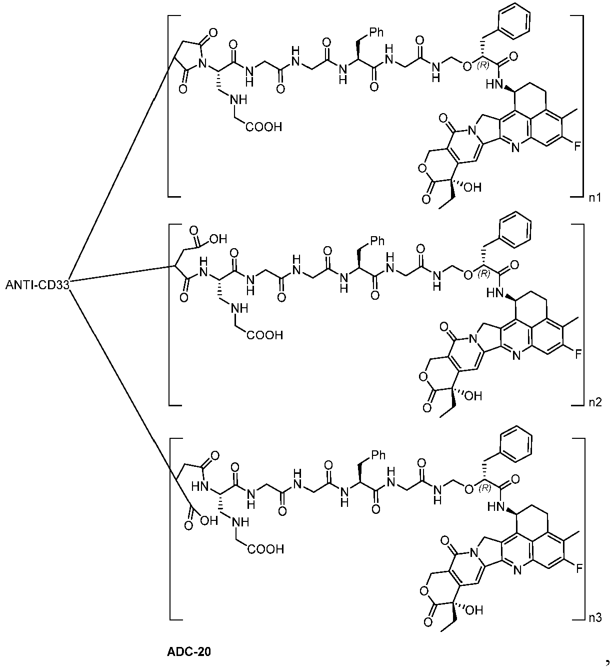 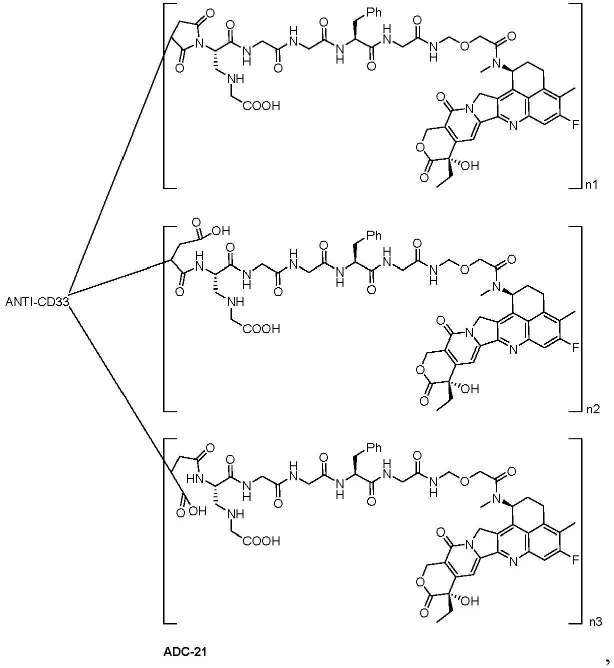 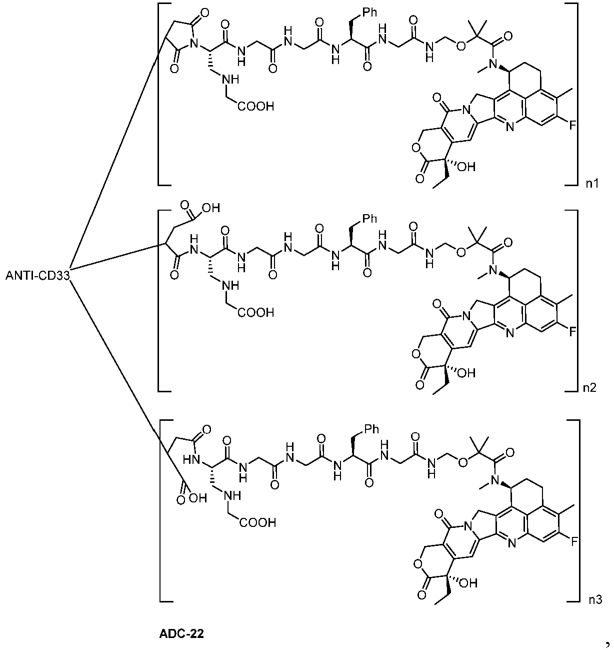 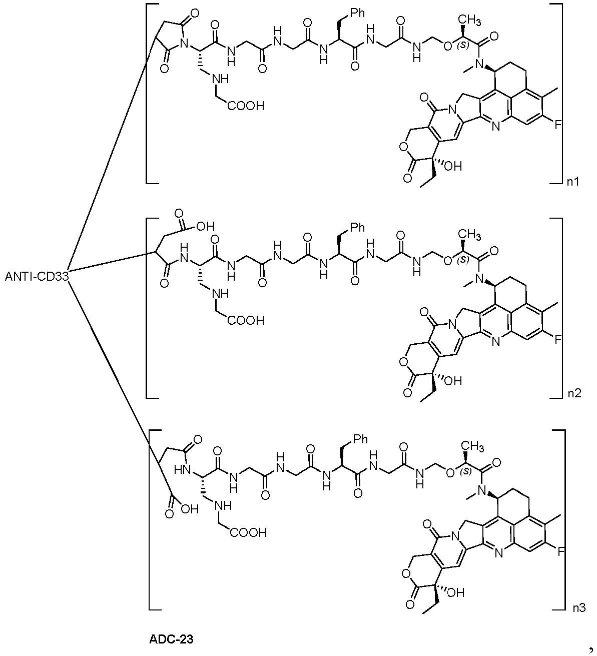 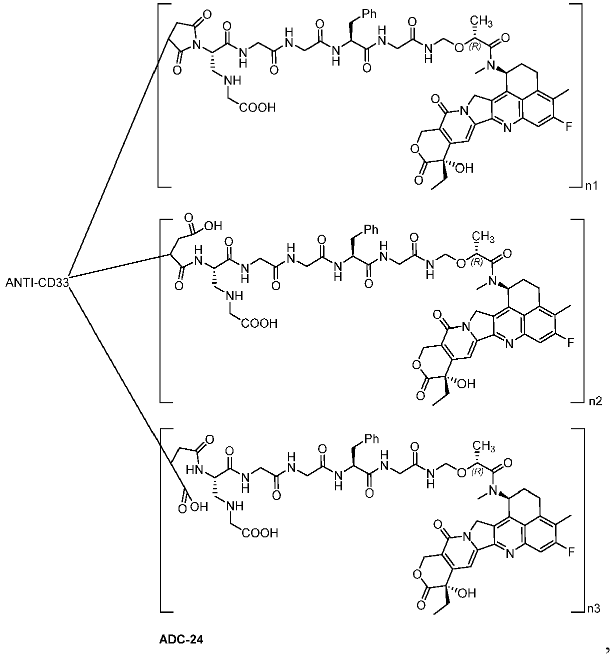 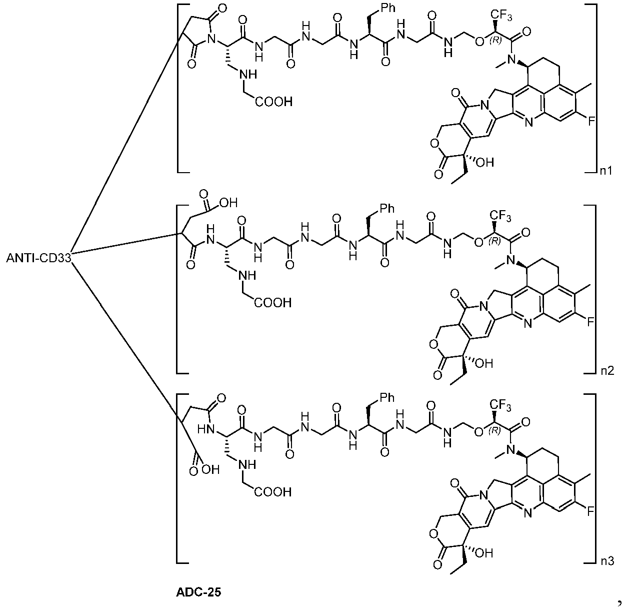 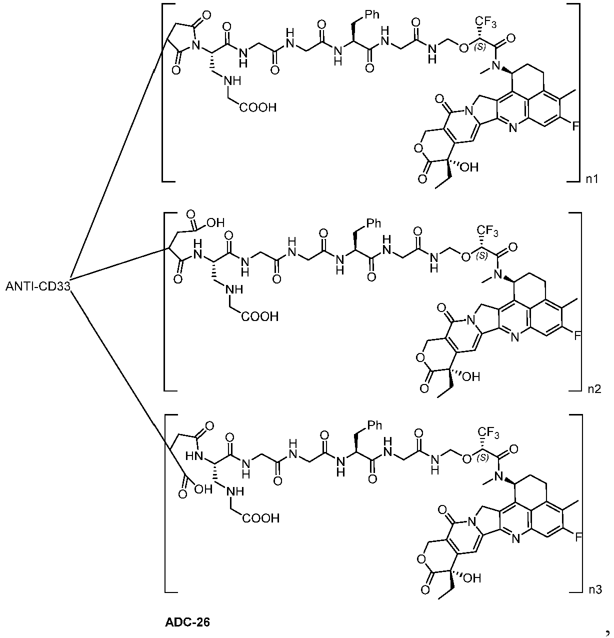 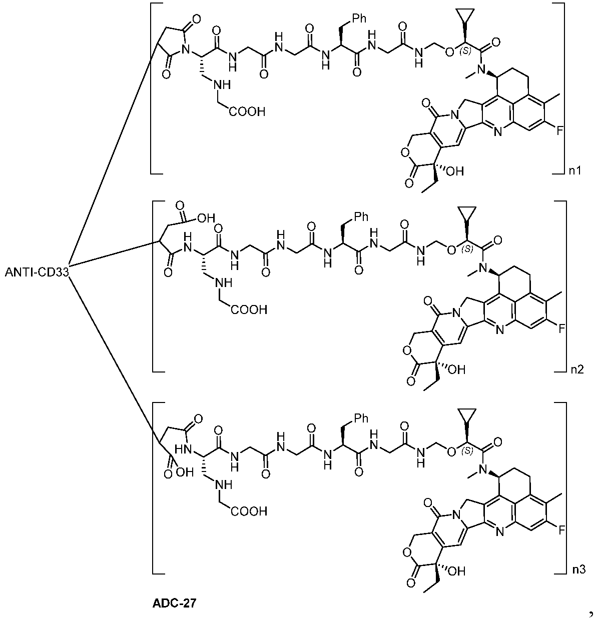 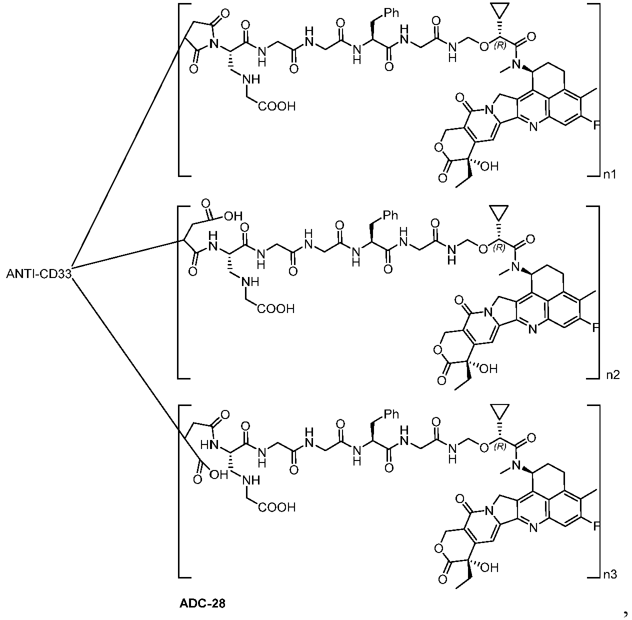 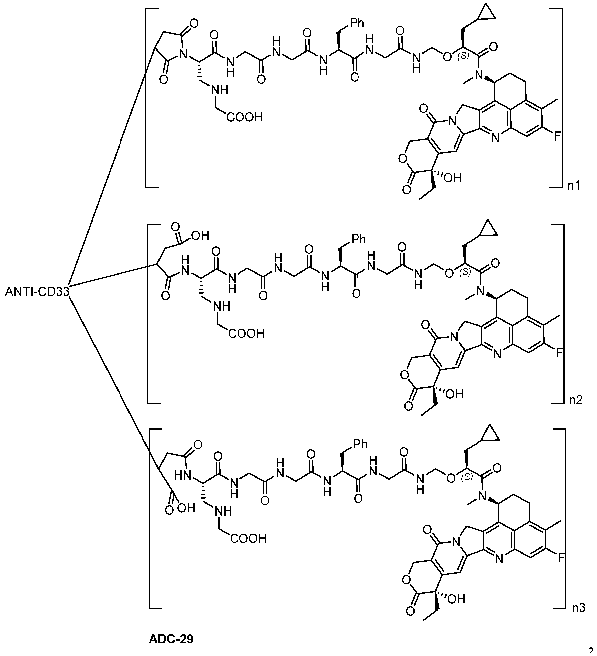 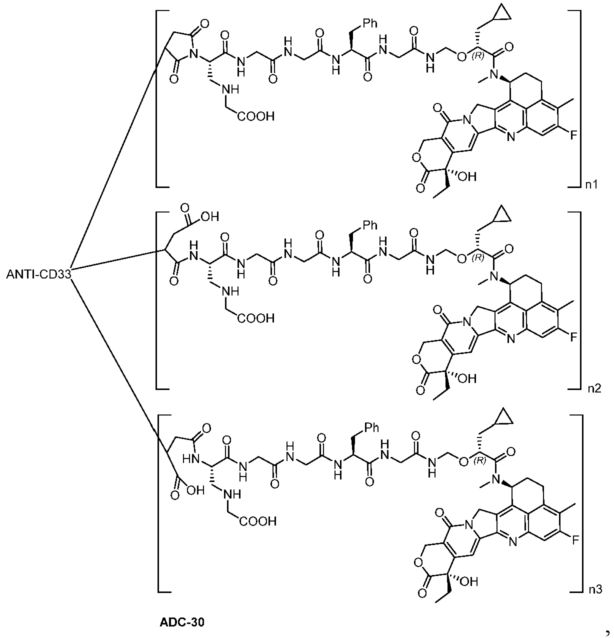 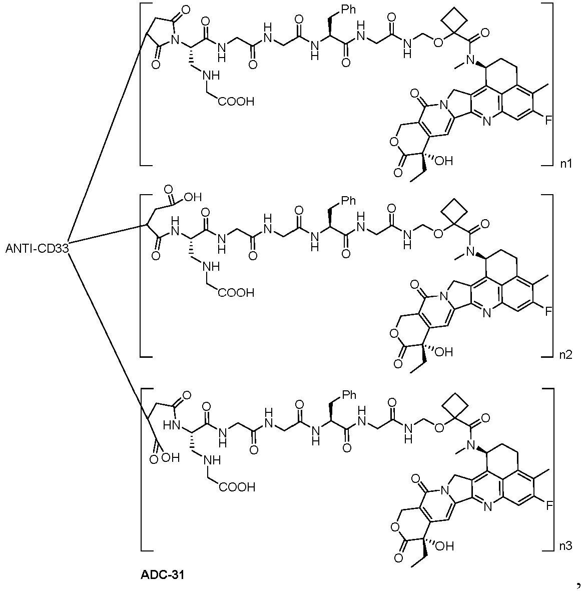 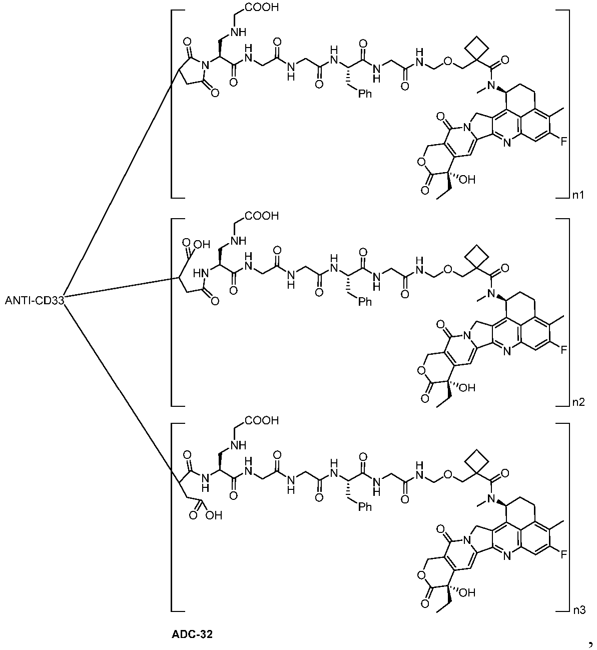 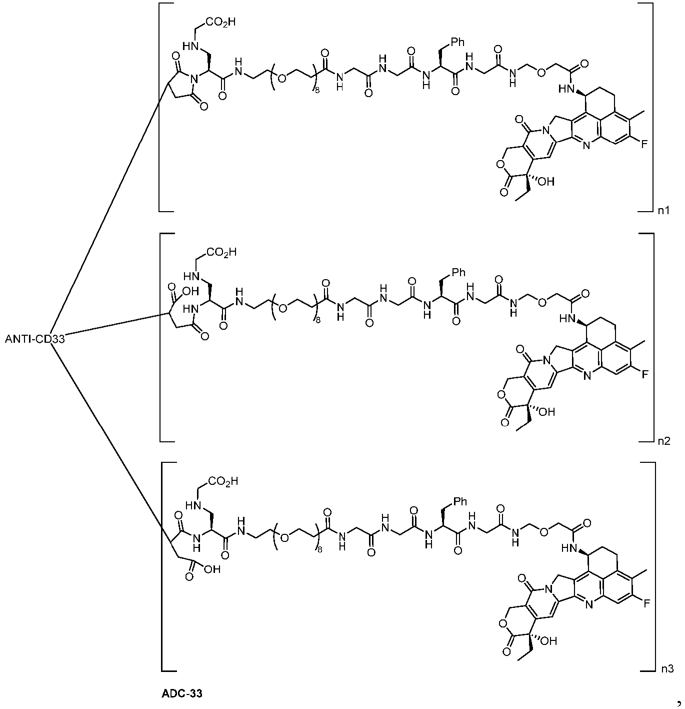 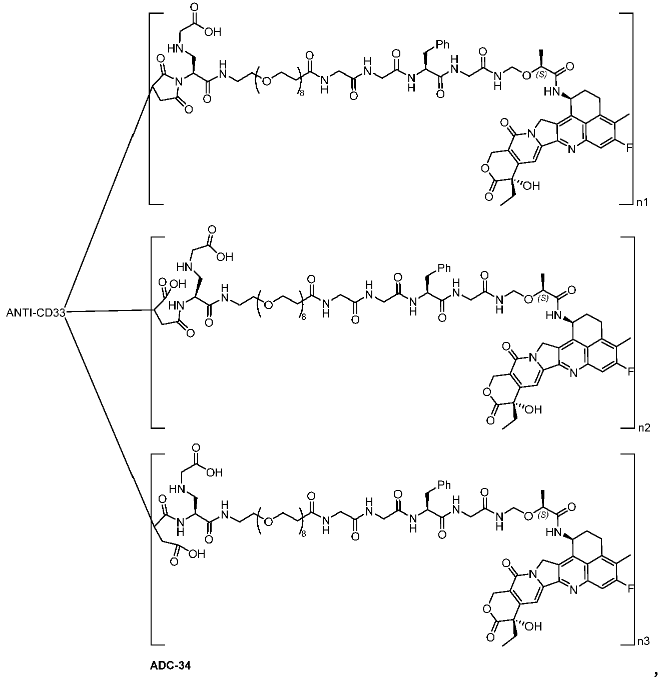 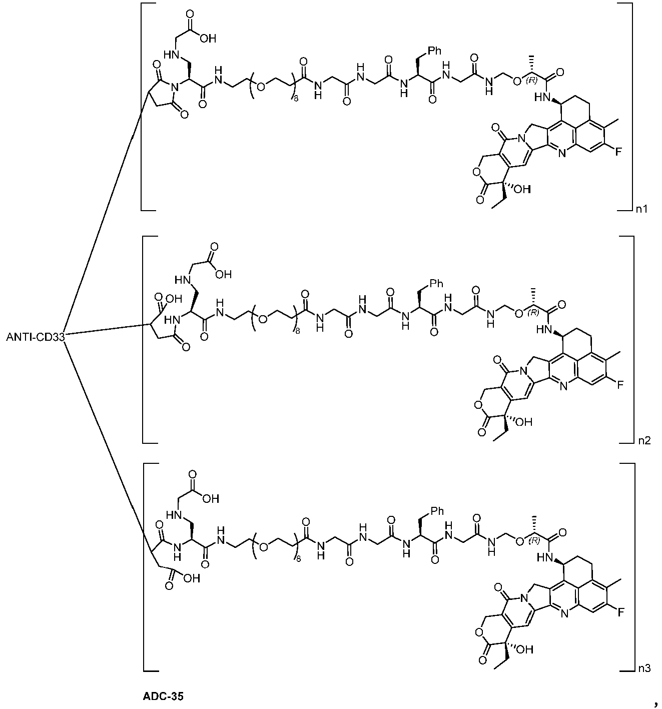 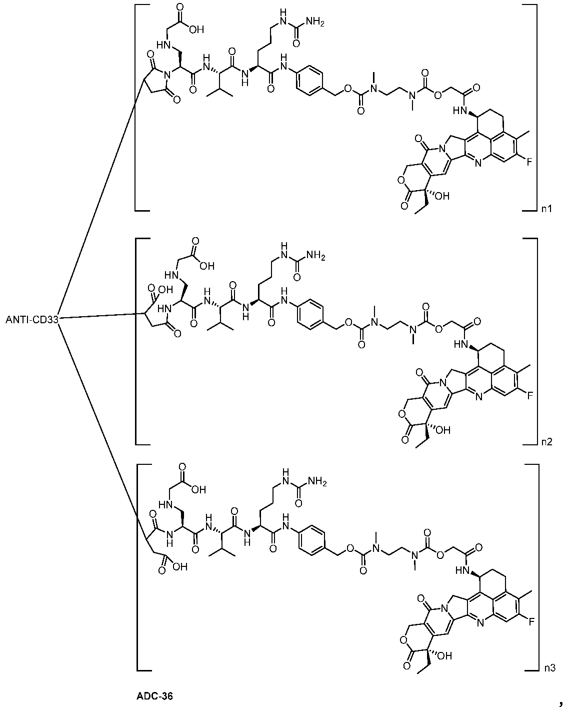 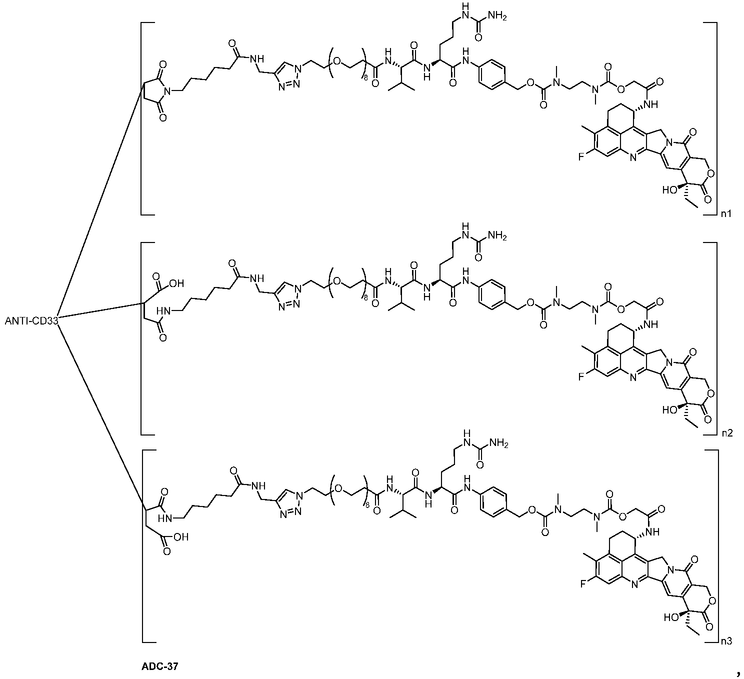 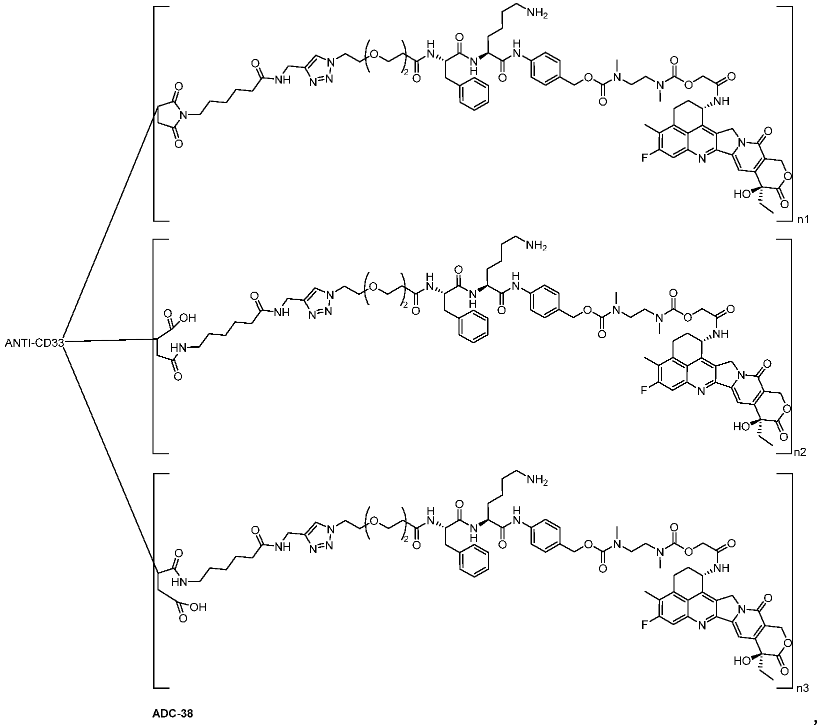 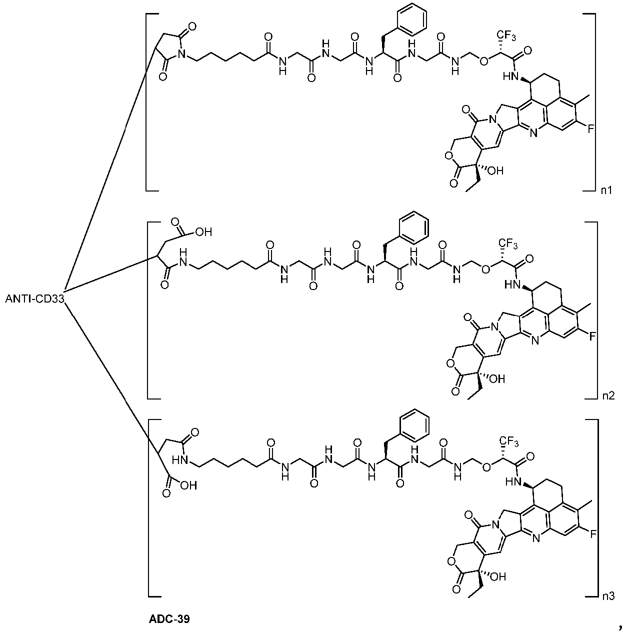 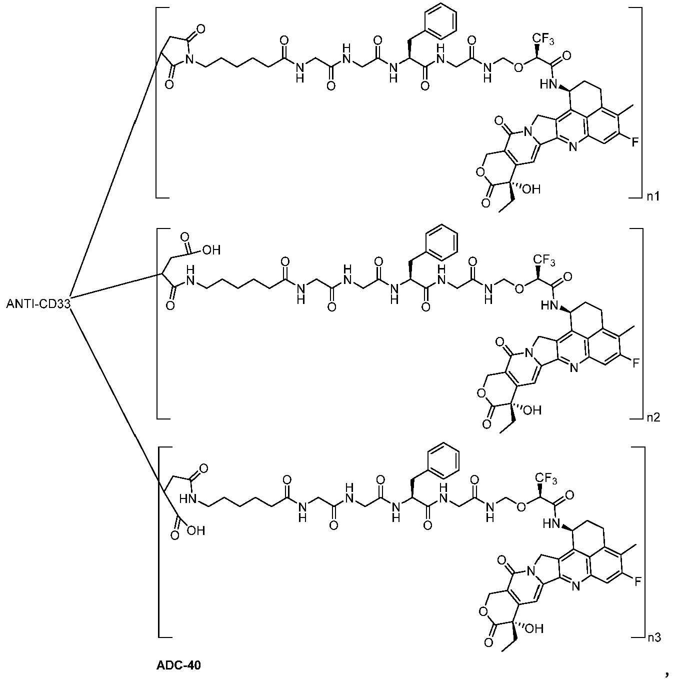 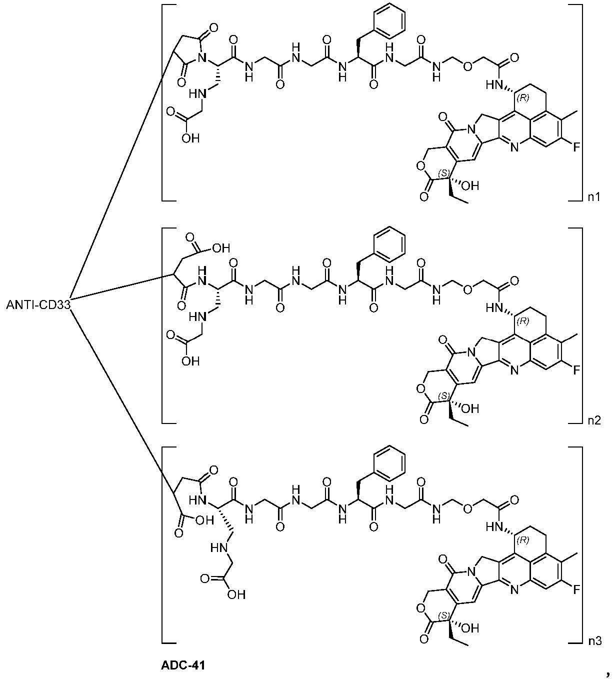 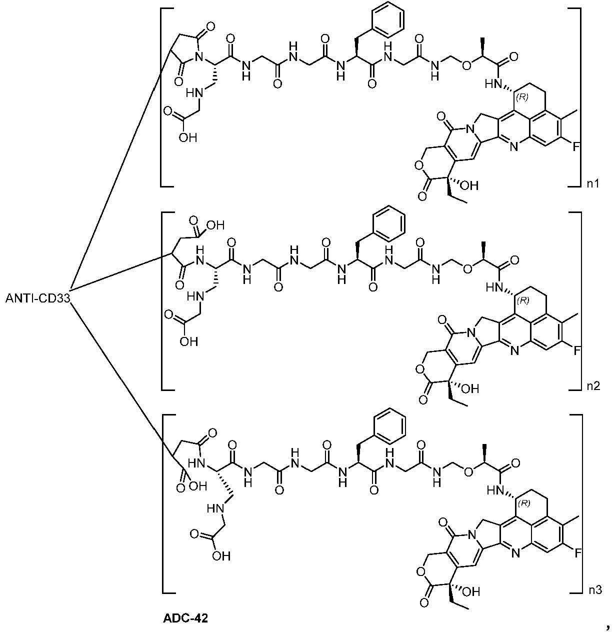 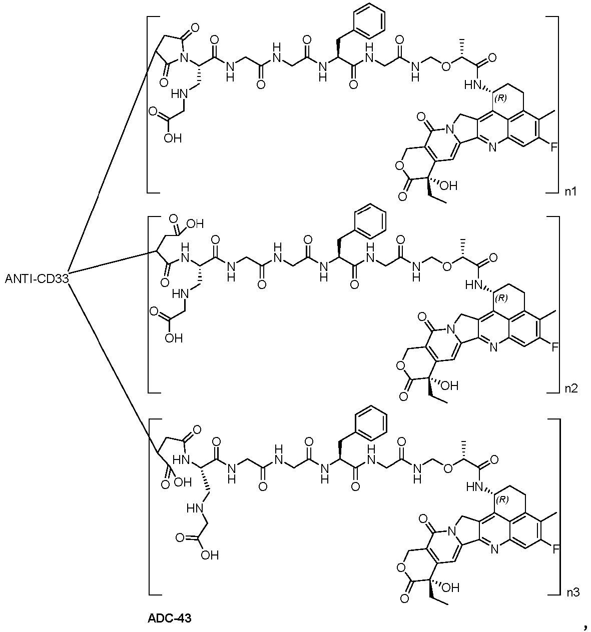 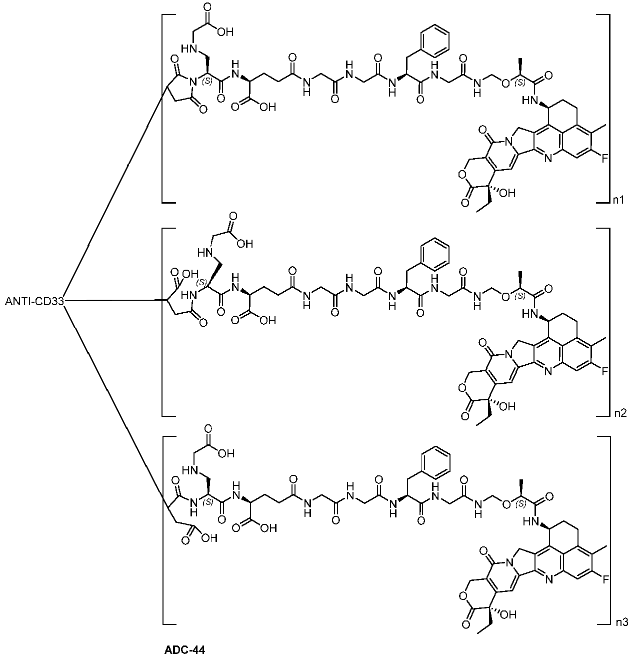 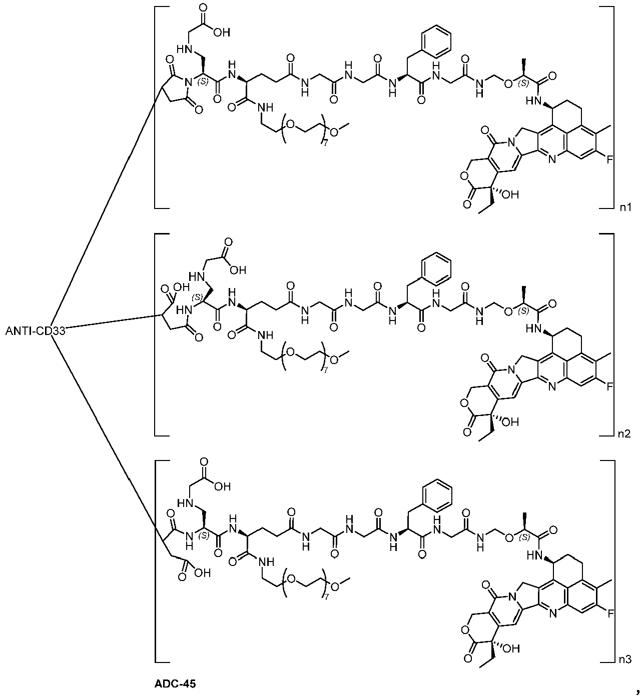 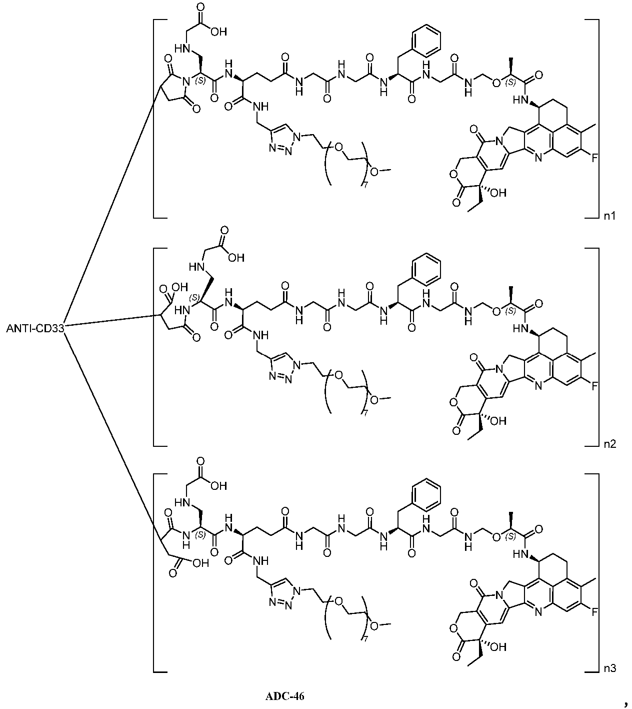 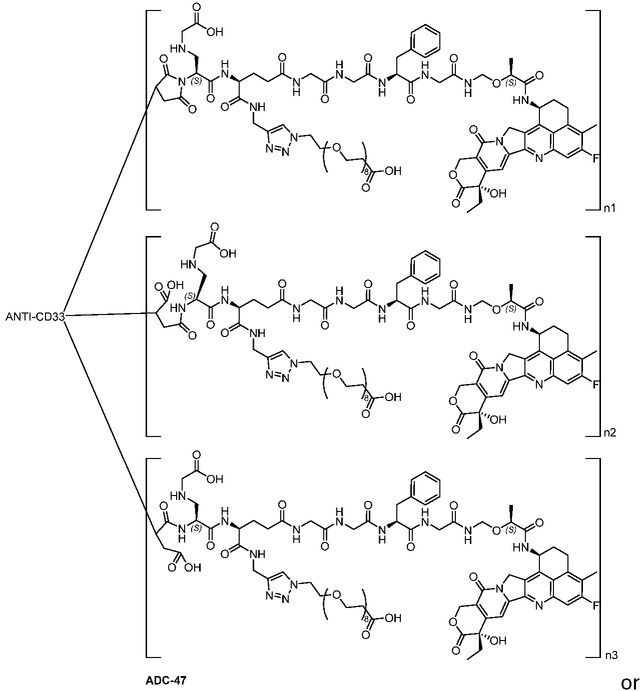 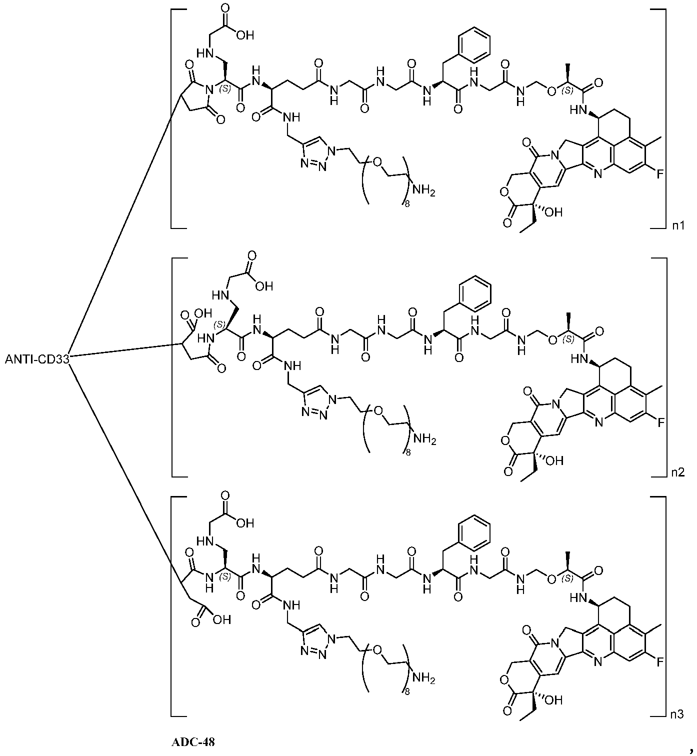 wherein:
ANTI-CD33 is anti-CD33 antibody or antigen-binding fragment thereof;
n1, n2, and n3 are each independently chosen from any integer or any decimal from 0 to 10. n1, n2, and n3 are not simultaneously 0 and 1 ≤ n1+n2+n3 ≤ 10 (e.g., n1+n2+n3 are 1, 1.5, 2, 2.5, 3, 3.5, 4, 4.5, 5, 5.5, 6, 6.5, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10; or for example, n1 + n2 + n3 is 8.01, 8.32, 8.56, 8.63, 8.66 or 9.40; or for example n1+n2+n3 is selected from any integer or any decimal from 7-7.5, 7.5-8, 8-8.5, 8.5-9, 9-9.5 or 9.5-10; preferably for example n1+n2+n3 is selected from any integer or any decimal from 7-10, i.e., 7≤ n1+n2+n3≤ 10), and preferably any integer or any decimal from 8-10, i.e., 8≤ n1+n2+n3≤ 10.

In some embodiments, the antibody to Ab comprises two light chains and two heavy chains, wherein said light chains and heavy chains form IgG with binding specificity for CD33.

In some embodiments, the light chain variable region of said anti-CD33 antibody or antigen-binding fragment thereof comprises CDR1, CDR2, CDR3 as shown in SEQ ID NO: 8, SEQ ID NO: 9, SEQ ID NO: 10, and the heavy chain variable region comprises CDR1 as shown in SEQ ID NO: 2, SEQ ID NO: 3, SEQ ID NO: 4, CDR2, CDR3.

In some embodiments, said anti-CD33 antibody or antigen-binding fragment thereof has a light chain variable region as shown in SEQ ID NO: 7 and a heavy chain variable region as shown in SEQ ID NO: 1.

In some embodiments, said anti-CD33 antibody comprises a constant region derived from human immunoglobulin;
Preferably, the light chain of said anti-CD33 antibody comprises a light chain constant region derived from a human immunoglobulin (e.g., kappa or lambda); and the heavy chain of said antibody comprises a heavy chain constant region derived from a human immunoglobulin (e.g., IgG1, IgG2, IgG3, or IgG4);
Preferably, the amino acid sequence of the light chain of said anti-CD33 antibody is SEQ ID NO: 11 and the amino acid sequence of the heavy chain is SEQ ID NO: 5;
Preferably, the nucleic acid coding sequence for the light chain of said anti-CD33 antibody is SEQ ID NO: 23 and the nucleic acid coding sequence for the heavy chain is SEQ ID NO: 17.
In some embodiments, said anti-CD33 antibody further comprises a cysteine site-specific insertion;
Preferably, the insertion site of said cysteine is in the light chain constant region;
Preferably, the insertion site of said cysteine is position 206 (Kabat numbering) of the kappa light chain constant region;
Preferably, said anti-CD33 antibody has a light chain amino acid sequence of SEQ ID NO: 25 and a heavy chain amino acid sequence of SEQ ID NO: 5;
Preferably, the nucleic acid coding sequence for the light chain of said anti-CD33 antibody is SEQ ID NO: 27 and the nucleic acid coding sequence for the heavy chain is SEQ ID NO: 17.

In some embodiments, said pharmaceutically acceptable salt comprises a sodium, potassium, calcium, or magnesium salt formed with an acidic functional group in the structural formula, and an acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed with basic functional groups in the structure.

In a second aspect of the present disclosure, there is provided a linker-payload or a stereoisomer, pharmaceutically acceptable salt or solvate thereof, as shown in formula III. 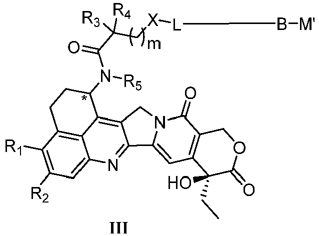 wherein:
M' is selected from 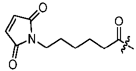 or the connector unit shown in formula A' below; 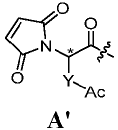
wherein Y is a scaffold selected from C1-C6 alkyl, substituted C1-C6 alkyl or C3-C8 cycloalkyl; preferably Y is C1-C6 alkyl; Ac is a hydrophilic structural unit; and the position shown by the wavy line on the right side is connected to B or L;
B is present or absent, and when B is present it is selected from modifier units;
L is selected from linker units, preferably peptide-containing linker units;
X is selected from -NH-, -O- or -S-, preferably -O-;
R₁ and R₂ are the same or different and are each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, 5- 10 heteroaryl, and substituted 5-10 heteroaryl;
Preferably, R₁ and R₂ are the same or different and are each independently selected from halogen, C1-C6 alkyl;
More preferably, R₁ is selected from C1-C6 alkyl and R₂ is selected from halogen;
Most preferably, R₁ is methyl and R₂ is fluorine;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclic, substituted 3-7-membered heterocyclic, C6-C10 aryl, substituted C6-C10 aryl, C6- C10 aryl C1-C6 alkyl, 5-10-membered heteroaryl, and substituted 5-10-membered heteroaryl;
Preferably, R₃, R₄ are the same or different, each independently selected from hydrogen, C1-C6 alkyl, substituted C1-C6 alkyl, C3-C8 cycloalkyl, C3-C8 cycloalkyl C1-C6 alkyl and C6-C10 aryl C1-C6 alkyl;
Further preferably, R₃, R₄ are the same or different, each independently selected from hydrogen, C1-C6 alkyl, halo-C1-C6 alkyl, C3-C6 cycloalkyl, C3-C6 cycloalkyl C1-C6 alkyl, and phenyl C1-C6 alkyl;
More preferably, R₃, R₄ are the same or different, each independently selected from hydrogen, methyl, ethyl, trifluoromethyl, cyclopropyl, cyclopropylmethyl, and benzyl;
Most preferably, of R₃, R₄, any one is selected from hydrogen, methyl, and the other is selected from hydrogen, methyl, ethyl, trifluoromethyl, cyclopropyl, cyclopropylmethyl, benzyl;
Alternatively, R₃, R₄ and the carbon atoms to which they are attached constitute a C3-C8 cycloalkyl group, a 3-7-membered heterocyclic group, a substituted 3-7-membered heterocyclic group;
Preferably, R₃, R₄ and the carbon atoms to which they are attached form a C3-C8 cycloalkyl group;
More preferably, R₃, R₄ and the carbon atoms to which they are attached constitute a C3-C6 cycloalkyl group;
Most preferably, R₃, R₄ and the carbon atom to which it is attached constitute a cyclopropyl, cyclobutyl or cyclopentyl group;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl, R₅ is preferably selected from hydrogen, C1-C6 alkyl, R₅ is more preferably selected from hydrogen, methyl;
m is selected from an integer from 0 to 5, preferably selected from 0, 1;
The chiral carbon atom shown at position * has either R or S absolute configuration;
and the following condition is met: when M' is 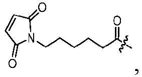 B exists and is selected from a modifier unit.

In some embodiments, said M' is selected from 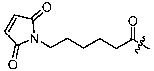 or a connector unit as shown in formula A' below, preferably selected from a connector unit as shown in formula A' below; 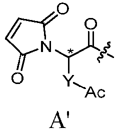
wherein Y is a scaffold selected from C1-C6 alkyl, substituted C1-C6 alkyl, and C3-C8 cycloalkyl; preferably Y is C1-C6 alkyl, more preferably C1-C3 alkyl, and most preferably methylene;
Position * has two chiralities, either R or S absolute configuration;
The position shown by the wavy line on the right is connected to B or connected to L;

The hydrophilic structural unit Ac is selected, without limitation, from natural or non-natural amino acids, 1-20 polyethylene glycols, phosphate groups, carboxylic acid groups, sulfonic acid groups, sulfinic acid groups, or the following structures. 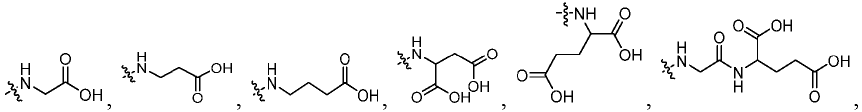 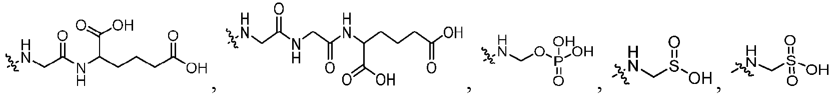 or 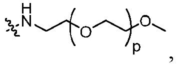
wherein p is selected as an integer from 0 to 10; the position shown by the wavy line is connected to the bracket Y;
Preferably, the hydrophilic structural unit Ac is selected from 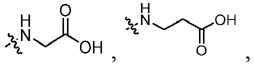 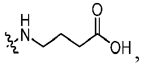 and is connected to the bracket Y at the position shown by the wavy line;
More preferably, the hydrophilic structural unit Ac is 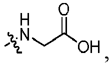 connected to the bracket Y at the position shown by the wavy line.

In some embodiments, said connector unit M' is selected, without limitation, from the structure shown in the following formula, the

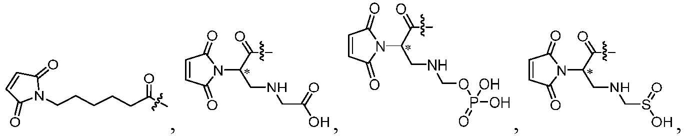

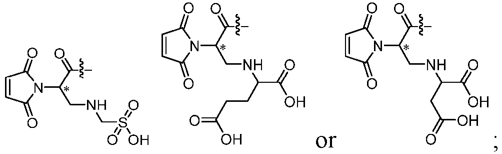

Preferably, said connector unit M' is selected from

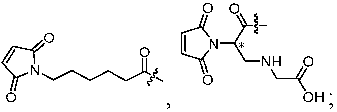

More preferably, said connector unit M' is 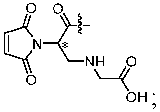 wherein:
Position * has two chiralities, either R or S absolute configuration;
The position shown by the wavy line on the right is connected to modifier unit B or linker unit L.

In some embodiments, the modifier unit B is present or absent, and when said modifier unit B is present, it is selected, without limitation, from a structure that enhances hydrophilicity or a stereoisomer thereof.

In some embodiments, the modifier unit B is present or absent, and when said modifier unit B is present, said modifier unit B is selected, without limitation, from a structure that enhances hydrophilicity or a stereoisomer thereof as shown in the following formula.

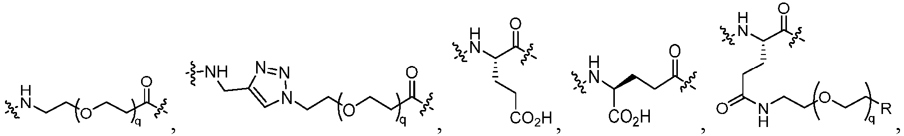

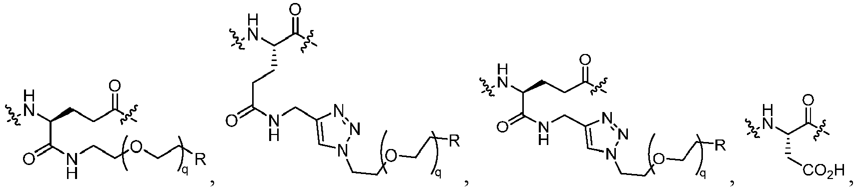

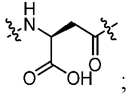

Preferably, the modifier unit B is present or absent, and when said modifier unit B is present, said modifier unit B is selected, without limitation, from a structure that enhances hydrophilicity or a stereoisomer thereof as shown in the following formula, 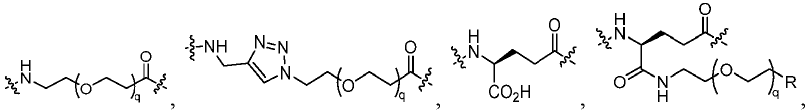 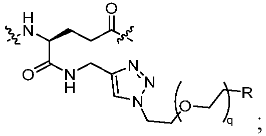 wherein:
R is selected, without limitation, from one or a combination of hydroxyl, amino, polyethylene glycol, carboxylic acid group, sulfonic acid group, sulfinic acid group, phosphoric acid group, **C1-**C6 alkyl group, C1-C6 alkoxy group, natural or non-natural amino acid residue, sugar, or a derivative thereof;
Preferably, R is selected from C1-C6 alkoxy, carboxylic acid group, and amino group;
More preferably, R is selected from methoxy, carboxylic acid group, and amino group;
The position shown by the wavy line on the left is connected to the connector unit M';
The position shown by the wavy line on the right is connected to the linker unit L;
q is selected from integers 1-10 (preferably from integers 2-8), preferably from 2, 7, 8;
Most preferably, said modifier unit B is selected from a structure that enhances hydrophilicity or a stereoisomer thereof as shown in the following formula: 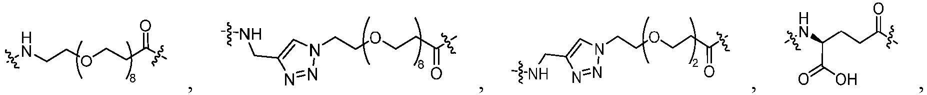 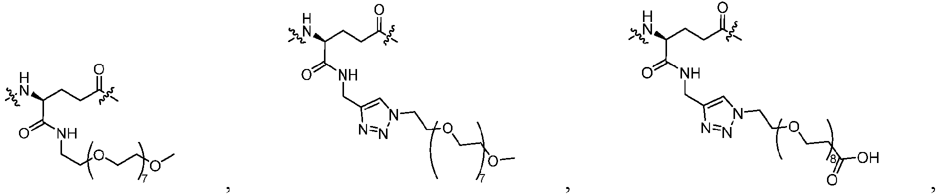 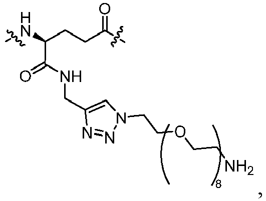 the position shown by the wavy line on the left is connected to the connector unit M', and the position shown by the wavy line on the right is connected to the linker unit L.

In some embodiments, said linker unit L is L₁-L₂,
L₁ is a peptide residue comprising 2-10 amino acid residues, said peptide residue having the amino terminal connected to the connector unit M' or the modifier unit B and the carbonyl terminal connected to L₂;
Preferably, L₁ is a peptide residue comprising 2-4 amino acid residues, said peptide residue having an amino terminal connected to a connector unit M' or a modifier unit B and a carbonyl terminal connected to L₂; preferably, said amino acid is selected from valine, alanine, phenylalanine, glycine, lysine, citrulline, serine, glutamic acid, and aspartic acid; more preferably, said amino acid is selected from valine, alanine, phenylalanine, glycine, lysine, and citrulline; most preferably, said amino acid is selected from valine, phenylalanine, glycine, lysine, and citrulline;
More preferably, L₁ is selected from the following peptide residues: glycine-glycine-phenylalanine-glycine, valine-citrulline, phenylalanine-lysine, valine-alanine, alanine-alanine-alanine (preferably selected from glycine-glycine-phenylalanine glycine-glycine, valine-citrulline, phenylalanine-lysine), and the amino terminal of said peptide residues is connected to the connector unit M ' or the modifier unit B and the carbonyl terminal is connected to L₂;
Most preferably, L₁ is selected from the following peptide residues: 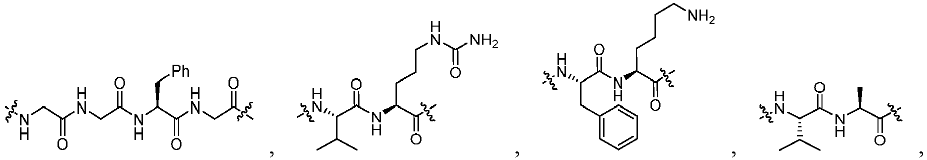 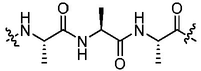 (preferably from 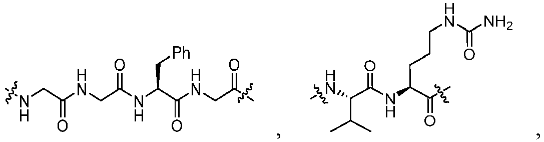 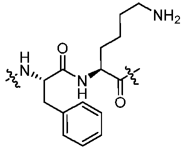 ), said peptide residues having an amino terminal connected to a connector unit M' or a modifier unit B and a carbonyl terminal connected to L₂;
L₂ is selected from 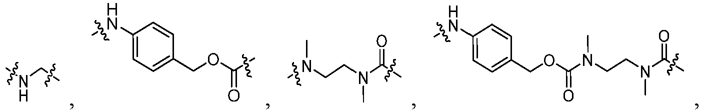 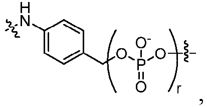 and is connected to L₁ at the position shown by the left wavy line, and to X at the position shown by the right wavy line;
Preferably, L₂ is selected from 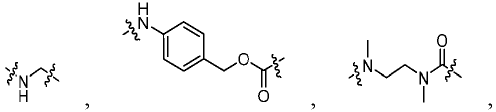 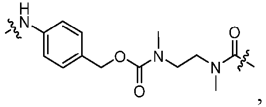 and is connected to L₁ at the position shown by the left wavy line, and is connected to X at the position shown by the right wavy line;
More preferably, L₂ is selected from 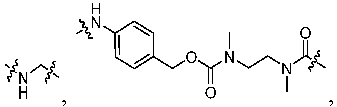 and is connected to L₁ at the position shown by the left wavy line, and is connected to X at the position shown by the right wavy line;
Preferably, said linker unit L is selected, without limitation, from the following structures or stereoisomers thereof. 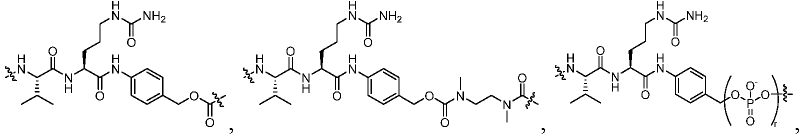 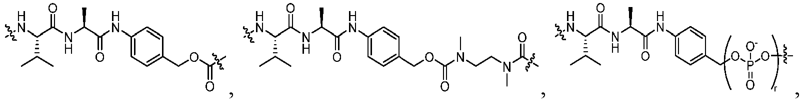 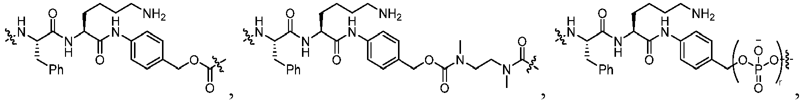 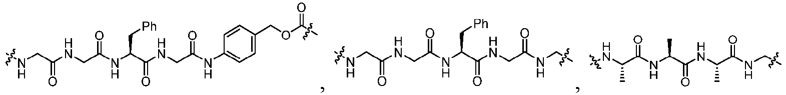 or 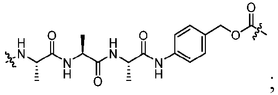 wherein:
   r is chosen from the integers 1-10;
   More preferably, said linker unit L is selected from the following structures or stereoisomers thereof: 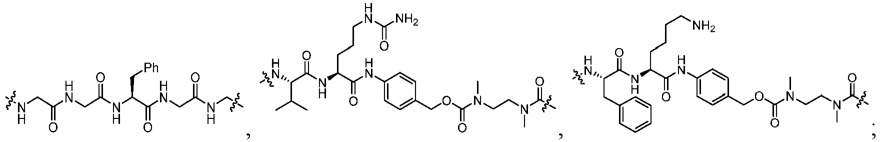
   Said linker unit L is connected to the connector unit M' or the modifier unit B at the position shown by the wavy line on the left side;
   Said linker unit L is connected to X at the position shown by the wavy line on the right side.

In some embodiments, said linker-payload is selected, without limitation, from the following structures or stereoisomers thereof.

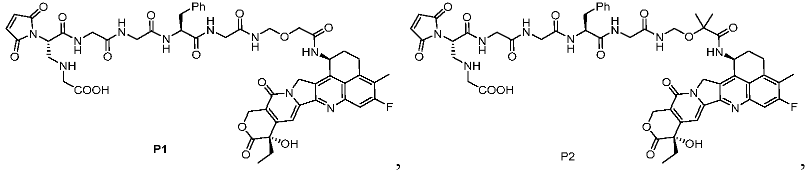

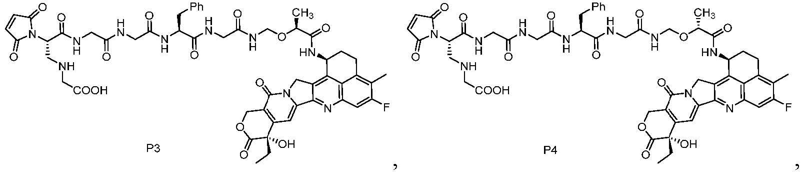

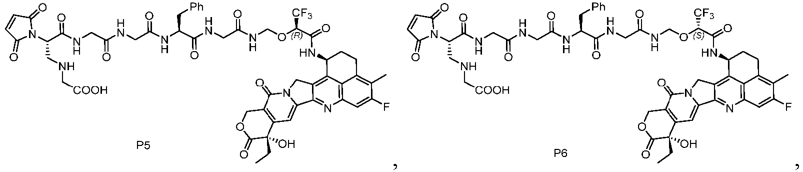

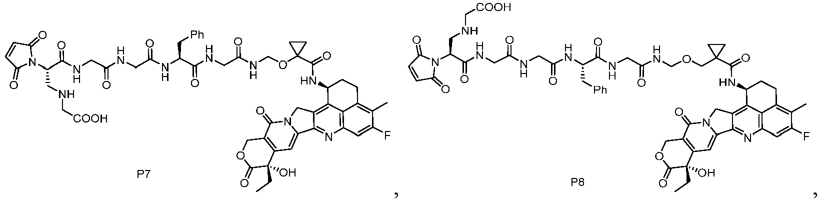

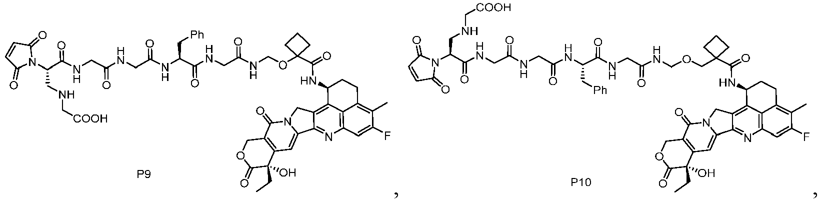

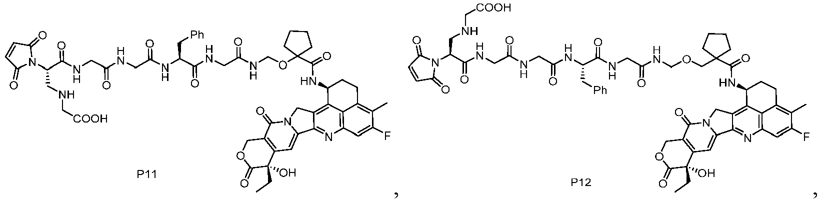

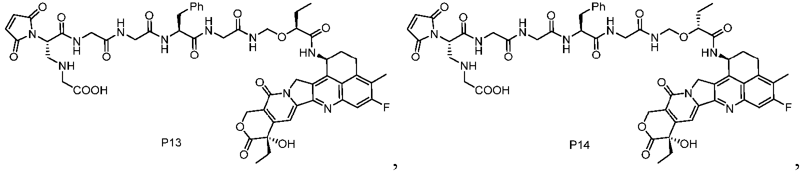

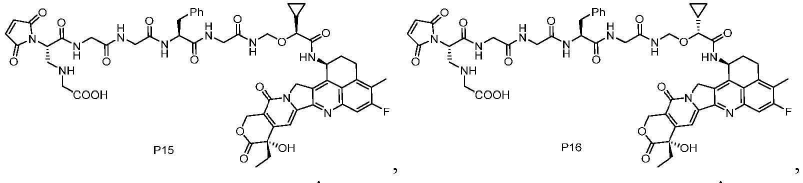

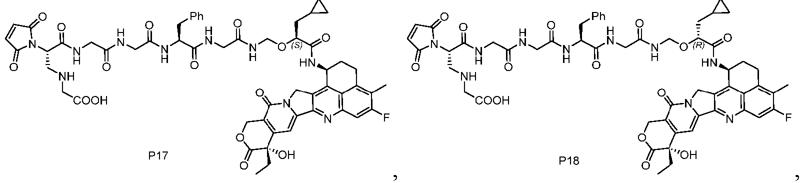

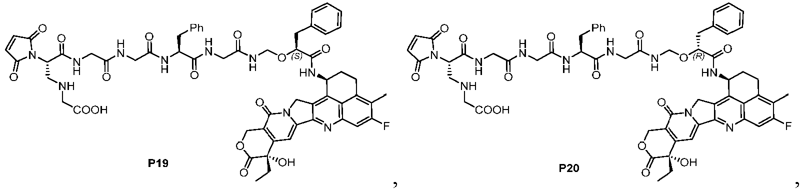

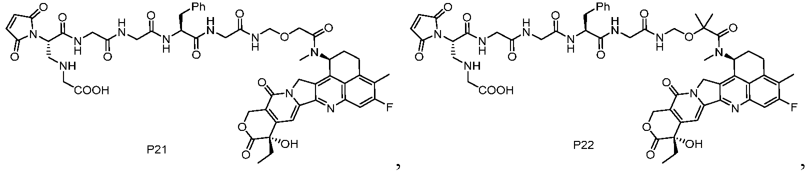

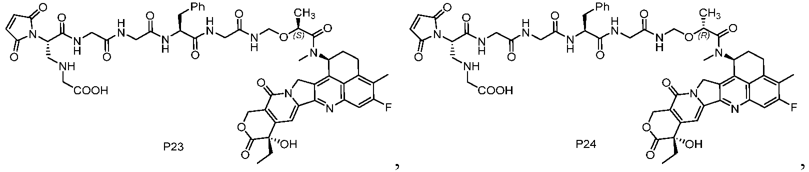

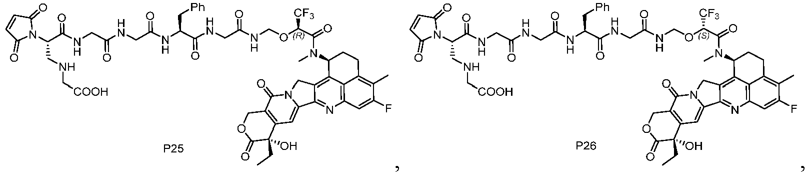

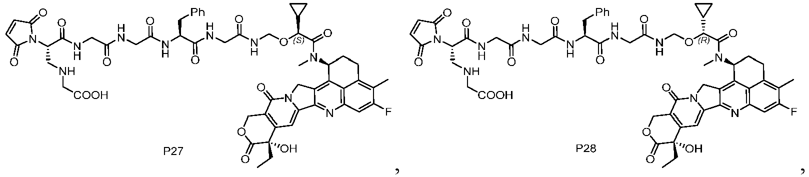

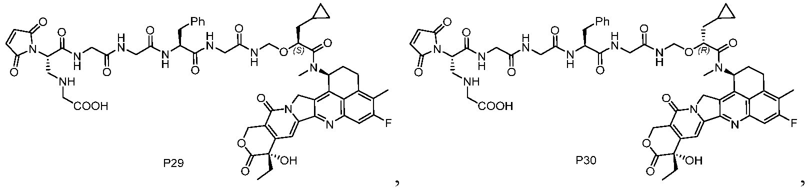

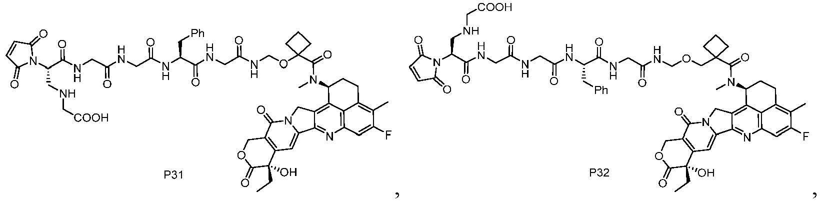

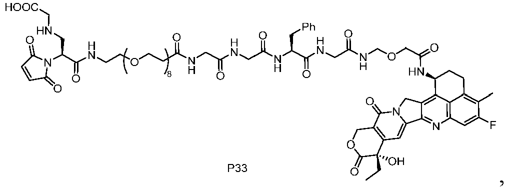

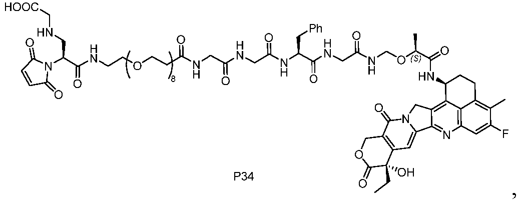

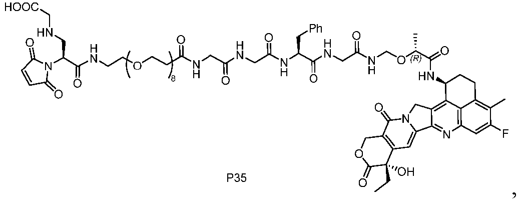

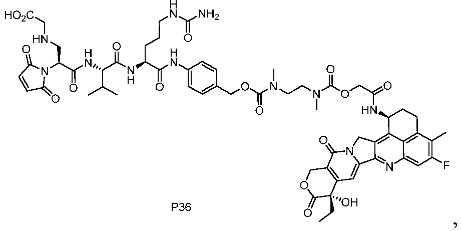

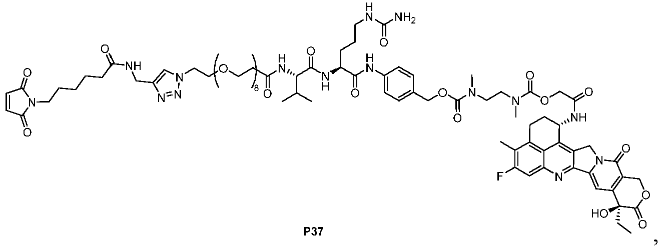

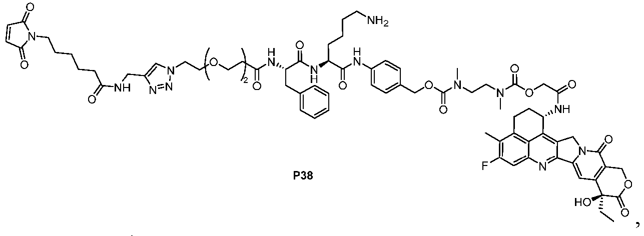

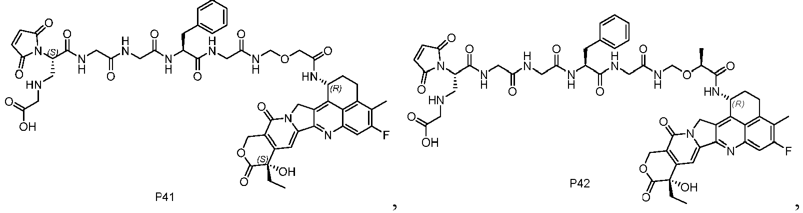

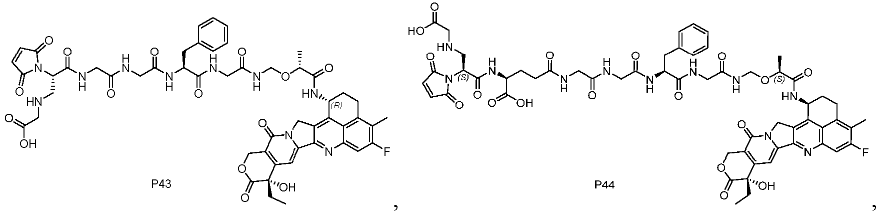

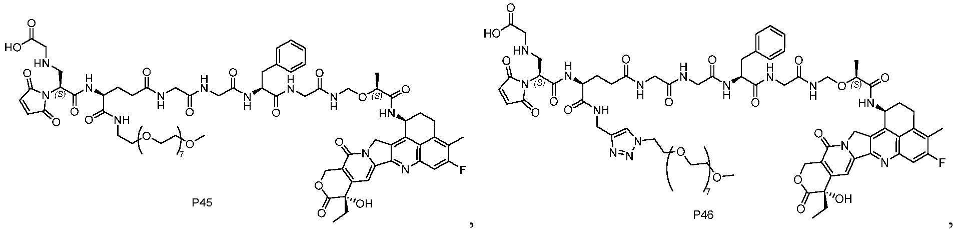

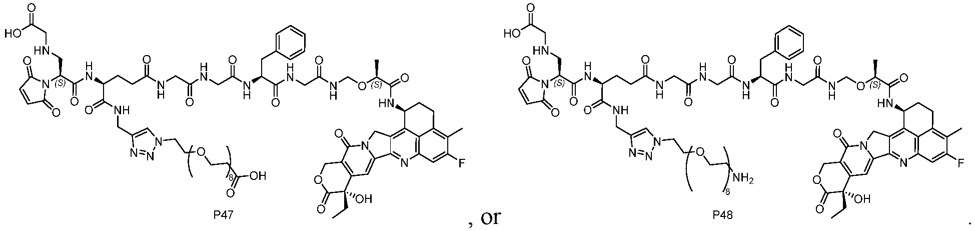

The third aspect of the present disclosure provides a method for preparing the antibody-drug conjugate or the stereoisomer, pharmaceutically acceptable salt or solvate thereof according to the first aspect, the method comprising:

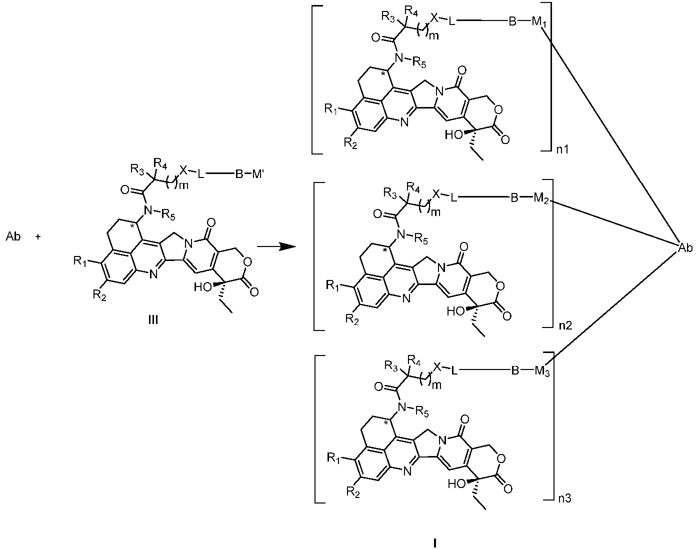

Ab is coupled with a linker-payload shown in general formula **III** to prepare to obtain an antibody-drug conjugate or stereoisomer, pharmaceutically acceptable salt or solvate thereof as shown in general formula **I** of the preceding first aspect;
wherein:
Ab is selected from anti-CD33 antibodies and antigen-binding fragments thereof;
Preferably, said anti-CD33 antibody is as defined in the preceding first aspect;
M₁, M₂, M₃, B, L, X, R₁, R₂, R₃, R₄, R₅, m, n1, n2, n3 are as defined in the preceding first aspect, and M' is as defined in the preceding second aspect;
The chiral carbon atom at the position indicated by * has two chiralities, namely, R absolute configuration or S absolute configuration.

The fourth aspect of the present disclosure provides a pharmaceutical composition, which comprises the antibody-drug conjugate or thestereoisomer, pharmaceutically acceptable salt or solvate thereof described in the first aspect, or the linker-payload or thestereoisomer, pharmaceutically acceptable salt or solvate thereof described in the second aspect, and an optional pharmaceutically acceptable carrier.

The fifth aspect of the present disclosure provides a pharmaceutical formulation, which comprises the antibody-drug conjugate or the stereoisomer, pharmaceutically acceptable salt or solvate thereof described in the first aspect, or the linker-payload or the stereoisomer, pharmaceutically acceptable salt or solvate thereof described in the second aspect.

The sixth aspect of the present disclosure provides the use of the antibody-drug conjugate or the stereoisomer, pharmaceutically acceptable salt or solvate thereof described in the first aspect, or the linker-payload or the stereoisomer, pharmaceutically acceptable salt or solvate thereof described in the second aspect, or the pharmaceutical composition described in the fourth aspect and/or the pharmaceutical formulation described in the fifth aspect in the preparation of a drug for treating or preventing cancer or tumors.

In some embodiments, said cancer or tumor expresses CD33.

In some embodiments, said cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer (such as non-small cell lung cancer), colon cancer, breast cancer (such as triple-negative breast cancer), rectal cancer, colorectal cancer, bone cancer, skin cancer (such as epidermal cancer), thyroid cancer, pancreatic cancer, melanoma, glioma, neuroblastoma, multiforme glioma, sarcoma, lymphoma, myeloma and leukemia and other solid tumors or blood tumors.

In some embodiments, the cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer, colon cancer, triple-negative breast cancer, rectal cancer, colorectal cancer, bone cancer, skin cancer, thyroid cancer, pancreatic cancer, melanoma, glioma, neuroblastoma, multiforme glioma, sarcoma, lymphoma and leukemia and other solid tumors or blood tumors.

The seventh aspect of the present disclosure provides the antibody-drug conjugate or the stereoisomer, pharmaceutically acceptable salt or solvate thereof described in the first aspect, or the linker-payload or the stereoisomer, pharmaceutically acceptable salt or solvate thereof described in the second aspect, or the pharmaceutical composition described in the fourth aspect and/or the pharmaceutical formulation described in the fifth aspect, which are used to treat or prevent cancer or tumors.

In some embodiments, said cancer or tumor expresses CD33.

In some embodiments, said cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer (such as non-small cell lung cancer), colon cancer, breast cancer (such as triple-negative breast cancer), rectal cancer, colorectal cancer, bone cancer, skin cancer (such as epidermal cancer), thyroid cancer, pancreatic cancer, melanoma, glioma, neuroblastoma, multiforme glioma, sarcoma, lymphoma, myeloma and leukemia and other solid tumors or blood tumors.

In some embodiments, said cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer, colon cancer, triple-negative breast cancer, rectal cancer, colorectal cancer, bone cancer, skin cancer, thyroid cancer, pancreatic cancer, melanoma, glioma, neuroblastoma, multiforme glioma, sarcoma, lymphoma and leukemia and other solid tumors or blood tumors.

In the eighth aspect of the present disclosure, a method for treating or preventing cancer or tumor is provided, which comprises administering to a subject in need an effective amount of the antibody-drug conjugate or the stereoisomer, pharmaceutically acceptable salt or solvate thereof described in the first aspect, or the linker-payload or the stereoisomer, pharmaceutically acceptable salt or solvate thereof described in the second aspect, or the pharmaceutical composition described in the fourth aspect and/or the pharmaceutical formulation described in the fifth aspect.

In some embodiments, said cancer or tumor expresses CD33.

In some embodiments, said cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer (such as non-small cell lung cancer), colon cancer, breast cancer (such as triple-negative breast cancer), rectal cancer, colorectal cancer, bone cancer, skin cancer (such as epidermal cancer), thyroid cancer, pancreatic cancer, melanoma, glioma, neuroblastoma, multiforme glioma, sarcoma, lymphoma, myeloma and leukemia and other solid tumors or blood tumors.

In some embodiments, said cancer or tumor is selected from adenocarcinoma, ovarian cancer, cervical cancer, uterine cancer, prostate cancer, kidney cancer, urothelial cancer, bladder cancer, liver cancer, gastric cancer, endometrial cancer, salivary gland cancer, esophageal cancer, lung cancer, colon cancer, triple-negative breast cancer, rectal cancer, colorectal cancer, bone cancer, skin cancer, thyroid cancer, pancreatic cancer, melanoma, glioma, neuroblastoma, glioblastoma multiforme, sarcoma, lymphoma and leukemia and other solid tumors or blood tumors.

### BRIEF DESCRIPTION OF THE DRAWINGS

Figure 1A shows the aggregation of ADC-46 detected by SEC-HPLC;
Figure 1B shows the aggregation of ADC-47 detected by SEC-HPLC;
Figure 1C shows the aggregation of ADC-48 detected by SEC-HPLC;
Figure 1D shows the aggregation of ADC-50 detected by SEC-HPLC;
Figure 1E shows the aggregation of ADC-3 detected by SEC-HPLC;
Figure 1F shows the aggregation of ADC-34 detected by SEC-HPLC;
Figure 1G shows the aggregation of ADC-44 detected by SEC-HPLC;
Figure 1H shows the aggregation of ADC-49 detected by SEC-HPLC;
Figure 2A shows the drug-to-antibody ratio (DAR) of ADC-46 detected by RP-HPLC;
Figure 2B shows the drug-to-antibody ratio (DAR) of ADC-47 detected by RP-HPLC;
Figure 2C shows the drug-to-antibody ratio (DAR) of ADC-48 detected by RP-HPLC;
Figure 2D shows the drug-to-antibody ratio (DAR) of ADC-50 detected by RP-HPLC;
Figure 2E shows the drug-to-antibody ratio (DAR) of ADC-3 detected by RP-HPLC;
Figure 2F shows the drug-to-antibody ratio (DAR) of ADC-34 detected by RP-HPLC;
Figure 2G shows the drug-to-antibody ratio (DAR) of ADC-44 detected by RP-HPLC;
Figure 2H shows the drug-to-antibody ratio (DAR) of ADC-49 detected by RP-HPLC;
Figure 3 shows the measurement of molecular weight of ADC-3;
Figure 4A shows that ADC-3 maintains the same affinity as naked antibody for CD33 antigen;
Figure 4B shows that ADC-48 maintains the same affinity as naked antibody for CD33 antigen;
Figure 4C shows that ADC-46 and ADC-47 maintain the same affinity as naked antibody for CD33 antigen;
Figure 5A shows the killing curves of anti-CD33, ADC-3, ADC-47 and ADC-48 on HL-60 cells;
Figure 5B shows the killing curves of anti-CD33, ADC-3, ADC-47, and ADC-48 on HEL92.1.7 cells;
Figure 5C shows the killing curves of anti-CD33, ADC-3, ADC-47, and ADC-48 on TF-1 cells;
Figure 5D shows the killing curves of anti-CD33, ADC-3, ADC-47, and ADC-48 on MV4-11 cells;
Figure 5E shows the killing curves of anti-CD33, ADC-3, ADC-47, and ADC-48 on MOLM-13 cells;
Figure 5F shows the killing curves of anti-CD33, ADC-3, ADC-47, and ADC-48 on U937 cells;
Figure 5G shows the killing curves of anti-CD33, ADC-3, ADC-47, and ADC-48 on CMK cells;
Figure 6A shows the effect of tumor inhibition by anti-CD33 antibodies and antibody-drug conjugates to HEL92.1.7 cell subcutaneous transplant tumor in mice;
Figure 6B shows the effect of tumor inhibition by anti-CD33 antibodies and antibody-drug conjugates to MV4-11 cell subcutaneous transplant tumor in mice;
Figure 7A shows the killing curves of anti-CD33, small molecule drugs D3, ADC-3, and ADC-34 on HL-60 cells;
Figure 7B shows the killing curves of anti-CD33, small molecule drugs D3, ADC-3, and ADC-34 on JVM-3 cells; and
Figure 7C shows the killing curves of anti-CD33, small molecule drugs D3, ADC-3, and ADC-34 on Raji cells.

### DETAILED DESCRIPTION

### Abbreviations and definitions

Unless otherwise indicated, the following terms and phrases, as used herein, are intended to have the meanings set forth below. When a trade name is used herein, unless otherwise indicated in the context, the trade name includes the product formulation, generic drug and active ingredient of said trade name product.

Unless stated to the contrary, terms used in the claims and specification herein have the meanings set forth below.

The term "antibody" or "antigen-binding fragment" includes, to the extent that it is used, any part of an antibody structure. This unit may bind, reactively associate or complex with a receptor, antigen or other receptor unit possessed by the target cell population. The antibody may be any protein or protein-like molecule that binds, complexes or reacts with a portion of the cell population to be treated or biologically modified. The antibodies comprising the antibody-drug couplings in the present invention maintain their original antigen-binding capacity in the wild. Thus, the antibodies of the present invention are capable of binding exclusively to antigens. Antigens involved include, for example, tumor-associated antigens (TAA), cell surface receptor proteins and other cell surface molecules, cell survival regulators, cell proliferation regulators, molecules associated with tissue growth and differentiation (such as are known or foreseen to be functional), lymphokines, cytokines, molecules involved in the regulation of cellular cycling, molecules involved in angiogenic molecules (if known or predicted to be functional). Tumor-associated factors may be cluster differentiation factors (e.g., CD proteins).

Antibodies applied in antibody-drug conjugates include, but are not limited to, antibodies directed against cell surface receptors and tumor-associated antigens. Such tumor-associated antigens are well known in the industry and can be prepared by methods and information for antibody preparation well known in the industry. To develop effective cellular level targets that can be used in cancer diagnosis and therapy, researchers seek to find transmembrane or other tumor-associated peptides. These targets can be specifically expressed on the surface of one or more cancer cells with little or no expression on the surface of one or more non-cancer cells. Typically, such tumor-associated polypeptides are more overexpressed on the surface of cancer cells relative to the surface of non-cancer cells. Identification of such tumor-associated factors can greatly enhance the specific targeting properties of antibody-based cancer therapies. For convenience, information related to antigens known to the industry is labeled below, including name, other names, and GenBank accession number. Nucleic acid and protein sequences corresponding to the tumor-associated antigens can be found in publicly available databases, such as Genbank, and the antibody-targeted tumor-associated antigens include all amino acid sequence variants and isoforms having at least 70%, 80%, 85%, 90%, or 95% homology to the sequences identified in the references, or possessing biological properties and characteristics that are identical to the tumor-associated antigen sequences of the cited literature.

The "antibody" in the present invention refers to immunoglobulin, which is a tetrapeptide chain structure consisting of two identical heavy chains and two identical light chains connected by interchain disulfide bonds. The amino acid composition and arrangement order of the constant region of the heavy chain of the immunoglobulin are different, so its antigenicity is also different. Accordingly, immunoglobulins can be categorized into five classes, or isoforms of immunoglobulins, i.e., IgM, IgD, IgG, IgA, and IgE, whose corresponding heavy chains are *µ*, *δ, γ, α,* and *ε* chains, respectively. The same class of Ig can be further divided into different subclasses according to the differences in the amino acid composition of its hinge region and the number and position of disulfide bonds of the heavy chain, such as IgG can be divided into IgG1, IgG2, IgG3, IgG4. The light chain is divided into κ-chain or λ-chain through the differences in the constant region. Each of the five classes of Ig may have either a *κ* chain or *a λ* chain. The antibodies described in the present invention are preferably specific antibodies against cell surface antigens on the target cells, specifically anti-CD33 antibodies.

The three-letter codes and single-letter codes for amino acids used in the present invention are as described in *J*. *biol. chem.* 1968. 243. 3558.

The term "drug" refers to cytotoxic drugs, drug denoted D, which are chemical molecules that have a strong ability to disrupt normal growth within cells.

The term "antibody-drug coupling" refers to the attachment of an antibody to a biologically active drug by means of a junction unit, optionally a modification unit and a linker unit.

The term "connector unit" refers to a chemical structure fragment or bond that is attached to an antibody or antigen-binding fragment thereof at one end and to a modifier or linker unit at the other end or may be attached to other junctions and then to the drug.

The term "linker unit" includes an extension, a spacer or an amino acid unit, which can be synthesized by methods known in the art, such as those described in US2005-0238649A1.

According to the mechanism of intracellular drug release, as used herein, "linker units" or "linkers" can be categorized into two types: unbreakable linker units and breakable linker units. For an antibody-drug conjugate containing an unbreakable linker, the mechanism of drug release is as follows: after the coupling binds to the antigen and is endocytosed by the cell, the antibody is enzymatically cleaved in the lysosome, releasing an active molecule consisting of the small molecule of the drug, the linker and the amino acid residues of the antibody. The resulting structural change in the drug molecule does not diminish its cytotoxicity, but because the active molecule is electrically charged (amino acid residues), this results in its inability to penetrate neighboring cells. Therefore, such active drugs cannot kill neighboring tumor cells that do not express the targeted antigen (antigen-negative cells) (bystander effect) (Ducry et al., 2010, Bioconjugate Chem. 21: 5-13). The linker unit may also be a "breakable linker" that facilitates the release of the drug in the cell. For example, acid-unstable linkers (e.g., hydrazone), protease-sensitive (e.g., peptidase-sensitive) linkers, light-unstable linkers, or disulfide-containing linkers can be used (Chari et al. Cancer Research 52:127-131, 1992); U.S. Patent No. 5, 208, 020.

The term "modifier unit" means a chemical bond or extension, a spacer, an amino acid unit with a branching structure, a sugar unit, a polyethylene glycol unit, a carbonyl group, an amide group, a carboxylic acid group, a phosphoric acid group, a sulphonic acid group, a sulfinic acid group, an alkyl group, a cycloalkyl group, a heterocycloalkyl group, an aryl group, or a combination of one or more of the following, one or more of them, which is connected to the connector unit at one end and to the linking unit at the other end. aryl or heteroaryl, or a combination of one or more of these. The role of the modifier unit is to enhance the hydrophilicity of the antibody-drug coupling or to have a branching structure to increase drug loading.

In the formula I 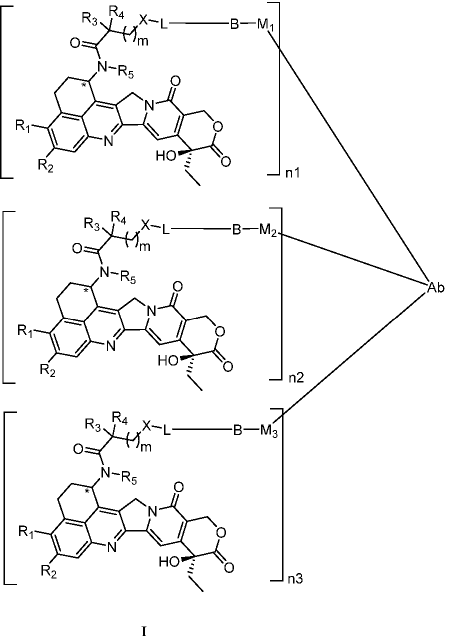 of the present invention, B is present or absent, then the person skilled in the art can understand that if B is present, the structural formula of formula **I** is as before, and if B is absent, the structural formula of formula **I** changes to 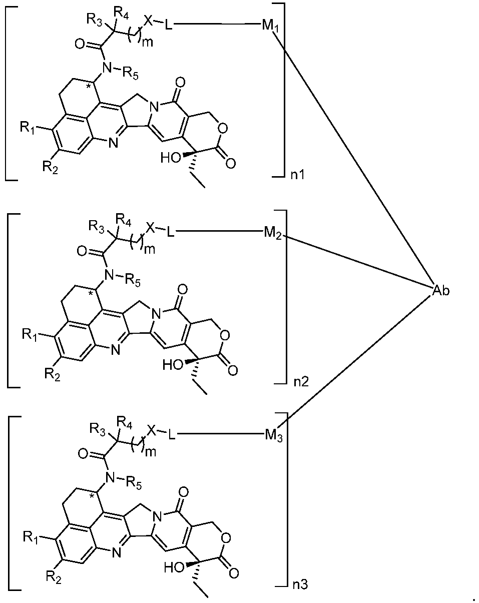 The remaining similar definitions can be understood with reference to the foregoing.

Accordingly, in the present invention, if the connector units M₁, M₂, M₃ are each independently selected from 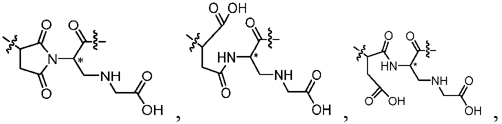 and the position shown by the wavy line on the right side is connected to the modifier unit B or to the connection unit L, a person skilled in the art will understand that it means that if said modifier unit B exists, the position shown by the wavy line on the right side will be connected to the modifier unit B. However, if said modifier unit B is not existent, the position shown by the wavy line on the right side will be connected to the modifier unit B. But if said modifier unit B is not existent, the position will be connected to the linker unit L. The remaining similar definitions can be understood with reference to the foregoing.

The term "drug loading" or "drug-antibody ratio (DAR)" refers to the average number of cytotoxic drugs loaded on each antibody in formula I (e.g., n1+n2+n3 in formula I), and can also be expressed as the ratio of the amount of drug to the amount of antibody, which can be an integer or a decimal. The range of drug loading (e.g., n1+n2+n3 in formula I) can be that each antibody is connected to an average of 1-10 (i.e., any integer selected from 1-10 including the endpoints 1 and 10, or any decimal between 1-10), preferably 8-10 (i.e., any integer selected from 8-10 including the endpoints 8 and 10, or any decimal between 8-10) cytotoxic drugs (D).

When the antibody drug coupling compound provided by the present invention is prepared, the linker-payload shown in general formula **III** can be hydrolyzed under easy hydrolysis conditions when coupling with Ab, and its hydrolysis site is in the maleimide part. When Ab coupled with multiple linker-payloads, with different degrees of hydrolysis, the following situations can occur:
(1) the maleimide is not hydrolyzed, i.e., the maleimides are all in the form of a closed ring 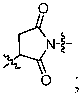
(2) the maleimide is incompletely hydrolyzed, i.e., some of the maleimides are in the form of a closed ring 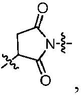 and another part of the maleimide is in the form of an open ring 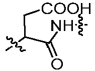 or 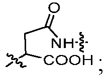
(3) the maleimide is completely hydrolyzed, i.e., the maleimides are all in the form of a ring-open 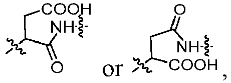 therefore, in the antibody-drug conjugate shown in general formula I , n1, n2, and n3 are each independently selected from any integer or any decimal from 0 to 10, and n1, n2, and n3 are not 0 at the same time. At the same time, it can be understood by those skilled in the art that, as mentioned above, the number of linker-payloads coupled to each Ab can be the same or different, and therefore, the average number of linker-payloads coupled to each Ab can be an integer or a decimal. Accordingly, when the linker-payload is coupled to the Ab, the maleimide part can be hydrolyzed under the condition of easy hydrolysis, and the degree of hydrolysis can be the same or different. Therefore, n1, n2, and n3 can be integers or decimals.

The term "pharmaceutically acceptable salt" refers to a salt of the antibody-drug conjugate or linker-payload of the present invention, or a salt of the compound described in the present invention, which is safe and effective when used in mammals and has the desired biological activity. For example, the carboxylic acid group in the antibody-drug conjugate or linker-payload of the present invention forms a salt with a base, non-limiting examples of which include sodium salt, potassium salt, calcium salt or magnesium salt. It can also be that the amino group in the antibody-drug conjugate or linker-payload of the present invention forms a salt with an acid, non-limiting examples of which include hydrochloride, hydrobromide, hydroiodide, sulfate, bisulfate, citrate, acetate, succinate, ascorbate, oxalate, nitrate, sorbate, hydrogen phosphate, phosphate, etc. In some embodiments, the pharmaceutically acceptable salts include sodium, potassium, calcium or magnesium salts formed with acidic functional groups in the structural formula and acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methane sulfonate, ethanesulfonate, benzenesulfonate or p-toluenesulfonate formed with basic functional groups in the structure.

The term "amino acid residue" refers to the incomplete amino acid structure remaining after the amino group of an amino acid loses a hydrogen and the carboxyl group loses a hydroxyl and has an amino terminal and a carbonyl terminal. In the present invention, L₁ is a peptide residue composed of 2-10 (preferably 2-4) amino acid residues, wherein the types of the 2-10 (preferably 2-4) amino acids may be the same or different from each other. For example, if L₁ is a peptide residue comprising 4 amino acid residues, and the amino acids are selected from glycine, phenylalanine, then L₁ may be the following peptide residue: glycine-glycine-phenylalanine-glycine (Gly-Gly-Phe-Gly), which may be specified as

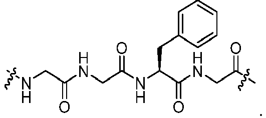

The term "solvate" refers to a pharmaceutically acceptable solvate formed by the antibody-drug conjugate or linker-payload of the present invention and one or more solvent molecules, and non-limiting examples of solvent molecules include water, ethanol, acetonitrile, isopropanol, DMSO, and ethyl acetate.

The term "absolute configuration" refers to the real spatial arrangement relationship of each group in a chiral molecule, that is, the absolute spatial relationship. In the present invention, a configuration labeling method characterized by the arrangement of the groups connected to the chiral carbon atoms in different directions in space is adopted, that is, the R and S configuration labeling method.

The term "natural amino acid" refers to an amino acid synthesized by biology. Natural amino acids are generally L-type, but there are a few exceptions, such as glycine, including natural and biologically synthesized ones.

The term "unnatural amino acid" refers to an amino acid obtained by synthetic means.

The term "stereoisomer" refers to a compound molecule with the same molecular formula in which the order of atoms or substituents are connected to each other is the same, but the arrangement in space is different, which belongs to the isomerism phenomenon in the category of organic chemistry, the same as structural isomerism.

The term "hydroxyl" refers to -OH.

The term "amino" refers to -NH2.

The term "halogen" refers to fluorine, chlorine, bromine or iodine.

The term "polyethylene glycol" refers to an oligomer or polymer of ethylene oxide.

The term "carboxylic acid" refers to -COOH.

The term "sulfonic acid" refers to -S(O)2OH.

The term "sulfinic acid" refers to -S(O)OH.

The term "phosphate" refers to -OP(O)2OH.

The term "sugar" refers to a polyhydroxy (2 or more) aldehyde or ketone compound or an organic compound that can be converted into either of the above after hydrolysis.

The term "derivative" refers to a product generated by replacing an atom or group in a compound molecule with another atom or group.

The term "alkyl" refers to a saturated aliphatic hydrocarbon group, which is a straight or branched chain group containing 1 to 20 carbon atoms (i.e., "C1-C20 alkyl"), preferably an alkyl group containing 1 to 12 carbon atoms (i.e., "C1-C12 alkyl"), more preferably an alkyl group containing 1 to 10 carbon atoms (i.e., "C1-C10 alkyl"), further preferably an alkyl group containing 1 to 6 carbon atoms (i.e., "C1-C6 alkyl"), further preferably an alkyl group containing 1 to 4 carbon atoms (i.e., "C1-C4 alkyl"), and most preferably an alkyl group containing 1 to 3 carbon atoms (i.e., "C1-C3 alkyl"). Examples of "C1-C6 alkyl" include, but are not limited to, methyl, ethyl, propyl (e.g., n-propyl, isopropyl), butyl (e.g., n-butyl, isobutyl, tert-butyl), pentyl (e.g., n-pentyl, isopentyl, neopentyl), and the like. Examples of "C1-C4 alkyl" include, but are not limited to, methyl, ethyl, propyl (e.g., n-propyl, isopropyl), butyl (e.g., n-butyl, isobutyl, tert-butyl), etc. Examples of "C1-C3 alkyl" include methyl, ethyl, propyl (e.g., n-propyl, isopropyl), etc.

The term "substituted" refers to the replacement of hydrogen in a compound by a substituent group. Unless otherwise specified herein, the substituent group can be a variety of groups selected from the following groups: -halogen, -OR', -NR'R", -SR', -SiR'R"R"', -OC(O)R', -C(O)R', -CO₂R', -CONR'R", -OC(O)NR'R", -NR"C(O)R', -NR'-C(O) NR"R"', -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NO₂, with the number of substituents being 1 to (2m'+1), where m' is the total number of carbon atoms in the group. R', R" and R‴ independently represent hydrogen, unsubstituted C1-C6 alkyl, unsubstituted C6-C12 aryl (or C6-C10 aryl), C6-C12 aryl (or C6-C10 aryl) substituted by 1-3 halogens, unsubstituted C1-C6 alkoxy or C1-C6 thioalkoxy, or unsubstituted C6-C12 aryl (or C6-C10 aryl) C1-C6 alkyl. When R' and R" are attached to the same nitrogen atom, they may form a 3-, 4-, 5-, 6- or 7-membered ring together with the atom.

The term "haloalkyl" refers to a group in which one or more hydrogen atoms in any of the above alkyl groups (e.g., C1-C20 alkyl, C1-C12 alkyl, C1-C10 alkyl, C1-C6 alkyl, C1-C4 alkyl, C1-C3 alkyl, etc.) are replaced by halogen (preferably fluorine). Examples of "halogenated C1-C6 alkyl" include monofluoromethyl, difluoromethyl, difluoroethyl, trifluoromethyl, and the like.

The term "cycloalkyl" refers to a saturated or partially unsaturated monocyclic or polycyclic hydrocarbon substituent, and the cycloalkyl ring contains 3 to 20 carbon atoms, preferably 3 to 12 carbon atoms, more preferably 3 to 10 carbon atoms, further preferably 3 to 8 carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl includes cycloalkyl of spiro ring, condensed ring and bridged ring. Examples of "C3-C6 cycloalkyl" include cyclopropyl, cyclobutyl, cyclopentyl or cyclohexyl, etc.

The term "heterocyclyl" refers to a saturated or partially unsaturated monocyclic or polycyclic hydrocarbon substituent containing 3 to 20 ring atoms (i.e., "3-20-membered heterocyclyl"), wherein one or more ring atoms are heteroatoms selected from nitrogen, oxygen or sulfur, but excluding the ring part of -O-O-, -O-S- or -S-S-, and the remaining ring atoms are carbon. Preferably, it contains 3 to 12 ring atoms (i.e., "3-12-membered heterocyclyl"), of which 1 to 4 are heteroatoms; more preferably, the heterocyclyl ring contains 3 to 10 ring atoms (i.e., "3-10-membered heterocyclyl"). Non-limiting examples of monocyclic heterocyclyls (e.g., 3-7-membered heterocyclyls) include pyrrolidinyl, piperidinyl, piperazinyl, morpholinyl, thiomorpholinyl, homopiperazinyl, etc. Polycyclic heterocyclyls include spirocyclic, fused ring, and bridged heterocyclyls.

The term "C6-C10 aryl" refers to a group of a carbocyclic aromatic system having 6-10 carbon atoms, such as benzene and naphthalene.

The term "cycloalkylalkyl" refers to an alkyl group substituted by one or more cycloalkyl groups, preferably by one cycloalkyl group, wherein the alkyl group is as defined above, wherein the cycloalkyl group is as defined above. For example, C3-C8 cycloalkylC1-C6 alkyl or C3-C6 cycloalkylC1-C6 alkyl. Similarly, the term "C6-C10 arylC1-C6 alkyl" refers to a C1-C6 alkyl group substituted by one or more C6-C10 aryl groups, preferably by one C6-C10 aryl group, wherein the alkyl group is as defined above, wherein the C6-C10 aryl group is as defined above. Specific examples such as "phenylC1-C6 alkyl" means that the C1-C6 alkyl group is substituted by one phenyl group, and specific examples such as benzyl. Other similar definitions can be understood with reference to the above content.

The term "alkoxy" refers to -O-(alkyl), wherein alkyl is as defined above. Non-limiting examples of C1-C6 alkoxy include: methoxy, ethoxy, propoxy, butoxy. The term "cycloalkoxy" refers to -O-(cycloalkyl), wherein cycloalkyl is as defined above. Non-limiting examples of C3-C6 cycloalkoxy include: cyclopropyloxy, cyclobutyloxy, cyclopentyloxy, cyclohexyloxy. Alkoxy or cycloalkoxy may be optionally substituted or unsubstituted, and when substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "heteroaryl" refers to an aromatic heterocyclic ring, typically a 5-, 6-, 7-, 8-, 9-, 10-membered heterocyclic ring having 1 to 3 heteroatoms selected from N, O or S; the heteroaryl ring may optionally be further fused or linked to aromatic and non-aromatic carbocyclic and heterocyclic rings.

In one embodiment of the present invention, the cytotoxic drug is coupled to the thiol-SH of the opened cysteine residues and/or the thiol-SH of the site-directed mutated cysteine residues between the antibody chains through the linker unit, the optional modification unit and the linker unit. Generally, the number of drug molecules that can be coupled to the antibody in the coupling reaction will be less than or equal to the theoretical maximum value.

The loading of the ligand cytotoxic drug conjugate can be controlled by the following non-limiting methods, including:
(1) controlling the molar ratio of the linker and the monoclonal antibody,
(2) controlling the reaction time and temperature,
(3) selecting different reaction reagents.

The term "pharmaceutical composition" refers to a mixture containing one or more antibody-drug conjugates or their stereoisomers, pharmaceutically acceptable salts or solvates or linker-payloads or their stereoisomers, pharmaceutically acceptable salts or solvates and other chemical components described herein, as well as other components such as physiologically or pharmaceutically acceptable carriers and excipients. The purpose of the pharmaceutical composition is to facilitate administration to the organism, facilitate the absorption of the active ingredient and thus exert biological activity.

The preparation of conventional pharmaceutical compositions can be found in the Chinese Pharmacopoeia.

The term "carrier" refers to a system that can change the way a drug enters the human body and its distribution in the body, control the release rate of the drug, and deliver the drug to the targeted organ. The drug carrier release and targeting system can reduce drug degradation and loss, reduce side effects, and improve bioavailability. For example, polymer surfactants that can be used as carriers can self-assemble to form various forms of aggregates due to their unique amphiphilic structure. Preferred examples are micelles, microemulsions, gels, liquid crystals, vesicles, etc. These aggregates can encapsulate drug molecules and have good permeability to membranes and can be used as excellent drug carriers.

The term "treatment" generally refers to obtaining the desired pharmacological and/or physiological effects. The effect can be preventive based on the complete or partial prevention of the disease or its symptoms; and/or can be therapeutic based on the partial or complete stabilization or cure of the disease and/or the side effects caused by the disease. As used herein, "treatment" encompasses any treatment of a disease in a patient, including: (a) preventing the disease or symptoms from occurring in a patient who is susceptible to the disease or symptoms but has not yet been diagnosed with the disease; (b) suppressing the symptoms of the disease, i.e., arresting its development; or (c) relieving the symptoms of the disease, i.e., causing regression of the disease or symptoms.

The term "subject" includes humans or non-human animals. Exemplary human subjects include humans (referred to as patients) or normal individuals suffering from diseases (e.g., the diseases described herein). The term "non-human animals" as used in the present invention includes all vertebrates, such as non-mammals (e.g., birds, amphibians, reptiles) and mammals, such as non-human primates, livestock and/or domesticated animals (e.g., sheep, dogs, cats, cows, pigs, etc.).

The term "effective amount" refers to the amount of a drug that, after administration, will relieve one or more symptoms of the treated condition to a certain extent.

The present invention is further elaborated below in connection with specific embodiments which, it should be understood, are intended only to illustrate the present invention and are not intended to limit the scope of the invention. Test methods for which specific conditions are not indicated in the following embodiments are generally performed in accordance with conventional conditions or in accordance with conditions recommended by the manufacturer. Unless otherwise specified, all percentages, ratios, ratios, or portions are by weight.

### Example 1: Synthesis of compound M1

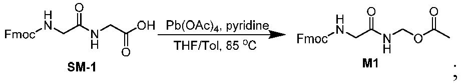

In a 5000 mL single-necked vial, Fmoc-Gly-Gly-OH (100 g, 282 mmol, 1.0 eq), lead tetraacetate (175 g, 395 mmol, 1.4 eq), 2000 mL of dry tetrahydrofuran and 670 mL of toluene were added, stirred uniformly under nitrogen, and the reaction was heated to 85 °C for 2.5 h. The reaction was monitored by TLC until the raw materials were finished reacting. The reaction was cooled to room temperature, filtered, and the filtrate was concentrated under reduced pressure, the residue was purified by column chromatography to obtain compound **M1** (87 g); LC-MS: [M+NH₄] ⁺ =386.0.

### Example 2: Synthesis of compound M3

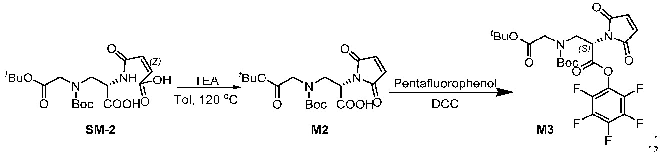

In a 1000 mL vial, compound **SM-2** (synthesized according to the method published in CN108452321A) (40 g, 96 mmol, 1.0 eq), triethylamine (26.7 mL, 2.0 eq), and toluene (400 mL) were added, and the reaction was carried out by refluxing at a temperature of 120 °C for 2 h. The reaction was monitored to be completely reacted by TLC, and then the solvent was concentrated by decompression at a temperature of 50 °C. The residue was dissolved with ethyl acetate (150 mL), water (40 mL), adjust pH to 2-3 with 1M HCl under ice bath by stirring, and partition. The aqueous layer was extracted once more with ethyl acetate, the organic layers were combined and dried by adding anhydrous sodium sulfate. After filtration, a light yellow oily crude was obtained by concentration, and the crude was purified by column chromatography (DCM:MeOH=40:1) to obtain compound **M2** (26.6 g); LC-MS: [M+H]⁺ =399.3.

In a 1000 mL single-necked vial, compound **M2** (26.5 g, 60.5 mmol, 1.0 eq), pentafluorophenol (12.2 g, 66.5 mmol, 1.1 eq), DCC (13.7 g, 66.5 mmol, 1.1 eq), and THF (300 mL) were added, and the reaction was carried out at room temperature for 30 min (monitored by TLC), and filtered to remove the insoluble material. The reaction solution was directly purified by Prepare-HPLC, and the prepared solution was concentrated at 35 °C by water pump under reduced pressure to remove acetonitrile, and lyophilized to obtain compound **M3** (31.5 g) with 64% yield; LC-MS: [M+H]⁺ =565.1.

### Example 3: Synthesis of compound P1

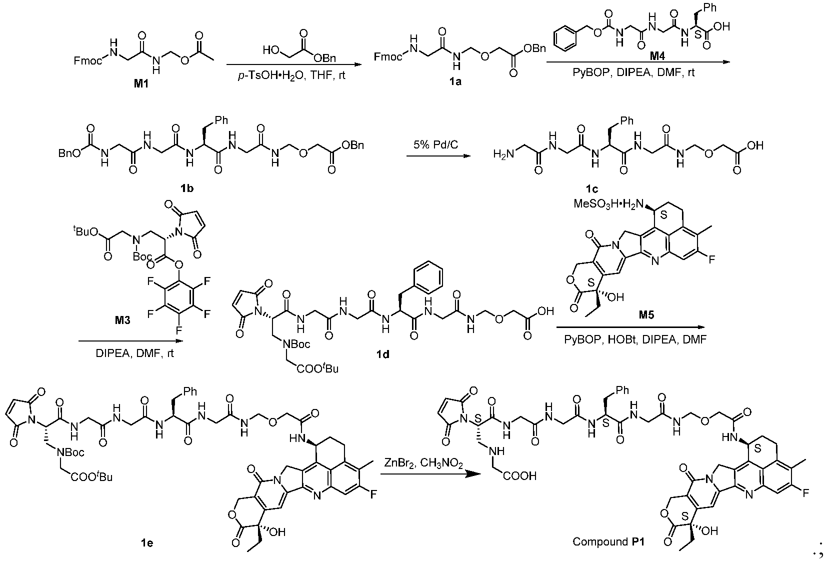

### Step 1: Compound 1a

In a 250 mL single-necked vial, **M1** (6 g, 16.3 mmol), 100 mL THF, p-toluenesulfonic acid monohydrate (0.31 g, 1.63 mmol) was added, stirred and cooled to 0 °C, benzyl 2-hydroxyacetate (5.4 g, 32.6 mmol) was added dropwise, and the reaction was naturally warmed to room temperature (about 2-4 h), which was monitored by TLC. After reaction finished, saturated NaHCO₃ solution was added, extracted with ethyl acetate, washed with saturated sodium chloride solution, dried with anhydrous sodium sulfate, filter, concentrate, and purify the residue with silica gel column (PE:EA=10:1-5:1-1:1) to give **1a** (4 g) in 52% yield; LC-MS: [M+H]⁺ =475.18.

### Step 2: Compound 1b

Compound **1a** (2 g, 4.2 mmol) and10 mL DMF were added into a 25 mL single-necked vial, the reaction wasstirred at 0 °C, then DBU (766 mg, 5.04 mmol) was added, the reaction was carried out for 1 h until the Fmoc group was fully deprotected monitored by TLC and set aside.

In another 25 mL single-necked vial, **M4** (prepared according to the method published in CN111051330 A, 1.73 g, 4.2 mmol), PyBOP (2.61 g, 5.04 mmol), HOBt (680 mg, 5.04 mmol) and 10 mL of DMF were added, then DIPEA (830 uL, 5.04 mmol) was added under an ice-water bath, stirred for 30 min, the above reaction solution was added to the reaction vial and raised to room temperature. The reaction was monitored by HPLC, the reaction solution was purified by preparative-HPLC to obtain the product preparation, the preparation was extracted by dichloromethane, washed by saturated sodium chloride solution, dried by anhydrous sodium sulfate, filtered, and the filtrate was concentrated under reduced pressure to obtain the solid **1b** (1.7 g), 63% yield; LC-MS : [M+H]⁺ =648.26.

### Step 3: Compound 1c

**1b** (900 mg, 1.39 mmol) was added to a 25 mL single-necked vial, and after dissolved in 15 mL of DMF, 900 mg of 5% Pd/C was added, and the reaction was hydrogenated for 2 h. After the reaction was completed, the filtrate was filtered and used directly in the next step without purification.

### Step 4: Compound 1d

The crude product **1c** was placed in an ice-water bath, DIPEA (235 uL, 1.39 mmol) and compound **M3** (784 mg, 1.39 mmol) were added, and the reaction was brought to room temperature for 1 h. The reaction was monitored for completion by HPLC, and the reaction solution was purified by preparative-HPLC to obtain the preparative solution, which was lyophilized to obtain **1d** (504 mg); LC-MS: [M+H]⁺ = 804.4.

### Step 5: Compound 1e

**1d** (500 mg, 0.62 mmol), **M5** (310 mg, 0.62 mmol), PyBOP (448 mg, 0.86 mmol), HOBt (116 mg, 0.86 mmol) and 15 mL of DMF were added to a 50 mL single-necked vial under an ice-water bath, and DIPEA (378 uL, 2.29 mmol) was added and the reaction was brought to room temperature for 2 h. After the the reaction was completed by HPLC monitoring, the reaction solution was purified by preparative-HPLC to obtain the preparative solution of compound **1e**, which was lyophilized to obtain **1e** (210 mg); LC-MS: [M+H]⁺ =1221.6.

### Step 6: Compound P1

**1e** (100 mg, 0.081 mmol), zinc bromide (368 mg, 1.63 mmol) and 5 mL of nitromethane were added to a 25 mL single-necked vial and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain solid compound **P1** (60 mg); LC-MS: [M+H]⁺ =1065.3.

### Example 4: Synthesis of compound P2

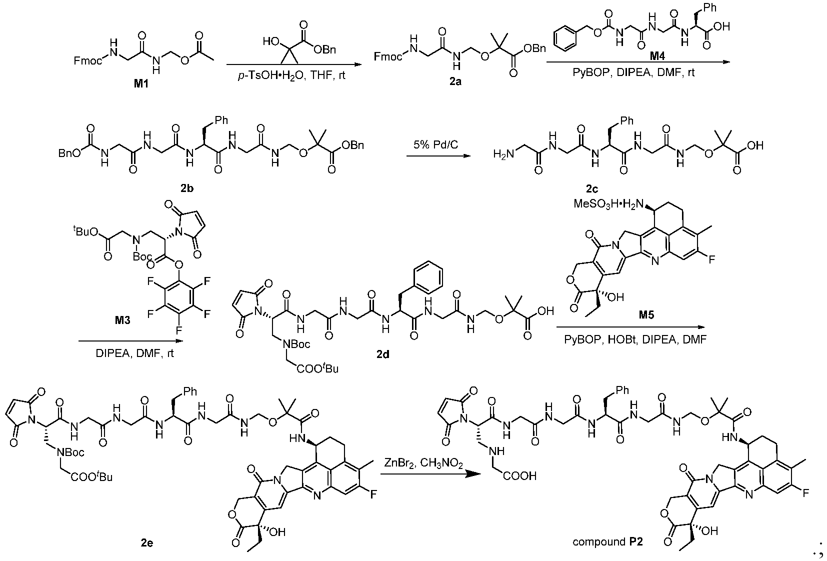

### Step 1: Compound 2a

In a 250 mL single-necked bottle, **M1** (6 g, 16.3 mmol), 100 mL THF, p-toluenesulfonic acid monohydrate (0.31 g, 1.63 mmol) were added, then cooled to 0 °C with stirring, benzyl 2-hydroxy-2-methylpropionate (6.3 g, 32.6 mmol) was added dropwise, and naturally warmed to room temperature for reaction (about 2-4 h) monitored by TLC. At the end of the reaction, saturated NaHCO₃ solution was added, extracted with ethyl acetate, washed with saturated sodium chloride solution, dried with anhydrous sodium sulfate, filtered, concentratee, and the residue was purified with silica gel column (PE:EA=10:1-5:1-2:1) to obtain **2a** (4.2 g), yield 52%; LC-MS: [M+H]⁺ =503.3.

### Step 2: Compound 2b

In a 25 mL single-necked vial, **2a** (2 g, 4.0 mmol) and 10 mL DMF were added, stirred at 0 °C, DBU (760 mg, 5.0 mmol) was added, reacted for 1 h, Fmoc was fully deprotected monitored by TLC, and set aside;
In another 25 mL single-necked bottle, **M4** (1.65 g, 4.0 mmol), PyBOP (2.59 g, 5.0 mmol), HOBt (675 mg, 5.0 mmol) and 10 mL of DMF were added, and then was added DIPEA (823 uL, 5.04 mmol) under ice-water bath, the reaction was stirred for 30 min, the above reaction solution was added to the reaction vial and raised to room temperature. After the completion of the reaction by HPLC monitoring, the reaction solution was purified by preparative-HPLC to obtain the product preparation solution, which was extracted by dichloromethane, washed with saturated sodium chloride solution, dried over anhydrous sodium sulfate, filtered, and the filtrate was concentrated under reduced pressure to obtain the solid **2b** (1.4 g), with a yield of 53%; LC-MS: [M+H]⁺ =676.2.

### Step 3: Compound 2c

In a 25 mL single-necked vial, **2b** (700 mg, 1.04 mmol) was added. After dissolving 10 mL DMF, 700 mg of 5% Pd/C was added. The hydrogenation reaction was carried out for 1.5 h. After the reaction was completed, the filtered filtrate was obtained and used directly for the next reaction without purification.

### Step 4: Compound 2d

The crude product **2c** was placed in an ice-water bath, DIPEA (210 uL, 1.25 mmol) and compound **M3** (704 mg, 1.25 mmol) were added, and the reaction was brought to room temperature for 1 h. The completion of the reaction was monitored by HPLC, and the reaction solution was purified by preparative-HPLC to give the preparative solution, which was lyophilized to obtain **2d** (486 mg); LC-MS: [M-H]⁻ = 830.5.

### Step 5: Compound 2e

In a 50 mL single-necked vial, **2d** (300 mg, 0.36 mmol), M5 (180 mg, 0.36 mmol), PyBOP (260 mg, 0.5 mmol), HOBt (67 mg, 0.5 mmol) and 10 mL of DMF were added, DIPEA (219.5 uL, 1.33 mmol) was added to the vial under an ice-water bath, and the reaction was carried out at room temperature for 3 h. The reaction was monitored by HPLC, and the reaction solution was purified by preparative-HPLC to obtain the preparative solution of compound **2e,** and the preparative solution was lyophilized to give **2e** (157 mg); LC-MS: [M+H]⁺ =1249.6.

### Step 6: Compound P2

In a 25 mL single-necked vial, **2e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was concentrated under reduced pressure, and the crude product was obtained. The crude product was purified by preparative-HPLC to obtain the product preparation solution, and the preparation solution, which was lyophilized to obtain solid compound **P2** (64 mg); LC-MS: [M+H]⁺ =1093.1.

### Example 5: Synthesis of compound P3

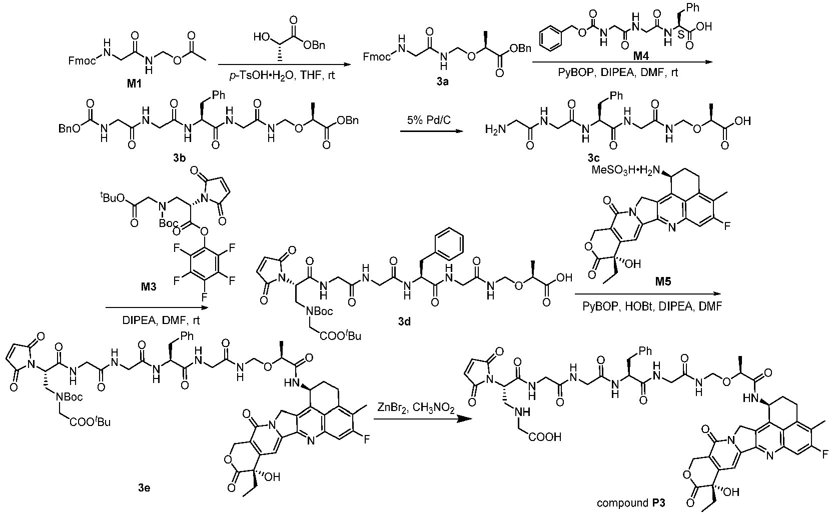

### Step 1: Compound 3a

In a 25 mL single-necked vial, **M1** (500 mg, 1.4 mmol), p-toluenesulfonic acid monohydrate (26 mg, 0.1 mmol) and 10 mL of THF were added, stirred well, and then cooled to 0 °C, and then slowly added L-lactic acid benzyl ester (1.2 g, 7.0 mmol), and the reaction was raised to room temperature after the addition of the product. The reaction was monitored by TLC, and at the end of the reaction, saturated NaHCO₃ solution was added, extracted with ethyl acetate, dried over anhydrous sodium sulfate, filtered, concentrated, and the residue was purified by reversed-phase column to obtain **3a** (400 mg); LC-MS: [M+NH₄] ⁺ =506.2. ¹H NMR (400 Mz, CDCl₃/CD₃OD):1.39 (3H, d, *J* = 6.8 Hz), 3.78 (2H, t, *J* = 4.0 Hz), 4.17-4.27 (2H, m), 4.42 (2H, d, *J* = 4.0 Hz), 4.72-4.85 (2H, m), 5.11-5.58 ( 2H, m), 5.43 (1H, s), 7.06 (1H, t, *J* = 8.0 Hz), 7.25-7.33 (6H, m), 7.38 (2H, t, J = 8.0 Hz), 7.57 (2H, d, *J* = 8.0 Hz), 7.75 (2H, d, *J* = 8.0 Hz).

### Step 2: Compound 3b

In a 25 mL single-necked vial, compound **3a** (400 mg, 0.8 mmol, 1.0 eq) and 4 mL of DMF were added, stirred well, and then cooled to 0 °C, and then DBU (137 mg, 0.9 mmol, 1.1 eq) was added slowly, and the reaction was raised to room temperature after the addition of compound **3a.** TLC was performed to monitor the end of the reaction, and the reaction was recorded as reaction solution ①;
In another 25 mL single-necked vial, **M4** (372 mg, 0.9 mmol, 1.1 eq), PyBOP (852 mg, 1.6 mmol, 2.0 eq) and 3 mL of DMF were added, stirred at room temperature for 5 min, and reaction solution ① was added, the reaction was carried out at room temperature, and monitored by HPLC. After the reaction was completed, the reaction solution was purified by preparative-HPLC to yield compound **3b** (326 mg); LC-MS: [M+NH₄] ⁺ =679.2.

### Step 3: Compound 3c

In a 100 mL single-necked vial, **3b** (4.0 g, 6.05 mmol, 1.0 eq) was added, DMF (60 mL) was added to dissolve, 5% Pd/C (4 g) was added, and the hydrogenation reaction was carried out at room temperature for 4 h (HPLC was used to monitor the progress of the reaction). The Pd/C was filtered and the filtrate was placed directly in an ice-water bath (ca. 0 °C) without concentration and set aside.

### Step 4: Compound 3d

The crude product **3c** was placed in an ice-water bath, DIPEA (1.1 mL, 1.1 eq) was added, and compound **M3** (3.4 g, 6.05 mmol) was added, and the reaction was brought to room temperature for 2 h. The completion of the reaction was monitored by HPLC, and the reaction solution was purified by preparative-HPLC to obtain the preparative solution, which was lyophilized to give **3d** (3.15 g); LC-MS: [M-H]⁻= 816.3.

### Step 5: Compound 3e

**3d** (2.07 g, 2.53 mmol, 1.0 eq), **M5** (1.35 g, 2.53 mmol, 1.0 eq), PyBOP (1.98 g, 3.79 mmol, 1.5 eq), HOBt (0.51 g, 3.79 mmol, 1.5 eq) and DMF (40 mL) were added to a 100 mL single-necked vial, and the reaction was brought to room temperature for 2 h. The reaction was monitored by HPLC. DIPEA (1.05 mL, 1.5 eq) was added, and the reaction was brought to room temperature for 2 h (monitored by HPLC). The reaction solution was prepared directly for purification, the preparative solution was concentrated by water pump under reduced pressure at 35 °C to remove acetonitrile, and lyophilized to obtain compound **3e** (1.92 g) in 61% yield; LC-MS: [M+H]⁺ =1235.4.

### Step 6: Compound P3

In a 100 mL single-necked vial, compound **3e** (1.0 g, 0.8 mmol, 1.0 eq), 35 mL nitromethane was added. After dissolution, zinc bromide was added (3.64 g, 16 mmol, 20.0 eq). The mixture was reacted in an oil bath at 40 °C for 30 min (preheated for stability). The mixture was concentrated in a water bath at 45 °C under reduced pressure to remove the nitromethane, yielding a yellow solid residue (monitored by HPLC). After acid preparation, the preparative solution was obtained, and the preparative solution was concentrated in a water bath at 35 °C with a water pump to spun off acetonitrile, and then lyophilized to obtain compound **P3** (786 mg) in 90% yield.LC-MS: [M+H]⁺ =1079.4;
¹H NMR (400 MHz, d6DMSO) δ 9.39- 9.02 (m, 1H), 8.70 (t, *J=* 6.5 Hz, 1H), 8.64 (t, *J =* 5.7 Hz, 1H), 8.56 (d, *J=* 8.8 Hz, 1H), 8.34 (t, *J* = 5.7 Hz, 1H), 8.16 (d, *J* = 8.2 Hz, 1H), 8.01 (t, *J* = 5.5 Hz, 1H), 7.71 (d, J = 10.9 Hz, 1H), 7.30 (s, 1H) 1H), 8.01 (t, *J* = 5.5 Hz, 1H), 7.71 (d, *J* = 10.9 Hz, 1H), 7.30 (s, 1H), 7.28 - 7.15 (m, 4H), 7.14 (s, 2H), 5.53 (dd, *J* = 14.5, 6.4 Hz, 1H), 5.49 - 5.34 (m, 2H), 5.22 (d, *J* = 18.8 Hz, 1H), 5.09 (d, *J* = 18.7 Hz, 1H), 5.03 (dd, *J* = 9.6, 3.9 Hz, 1H), 4.73 (dd, *J =* 9.9, 6.9 Hz, 1H), 4.59 (dd, *J* = 10.1, 6.5 Hz, 1H), 4.49 (dd, *J* = 13.2, 8.6, 4.4 Hz, 1H), 4.14 (dd, *J =* 13.3, 6.6 Hz, 2H), 3.93 (s, 2H), 3.84 (dd, *J* = 16.5, 6.3 Hz, 1H), 3.76 (dd, *J* = 16.9, 5.7 Hz, 2H), 3.70 (d, J = 5.2 Hz, 2H), 3.70 (d, J = 5.2 Hz, 2H). *J* = 5.2 Hz, 2H), 3.60 (dd, *J* = 16.7, 5.4 Hz, 1 H), 3.52 (dd, *J* = 16.4, 5.1 Hz, 1H), 3.45 (dd, *J* = 12.8, 10.1 Hz, 1H), 3.25 - 3.15 (m, 1H), 3.14 - 3.05 (m, 1H), 3.01 (dd, *J* = 13.7, 4.1 Hz, 1H), 2.73 (dd, *J* = 13.5, 9.8 Hz, 1H), 2.54 - 2.47 (m, 1H), 2.33 (s, 2H), 2.17 (d, *J* = 5.5 Hz, 2H), 1.91 - 1.79 (m, 2H), 1.33 (d, *J* = 6.6 Hz, 2H), 0.87 (t, *J* = 7.3 Hz, 2H).

### Example 6: Synthesis of compound P4

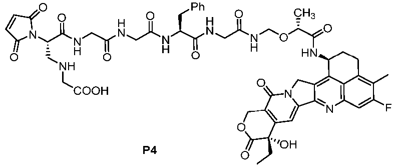

Compound **P4** (468 mg) was prepared according to the synthetic route in Example 5. LC-MS: [M+H]⁺ =1079.6.

### Example 7: Synthesis of compound P5

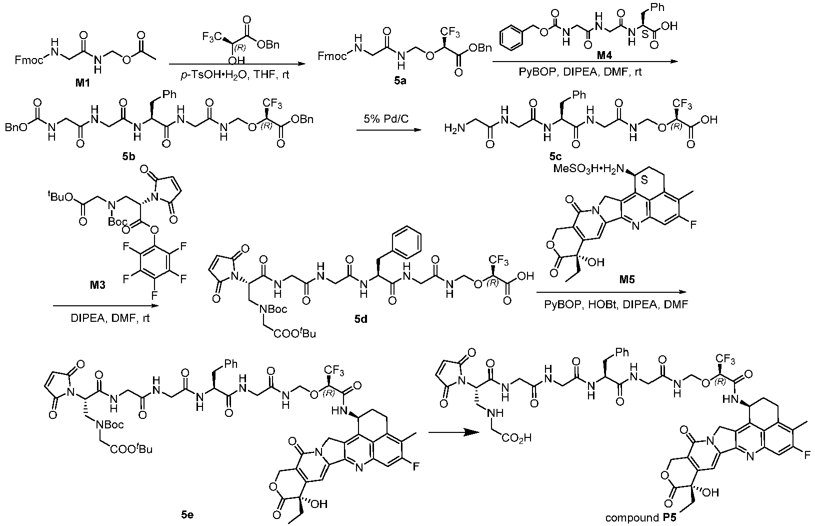

### Step 1: Compound 5a

In a 250 mL single-necked vial, **M1** (10 g, 27.1 mmol), R-3, 3, 3-trifluorolactic acid benzyl ester (prepared according to the method published in WO2020063673A1) (12.7 g, 54.3 mmol), zinc acetate (9.96 g, 54.3 mmol) and 100 mL of toluene were added and reacted at 100 °C for 4 h. After the reaction was completed, the reaction was cooled to room temperature, insoluble material was filtered, and the filtrate was concentrated to obtain a crude product. The crude product was purified by silica gel column chromatography (PE:EA=10:1-5:1-2:1) to obtain 5.15 g of the target product with a yield of 35.1%; LC-MS: [M+H]⁺ =543.17.

### Step 2: Compound 5b

In a 50 mL single-necked flask, **5a** (5 g, 9.2 mmol) and 15 mL of DMF were added, dissolved and clear, and then DBU (1.68 g, 11 mmol) was added in an ice-water bath and reacted for 1 h. This was recorded as reaction solution ①;
In another 50 mL vial, **M4** (3.8 g, 9.2 mmol), PyBOP (5.75 g, 11 mmol), HOBt (1.49 g, 11 mmol) and 10 mL of DMF were added, and after dissolved, DIPEA (1.82 mL, 11 mmol) was added in an ice-water bath, and the reaction was continued for 30 min, then reaction solution ① was added, and the reaction was brought to room temperature for 2 h. The reaction was monitored by HPLC after 2 h. After completion of the reaction, the reaction solution was purified by preparative-HPLC to obtain the preparative solution. The prepared solution was extracted with dichloromethane, washed with saturated sodium chloride solution, dried with anhydrous sodium sulfate, filtered, and concentrated to obtain 4.1 g of solid, with a yield of 62.3%; LC-MS: [M+H]⁺ =716.25.

### Step 3: Compound 5d

In a 25 mL single-necked vial, **5b** (900 mg, 1.26 mmol) was added and 15 mL of DMF was added to dissolve. Then, 900 mg 5% Pd/C was added, and the hydrogenation reaction was carried out for 2 h. After the reaction was completed and filtered, the filtrate was placed in an ice-water bath, DIPEA (228 uL, 1.38 mmol) was added, and then **M3** (712 mg, 1.26 mmol) was added. After the addition, the temperature was raised to room temperature and the reaction was allowed to react for 1 h. The reaction solution was purified by preparative-HPLC to obtain a preparative solution, which was lyophilized to obtain 525 mg of the product with a yield of 47.9%; LC-MS: [M-H]⁻ = 870.33.

### Step 4: Compound 5e

In a 50 mL single-necked vial, **5d** (500 mg, 0.57 mmol), **M5** (305 mg, 0.57 mmol), PyBOP (448 mg, 0.86 mmol), HOBt (116 mg, 0.86 mmol) and 15 mL of DMF were added. Then, DIPEA (378 uL, 2.29 mmol) was added under an ice-water bath, and the reaction was carried out at room temperature for 2 h. After the reaction was completed by HPLC monitoring, the reaction solution was purified by preparative-HPLC to obtain a preparative solution of compound **5e,** which was lyophilized to obtain 150 mg of compound **5e,** LC-MS: [M+H]⁺ =1289.46.

### Step 5: Compound P5

In a 25 mL single-necked vial, **5e** (100 mg, 0.077 mmol), zinc bromide (349 mg, 1.55 mmol) and 5 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain 52 mg of solid; TOF-MS: [M+H]⁺ = 1133.3613.

### Example 8: Synthesis of compound P6

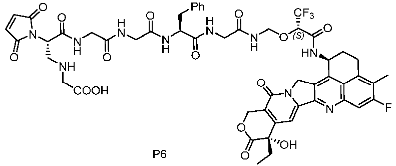

Compound **P6** (221 mg) was prepared according to the synthetic route in Example 7. LC-MS: [M+H]⁺ =1133.5.

### Example 9: Synthesis of compound P7

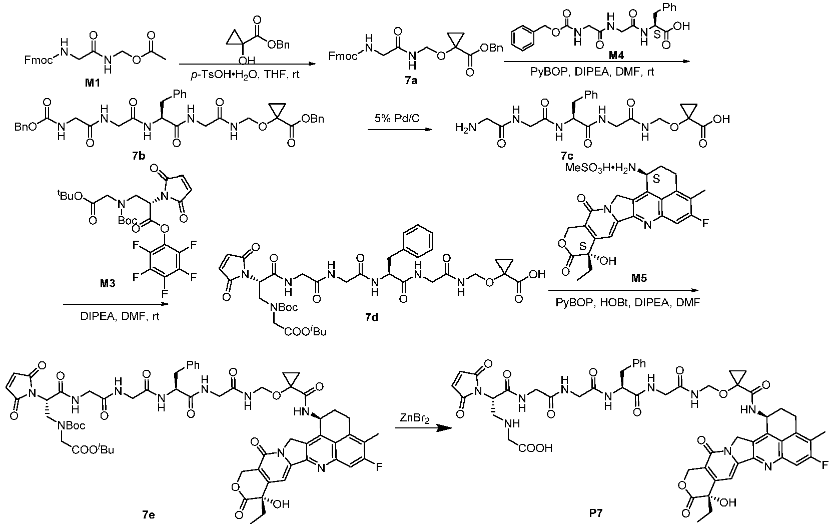

### Step 1: Compound 7a

In a 250 mL single-necked vial, **M1** (6 g, 16.3 mmol), 100 mL THF, p-toluenesulfonic acid monohydrate (0.31 g, 1.63 mmol) were added, cooled to 0 °C with stirring, benzyl 2-hydroxy-2-cyclopropyl acetate (prepared according to the method published in US20050020645 A1) (6.3 g, 32.6 mmol) was added. , and naturally warmed to room temperature after dropping (reaction for about 2-4 h), monitored by TLC. At the end of the reaction, saturated NaHCO₃ solution was added, extracted with ethyl acetate, washed with saturated sodium chloride solution, dried with anhydrous sodium sulfate, filtered, concentrated, and the residue was purified by silica gel column (PE:EA=10:1-5:1-2:1) to give **7a** (3.7 g) in 45% yield; LC-MS: [M+H]⁺ =501.5.

### Step 2: Compound 7b

In a 25 mL single-necked vial, **7a** (2 g, 4.0 mmol) and 10 mL DMF were added, stirred at 0 °C, DBU (760 mg, 5.0 mmol) was added, and reacted for 1 h. After the Fmoc-deprotection was completed monitored by TLC, the reaction was set aside;
In another 25 mL single-necked vial, **M4** (1.65 g, 4.0 mmol), PyBOP (2.59 g, 5.0 mmol), HOBt (675 mg, 5.0 mmol) and 10 mL of DMF, and DIPEA (823 uL, 5.04 mmol) were added under an ice-water bath and continued stirring for 30 min. Then the reaction solution was added, and the reaction was brought to room temperature. After the reaction was completed by HPLC monitoring, the reaction solution was purified by preparative-HPLC to obtain the product preparation solution, which was extracted by dichloromethane, washed with saturated sodium chloride solution, dried over anhydrous sodium sulfate, filtered, and the filtrate was concentrated under reduced pressure to obtain a solid of 1.5 g in a yield of 56%; LC-MS: [M+H]⁺ =674.7.

### Step 3: Compound 7c

In a 25 mL single-necked vial, **7b** (900 mg, 1.3 mmol) was added, 10 mL of DMF was added to dissolve, 900 mg of 5% Pd/C was added, and the reaction was hydrogenated for 1.5 h. The reaction was completed and filtered to obtain the filtrate, which was used directly for the next step of the reaction without purification.

### Step 4: Compound 7d

The crude product **7c** was placed in an ice-water bath, DIPEA (223 uL, 1.3 mmol) was added, and compound **M3** (750 mg, 1.3 mmol) was added, and the reaction was brought to room temperature for 1 h. The completion of the reaction was monitored by HPLC, and the reaction solution was purified by preparative-HPLC to obtain the preparative solution, which was lyophilized to give **7d** (529 mg); LC-MS: [M-H]⁻= 828.4. 828.4.

### Step 5: Compound 7e

In a 50 mL vial, **7d** (500 mg, 0.6 mmol), **M5** (300 mg, 0.6 mmol), PyBOP (416 mg, 0.8 mmol), HOBt (108 mg, 0.5 mmol) and 15 mL of DMF were added, DIPEA (351 uL, 2.13 mmol) was added under an ice-water bath, and the reaction was carried out for 3 h. After the reaction was completed by HPLC monitoring, the reaction solution was purified by preparative-HPLC to obtain the preparative solution of compound **7e,** which was lyophilized to obtain 7e (257 mg); LC-MS: [M+H]⁺ =1247.5.

### Step 6: Compound P7

In a 25 mL single-necked vial, **7e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was concentrated under reduced pressure to remove the solvent, and a crude product was obtained. The crude product was purified by preparative-HPLC to obtain a product preparation solution, and the preparation solution was lyophilized to obtain the solid compound **P7** (55 mg); LC-MS: [M+H]⁺ =1091.3.

### Example 10: Synthesis of compound P8

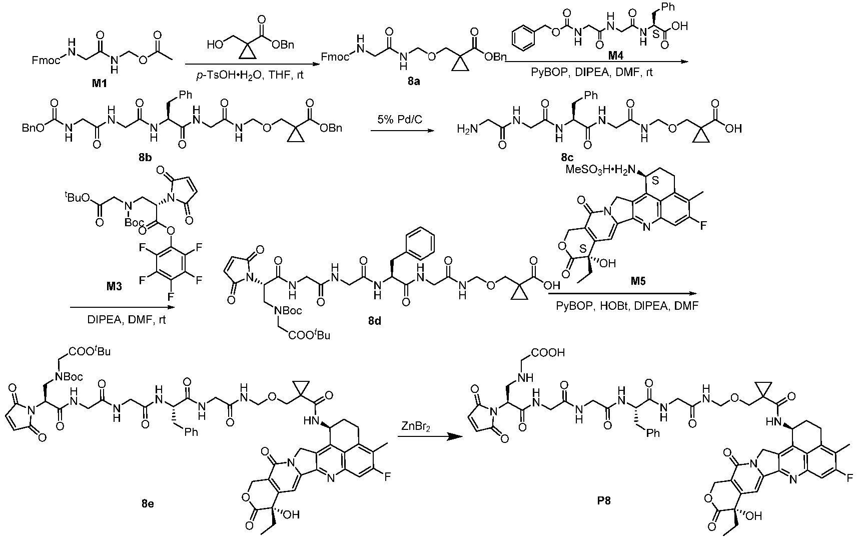

### Step 1: Compound 8a

In a 250 mL single-necked vial, **M1** (6 g, 16.3 mmol), 100 mL THF, p-toluenesulfonic acid monohydrate (0.31 g, 1.63 mmol) was added, stirred and cooled to 0 °C, benzyl 3-hydroxy-2-cyclopropylpropionate (prepared according to the method published in WO2013187496A1, 6.7 g, 32.6 mmol) was added, and naturally warmed to room temperature (about 2-4 h) by TLC monitoring. After the completed reaction, saturated NaHCO₃ solution was added, extracted with ethyl acetate, washed with saturated sodium chloride solution, dried with anhydrous sodium sulfate, filtered, concentratee, and purifiedthe residue on a silica gel column (PE:EA=10:1-5:1-2:1) to obtain **8a** (4.9 g) in 58% yield; LC-MS: [M+H]⁺ =515.4.

### Step 2: Compound 8b

In a 25 mL single-necked vial, **8a** (4 g, 7.8 mmol) and 10 mL DMF was added, stirred at 0 °C, DBU (1.2 g, 8.0 mmol) was added, and reacted for 1 h, monitored the completion of Fmoc-deprotection by TLC, and set aside;
In another 25 mL single-necked vial **M4** (3.3 g, 8.0 mmol), PyBOP (5.2 g, 10.0 mmol), HOBt (1.35 g, 10.0 mmol) and 10 mL of DMF were added, and DIPEA (1.65 mL, 10.1 mmol) was added in an ice-water bath with continued stirring for 50 min, and then the above reaction solution was added to the vial, and warmed to room temperature for reaction. After the reaction was completed by HPLC monitoring, the reaction solution was purified by preparative-HPLC to obtain a product preparation solution, which was extracted by dichloromethane, washed with saturated sodium chloride solution, dried with anhydrous sodium sulfate, filtered, and the filtrate was concentrated under reduced pressure to obtain 2.3 g of solid, with a yield of 42%; LC-MS: [M+H]⁺ =688.8.

### Step 3: Compound 8c

In a 25 mL single-necked vial, **8b** (1.0 g, 1.45 mmol) was added. After dissolving in 15 mL DMF, 1.0 g of 5% Pd/C was added. The hydrogenation reaction was carried out for 1.5 h. After the reaction was completed, it was filtered, and the filtrate was used directly for the next step without purification.

### Step 4: Compound 8d

The crude product **8c** was placed in an ice-water bath, DIPEA (258 uL, 1.5 mmol) was added, and compound **M3** (837 mg, 1.45 mmol) was added, and the reaction was brought to room temperature for 1 h. The completion of the reaction was monitored by HPLC, and the reaction solution was purified by preparative-HPLC to obtain a preparative solution, which was lyophilized to obtain **10d** (499 mg); LC-MS: [M-H]⁻= 842.4.

### Step 5: Compound 8e

In a 50 mL single-necked vial, **8d** (400 mg, 0.48 mmol), **M5** (240 mg, 0.48 mmol), PyBOP (250 mg, 0.48 mmol), HOBt (104 mg, 0.48 mmol) and 15 mL of DMF were added, and DIPEA (330 uL, 2.0 mmol) was added under an ice-water bath, and the mixture was heated to room temperature for 3 h. After the reaction was completed by HPLC monitoring, the reaction solution was purified by preparative-HPLC to obtain a preparative solution of compound **8e,** which was lyophilized to obtain **8e** (188 mg); LC-MS: [M+H]⁺ =1261.5.

### Step 6: Compound P8

In a 25 mL single-necked vial, **8e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, and the preparation solution was lyophilized to obtain the solid compound **P8** (61 mg); LC-MS: [M+H]⁺ =1105.6.

### Example 11: Synthesis of compound P9

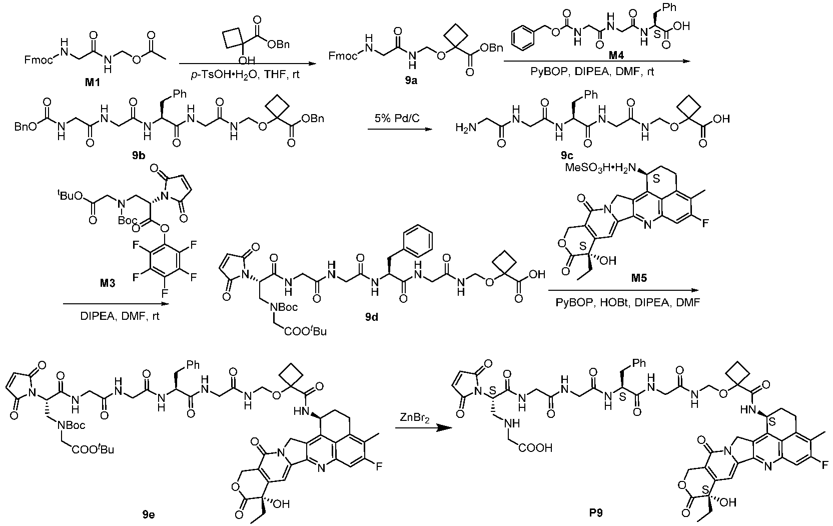

### Step 1: Compound 9a

In a 250 mL single-necked vial, **zyM1** (6 g, 16.3 mmol), 100 mL THF, *p*-toluenesulfonic acid monohydrate (0.31 g, 1.63 mmol, was added), stirred and cooled to 0 °C, benzyl 2-hydroxy-2-cyclobutyl acetate (synthesized according to the method published in Journal of Medicinal Chemistry, 2013, 56(13), 5541-5552., (6.7 g, 32.6 mmol) was added, naturally warmed to room temperature for the reaction after dropwise (the reaction was carried out for about 2-4 h) and monitored by TLC. After the reaction, saturated NaHCO₃ solution was added, extracted with ethyl acetate, washed with saturated sodium chloride solution, dried with anhydrous sodium sulfate, filtered, concentrated, and purified the residue by silica gel column (PE:EA=10:1-5:1-2:1) to obtain **9a** (5.1 g) in a yield of 62%; LC-MS: [M+H]⁺ =515.7.

### Step 2: Compound 9b

In a 25 mL single-necked vial was added **9a** (4 g, 7.8 mmol), 10 mL DMF, stirred at 0 °C, DBU (1.2 g, 8.0 mmol) was added, and reacted for 1 h. After monitoring the completion of Fmoc-deprotection by TLC, the reaction was set aside;
In another 25 mL single-necked vialwas added **M4** (3.3 g, 8.0 mmol), PyBOP (5.2 g, 10.0 mmol), HOBt (1.35 g, 10.0 mmol) and 10 mL of DMF, and added DIPEA (1.63 mL, 10.0 mmol) under an ice-water bath, continued stirring for 40 min, and then the above reaction solution was added to the vial, and warmed to room temperature for reaction. After the reaction was completed by HPLC monitoring, the reaction solution was purified by preparative-HPLC to obtain a product preparation solution, which was extracted by dichloromethane, washed with saturated sodium chloride solution, dried with anhydrous sodium sulfate, filtered, and the filtrate was concentrated under reduced pressure to obtain 2.3 g of solid, with a yield of 42%; LC-MS: [M+H]⁺ =688.3.

### Step 3: Compound 9c

In a 25 mL single-necked vial, **9b** (2.0 g, 2.9 mmol) was added. After dissolving in 25 mL DMF, 2.0 g 5% Pd/C was added. The hydrogenation reaction was carried out for 3 h. After completion of the reaction, it was filtered, and the filtrate was obtained and used directly for the next step of the reaction without purification.

### Step 4: Compound 9d

The crude product **9c** was placed in an ice-water bath, DIPEA (516 uL, 3.0 mmol) was added, and compound **M3** (1.7 g, 2.9 mmol) was added, and the reaction was brought to room temperature for 2 h. The completion of the reaction was monitored by HPLC, and the reaction solution was purified by preparative-HPLC to obtain a preparative solution, which was lyophilized to obtain **9d** (934 mg); LC-MS: [M-H]⁻= 842.4. 842.4.

### Step 5: Compound 9e

In a 50 mL single-necked vial, **9d** (800 mg, 0.96 mmol), **M5** (480 mg, 0.96 mmol), PyBOP (500 mg, 0.96 mmol), HOBt (208 mg, 0.96 mmol) and 30 mL of DMF were added, and DIPEA (660 uL, 4.0 mmol) was added under an ice-water bath, and the reaction was warmed to room temperature and carried out for 4 h. After the reaction was completed by HPLC monitoring, the reaction solution was purified by preparative-HPLC to obtain a preparative solution of compound **9e,** which was lyophilized to obtain **9e** (401 mg); LC-MS: [M+H]⁺ =1261.4.

### Step 6: Compound P9

In a 25 mL single-necked vial, **9e** (150 mg, 0.12 mmol), zinc bromide (532 mg, 2.4 mmol) and 10 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain a solid compound **P9** (86 mg); LC-MS: [M+H]⁺ =1105.5.

### Example 12: Synthesis of compound P10

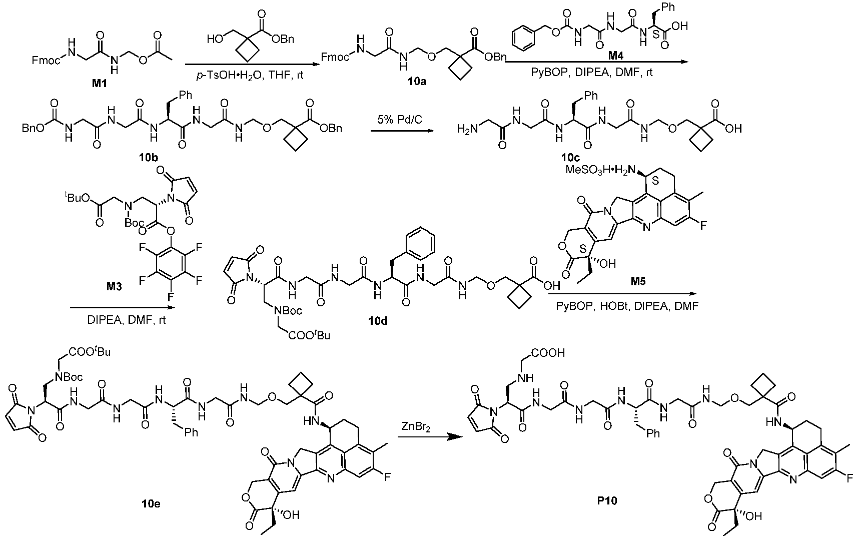

### Step 1: Compound 10a

In a 250 mL single-necked bottle, **M1** (6 g, 16.3 mmol) were added, 100 mL THF, π-toluenesulfonic acid monohydrate (0.31 g, 1.63 mmol), stirred and cooled to 0 °C, benzyl 3-hydroxy-2-cyclobutylpropionate (prepared according to the method published in WO2009011285A1, 7.2 g, 32.6 mmol) was added. After dropping, the reaction was naturally warmed up to room temperature (about 2-4 h) and monitored by TLC. After the reaction was completed, saturated NaHCO₃ solution was added, extracted with ethyl acetate, washed with saturated sodium chloride solution, dried with anhydrous sodium sulfate, filtered, concentrated, and purified by silica gel column (PE:EA=10:1-5:1-2:1) to obtain 10a (4.5 g), with a yield of 52%; LC-MS: [M+H]⁺ =529.4.

### Step 2: Compound 10b

In a 25 mL single-necked vial, **10a** (4 g, 7.6 mmol) was added, 10 mL DMF, stirred at 0 °C, added DBU (1.2 g, 8.0 mmol), reacted for 1 h, monitored the Fmoc-deprotection by TLC, and set aside;

In another 25 mL single-necked vial, **M4** (3.2 g, 7.6 mmol), PyBOP (4.7 g, 9.0 mmol), HOBt (1.22 g, 9.0 mmol) and 10 mL of DMF were added, DIPEA (1.49 mL, 0.9 mmol) was added under an ice-water bath, and continued stirring for 30 min, then the above reaction solution was added to the reaction vial, and warmed to room temperature for reaction. After the reaction was completed by HPLC monitoring, the reaction solution was purified by preparative-HPLC to obtain a product preparation solution, which was extracted by dichloromethane, washed with saturated sodium chloride solution, dried with anhydrous sodium sulfate, filtered, and the filtrate was concentrated under reduced pressure to obtain 2.0 g of solid, with a yield of 37%; LC-MS: [M+H]⁺ =702.8.

### Step 3: Compound 10c

In a single-necked vial, **10b** (1.0 g, 1.43 mmol) was added, after dissolving in 15 mL DMF, 1.0 g 5% Pd/C was added. The hydrogenation reaction was carried out for 1.5 h. After the reaction was completed, the filtrate was filtered and used directly in the next step without purification.

### Step 4: Compound 10d

The crude product **10c** was placed in an ice-water bath, DIPEA(258 uL, 1.5 mmol) was added, followed by compound **M3** (825 mg, 1.43 mmol), and the reaction was brought to room temperature for 1 h. The reaction was monitored by HPLC, and the reaction solution was purified by preparative-HPLC to obtain a preparative solution, which was lyophilized to obtain **10d** (522 mg); LC-MS: [M-H]⁽⁻⁾=856.4.

### Step 5: Compound 10e

In a 50 mL single-necked vial, **10d** (400 mg, 0.47 mmol), **M5** (240 mg, 0.47 mmol), PyBOP (250 mg, 0.47 mmol), HOBt (101 mg, 0.47 mmol) and 15 mL of DMF were added, and DIPEA (330 uL, 2.0 mmol) was added under an ice-water bath, and the mixture was heated to room temperature for 3 h. After the reaction was completed by HPLC monitoring, the reaction solution was purified by preparative-HPLC to obtain a preparation solution of compound **10e,** which was lyophilized to obatin **10e** (198 mg); LC-MS: [M+H]⁺ =1275.4.

### Step 6: Compound P10

In a 25 mL single-necked vial, **10e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was concentrated under reduced pressure to remove the solvent, and a crude product was obtained. The crude product was purified by preparative-HPLC to obtain a product preparation solution, which was lyophilized to obtain solid compound **P10** (55 mg); LC-MS: [M+H]⁺ =1119.5.

### Example 13: Synthesis of Compound P11

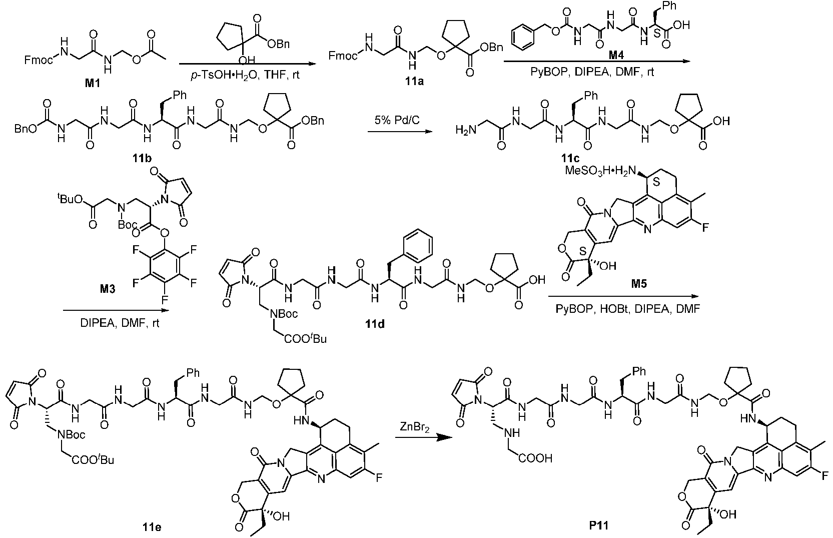

### Step 1: Compound 11a

In a 250 mL single-necked vial, **M1** (6 g, 16.3 mmol) and 100 mL THF, *p*-toluenesulfonic acid monohydrate (0.31 g, 1.63 mmol) were added, stirred and cooled to 0 °C, benzyl 1-hydroxycyclopentane-1-carboxylate (synthesized according to the method published in Journal of Medicinal Chemistry, 2013, 56(13), 5541-5552) (7.2 g, 32.6 mmol) was added, and naturally warmed to room temperature after dropwise for reaction (reaction for about 2-4 h) under TLC monitoring. After the reaction is completed, added saturated NaHCO₃ solution, extracted with ethyl acetate, washed with saturated sodium chloride solution, dried with anhydrous sodium sulfate, filtered, concentrated, and purified with silica gel column (PE:EA=10:1-5:1-2:1) to obtain **11a** (4.6 g) in a yield of 53%; LC-MS: [M+H]⁺ =529.5.

### Step 2: Compound 11b

In a 25 mL single-necked vial, **11a** (4 g, 7.6 mmol) and 10 mL DMF were added, stirred at 0 °C, DBU (1.17 g, 7.8 mmol) was added, and reacted for 1 h, monitor the completion of Fmoc deprotection by TLC, and set aside;
In another 25 mL single-necked vial, **M4** (3.14 g, 7.6 mmol), PyBOP (4.42 g, 8.5 mmol), HOBt (1.15 g, 8.5 mmol) and 10 mL of DMF were added, then DIPEA (1.39 mL, 0.85 mmol) was added under an ice-water bath, continued stirring for 30 min, the above reaction solution was added to the vial, and warmed to room temperature for reaction. After the reaction is completed by HPLC monitoring, the reaction solution was purified by preparative-HPLC to obtain a product preparation solution, which was extracted by dichloromethane, washed with saturated sodium chloride solution, dried with anhydrous sodium sulfate, filtered, and the filtrate was concentrated under reduced pressure to obtain 2.1 g of solid, with a yield of 39%; LC-MS: [M+H]⁺ =702.8.

### Step 3: Compound 11c

In a 25 mL single-necked vial, **11b** (1.5 g, 1.87 mmol) was added. After dissolving in 25 mL DMF, 1.5 g 5% Pd/C was added. The hydrogenation reaction was carried out for 3 h. After the reaction was completed, the filtrate was filtered and used directly in the next step without purification.

### Step 4: Compound 11d

The crude product **11c** was placed in an ice-water bath, DIPEA (333 uL, 1.93 mmol) was added, compound **M3** (1.1 g, 1.87 mmol) was added, and the reaction was brought to room temperature for 1 h. The completion of the reaction was monitored by HPLC, and the reaction solution was purified by preparative-HPLC to obtain a preparative solution, which was lyophilized to obtain **11d** (519 mg); LC-MS: [M-H] ⁻=856.6.

### Step 5: Compound 11e

In a 50 mL single-necked bottle, **11d** (400 mg, 0.47 mmol), M5 (240 mg, 0.48 mmol), PyBOP (250 mg, 0.48 mmol), HOBt (103 mg, 48 mmol) and 15 mL of DMF were added, and DIPEA (330 uL, 2.0 mmol) was added under an ice-water bath, and the reaction was heated to room temperature and carried out for 4 h. After the reaction was completed by HPLC monitoring, the reaction solution was purified by preparative-HPLC to obtain a preparation solution of compound **11e**, which was lyophilized to obtain **11e** (187 mg); LC-MS: [M+H]⁺ =1275.5.

### Step 6: Compound P11

In a 25 mL single-necked vial, **11e** (100 mg, 0.08 mmol), zinc bromide (355 mg, 0.16 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid compound **P11** (60 mg); LC-MS: [M+H]⁺ =1119.7.

### Example 14: Synthesis of compound P12

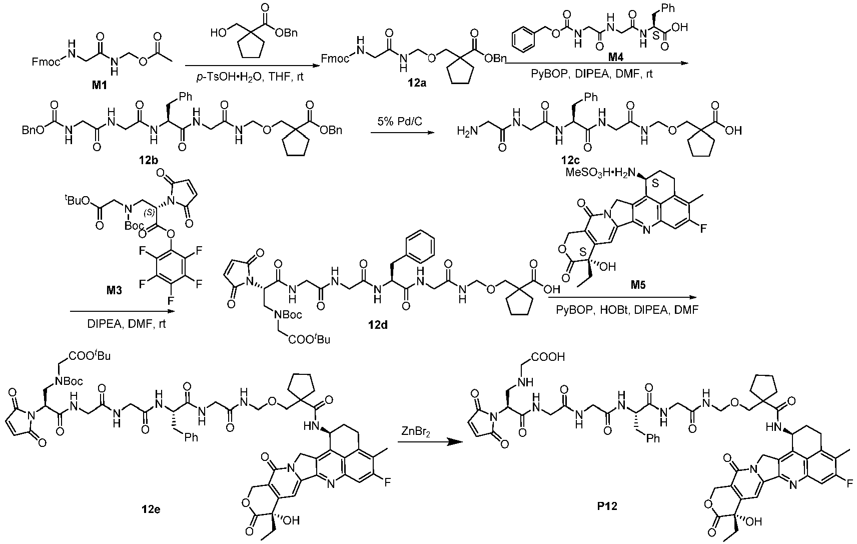

### Step 1: Compound 12a

In a 250 mL single-necked bottle, **M1** (6 g, 16.3 mmol), 100 mL THF, *p*-toluenesulfonic acid monohydrate (0.31 g, 1.63 mmol) were added, stirred and cooled to 0 °C, benzyl 1-(hydroxymethyl)cyclopentane-1-carboxylate (synthesized according to the method published in WO2009011285A1) (7.6 g, 32.6 mmol) was added, and naturally warmed to room temperature for reaction (reaction for about 2-4 h), monitored by TLC. After the reaction, saturated NaHCO₃ solution was added, extracted with ethyl acetate, washed with saturated sodium chloride solution, dried with anhydrous sodium sulfate, filtered, concentrated, and purified by silica gel column (PE:EA=10:1-5:1-2:1) to obtain **12a** (4.4 g), with a yield of 49%; LC-MS: [M+H]⁺ =543.6.

### Step 2: Compound 12b

In a 25 mL single-necked vial, **12a** (4 g, 7.4 mmol) and 10 mL DMF were added, stirred at 0 °C, DBU (1.2 g, 8.0 mmol) was added, reacted for 1 h, and monitored the completion of Fmoc deprotection by TLC, and set aside;

In another 25 mL single-necked vial, **M4** (3.1 g, 7.4 mmol), PyBOP (4.6 g, 8.8 mmol), HOBt (1.19 g, 8.8 mmol) and 10 mL of DMF were added, and DIPEA (1.49 mL, 9.0 mmol) was added under ice-water bath, and continued stirring for 30 min, then the above reaction solution was added to the reaction vial, and raised the temperature to room temperature. After the reaction was completed by HPLC monitoring, the reaction solution was purified by preparative-HPLC to obtain a product preparation solution, which was extracted by dichloromethane, washed with saturated sodium chloride solution, dried with anhydrous sodium sulfate, filtered, and the filtrate was concentrated under reduced pressure to obtain 2.6 g of solid, with a yield of 49%; LC-MS: [M+H]⁺ =716.4.

### Step 3: Compound 12c

In a 25 mL single-necked vial, **12b** (1.0 g, 1.4 mmol) was added. After dissolving in 15 mL DMF, 1.0 g 5% Pd/C was added. The hydrogenation reaction was carried out for 1.5 h. After the reaction was completed, the filtrate was filtered and used directly in the next step without purification.

### Step 4: Compound 12d

The crude product **12c** was placed in an ice-water bath, DIPEA (248 uL, 1.5 mmol) was added, and compound **M3** (808 mg, 1.4 mmol) was added, and the reaction was brought to room temperature for 1 h. The completion of the reaction was monitored by HPLC, and the reaction solution was purified by preparative-HPLC to obtain a preparative solution, which was lyophilized to obtain **12d** (500 mg); LC-MS: [M-H]⁻ = 870.5.

### Step 5: Compound 12e

In a 50 mL single-necked vial, **12d** (400 mg, 0.46 mmol), M5 (235 mg, 0.46 mmol), PyBOP (245 mg, 0.46 mmol), HOBt (99 mg, 0.46 mmol) and 15 mL of DMF were added, and DIPEA (331 uL, 2.0 mmol) was added in an ice-water bath, and the reaction was warmed to room temperature for 3 h. After the reaction was completed by HPLC monitoring, the reaction solution was purified by preparative-HPLC to obtain a preparation solution of compound **12e,** which was lyophilized to obtain**12e** (146 mg); LC-MS: [M+H]⁺ =1289.5.

### Step 6: Compound P12

In a 25 mL single-necked vial, **12e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid compound **P12** (52 mg); LC-MS: [M+H]⁺ =1133.7.

### Example 15: Synthesis of Compound P13

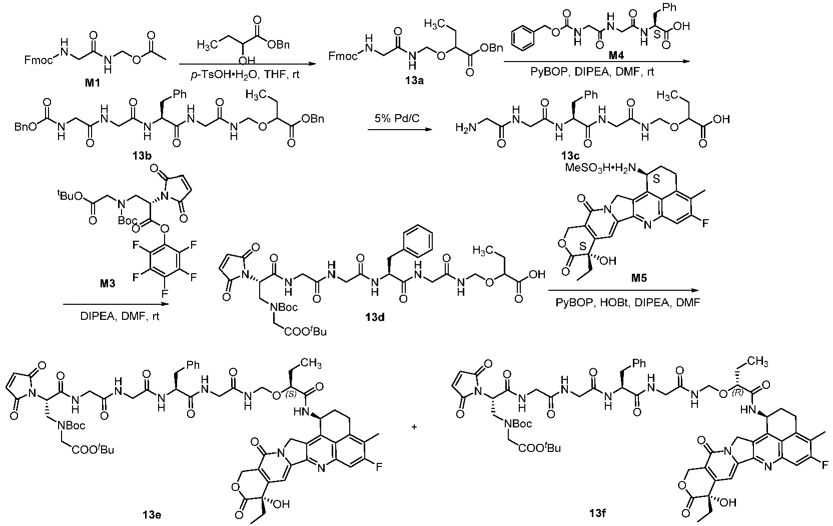

### Step 1: Compound 13a

In a 250 mL single-necked vial, **M1** (10 g, 27.1 mmol), benzyl 2-hydroxybutanoate (prepared according to the method published in Chemical Communications, 2019, 55(53), 7699-7702) (10.5 g, 54.3 mmol), zinc acetate (9.96 g, 54.3 mmol) and 100 mL of toluene were added and heated to 100 °C reaction for 4 h. After the reaction was completed, the temperature was cooled to room temperature, the insoluble material was filtered out, and the filtrate was concentrated to obtain a crude product. The crude product was purified by silica gel column chromatography (PE:EA=10:1-5:1-2:1) to obtain 5.67 g of the target product with a yield of 42%; LC-MS: [M+H]⁺ =503.5.

### Step 2: Compound 13b

In a 50 mL single-necked vial, **13a** (5 g, 9.95 mmol) and 15 mL of DMF were added. After dissolving, DBU (1.68 g, 11 mmol) was added under ice-water bath. The reaction was continued for 1 h, which was recorded as reaction solution ①;
In another 50 mL vial, **M4** (4.1 g, 10.0 mmol), PyBOP (5.75 g, 11 mmol), HOBt (1.49 g, 11 mmol) and 10 mL DMF were added, and after dissolved, DIPEA (1.82 mL, 11 mmol) was added under an ice-water bath, and the reaction was continued for 40 min, then reaction solution ① was added, and the reaction was brought to room temperature for 2 h. The reaction progress was monitored by HPLC. After the reaction was completed, the reaction solution was purified by high-performance liquid chromatography to obtain a preparation solution, which was extracted with dichloromethane, washed with saturated sodium chloride solution, dried over anhydrous sodium sulfate, filtered and concentrated to obtain 4.6 g of solid with a yield of 68%; LC-MS: [M+H]+=676.7.

### Step 3: Compound 13d

In a 25 mL single-necked vial, **13b** (2.0 g, 2.96 mmol) and 15 mL of DMF was added to dissolve it. Then, 2.0 g of 5% Pd/C was added. The hydrogenation reaction was carried out for 2 h. After the reaction was completed, the filtrate was filtered and placed in an ice-water bath. DIPEA (496 uL, 3.0 mmol) was added, and then M3 (1.7 g, 2.96 mmol) was added. After the addition, the temperature was raised to room temperature and the reaction was allowed to react for 1 h. The reaction was monitored by HPLC. The reaction solution was purified by preparative-HPLC to obtain a preparation solution, which was lyophilized to obtain 1120.0 mg of the product, with a yield of 45%; LC-MS: [M-H]-=830.3.

### Step 4: Compounds 13e and 13f

In a 50 mL single-necked vial, **13d** (500 mg, 0.60 mmol), **M5** (321 mg, 0.60 mmol), PyBOP (469 mg, 0.90 mmol), HOBt (121 mg, 0.90 mmol) and 15 mL of DMF were added, DIPEA (446 uL, 2.7 mmol) was added under an ice-water bath, and the reaction was carried out at room temperature for 2 h. After the reaction was completed by HPLC monitoring, the reaction solution was purified by preparative-HPLC to obtain the preparation solutions of compound **13e** and compound **13f.** The preparative solutions were lyophilized to obtain 138 mg of compound **13e,** LC-MS: [M+H]⁺ =1249.5, and 140 mg of compound **13f,** LC-MS: [M+H]⁺ =1249.5, respectively.

### Step 5: Compound P13

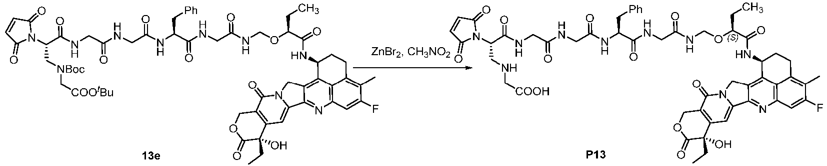

In a 25 mL single-necked vial, **13e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid of 59 mg; LC-MS: [M+H]+=1093.8.

### Example 16: Synthesis of compound P14

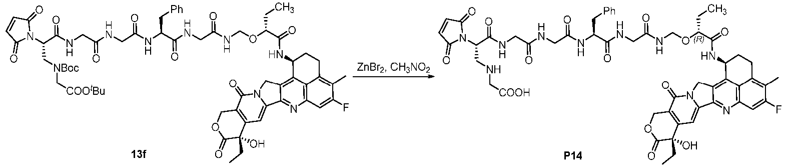

In a 25 mL single-necked vial, **13f** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain a solid of 60 mg; LC-MS: [M+H]+=1093.7.

### Example 17: Synthesis of Compound P15

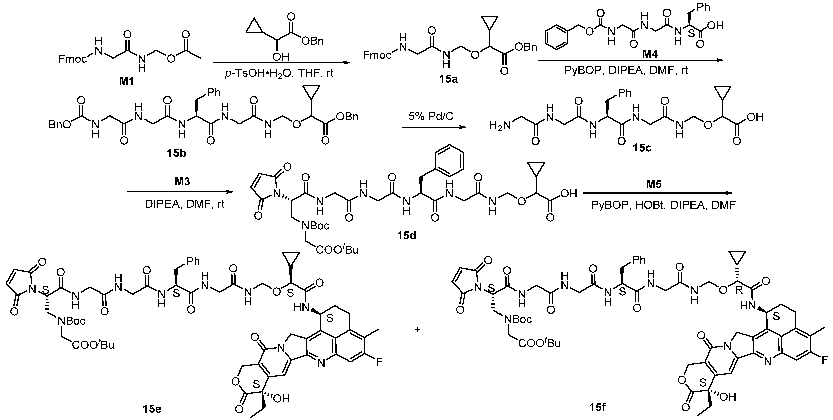

### Step 1: Compound 15a

In a 250 mL single-necked vial was added **M1** (10 g, 27.1 mmol), benzyl 2-cyclopropyl-2-hydroxyacetate (prepared according to the method published in WO2020244657A1) (11.2 g, 54.3 mmol), zinc acetate (9.96 g, 54.3 mmol) and 100 mL of toluene, and heated to 100 °C for 4 h. After the reaction was completed, the temperature was cooled to room temperature, the insoluble materials was filtered out, and the filtrate was concentrated to obtain a crude product. The crude product was purified by silica gel column chromatography (PE:EA=10:1-5:1-2:1) to obtain 4.97 g of the target product with a yield of 36%; LC-MS: [M+H]+=515.2.

### Step 2: Compound 15b

In a 50 mL single-necked flask, add **15a** (4 g, 7.8 mmol) and 10 mL of DMF. After dissolving, DBU (1.42 g, 9.3 mmol) was added under ice-water bath and reacted for 1 h. This was recorded as reaction solution ①.

In another 50 mL single-necked flask was added **M4** (3.2 g, 7.8 mmol), PyBOP (4.5 g, 8.6 mmol), HOBt (1.16 g, 8.6 mmol) and 10 mL of DMF, dissolve, DIPEA (1.65 mL, 10 mmol) was added under an ice-water bath, continued the reaction for 30 min, the reaction solution ① was added, and warmed to room temperature for 2 h. The reaction process was monitored by HPLC, and the preparation was purified by preparative-HPLC to obtain a preparative solution, which was extracted with dichloromethane, washed with saturated sodium chloride solution, dried with anhydrous sodium sulfate, filtered, and concentrated to obtain 4.2 g of solid, with a yield of 78%; LC-MS: [M+H]⁺ =688.3.

### Step 3: Compound 15d

In a 25 mL single-mouth vial, **15b** (1000 mg, 1.45 mmol) was added, 15 mL of DMF was added to dissolve it. Then, 1000 mg of 5% Pd/C was added. The hydrogenation reaction was carried out for 2 h. After the reaction was completed, the filtrate was filtered and placed in an ice-water bath. DIPEA (248 uL, 1.5 mmol) was added, and then **M3** (720 mg, 1.45 mmol) was added. After the addition, the temperature was raised to room temperature and the reaction was reacted for 1 h. The reaction was monitored by HPLC. The reaction solution was purified by preparative-HPLC to obtain a preparation solution. The preparation solution was lyophilized to obtain 503 mg of the product with a yield of 41%; LC-MS: [M-H]-=842.3.

### Step 4: Compounds 15e and 15f

In a 50 mL single-necked vial, **15d** (500 mg, 0.59 mmol), **M5** (317 mg, 0.59 mmol), PyBOP (339 mg, 0.65 mmol), HOBt (88 mg, 0.86 mmol) and 10 mL of DMF were added, DIPEA (292 uL, 1.77 mmol) was added under an ice-water bath, and the reaction was warmed to room temperature for 2 h. After the reaction was completed by HPLC monitoring, the reaction solution was purified by preparative-HPLC to obtain the preparations of compound **15e** and compound **15f.** The preparation solutions were lyophilized to obtain 112 mg of compound **15e,** LC-MS: [M+H]⁺ =1261.5, and 131 mg of compound **15f,** LC-MS: [M+H]⁺ =1261.5, respectively.

### Step 5: Compound P15

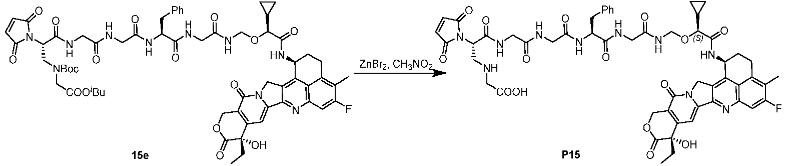

In a 25 mL single-necked bottle, **15e** (100 mg, 0.079 mmol), zinc bromide (357 mg, 1.59 mmol) and 5 mL of nitromethane were added and the reaction was carried out at 40 °C for 1 h. After the reaction was completed by HPLC monitoring, the solvent was concentrated under reduced pressure and removed to obtain a crude product. The crude product was purified by preparative-HPLC to obtain a product preparation solution, which was lyophilized to obtain 55 mg of solid; LC-MS: [M+H]⁺ =1105.5.

### Example 18: Synthesis of compound P16

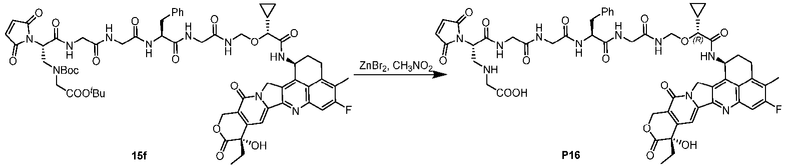

In a 25 mL single-necked vial, **15f** (100 mg, 0.079 mmol), zinc bromide (357 mg, 1.59 mmol) and 5 mL of nitromethane were added, and the reaction was carried out at 40 °C for 1 h. After the reaction was completed by HPLC monitoring, the solvent was concentrated under reduced pressure to obtain a crude product. The crude product was purified by preparative-HPLC to obtain a product preparation solution, which was lyophilized to obtain 58 mg of solid; LC-MS: [M+H]⁺ =1105.6.

### Example 19: Synthesis of compound P17

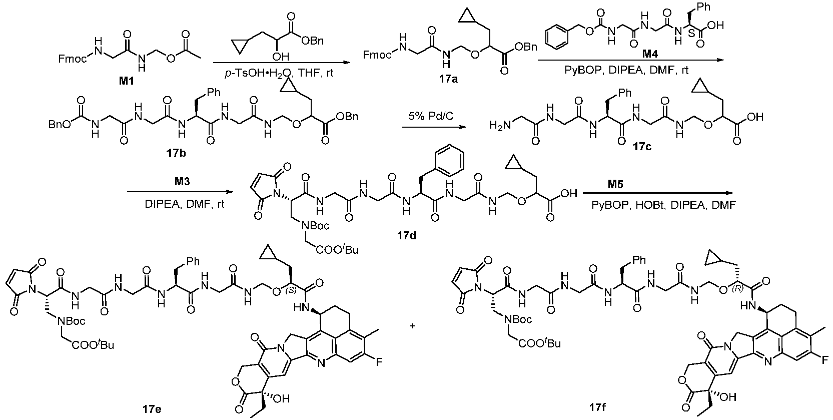

### Step 1: Compound 17a

In a 250 mL single-necked vial, **M1** (10 g, 27.1 mmol), benzyl 2-hydroxy-cyclopropylpropionate (synthesized according to the method published in WO2020063676A) (12.0 g, 54.3 mmol), zinc acetate (9.96 g, 54.3 mmol), and 100 mL of toluene were added and reacted at 100 °C for 4 h. After the reaction was completed, the temperature was cooled to room temperature, the insoluble material was filtered out, and the filtrate was concentrated to obtain a crude product. The crude product was purified by silica gel column chromatography (PE:EA=10:1-5:1-2:1) to obtain 5.09 g of the target material; LC-MS: [M+H]⁺ =529.2.

### Step 2: Compound 17b

In a 50 mL single-necked flask, **17a** (4 g, 7.6 mmol) and 10 mL of DMF were added. After dissolving, DBU (1.39 g, 9.1 mmol) was added in an ice-water bath. The reaction was continued for 1 h, which was recorded as reaction solution ①;
In another 50 mL single-necked vial, **M4** (3.12 g, 7.6 mmol), PyBOP (4.5 g, 8.6 mmol), HOBt (1.16 g, 8.6 mmol) and 10 mL of DMF were added. After dissolving, DIPEA (1.65 mL, 10 mmol) was added under an ice-water bath. The reaction was continued for 30 min, and then the reaction solution ① was added and the temperature was raised to room temperature for 2 h. The reaction progress was monitored by HPLC. After the reaction was completed, the reaction solution was purified by preparative-HPLC to obtain a preparative solution, which was extracted with dichloromethane, washed with saturated sodium chloride solution, dried with anhydrous sodium sulfate, filtered, and concentrated to obtain 4.5 g of solid, with a yield of 84%; LC-MS: [M+H]⁺ =702.3.

### Step 3: Compound 17d

In a 25 mL single-necked vial, **17b** (1000 mg, 1.42 mmol) was added, and 15 mL of DMF was added to dissolve it. Then,1000 mg of 5% Pd/C was added. The hydrogenation reaction was carried out for 2 h. After the reaction was completed, the filtrate was filtered and placed in an ice-water bath. DIPEA (248 uL, 1.5 mmol) was added, and then **M3** (708 mg, 1.42 mmol) was added, and the reaction was brought to room temperature for 1 h. The reaction was monitored by HPLC, and the reaction solution was purified by preparative-HPLC to obtain a preparative solution, which was lyophilized to obtain 443 mg of the product with a yield of 36%; LC-MS: [M- H]⁻=856.4.

### Step 4: Compounds 17e and 17f

In a 50 mL single-mouth bottle, **17d** (400 mg, 0.47 mmol), **M5** methanesulfonate (250 mg, 0.47 mmol), PyBOP (223 mg, 0.56 mmol), HOBt (83 mg, 0.56 mmol) and 10 mL of DMF were added, and DIPEA (248 uL, 1.5 mmol) was added under an ice-water bath, and the reaction was brought to room temperature for 2 h. After the reaction was completed by HPLC monitoring, the reaction solution was purified by preparative-HPLC to obtain the preparation solutions of compound **17e** and compound **17f,** which were lyophilized to obtain 103 mg of compound **17e,** LC-MS: [M+H]⁺ =1275.5, and 103 mg of compound 17f, LC-MS: [M+H]⁺ =1275.5, respectively.

### Step 5: Compound P17

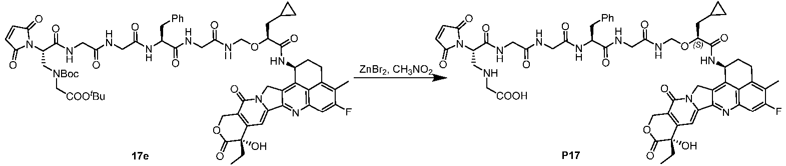

In a 25 mL single-necked vial, **17e** (100 mg, 0.078 mmol), zinc bromide (352 mg, 1.57 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain 51 mg of solid; LC-MS: [M+H]⁺ =1119.7.

### Example 20: Synthesis of compound P18

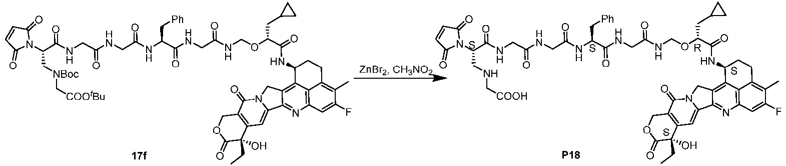

In a 25 mL single-necked vial, **17f** (100 mg, 0.079 mmol), zinc bromide (357 mg, 1.59 mmol) and 5 mL of nitromethane were added and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain 47 mg of a solid; LC-MS: [M+H]⁺ =1119.6.

### Example 21: Synthesis of Compound P19

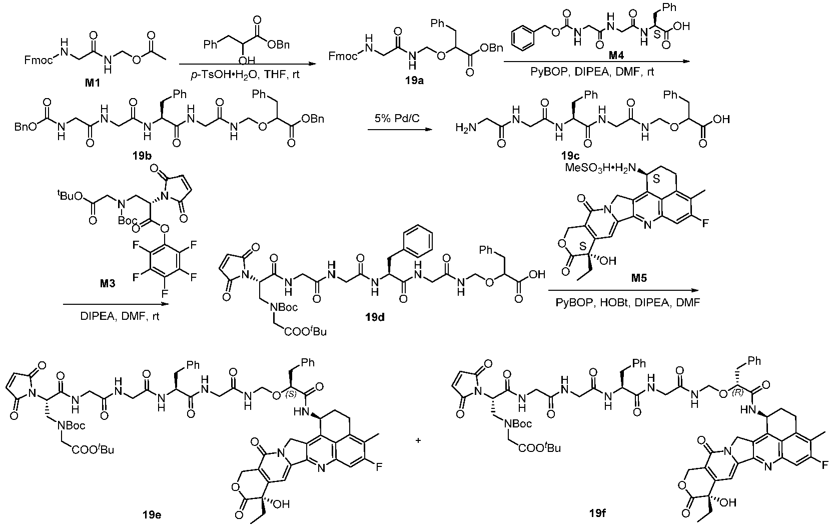

### Step 1: Compound 19a

In a 250 mL single-necked bottle, **M1** (10 g, 27.1 mmol), benzyl 2-hydroxy-3-phenylpropanoate (synthesized according to the method published in Nature Communications, 2020. 11(1), 56.) (14.7 g, 54.3 mmol), zinc acetate (9.96 g, 54.3 mmol) and 100 mL of toluene were added. The reaction was heated to 100 °C for 4 h. After the reaction was completed, it was reduced to room temperature, filtered to remove insoluble material, and the filtrate was concentrated to obtain a crude product. The crude product was purified by silica gel column chromatography (PE:EA=10:1-5:1-2:1) to obtain 6.13 g of the target material in 40% yield; LC-MS: [M+H]⁺ =565.6.

### Step 2: Compound 19b

In a 50 mL single-necked flask, **19a** (5 g, 8.86 mmol) and 15 mL of DMF were added. After dissolving and clearing, DBU (1.53 g, 10 mmol) was added under an ice-water bath and the reaction was continued for 1 h, which was recorded as reaction solution ①;
In another 50 mL single-mouth flask, **M4** (3.6 g, 8.86 mmol), PyBOP (5.23 g, 10 mmol), HOBt (1.36 g, 10 mmol) and 10 mL of DMF were added. After dissolving, DIPEA (1.65 mL, 10 mmol) was added under an ice-water bath. The reaction was continued for 30 min, then reaction solution ① was added, and the reaction was brought to room temperature for 2 h. The reaction was monitored by HPLC. After the reaction was completed, the reaction solution was purified by preparative-HPLC to obtain a preparation solution. The prepared solution was extracted with dichloromethane, washed with saturated sodium chloride solution, dried over anhydrous sodium sulfate, filtered and concentrated to obtain 5.0 g of solid with a yield of 77%; LC-MS: [M+H]+=738.3.

### Step 3: Compound 19d

In a 25 mL single-mouth bottle, **19b** (3.0 g, 4.07 mmol)and 15 mL of DMF was added, then 3.0 g of 5% Pd/C was added. The hydrogenation reaction was carried out for 2 h. After the reaction was completed, the filtrate was filtered and placed in an ice-water bath. DIPEA (744 uL, 4.5 mmol) was added, and then M3 (2.34 g, 4.07 mmol) was added. After the addition, the temperature was raised to room temperature and the reaction was carried out for 1 h. The reaction was monitored by HPLC. The reaction solution was purified by preparative-HPLC to obtain a preparation solution. The preparation solution was lyophilized to obtain 1.2 g of the product, with a yield of 33%; LC-MS: [M-H]-=892.4.

### Step 4: Compounds 19e and 19f

In a 50 mL single-necked vial, **19d** (500 mg, 0.56 mmol), **M5** (300 mg, 0.56 mmol), PyBOP (438 mg, 0.84 mmol), HOBt (113 mg, 0.84 mmol) and 15 mL of DMF were added, DIPEA (330 uL, 2.0 mmol) was added under an ice-water bath, and the reaction was warmed to room temperature for 2 h. After the reaction was completed by HPLC monitoring, the reaction solution was purified by preparative-HPLC to obtain the preparation solutions of compound **19e** and compound **19f.** The preparative solutions were lyophilized to obtain 156 mg of compound **19e,** LC-MS: [M+H]⁺ =1311.6, and 150 mg of compound **19f,** LC-MS: [M+H]⁺ =1311.7, respectively.

### Step 5: Compound P19

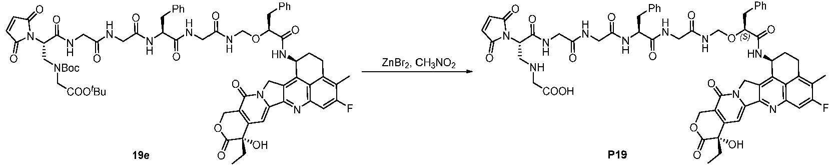

In a 25 mL single-necked vial, **19e** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain 43 mg of a solid; LC-MS: [M+H]+=1155.7.

### Example 22: Synthesis of compound P20

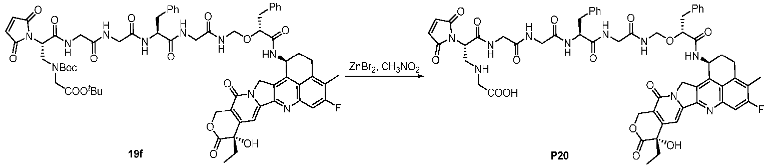

In a 25 mL single-necked bottle, **19f** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain 40 mg of a solid; LC-MS: [M+H]+=1155.8.

### Example 23: Synthesis of Compound 14

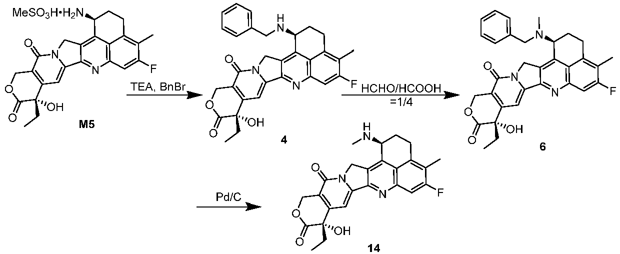

### Step 1: Synthesis of compound 4

To Exatecan mesylate **M5** (15 g, 28 mol, prepared by the method disclosed in EP0737683A1), 400 mL of DMF was added, the reaction was cooled to 0°C under an ice-water bath, triethylamine was added dropwise, the pH was adjusted to 7-8, and benzyl bromide (9.6 g, 56 mmol) was added dropwise under an ice-water bath, the reaction was warmed to room temperature (25°C) for 1 hour. The reaction was complete after TLC monitoring, and the reaction solution was concentrated under reduced pressure. The crude product was purified by preparative-HPLC (acetonitrile/pure water system), the target peak was collected, the acetonitrile was removed under reduced pressure, and then lyophilized to obtain about 11 g of compound **4** as a yellow solid with a yield of about 74%, MS m/z: [M+H]+ 526.3.

### Step 2: Synthesis of compound 6

At room temperature, compound **4** (11 g, 21 mol) was sequentially added into a 250 mL vial, 120 mL of formic acid was dissolved, 30 mL of formaldehyde (40% aqueous solution) was added into the resulting bright yellow solution, and the reaction was heated up to 50 °C for 1 hour. The reaction was complete after TLC monitoring. The reaction was cooled to room temperature, and the reaction solution was purified by preparative HPLC (acetonitrile/pure water system). The target peak was collected, and the acetonitrile was removed under reduced pressure and lyophilized to obtain about 4.5 g of compound **6** as a yellow powdery solid with a yield of about 40%. MS m/z: [M+H]+ 540.6.

### Step 3: Synthesis of compound 14:

At room temperature, compound **6** (2.3 g, 4.3 mol) was added to a 250 mL single-necked bottle, and 100 mL of DMF was added to dissolve it. 2.3 g of 5% Pd/C was added to the obtained, and the atmosphere in the system was replaced by a hydrogen balloon. After maintaining the reaction at room temperature for 1.5 hours, the reaction was completed after HPLC monitoring, and Pd/C was removed by filtration. The obtained reaction solution was concentrated and purified by preparative-HPLC (acetonitrile/pure water system). The target peak was collected, and after removing acetonitrile under reduced pressure, it was lyophilized to obtain about 1.0 g of compound **14** as a yellow powdery solid with a yield of about 52%. MS m/z: [M+H]⁺ 450.5.

### Example 24: Synthesis of Compound P21

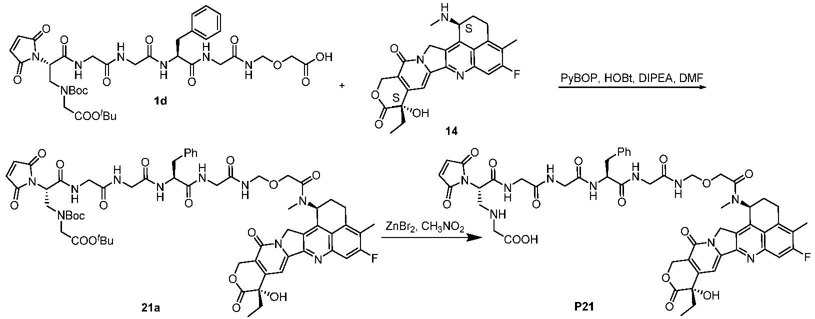

### Step 1: Compound 21a

In a 50 mL single-necked vial, **1d** (500 mg, 0.62 mmol), **14** (279 mg, 0.62 mmol), PyBOP (448 mg, 0.86 mmol), HOBt (116 mg, 0.86 mmol) and 15 mL of DMF were added, DIPEA (378 uL, 2.29 mmol) was added under an ice-water bath, and the reaction was warmed to room temperature for 2 h. After the reaction was completed, the reaction solution was purified by preparative-HPLC to obtain a preparative solution, which was lyophilized to obtain **21a** (166 mg); LC-MS: [M+H]+=1235.6.

### Step 2: Compound P21

In a 25 mL single-necked vial, **21a** (100 mg, 0.081 mmol), zinc bromide (368 mg, 1.63 mmol) and 10 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid compound **P21** (43 mg); LC-MS: [M+H]+=1079.5.

### Example 25: Synthesis of compound P22

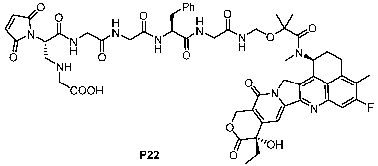

73 mg of compound **P22** was prepared from compound **2d** and compound **14** according to the route of Example 24,.; LC-MS: [M+H]⁺ =1107.6.

### Example 26: Synthesis of compound P23

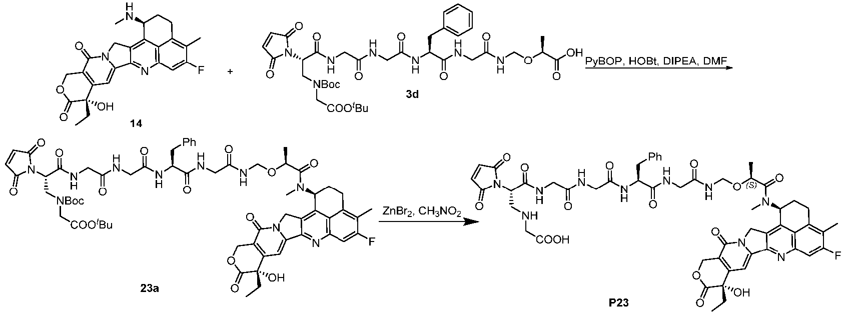

### Step 1: Compound 23a

In a 100 mL single-necked vial, **3d** (1.66 g, 2.02 mmol, 1.0 eq), **14** (0.91 g, 2.02 mmol, 1.0 eq), PyBOP (1.58 g, 3.03 mmol, 1.5 eq), HOBt (0.41 g, 3.03 mmol, 1.5 eq) and DMF (40 mL) were added, and DIPEA (0.84 mL, 1.5 eq) was added under an ice-water bath, and the mixture was warmed to room temperature for 2 h (monitored by HPLC). The reaction solution was directly purified by preparative-HPLC, and the preparative liquid was concentrated by water pump under reduced pressure at 35 °C in water bath to remove acetonitrile, and lyophilized to obtain compound **23a** (1.21 g); LC-MS: [M+H]+=1249.4.

### Step 2: Compound P23

In a 100 mL single-necked vial, compound **23a** (1.0 g, 0.8 mmol, 1.0 eq) and 35 mL of nitromethane were added, and zinc bromide (3.64 g, 16 mmol, 20.0 eq) was added after dissolution. The mixture was reacted in an oil bath at 40 °C (preheated for stability) for 30 min and concentrated in a water bath at 45 °C with a water pump to remove nitromethane to obtain a yellow solid residue (monitored by HPLC). The solution was prepared by an acid method to obtain a preparation solution, which was concentrated in a water bath at 35 °C with a water pump to remove acetonitrile, and lyophilized to obtain compound **P23** (786 mg), LC-MS: [M+H]+=1093.6.

### Example 27: Synthesis of Compound P24

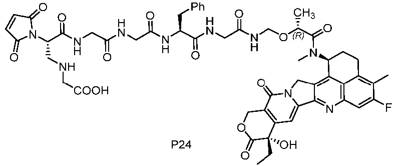

677 mg of compound **P24** was prepared from compound **14** according to the route of Example 26,; LC-MS: [M+H]⁺ =1093.7.

### Example 28: Synthesis of compound P25

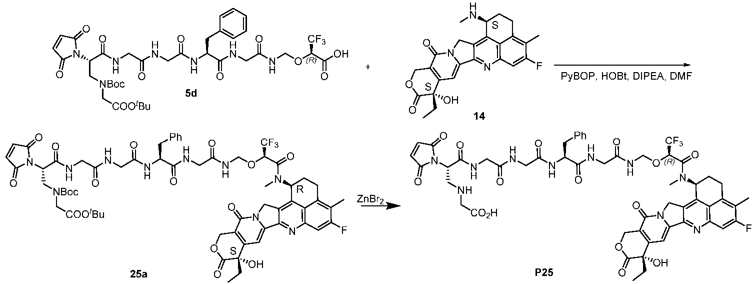

### Step 1: Compound 25a

In a 50 mL single-necked bottle, **5d** (500 mg, 0.57 mmol), **14** (256.8 mg, 0.57 mmol), PyBOP (448 mg, 0.86 mmol), HOBt (116 mg, 0.86 mmol) and 15 mL of DMF were added, and DIPEA (378 uL, 2.29 mmol) was added under an ice-water bath. The reaction was carried out at room temperature for 2 h. After the reaction was completed by HPLC monitoring, the reaction solution was purified by preparative-HPLC to obtain a preparation solution of compound **25a,** which was lyophilized to obtain 313 mg of compound **25a,** LC-MS: [M+H]+=1303.6.

### Step 2: Compound P25

In a 25 mL single-necked vial, **25a** (100 mg, 0.077 mmol), zinc bromide (349 mg, 1.55 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a preparative solution, which was lyophilized to obtain 49 mg of solid compound **P25;** LC-MS: [M+H]+=1147.6.

### Example 29: Synthesis of Compound P26

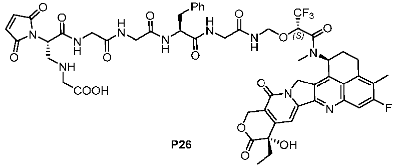

89 mg of compound **P26** was prepared from compound **14**according to the route of Example 28,; LC-MS: [M+H]⁺ =1147.7.

### Example 30: Synthesis of compounds P27 and P28

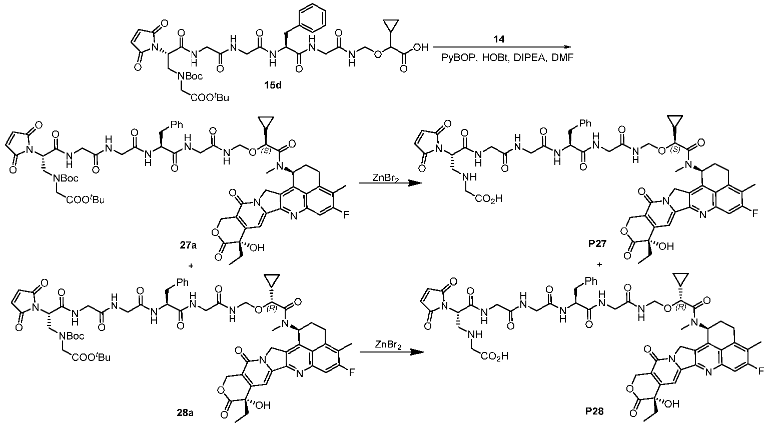

### Step 1: Compounds 27a and 28a

In a 50 mL single-necked vial, **15d** (500 mg, 0.59 mmol), **14** (266 mg, 0.59 mmol), PyBOP (339 mg, 0.65 mmol), HOBt (88 mg, 0.86 mmol) and 10 mL of DMF were added, DIPEA (292 uL, 1.77 mmol) was added under an ice-water bath, and the reaction was carried out at room temperature for 2 h. After the reaction was completed by HPLC monitoring, the reaction solution was purified by preparative-HPLC to obtain the preparation solutions of compounds **27a** and **28a,** which were lyophilized to obtain 109 mg of compound **27a,** LC-MS: [M+H]+=1275.5; 111 mg of compound **28,** LC-MS: [M+H]+=1275.7.

### Step 2: Compound P27

In a 25 mL single-necked vial, **27a** (100 mg, 0.078 mmol), zinc bromide (352 mg, 1.56 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid of 53 mg; LC-MS: [M+H]+=1119.5.

### Step 3: Compound P28

In a 25 mL single-necked bottle, **28a** (100 mg, 0.078 mmol), zinc bromide (352 mg, 1.56 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain 54 mg of solid; LC-MS: [M+H]+=1119.4.

### Example 31: Synthesis of compounds P29 and P30

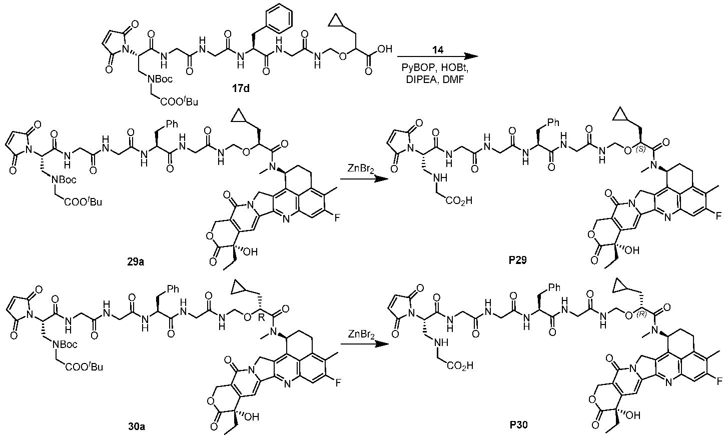

### Step 1: Compounds 29a and 30a

In a 50 mL single-necked vial, **17d** (400 mg, 0.47 mmol), **14** (211.7 mg, 0.47 mmol), PyBOP (223 mg, 0.56 mmol), HOBt (83 mg, 0.56 mmol) and 10 mL of DMF were added, and DIPEA (248 uL, 1.5 mmol) was added to the vial under an ice-water bath. The reaction was brought to room temperature for 2 h. After HPLC monitoring, the reaction solution was purified by preparative-HPLC to obtain the preparative solutions of compounds **29a** and **30a,** which were lyophilized to obtain 106 mg of compound **29a,** LC-MS: [M+H]⁺ =1289.5, and 101 mg of compound **30a,** LC-MS: [M+H]⁺ =1289.4, respectively.

### Step 2: Compound P29

In a 25 mL single-necked vial, **29a** (100 mg, 0.078 mmol), zinc bromide (352 mg, 1.57 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain 39 mg of a solid; LC-MS: [M+H]+=1133.4.

### Step 3: Compound P30

In a 25 mL single-necked vial, **30a** (100 mg, 0.078 mmol), zinc bromide (352 mg, 1.57 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain 35 mg of a solid; LC-MS: [M+H]+=1133.6.

### Example 32: Synthesis of Compound P31

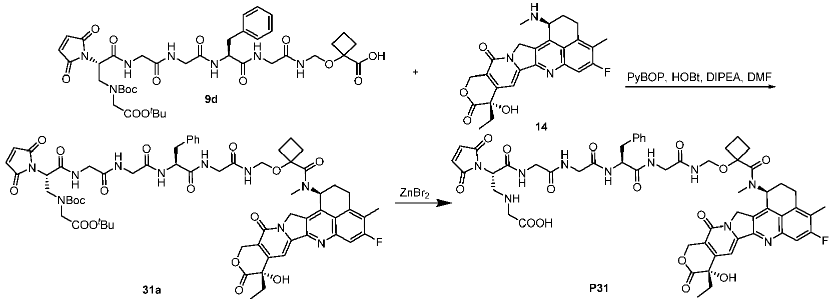

### Step 1: Compound 31a

In a 50 mL single-necked bottle, **9d** (800 mg, 0.96 mmol), **14** (432.5 mg, 0.96 mmol), PyBOP (500 mg, 0.96 mmol), HOBt (208 mg, 0.96 mmol) and 30 mL of DMF were added, and DIPEA (660 uL, 4.0 mmol) was added to the vial under an ice-water bath. The reaction was brought to room temperature for 4 h. After the reaction was completed by HPLC monitoring, the reaction solution was purified bypreparative-HPLC to obtain a preparation solution of compound **31a,** which was lyophilized to obtain **31a** (402 mg); LC-MS: [M+H]+=1275.4.

### Step 2: Compound P31

In a 25 mL single-necked bottle, **31a** (100 mg, 0.78 mmol), zinc bromide (356 mg, 1.57 mmol) and 10 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid compound **P31** (47 mg); LC-MS: [M+H]+=1119.6.

### Example 33: Synthesis of Compound P32

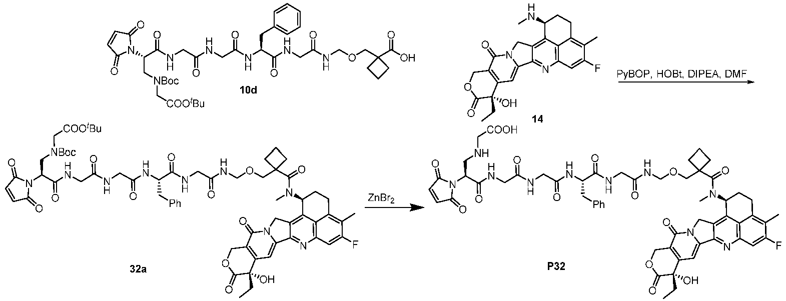

### Step 1: Compound 32a

In a 50 mL single-mouth bottle, **10d** (400 mg, 0.47 mmol), **14** (211.7 mg, 0.47 mmol), PyBOP (250 mg, 0.47 mmol), HOBt (101 mg, 0.47 mmol) and 15 mL of DMF were added, and DIPEA (330 uL, 2.0 mmol) was added to the bottle in an ice-water bath. The reaction was brought to room temperature for 3 h. After the reaction was completed by HPLC monitoring, the reaction solution was purified by preparative-HPLC to obtain a preparation solution of compound **32a,** which was lyophilized to obtain **32a** (177 mg); LC-MS: [M+H]+=1289.4.

### Step 2: Compound P32

In a 25 mL single-necked vial, **32a** (100 mg, 0.08 mmol), zinc bromide (360 mg, 1.6 mmol) and 5 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain a solid compound **P32** (45 mg); LC-MS: [M+H]+=1133.5.

### Example 34: Synthesis of compound M7

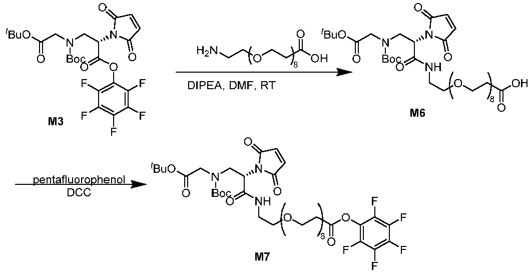

### Step 1: Compound M6:

In a 100 mL single-necked vial, compound **M3** (11.0 g, 19.5 mmol, 1.0 eq), DIPEA (2.8 g, 21.4 mmol, 1.1 eq), 27-amino-4, 7, 10, 13, 16, 19, 22, 25-octaoxaheptadecanoic acid (9.7 g, 20.5 mmol, 1.05 eq) and DMF (60 mL) were added, and reacted for 20 min at room temperature (monitored by TLC). The reaction solution was directly purified by preparative-HPLC, and the preparative liquid was concentrated by a water pump under reduced pressure in a water bath at 35 °C to remove acetonitrile and lyophilized to obtain compound **M6** (13.2 g) with a yield of 78%; LC-MS: [M-H]-=820.5.

### Step 2: Compound M7

In a 100 mL single-necked vial, compound **M6** (13.0 g, 15 mmol, 1.0 eq), pentafluorophenol (3 g, 16.5 mmol, 1.1 eq), DCC (3.4 g, 16.5 mmol, 1.1 eq) and THF (30 mL) were added, and the reaction was carried out at room temperature for 30 min (monitored by TLC), and filtered to remove insoluble material. The reaction solution was directly purified by preparative-HPLC, concentrated in a water bath at 35 °C to remove acetonitrile, and lyophilized to obtain compound **M7** (14.2 g) with a yield of 92%; LC-MS: [M+H]+=986.9.

### Example 35: Synthesis of Compound P33

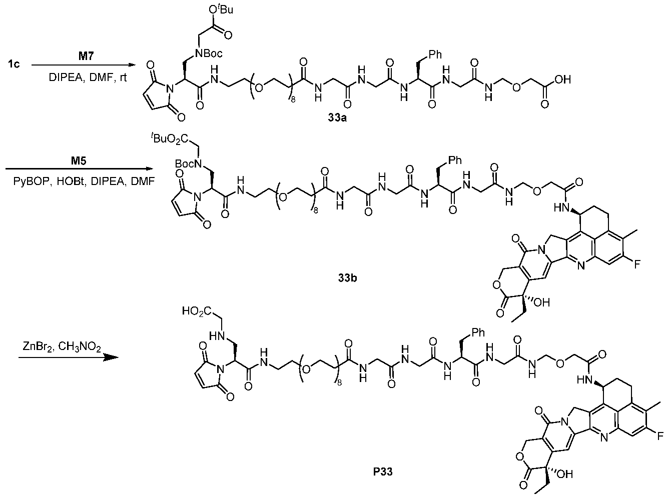

### Step 1: Synthesis of compound 33a

To **M7** (1 g, 0.79 mol), 10 mL of DMF was added, cooled to 0 °C in an ice-water bath, compound **1c** (334 mg, 0.79 mol), DIPEA (154 mg, 1.19 mol) were added, and the reaction was maintained under these conditions for 1 h. The reaction was completed by TLC monitoring. The reaction solution was purified by preparative HPLC (acetonitrile/pure water system), the target peak was collected, the acetonitrile was removed under reduced pressure and then lyophilized to obtain about 1.2 g of compound **33a,** MS m/z: [M-H]- =1225.8.

### Step 2: Synthesis of compound 33b

In a 25 mL single-necked bottle, **33a** (1.2 g, 0.94 mmol), **M5** (500 mg, 0.94 mmol), PyBOP (625 mg, 1.2 mmol), HOBt (162 mg, 1.2 mmol) and 15 mL of DMF were added, and DIPEA (310 mg, 2.4 mmol) was added to the vial under an ice-water bath, and the reaction was carried out for 2 h. After the reaction was completed, the reaction solution was purified by preparative-HPLC to obtain a preparative solution, which was lyophilized to obtain **33b** (709 mg); LC-MS: [M+H]+=1644.9.

### Step 3: Synthesis of compound P33:

In a 25 mL single-necked bottle, **33b** (200 mg, 0.116 mmol), zinc bromide (523 mg, 2.32 mmol) and 10 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain solid compound **P33** (88 mg); LC-MS: [M+H]+=1488.9.

### Example 36: Synthesis of Compound P34

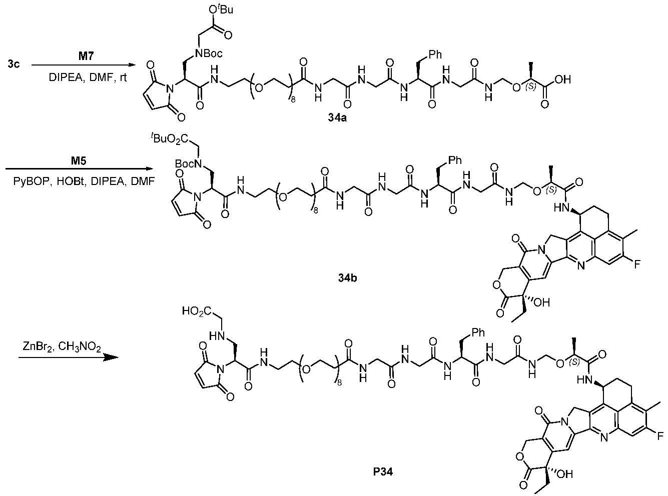

### Step 1: Synthesis of compound 34a

10 mL of DMF was added to **M7** (1 g, 0.79 mol), cooled to 0°C under an ice-water bath, and compound **3c** (345 mg, 0.79 mol) and DIPEA (154 mg, 1.19 mol) were added. The reaction was maintained under this condition for 1 h. The reaction was completed after monitoring by TLC. The reaction solution was purified by preparative HPLC (acetonitrile/pure water system), the target peak was collected, the acetonitrile was removed under reduced pressure and then lyophilized to obtain 0.9 g of compound **34a;** MS m/z: [M-H]-=1239.8.

### Step 2: Synthesis of compound 34b

In a 25 mL single-necked bottle, **34a** (700 mg, 0.54 mmol), **M5** (289 mg, 0.54 mmol), PyBOP (313 mg, 0.6 mmol), HOBt (81 mg, 0.6 mmol) and 10 mL of DMF were added, and DIPEA (155 mg, 1.2 mmol) was added under an ice-water bath, and the reaction was carried out for 2 h. After the reaction was completed, the reaction solution was purified by preparative-HPLC to obtain a preparative solution, which was lyophilized to obtain **34b** (304 mg); LC-MS: [M+H]+=1658.9.

### Step 3: Synthesis of compound P34:

In a 25 mL single-necked bottle, **34b** (200 mg, 0.116 mmol), zinc bromide (523 mg, 2.32 mmol) and 10 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain solid compound **P34** (96 mg); LC-MS: [M+H]+=1502.8.

### Example 37: Synthesis of Compound P35

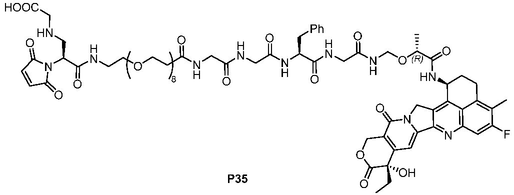

Referring to the synthetic route of Example 36, 80 mg of compound **P35** was prepared; LC-MS: [M+H]⁺ =1502.7.

### Example 38: Synthesis of Compound 16

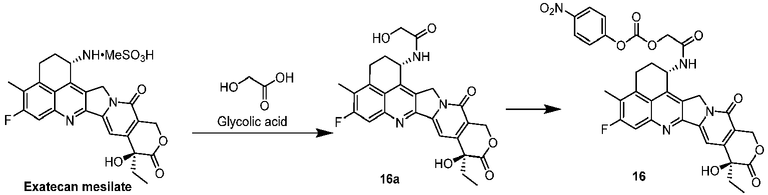

### Step 1: Preparation of compound 16a

Compound **16a** was prepared by the method disclosed in Example 76 on page 147 of the specification of EP2907824A1.

### Step 2: Preparation of compound 16

In a 100 mL single-necked vial, compound **16a** (3 g, 6.08 mmol) was added, 50 mL DCM was dissolved, cooled to 0 °C in an ice-water bath, and then DIPEA (2.03 mL, 12.16 mmol) and NPC (3.78 g, 12.16 mmol) were added sequentially at the maintained temperature, and the reaction was slowly warmed up to room temperature for 5 h, and the endpoint of the reaction was monitored by TLC. After the reaction was completed, the solvent was removed by water pump at 45 °C to obtain a residue. The residue was purified by silica gel column chromatography (DCM/MeOH=100/1-50:1-10:1) to obtain compound **16** (3.4 g), LC-MS: [M+H]+=659.4.

### Example 38: Synthesis of Compound 18

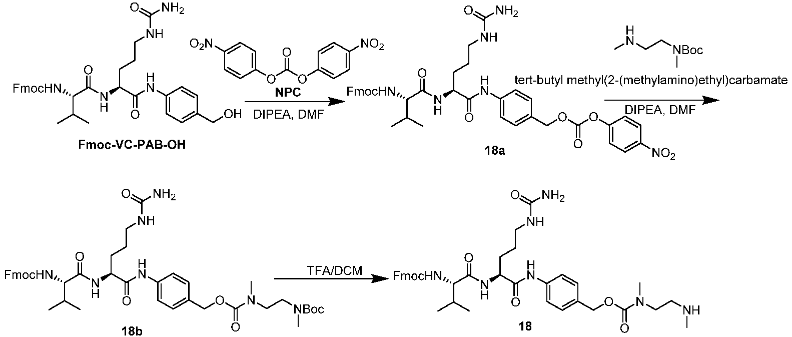

### Step 1: Synthesis of compound 18a

In a 5000 mL single-necked vial, **Fmoc-VC-PAB-OH** (30 g, 49.92 mmol), DMF (2 L) were added and stirred to dissolve, then **NPC** (22.76 g, 74.87 mmol) and DIPEA (9.68 g, 74.87 mmol) were added, stirred at room temperature for 1.5 h. The raw material was detected by TLC. The reaction was stopped when the reaction was complete. Under vigorous stirring, add 1.5 L of isopropyl ether to the reaction flask, stir for 2 h, pour off the upper liquid layer, add 800 mL of isopropyl ether to the residue, and continue to stir vigorously for l h, filter, place the filter cake in 600 mL of isopropyl ether and stirred overnight, filtered, and obtain 31.2 g of yellowpowder solid, with a yield of 82%, LC-MS: [M+H]+=767.6.

### Step 2: Synthesis of compound 18b

In a 5000 mL single-necked vial was added compound **18a** (31.2 g, 40.73 mmol), DMF (800 mL), and then stirred to dissolve, added 50 mL of DMF dissolved tert-butyl methyl (2-(methylamino) ethyl) carbamate (8.1 g, 36.66 mmol), and the reaction was stirred at room temperature for 3.5 h. The reaction was stopped when TLC detected that the reaction was complete. 2 L of isopropyl ether was added to the reaction solution and stirred. A viscous oil precipitated on the wall of the bottle. The supernatant was poured off, and 1 L of isopropyl ether was added. The mixture was stirred vigorously, and the supernatant was poured off again. 500 mL of isopropyl ether was added and stirred overnight. The mixture was filtered to obtain 27.2 g of a yellowish brown powdery solid with a yield of 81.9%. LC-MS: [M+H]+=816.7.

### Step 3: Synthesis of compound 18

In a 1000 mL vial was added compound **18b** (27.2 g), DCM (200 mL), and stirred to dissolve, 400 mL of TFA was added, stirred the reaction at room temperature for 1.5 h. TFA and DCM were removed by concentrating the mixture in a water bath at 45 °C with a water pump to obtain a yellow liquid residue, which was used directly in the next step without purification.

### Example 39: Synthesis of Compound P36

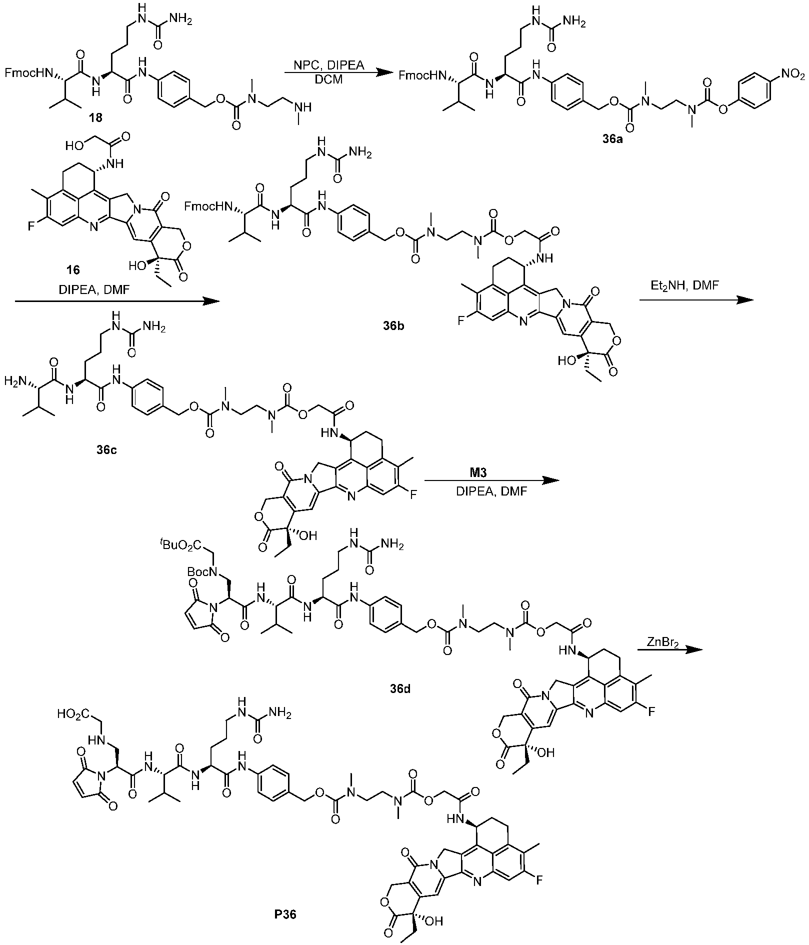

### Step 1: Preparation of compound 36a

In a 50 mL single-necked vial was added compound **18** (2 g, 2.8 mmol) dissolved in 30 mL of DCM, cooled to 0 °C under an ice-water bath, DIPEA (2.32 mL, 14 mmol) and NPC (4.32 g, 14 mmol) were added sequentially at the maintained temperature, and the temperature was slowly increased to room temperature for 5 h, and the reaction endpoint was monitored by TLC. After the reaction was completed, the solvent was removed by water pump at 45 °C to obtain a residue. The residue was purified by silica gel column chromatography (DCM/MeOH=100/1-50:1-10:1) to obtain compound **36a** (1.98 g), LC-MS: [M+H]+= 881.6.

### Step 2: Preparation of compound 36b

In a 50 mL single-necked vial, compound **36a** (1.98 g, 2.24 mmol) and compound **16** (1.11 g, 2.24 mmol) were dissolves in 25 mL of DMF, and cooled to 0 °C under an ice-water bath, then DIPEA (0.745 mL, 4.48 mmol) was added, slowly warmed up to room temperature for 1 h, and monitored the end point of the reaction by HPLC. After the reaction, the solvent was removed by concentrating at 45 °C in a water bath with an oil pump under reduced pressure to obtain a residue. The residue was purified by preparative HPLC, and the product preparation solution was collected and lyophilized to obtain compound **36b** (2.13 g). LC-MS: [M+H]+=1135.7.

### Step 3: Preparation of compound 36c

In a 50 mL single-necked bottle, compound **36b** (2.1 g, 1.85 mmol) was dissolved in 20 mL of DMF, cooled to 0 °C under an ice-water bath, and then added with diethylamine (5 mL), and slowly warmed up to room temperature for 4 h, and the reaction endpoint was monitored by HPLC. After the reaction, the solvent was removed by concentrating with an oil pump at 45 °C in a water bath to obtain the residue, which was directly used in the next step.

### Step 4: Preparation of compound 36d

In a 50 mL single-necked vial, crude compound **36c** (1.85 mmol, according to the **36b** molar number of feed) and compound **M3** (1.05 g, 1.85 mmol) were dissolved in 20 mL DMF, cooled to 0 °C under an ice water bath, DIPEA (615 uL, 3.7 mmol) was added, slowly warmed to room temperature reaction for 2 h, and monitored the end point of the reaction by HPLC. After the reaction was completed, the solvent was removed by concentrating in a water bath at 45 °C with an oil pump under reduced pressure to obtain a residue. The residue was purified by preparative-HPLC, and the product preparation solution was collected and lyophilized to obtain compound **36d** (2.3 g). LC-MS: [M+H]+=1393.9.

### Step 4: Preparation of compound P36

In a 10 mL single-necked bottle, compound **36d** (200 mg, 0.144 mmol) was dissolved in 15 mL of nitromethane, and then added zinc bromide (652 mg, 2.87 mmol), maintain the reaction at room temperature for 30 min. The nitromethane was removed by concentrating in a water bath at 45 °C with a water pump to obtain a yellow solid residue. The product was purified by preparative HPLC and the preparative solution was lyophilized to obtain compound **P36** (172 mg). LC-MS: [M+H]+=1237.8.

### Example 40: Synthesis of Compound P37

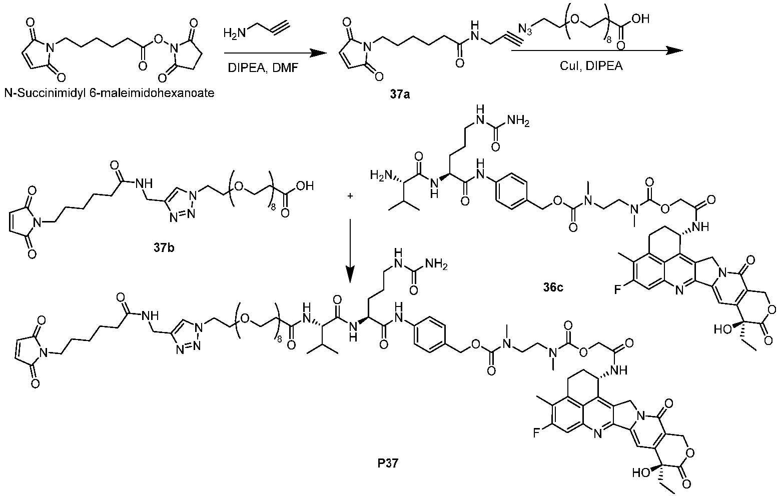

### Step 1: Preparation of compound 37a

In a 100 mL single-necked bottle, compound N-Succinimidyl 6-maleimidohexanoate (2 g, 6.8 mmol)was dissolved in 50 mL DMF, cooled to 0 ° C under an ice-water bath, maintained this temperature, added propargylamine (0.44 mL, 6.8 mmol) and DIPEA (2.27 mL, 13.6 mmol) in sequence, slowly warmed to room temperature and reacted overnight, and monitored the reaction end point by TLC. After the reaction was completed, the solvent was removed by water pump at 45 ° C to obtain a residue. The residue was purified by silica gel column chromatography (PE/EA=50/1-20:1-5:1) to obtain compound **37a** (1.3 g), LC-MS: [M+H]+= 249.3.

### Step 2: Preparation of compound 37b

In a 100 mL single-necked bottle, Compound **37a** (1.3 g, 5.3 mmol) was dissolved in 50 mL ultra-dry oxygen-free THF. The atmosphere in the system was replaced with nitrogen three times. N3-PEG8-CH2CH2COOH (2.6 g, 5.3 mmol), DIPEA (0.914 mL, 5.5 mmol) and CuI (110 mg, 0.55 mmol) were added in sequence at room temperature. The temperature was slowly raised to room temperature for 4 h. The reaction endpoint was monitored by HPLC. The reaction solution was directly purified by preparative HPLC (acid method), and the preparative solution was collected and lyophilized to obtain compound **37b** (2.7 g). LC-MS: [M-H]- = 714.3.

### Step 3: Preparation of compound P37

In a 25 mL single-necked bottle was added compound **37b** (200 mg, 0.285 mmol), compound **36c** (289 mg, 0.285 mmol), PyBOP (301 mg, 0.57 mmol), HOBt (78 mg, 0.57 mmol), and DMF (6 mL), and then lower the temperature to 0 °C in an ice-water bath. DIPEA (95 uL, 0.57 mmol) was added, and the mixture was heated to room temperature for 2 h. The reaction was monitored by HPLC. The reaction solution was directly purified by preparative HPLC, and the product preparation solution was collected and lyophilized to obtain compound **P37** (288 mg). TOF-MS: [M+H]+=1710.8014.

### Example 41: Synthesis of Compound P38

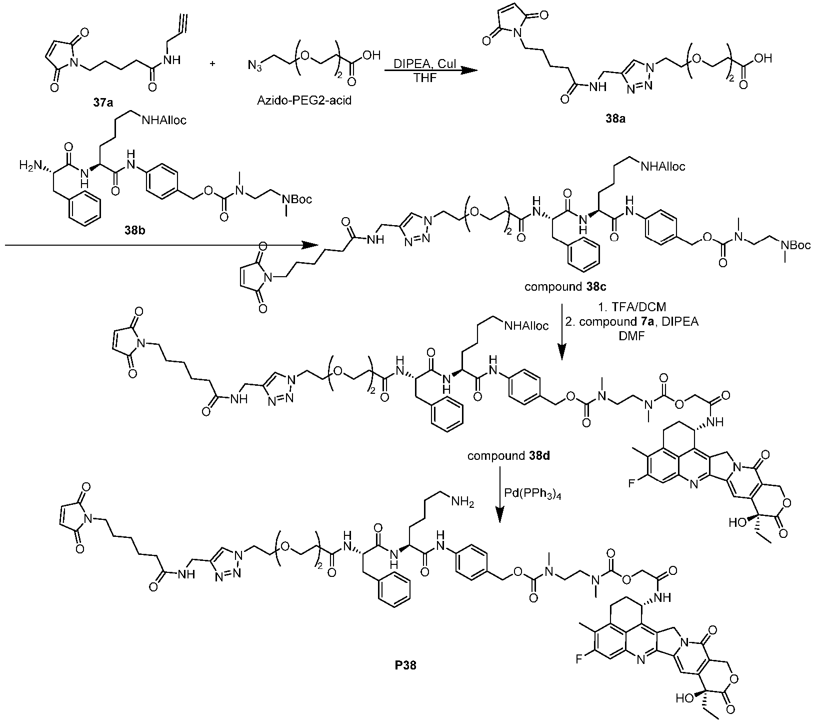

### Step 1: Preparation of compound 38a

In a 100 mL single-necked bottle, compound **37a** (1 g, 4.1 mmol) was added and dissolved in 50 mL ultra-dry oxygen-free THF. The atmosphere in the system was replaced with nitrogen three times. Azide-ethylene glycol-acetic acid (872 mg, 4.1 mmol), DIPEA (0.75 mL, 4.5 mmol) and CuI (82 mg, 0.41 mmol) were added in sequence at room temperature. The temperature was slowly raised to room temperature for 4 h, and the reaction endpoint was monitored by HPLC. The reaction solution was directly purified by preparative HPLC (acid method), and the preparative solution was collected and lyophilized to obtain compound **38a** (1.1 g), LC-MS: [M-H]- = 436.3.

### Step 2: Preparation of compound 38c

In a 100 mL single-necked vial, compound **38b** (0.44 g, 1 mmol, prepared according to the synthetic route of compound **12b** with reference to Chemistry - A European Journal. 2015. 21, 6921-6929), **38a** (0.7 g, 1 mmol), PyBOP (1.06 g, 2 (0.7 g, 1 mmol), PyBOP (1.06 g, 2 mmol), HOBt (273 mg, 2 mmol) and DMF (30 mL) were added, the temperature of an ice-water bath was lowered to 0 °C, DIPEA (225 uL, 2 mmol) was added, and the reaction was raised to room temperature for 2 h. The end of the reaction was monitored by HPLC. The reaction solution was purified directly by preparative HPLC, and the product preparation was collected and lyophilized to obtain compound **38c** (521 mg). LC-MS: [M-Boc+H]⁺ =1016.6.

### Step 3: Synthesis of compound 38d

In a 25 mL single-necked bottle, compound **38c** (500 mg, 0.5 mmol) was added, dissolved by 5 mL of DCM, and TFA (10 mL) was added dropwise under the cooling of ice-water bath, and the reaction was maintained at 0 °C for 2 h after completion of the dropwise addition, and the endpoint was monitored by HPLC. The reaction solution was directly concentrated by a water pump at 45 °C water bath, and the obtained yellow oil was directly used in the next reaction.

In a 25 mL single-necked vial, the above crude compound (0.5 mmol, according to the amount of compound **38c** feeding), compound **7a** (324 mg, 0.5 mmol) and DMF (6 mL) were added, the ice-water bath was lowered to 0 °C, and DIPEA (225 uL, 2 mmol) was added, and after addition, the reaction was elevated to room temperature for 2 h. The end of the reaction was monitored by HPLC. The reaction solution was purified directly by preparative-HPLC preparation, the product preparation was collected and lyophilized to obtain compound **38d** (446 mg).LC-MS: [M+H]⁺ =1549.6.

### Step 4: Synthesis of compound P38

Under the protection of nitrogen, compound **38d** (50 mg, 0.033 mmol), ultra-dry grade DMF (6 mL) and AcOH (0.2 mL) were added to a 25 mL single-mouth vial, and tetrakis (triphenylphosphine)palladium (37 mg, 0.033 mmol) and Bu₃SnH (19 mg, 0.066 mmol) were added sequentially at room temperature, and then the reaction was brought to room temperature. The reaction was carried out overnight and the end of the reaction was monitored by HPLC. The reaction solution was purified directly by preparative-HPLC, and the product preparation was collected and lyophilized to obtain compound **P38** (39 mg). LC-MS: [M+Na]⁺ =1487.8.

### Example 42: Synthesis of Compound P39

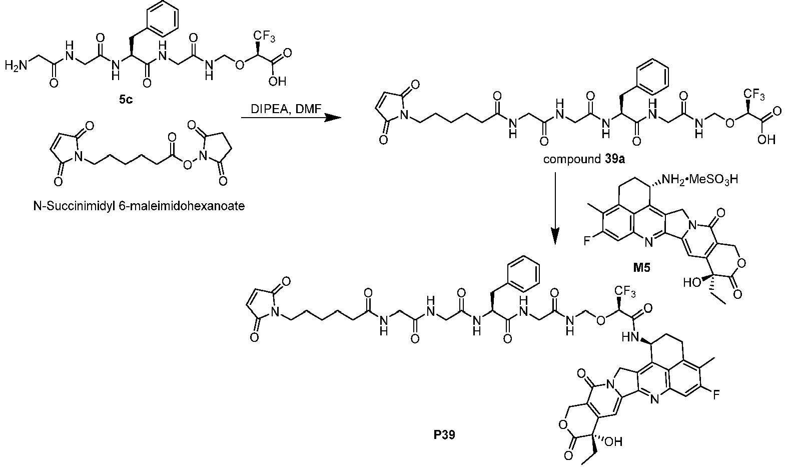

### Step 1: Preparation of compound 39a

In a 100 mL single-necked bottle, compound **5c** (1 g, 3.2 mmol) was added and dissolved in 30 mL of DMF, compound N-Succinimidyl 6-maleimidohexanoate (1.6 g, 3.2 mmol) and DIPEA (1.06 mL, 6.4 mmol) were added sequentially, the reaction was maintained at room temperature for 4 h. The reaction endpoint was monitored by HPLC. The reaction solution was directly purified by preparative HPLC (acid method), and the preparative solution was collected and lyophilized to obtain compound **39a** (1.9 g), LC-MS: [M-H]-=683.2.

### Step 2: Synthesis of compound P39

In a 25 mL single-necked bottle, Compound **39a** (200 mg, 0.29 mmol), M5 exatecan mesylate (155 mg, 0.29 mmol), PyBOP (187 mg, 0.36 mmol), HOBt (48 mg, 0.36 mmol), and DMF (6 mL) were added, and the reaction was lowered to 0 °C under an ice-water bath. DIPEA (62 mg, 0.48 mmol) was added, and the reaction was raised to 20 °C for 2 h reaction. The reaction was monitored by HPLC. The reaction solution was directly prepared and purified by preparative-HPLC, and the product preparation solution was collected and lyophilized to obtain compound **P39** (156 mg); LC-MS: [M+H]⁺ =1102.7.

### Example 43: Synthesis of Compound P40

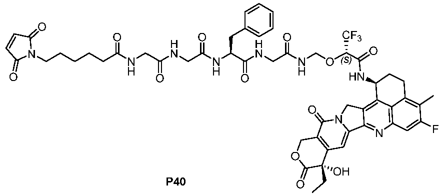

Referring to the synthetic route of Example 42, 134 mg of compound **P40** was prepared; LC-MS: [M+H]⁺ =1102.3.

### Example 44: Synthesis of Compound P41

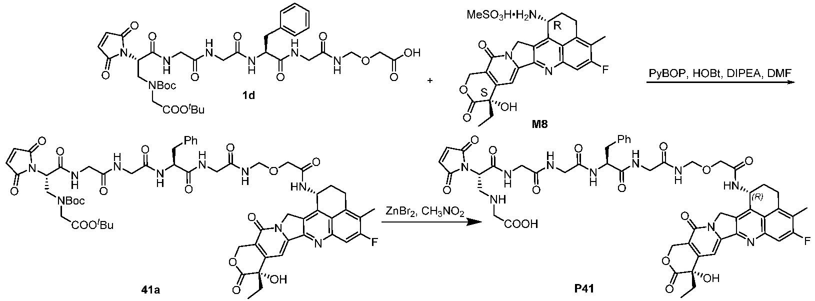

### Step 1: Compound 41a

In a 50 mL single-necked vial, add **1d** (500 mg, 0.62 mmol), **M8** (310 mg, 0.62 mmol) (compounds shown in formula 31 were prepared according to the method disclosed in CN111065621A, and subsequent preparations were consistent with Examples 7-2 of that reference), PyBOP (448 mg, 0.86 mmol), and HOBt (116 mg, 0.86 mmol), 15 mL DMF, DIPEA (378 uL, 2.29 mmol) was added under an ice-water bath, and the reaction was brought to room temperature for 2 h. After the reaction was completed by HPLC monitoring, the reaction solution was purified by preparative-HPLC to obtain a preparation solution, and the preparation solution was lyophilized to obtain **41a** (210 mg); LC-MS: [M+H]+=1221.6.

### Step 2: Compound P41

In a 25 mL single-necked vial, **41a** (200 mg, 0.162 mmol), zinc bromide (736 mg, 3.26 mmol) and 10 mL of nitromethane were added, and the reaction was carried out at 40 °C for 1 h. After the reaction was completed by HPLC monitoring, the solvent was removed by concentration under reduced pressure to obtain a crude product. The crude product was purified by preparative-HPLC to obtain a product preparation solution, which was lyophilized to obtain solid compound **P41** (120 mg); LC-MS: [M+H]+=1065.3.

### Example 45: Synthesis of Compound P42

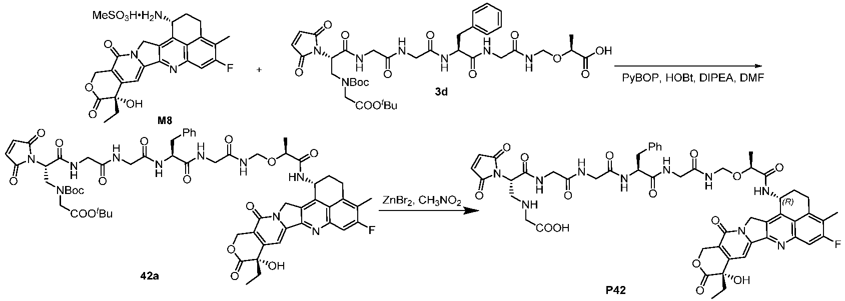

### Step 1: Compound 42a

In a 100 mL single-necked vial, **3d** (1.66 g, 2.02 mmol, 1.0 eq), **M8** (1.08 g, 2.02 mmol, 1.0 eq), PyBOP (1.58 g, 3.03 mmol, 1.5 eq), HOBt (0.41 g, 3.03 mmol, 1.5 eq), and DMF (40 mL) were added, DIPEA (0.84 mL, 1.5 eq) was added under an ice-water bath, and the mixture was heated to room temperature for 2 h (monitored by HPLC). The reaction solution was directly purified by preparative-HPLC, and the preparative liquid was concentrated by water pump under reduced pressure at 35 °C in water bath to remove acetonitrile, and lyophilized to obtain compound **42a** (1.54 g) with a yield of 61%; LC-MS: [M+H]+=1235.4.

### Step 6: Compound P42

In a 100 mL single-necked vial, compound **42a** (1.0 g, 0.8 mmol, 1.0 eq) was added, 35 mL of nitromethane was added to dissolve, and then zinc bromide (3.64 g, 16 mmol, 20.0 eq) was added. The reaction was carried out in an oil bath at 40 °C (preheated for stability) for 30 min and concentrated in a water bath at 45 °C with a water pump to remove nitromethane to obtain a yellow solid residue (monitored by HPLC). The solution was prepared by an acid method to obtain a preparation solution, which was concentrated in a water bath at 35 °C with a water pump to remove acetonitrile, and freeze-dried to obtain compound **P42** (786 mg) with a yield of 90%; LC-MS: [M+H]+=1079.8.

### Example 46: Synthesis of Compound P43

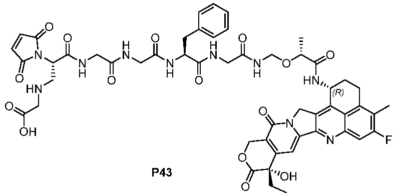

Referring to the synthetic route of Example 45, 442 mg of compound **P43** was prepared; LC-MS: [M+H]⁺ =1079.6.

### Example 47: Synthesis of Compound P44

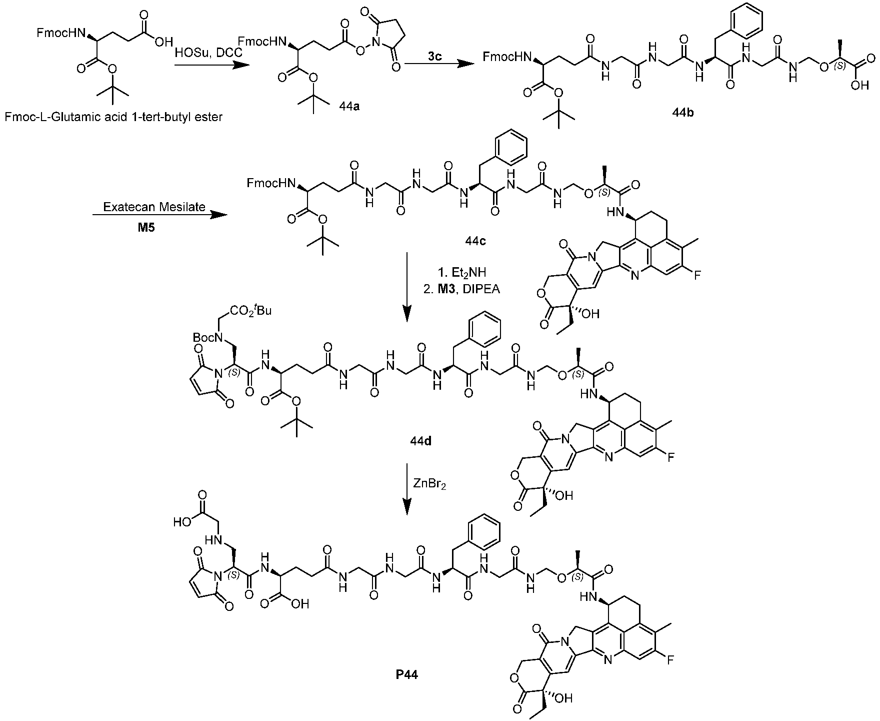

### Step 1: Preparation of compound 44a

In a 250 mL single-necked bottle, compound Fmoc-L-glutamic acid 1-tert-butyl ester (4 g, 9.35 mmol) was added, 100 mL of DCM was added to dissolve it at room temperature, and HOSu (1.17 g, 10 mmol) and DCC (2.92 g, 14 mmol) were added in sequence and reacted at room temperature for 2 h. After the reaction was completed by TLC monitoring, insoluble white solids were filtered out, and the filter cake was washed with DCM and the resulting organic phase was dried by water pumping in a water bath at 45 °C. The filter cake was washed with DCM, and the resulting organic phase was spun-dried by pumping water at 45 °C. The resulting yellowish residue was purified by silica gel column chromatography (PE/EA=50/1-20/1) to obtain 4.1 g of compound **44a.**

### Step 2: Preparation of compound 44b

In a 100 mL single-necked bottle, compound **44a** (2 g, 3.83 mmol) and 40 mL of DMF were added, and DIPEA (1.28 mL, 7.66 mmol) and compound **3c** (1.7 g, 3.83 mmol) were added to the bottle under an ice-water bath cooling, and the reaction was slowly brought to room temperature for 2 h. The completion of the reaction was detected by HPLC, and the reaction solution was purified by preparative-HPLC. Lyophilization yielded 2.8 g of compound **44b,** LC-MS: [M-H]⁻ = 844.0.

### Step 3: Preparation of compound 44c

In a 50 mL single-mouth bottle, compound **44b** (500 mg, 0.59 mmol) was added in 25 mL of DMF, and M5 exatecan mesilate (319 mg, 0.59 mmol), PyBOP (625 mg, 1.2 mmol), HOBt (164 mg, 1.2 mmol), and DIPEA (400 uL, 2.4 mmol) were added in sequence at room temperature, and the temperature was raised to room temperature for 5 h. After the reaction was completed by HPLC monitoring, the reaction solution was purified by preparative-HPLC, and the prepared solution was lyophilized to obtain compound **44c** (502 mg), LC-MS: [M+H]+= 1262.5.

### Step 4: Preparation of compound 44d

In a 50 mL single-necked bottle, compound **44c** (500 mg, 0.4 mmol) was added in 20 mL of DMF, and diethylamine (5 mL) was added under an ice-water bath cooling, and the reaction was slowly brought to room temperature for reaction 5 h. After the reaction was completed by HPLC monitoring, the reaction liquid was pumped dry in a 45 °C water bath, and the obtained crude product was directly used for the next step reaction.

The above crude compound (0.4 mmol) and 20 mL DMF were added to a 50 mL single-mouth bottle, DIPEA (145 uL, 0.8 mmol) and M3 (227 mg, 0.4 mmol) were added under ice-water bath cooling, and the temperature was raised to room temperature for reaction for 1 h. After the reaction was completed by HPLC monitoring, the reaction solution was purified by preparative-HPLC, and the prepared solution was lyophilized to obtain compound **44d** (358 mg), LC-MS: [M+H]+= 1420.7.

### Step 5: Preparation of compound P44

In a 25 mL single-necked vial, compound **44d** (150 mg, 0.106 mmol), zinc bromide (476 mg, 2.11 mmol) and 5 mL of nitromethane were added, and the reaction was carried out at 40 °C for 1 h. After the reaction was completed by HPLC monitoring, the solvent was removed by vacuum concentration to obtain a crude product. The crude product was purified by preparative-HPLC to obtain a product preparation solution, which was lyophilized to obtain a solid compound **P44** (98 mg); TOF: [M+H]+ = 1208.4281.

### Example 48: Synthesis of Compound P45

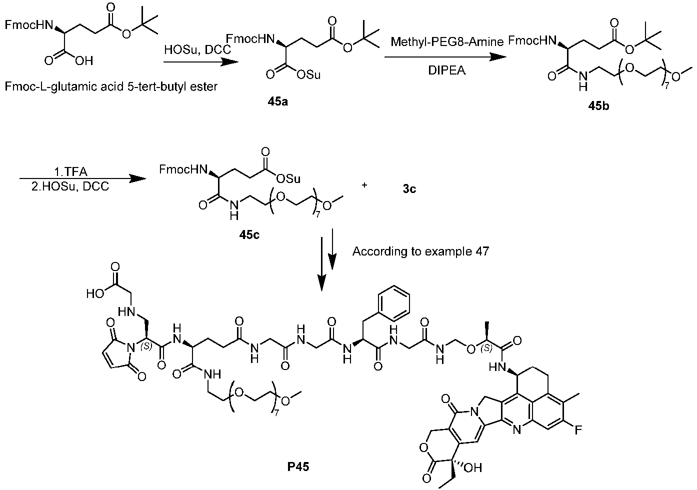

### Step 1: Preparation of compound 45a

In a 250 mL single-necked bottle, Fmoc-O-tert-butyl-L-glutamic acid (4 g, 9.35 mmol) was added and dissolved by adding 100 mL of DCM at room temperature, and then HOSu (1.17 g, 10 mmol) and DCC (2.92 g, 14 mmol) were added sequentially, and the reaction was carried out at room temperature for 2 h. After the reaction was completed by TLC monitoring, the insoluble white solid was removed by filtration, the filter cake was washed with DCM, the obtained organic phase was dried by water pump in a water bath at 45 °C, and the obtained light yellow residue was purified by silica gel column chromatography (PE/EA=50/1-20/1) to obtain 4.12 g of compound **45a.**

### Step 2: Preparation of compound 45b

In a 100 mL single-necked bottle, compound **45a** (2 g, 7.66 mmol) and 50 mL of DMF were added, and DIPEA (2.46 mL, 15.32 mmol) and compound m-PEG8-Amine (3.1 g, 7.66 mmol) were added under cooled condition in an ice-water bath, and the reaction was brought to room temperature slowly for 2 h. After the reaction was completed by HPLC monitoring, the reaction solution was purified by preparative-HPLC, and the prepared solution was lyophilized to obtain 3.7 g of compound **45b,** LC-MS: [M+H]+=791.9.

### Step 3: Synthesis of compound 45c

In a 100 mL single-necked bottle, compound **45b** (3.7 g) and 30 mL of DCM were added to a 100 mL vial, and TFA (30 mL) was added under an ice-water bath cooling, and the reaction was maintained at the same temperature for 2 h. After the completion of the reaction was detected by HPLC, the reaction solution was dried by pumping the solvent under reduced pressure at 45 °C with a water bath and heating and was used directly in the next step.

Add 50 mL DCM to the above crude product at room temperature to dissolve, and add HOSu (2.4 g, 20 mmol) and DCC (4.6 g, 22 mmol) in turn to react at room temperature overnight. After the reaction was completed by HPLC monitoring, the reaction solution was purified by preparative-HPLC, and the prepared solution was lyophilized to obtain 2.1 g of compound **45c,** LC-MS: [M+H]+=833.2.

### Step 4: Synthesis of compound P45

102 mg of compound **P45** was prepared from compound **45c** and compound **3c** according to the route of reference to Example 47; LC-MS:[M+H]⁺ = 1573.7.

### Example 49: Synthesis of Compound 22

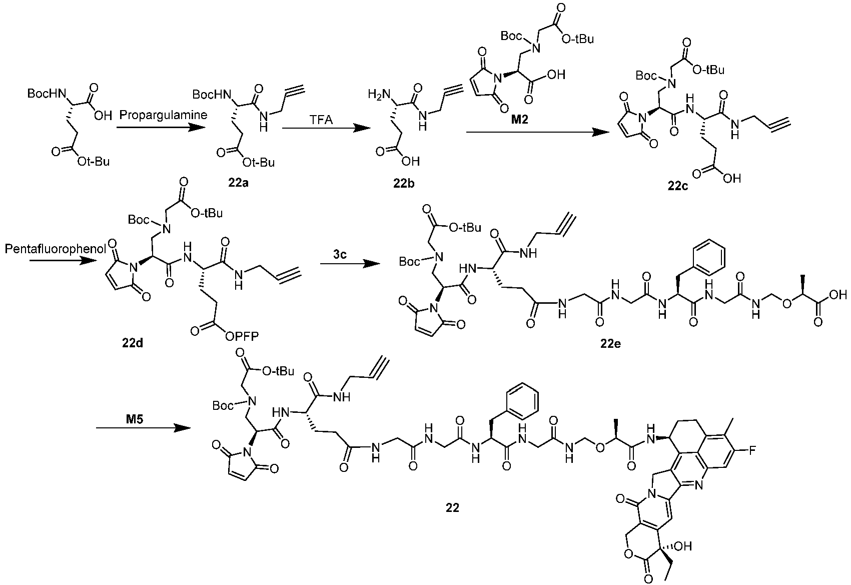

### Step 1: Preparation of compound 22a

In a 1 L single-mouth bottle, Boc-L-glutamic acid 5-tert-butyl ester (50 g, 164.8 mmol, 1.0 eq) was dissolved in 250 mL THF, propargylamine (9.97 g, 181.3 mmol, 1.1 eq), HOBt (22.25 g, 164.8 mmol, 1.0 eq), DIPEA (54.3 mL, 329.6 mmol, 2.0 eq), EDCI (37.7 g, 197.7 mmol, 1.2 eq) were added under an ice-water bath, warmed to room temperature for 2 h, and monitored the end point of the reaction by TLC. After the reaction was completed, poured the reaction solution into 600 mL ice water, added ethyl acetate to extract three times (200 mL×3), combined the organic phases, washed twice with saturated brine (300 mL×2), dried over anhydrous sodium sulfate, filtered, and concentrated to obtain a crude product. The crude product was purified by petroleum ether/ethyl acetate (2/1) to obtain 35.5 g of compound **22a;** LC-MS: [M+H]+=341.2.

### Step 2: Preparation of compound 22b

In a 1L single-mouth bottle, compound **22a** (20 g) was added and 150 mL of dichloromethane was added to dissolve it. 150 mL of trifluoroacetic acid was added, and the reaction was allowed to react at room temperature for 1 h. The reaction end point was monitored by TLC. After the reaction was completed, the reaction solution was concentrated at 45 °C by water pump to obtain a crude oil. The crude product was added with tertiary methyl ether for crystallization overnight, filtered, and the filter cake was dried at 45 °C for 4 h by blowing air, and weighed to obtain 7.68 g of the product; LC-MS: [M+H]+=184.2.

### Step 3: Preparation of compound 22c

In a 50 mL single-necked bottle, compound **22b** (5.0 g, 27.1 mmol) and compound **M2** (10.8 g, 27.1 mmol) were added and dissolved in 20 mL DMF, and EEDQ (13.4 g, 54.2 mmol) was added under an ice-water bath. After addition, the mixture was heated to room temperature for 2 h, and the reaction endpoint was monitored by HPLC. The reaction solution was purified by preparative-HPLC and lyophilized to obtain product **22c** (7.84 g); LC-MS m/z (M+H)+: 465.2.

### Step 4: Preparation of compound 22d

In a 25 mL single-necked vial, compound **22c** (0.85 g, 1.51 mmol) was added and dissolved in 15 mLDMF, and DCC (1.55 g, 7.56 mmol) and pentafluorophenol (0.31 g, 1.66 mmol) were added under an ice-water bath, the reaction was carried out at room temperature for 2 h. The end point of the reaction was monitored by TLC. After the reaction was completed, the mixture was filtered, and the filtrate was spin-dried and used directly in the next step.

### Step 5: Preparation of compound 22e

In an ice-water bath, compound **3c** (661 mg, 1.51 mmol) was dissolved in 15 mL DMF, DIPEA (0.37 mL, 2.26 mmol) was added dropwise, and then 3 mL DMF solution of the crude compound was added, and the mixture was heated to room temperature for 1 h. The reaction endpoint was monitored by HPLC. After the reaction was completed, the reaction solution was purified by preparative-HPLC to obtain a product preparation solution, which was lyophilized to obtain 502 mg of white solid compound **22e;** LC-MS: [M+H]+=985.2.

### Step 6: Preparation of Compound 22

In a 50 mL single-necked vial, compound **22e** (500 mg, 0.51 mmol) was added and dissolved in 10 mL DMF, and PyBOP (396 mg, 0.76 mmol), HOBt (103 mg, 0.76 mmol), and M5 Exatecan mesylate (270 mg, 0.51 mmol) were added sequentially to the vial under an ice-water bath, followed by DIEA (252 uL, 1.52 mmol), and the reaction was brought to room temperature for 2 h. The endpoint was monitored by HPLC. After the reaction was completed, the reaction solution was purified by preparative-HPLC to obtain the preparative solution, which was lyophilized to give 500 mg of white solid compound **22;** LC-MS: [M+H]⁺ =1402.2.

### Example 50: Synthesis of Compound P46

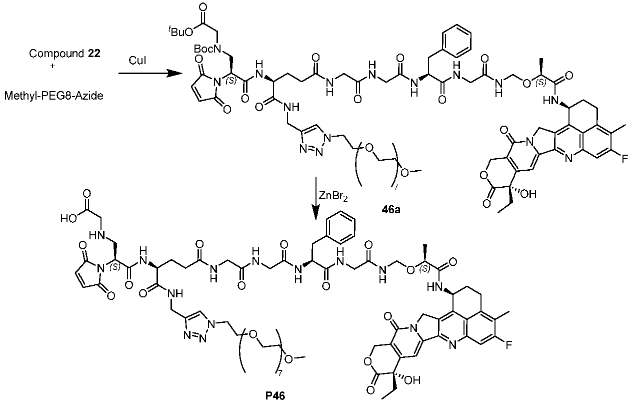

### Step 1: Synthesis of compound 46a

In a 25 mL single-necked vial, compound **22** (50 mg, 0.036 mmol) and Methyl-PEG8-Azide (156 mg, 0.036 mmol) were added, 5 mL DMF was added to dissolve, and then cuprous iodide (2 mg, 0.007 mmol) and TEA (10 uL, 0.07 mmol) were added, and the reaction was warmed up to 50 °C for 10 h reaction. The reaction was monitored by HPLC. At the end of the reaction, the reaction solution was purified by preparative-HPLC to obtain the product preparation solution, which was lyophilized to obtain 59 mg of compound **46a;** TOF-MS: [M+Na]+=1833.9517.

### Step 2: Synthesis of compound P46

In a 25 mL single-necked bottle, compound **46a** (50 mg, 0.028 mmol) was added, 5 mL nitromethane was added to dissolve, and then zinc bromide (130 mg, 0.56 mmol) was added, the reaction was carried out at room temperature for 1h, and the progress of the reaction was monitored by HPLC. At the end of the reaction, the reaction solution was concentrated under reduced pressure at 45 °C by water pump to obtain a crude product. The crude product was purified by preparative-HPLC to obtain a preparative solution, which was lyophilized and weighed to obtain 33 mg of compound **P46;** TOF-MS :[M+H]⁺ =1654.9.

### Example 51: Synthesis of Compound P47

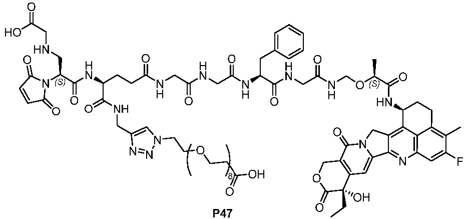

42 mg of compound **P47** was prepared from compound N₃-PEG₈-CH₂CH₂COOH according to the synthetic route of Example 50; TOF-MS : [M+H]⁺ =1712.7061.

### Example 52: Synthesis of Compound P48

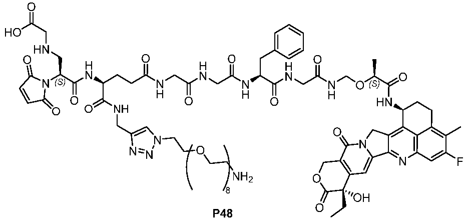

42 mg of compound **P48** was prepared from compound O-(2-aminoethyl)-O'-(2-azidoethyl) heptapolyethylene glycol according to the synthetic route of Example 50; TOF-MS :[M+H]⁺ =1683.7627.

### Example 53: Expression and purification of antibodies

### 1) Transient expression

Expi293 suspension cells (Shanghai OPM Biotech Co., Ltd.) were used to express antibodies targeting CD33: one day before transfection, cells were inoculated at a density of 0.9×10⁶ cells/mL in a 1 L shake flask containing 300 mL OPM-293 CD05 Medium (81075-001, Shanghai OPM Biotech Co., Ltd.) and placed in a cell culture shaker at 37°C, 5% CO₂, and 120 rpm for overnight culture. The next day, PEI-MAX was used for transfection of the antibody expression plasmid, where the mass ratio of plasmid: PEI-MAX was 1:3. OPM-293 ProFeed was added at 5% (v/v) on the first day after transfection, and OPM-293 ProFeed was added again at 5% (v/v) on the third day after transfection. The supernatant was collected by centrifugation on the sixth day after transfection.

### 2) Purified Antibodies

The cell expression supernatants were collected and purified using a Protein A affinity chromatography column (UniMab 50, Suzhou Nano Technology Co., Ltd.), eluted with 0.05 M sodium acetate (pH 3.6), and the captured antibody was adjusted to pH 7.0 with 1 M Tris-HCl (pH 8.8) at 0.7/10 (v/v), and then passed through a gel filtration chromatography column SEC (Superdex 200, GE) to remove impurities such as polymers.

### Antibody 1:

SEQ ID No.1 (Heavy chain variable region)
SEQ ID No.2 (Heavy chain CDR1)
   DSNIH
SEQ ID No.3 (Heavy chain CDR2)
   YIYPYNGGTDYNQKFKN
SEQ ID No.4 (Heavy chain CDR3)
   GNPWLAY
SEQ ID No.5 (Heavy chain) 
EVQLVQSGAEVKKPGSSVKVSCKASGYTITDSNIHWVRQAPGQSLEWIGYIYPYNGGTD
YNQKFKNRATLTVDNPTNTAYMELSSLRSEDTAFYYCVNGNPWLAYWGQGTLVTVSSA
STKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPS
VFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSR
WQQGNVFSCSVMHEALHNHYTQKSLSLSPG
SEQ ID No.6 (Heavy chain constant region) 
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPG
SEQ ID No.7 (Light chain variable region)
SEQ ID No.8 (Light chain variable region CDR1)
   RASESLDNYGIRFLT
SEQ ID No.9 (Light chain variable region CDR2)
   AASNQGS
SEQ ID No.10 (Light chain variable region CDR3)
   QQTKEVPWS
SEQ ID No. 11 (Light chain) 
DIQLTQSPSTLSASVGDRVTITCRASESLDNYGIRFLTWFQQKPGKAPKLLMYAASNQGS
GVPSRFSGSGSGTEFTLTISSLQPDDFATYYCQQTKEVPWSFGQGTKVEVKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID No.12 (Light chain constant region)
SEQ ID No.13 (Heavy chain variable region - Nucleic acid coding sequence) 
GAGGTGCAGCTGGTGCAGAGTGGAGCTGAGGTGAAGAAGCCAGGCTCCTCTGTGAA
GGTGTCCTGCAAGGCCTCTGGCTACACCATCACAGACAGCAACATCCACTGGGTGAG
GCAGGCCCCAGGCCAGAGCCTGGAGTGGATTGGCTACATCTACCCCTACAATGGAGG
CACAGACTACAACCAGAAGTTCAAGAACAGAGCCACCCTGACTGTGGACAACCCCA
CCAACACAGCCTACATGGAGCTGAGCTCCCTGAGAAGTGAAGACACAGCCTTCTACT
ACTGTGTGAATGGGAACCCCTGGCTGGCCTACTGGGGCCAGGGCACCCTGGTGACTG
TGTCCTCT
SEQ ID No.14 (Heavy chain CDR1 - Nucleic acid coding sequence)
   GACAGCAACATCCAC
SEQ ID No.15 (Heavy chain CDR2 - Nucleic acid coding sequence) TACATCTACCCCTACAATGGAGGCACAGACTACAACCAGAAGTTCAAGAAC
SEQ ID No.16 (Heavy chain CDR3 - Nucleic acid coding sequence)
   GGGAACCCCTGGCTGGCCTAC
SEQ ID No.17 (Heavy chain - Nucleic acid coding sequence) 
GAGGTGCAGCTGGTGCAGAGTGGAGCTGAGGTGAAGAAGCCAGGCTCCTCTGTGAA
GGTGTCCTGCAAGGCCTCTGGCTACACCATCACAGACAGCAACATCCACTGGGTGAG
GCAGGCCCCAGGCCAGAGCCTGGAGTGGATTGGCTACATCTACCCCTACAATGGAGG
 
CACAGACTACAACCAGAAGTTCAAGAACAGAGCCACCCTGACTGTGGACAACCCCA
CCAACACAGCCTACATGGAGCTGAGCTCCCTGAGAAGTGAAGACACAGCCTTCTACT
ACTGTGTGAATGGGAACCCCTGGCTGGCCTACTGGGGCCAGGGCACCCTGGTGACTG
TGTCCTCTGCTAGCACCAAGGGCCCCTCTGTGTTCCCTCTGGCTCCCTCCTCCAAGAG
CACCTCTGGAGGCACAGCTGCCCTGGGCTGCCTGGTGAAGGACTACTTCCCAGAGCC
TGTGACTGTGTCCTGGAACTCTGGAGCCCTGACCTCTGGAGTGCACACCTTCCCAGC
TGTGCTGCAGAGCTCTGGCCTCTACAGCCTGTCCTCTGTGGTGACTGTGCCCTCCTCC
TCCCTGGGAACCCAGACCTACATCTGCAATGTGAACCACAAGCCCTCCAACACCAAG
GTGGACAAGAGAGTGGAGCCCAAGAGCTGTGACAAGACCCACACCTGCCCACCCTG
CCCAGCCCCAGAGCTGCTGGGAGGACCCTCTGTGTTCCTCTTTCCACCCAAGCCCAA
GGACACCCTGATGATCTCCAGAACCCCAGAGGTGACCTGTGTGGTGGTGGATGTGTC
CCATGAAGACCCAGAAGTGAAGTTCAACTGGTATGTGGATGGAGTGGAAGTGCACA
ATGCCAAGACCAAGCCCAGAGAAGAGCAGTACAACAGCACCTACAGAGTGGTGTCT
GTGCTGACTGTGCTGCACCAGGACTGGCTGAATGGCAAAGAGTACAAGTGCAAAGT
GTCCAACAAGGCCCTGCCAGCTCCCATTGAGAAGACCATCTCCAAGGCCAAGGGCC
AGCCCAGAGAGCCTCAGGTGTACACCCTGCCACCATCCAGAGATGAGCTGACCAAG
AACCAGGTGTCCCTGACCTGCCTGGTGAAAGGCTTCTACCCCTCTGACATTGCTGTG
GAGTGGGAGAGCAATGGCCAGCCAGAGAACAACTACAAGACCACACCACCTGTGCT
GGACTCTGATGGCAGCTTCTTCCTGTACAGCAAGCTGACTGTGGACAAGAGCAGATG
GCAGCAGGGCAATGTCTTCTCCTGCTCTGTGATGCATGAAGCTCTGCACAACCACTAC
ACCCAGAAGAGCCTGTCCCTGTCTCCAGGGTGA
SEQ ID No.18 (Heavy chain constant region - Nucleic acid coding sequence) 
GCTAGCACCAAGGGCCCCTCTGTGTTCCCTCTGGCTCCCTCCTCCAAGAGCACCTCTG
GAGGCACAGCTGCCCTGGGCTGCCTGGTGAAGGACTACTTCCCAGAGCCTGTGACTG
TGTCCTGGAACTCTGGAGCCCTGACCTCTGGAGTGCACACCTTCCCAGCTGTGCTGC
AGAGCTCTGGCCTCTACAGCCTGTCCTCTGTGGTGACTGTGCCCTCCTCCTCCCTGGG
AACCCAGACCTACATCTGCAATGTGAACCACAAGCCCTCCAACACCAAGGTGGACAA
GAGAGTGGAGCCCAAGAGCTGTGACAAGACCCACACCTGCCCACCCTGCCCAGCCC
CAGAGCTGCTGGGAGGACCCTCTGTGTTCCTCTTTCCACCCAAGCCCAAGGACACCC
TGATGATCTCCAGAACCCCAGAGGTGACCTGTGTGGTGGTGGATGTGTCCCATGAAG
ACCCAGAAGTGAAGTTCAACTGGTATGTGGATGGAGTGGAAGTGCACAATGCCAAG
ACCAAGCCCAGAGAAGAGCAGTACAACAGCACCTACAGAGTGGTGTCTGTGCTGAC
TGTGCTGCACCAGGACTGGCTGAATGGCAAAGAGTACAAGTGCAAAGTGTCCAACA
AGGCCCTGCCAGCTCCCATTGAGAAGACCATCTCCAAGGCCAAGGGCCAGCCCAGA
GAGCCTCAGGTGTACACCCTGCCACCATCCAGAGATGAGCTGACCAAGAACCAGGTG
TCCCTGACCTGCCTGGTGAAAGGCTTCTACCCCTCTGACATTGCTGTGGAGTGGGAG
AGCAATGGCCAGCCAGAGAACAACTACAAGACCACACCACCTGTGCTGGACTCTGAT
GGCAGCTTCTTCCTGTACAGCAAGCTGACTGTGGACAAGAGCAGATGGCAGCAGGG
CAATGTCTTCTCCTGCTCTGTGATGCATGAAGCTCTGCACAACCACTACACCCAGAAG
 AGCCTGTCCCTGTCTCCAGGGTGA
SEQ ID No.19 (Light chain variable region - Nucleic acid coding sequence) 
GACATCCAGCTGACCCAGAGCCCCTCCACCCTGTCTGCCTCTGTGGGAGACAGAGTG
ACCATCACCTGCAGAGCCTCTGAGAGCCTGGACAACTATGGCATCAGATTCCTGACCT
GGTTCCAGCAGAAGCCAGGGAAAGCCCCCAAGCTGCTGATGTATGCTGCCTCCAACC
AGGGCAGTGGAGTGCCCTCCAGATTCTCTGGCTCTGGCTCTGGCACAGAGTTCACCC
TGACCATCTCCTCCCTGCAGCCAGATGACTTTGCCACCTACTACTGCCAGCAGACCAA
GGAGGTGCCCTGGAGCTTTGGACAGGGCACCAAGGTGGAGGTGAAG
SEQ ID No.20 (Light chain variable region CDR1- Nucleic acid coding sequence) AGAGCCTCTGAGAGCCTGGACAACTATGGCATCAGATTCCTGACC
SEQ ID No.21 (Light chain variable region CDR2 - Nucleic acid coding sequence) GCTGCCTCCAACCAGGGCAGT
SEQ ID No.22 (Light chain variable region CDR3 - Nucleic acid coding sequence) CAGCAGACCAAGGAGGTGCCCTGGAGC
SEQ ID No.23 (Light chain - Nucleic acid coding sequence) 
GACATCCAGCTGACCCAGAGCCCCTCCACCCTGTCTGCCTCTGTGGGAGACAGAGTG
ACCATCACCTGCAGAGCCTCTGAGAGCCTGGACAACTATGGCATCAGATTCCTGACCT
GGTTCCAGCAGAAGCCAGGGAAAGCCCCCAAGCTGCTGATGTATGCTGCCTCCAACC
AGGGCAGTGGAGTGCCCTCCAGATTCTCTGGCTCTGGCTCTGGCACAGAGTTCACCC
TGACCATCTCCTCCCTGCAGCCAGATGACTTTGCCACCTACTACTGCCAGCAGACCAA
GGAGGTGCCCTGGAGCTTTGGACAGGGCACCAAGGTGGAGGTGAAGCGTACGGTGG
CTGCTCCCTCTGTGTTCATCTTCCCACCCTCTGATGAGCAGCTGAAGTCTGGCACAGC
CTCTGTGGTGTGCCTGCTGAACAACTTCTACCCCAGAGAAGCCAAGGTGCAGTGGAA
AGTGGACAATGCCCTGCAGAGTGGGAACAGCCAGGAGAGTGTGACAGAGCAGGAC
AGCAAGGACAGCACCTACAGCCTGTCCTCCACCCTGACCCTGTCCAAAGCTGACTAT
GAGAAGCACAAAGTGTATGCCTGTGAAGTGACCCACCAGGGCCTGTCCTCTCCTGTG
ACCAAGAGCTTCAACAGAGGAGAGTGCTGA
SEQ ID No.24 (Light Chain constant region - Nucleic acid coding sequence)

CGTACGGTGGCTGCTCCCTCTGTGTTCATCTTCCCACCCTCTGATGAGCAGCTGAAGT
CTGGCACAGCCTCTGTGGTGTGCCTGCTGAACAACTTCTACCCCAGAGAAGCCAAGG
TGCAGTGGAAAGTGGACAATGCCCTGCAGAGTGGGAACAGCCAGGAGAGTGTGACA
GAGCAGGACAGCAAGGACAGCACCTACAGCCTGTCCTCCACCCTGACCCTGTCCAA
AGCTGACTATGAGAAGCACAAAGTGTATGCCTGTGAAGTGACCCACCAGGGCCTGTC
CTCTCCTGTGACCAAGAGCTTCAACAGAGGAGAGTGCTGA

### Antibody 2:

Except for the amino acid sequence of the light chain constant region and its nucleic acid coding sequence, and the amino acid sequence of the light chain and its nucleic acid coding sequence, the remaining sequences are the same as those of Antibody 1.
SEQ ID No.25 (Light chain) 
DIQLTQSPSTLSASVGDRVTITCRASESLDNYGIRFLTWFQQKPGKAPKLLMYAASNQGS
GVPSRFSGSGSGTEFTLTISSLQPDDFATYYCQQTKEVPWSFGQGTKVEVKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVCTKSFNRGEC
SEQ ID No.26 (Light chain constant region) 
RTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVCTKSFNRGEC
SEQ ID No.27 (Light chain - Nucleic acid coding sequence) 
GACATCCAGCTGACCCAGAGCCCCTCCACCCTGTCTGCCTCTGTGGGAGACAGAGTG
ACCATCACCTGCAGAGCCTCTGAGAGCCTGGACAACTATGGCATCAGATTCCTGACCT
GGTTCCAGCAGAAGCCAGGGAAAGCCCCCAAGCTGCTGATGTATGCTGCCTCCAACC
AGGGCAGTGGAGTGCCCTCCAGATTCTCTGGCTCTGGCTCTGGCACAGAGTTCACCC
TGACCATCTCCTCCCTGCAGCCAGATGACTTTGCCACCTACTACTGCCAGCAGACCAA
GGAGGTGCCCTGGAGCTTTGGACAGGGCACCAAGGTGGAGGTGAAGCGTACGGTGG
CTGCTCCCTCTGTGTTCATCTTCCCACCCTCTGATGAGCAGCTGAAGTCTGGCACAGC
CTCTGTGGTGTGCCTGCTGAACAACTTCTACCCCAGAGAAGCCAAGGTGCAGTGGAA
AGTGGACAATGCCCTGCAGAGTGGGAACAGCCAGGAGAGTGTGACAGAGCAGGAC
AGCAAGGACAGCACCTACAGCCTGTCCTCCACCCTGACCCTGTCCAAAGCTGACTAT
GAGAAGCACAAAGTGTATGCCTGTGAAGTGACCCACCAGGGCCTGTCCTCTCCTGTG
TGCACCAAGAGCTTCAACAGAGGAGAGTGCTGA
SEQ ID No.28 (Light chain constant region - Nucleic acid coding sequence)

CGTACGGTGGCTGCTCCCTCTGTGTTCATCTTCCCACCCTCTGATGAGCAGCTGAAGT
CTGGCACAGCCTCTGTGGTGTGCCTGCTGAACAACTTCTACCCCAGAGAAGCCAAGG
TGCAGTGGAAAGTGGACAATGCCCTGCAGAGTGGGAACAGCCAGGAGAGTGTGACA
GAGCAGGACAGCAAGGACAGCACCTACAGCCTGTCCTCCACCCTGACCCTGTCCAA
AGCTGACTATGAGAAGCACAAAGTGTATGCCTGTGAAGTGACCCACCAGGGCCTGTC
CTCTCCTGTGTGCACCAAGAGCTTCAACAGAGGAGAGTGCTGA

### Example 54: Generic preparation of Antibody-Drug Conjugates

### Preparation method of antibody-drug conjugates

After cell expression and purification by Protein A affinity chromatography and molecular sieve chromatography, Antibody 2 of Example 53 was replaced in 20 mM NaAc-HAc, pH 6.0 buffer, and Antibody 2 was concentrated or diluted to a protein concentration of 5 mg/mL. The payload was a white or light-yellow powder, which was dissolved to 10 mg/mL using N, N-dimethylacetamide (DMA) for use.

To open the interchain disulfide bond of Antibody 2, 20 times tris (2-carboxyethyl) phosphine (TCEP) was added according to the molecular ratio and reacted at room temperature for 2 h. 25 times the payload solution was added according to the molecular ratio and reacted at room temperature for 8 h. After the reaction, the liquid was replaced using a 30 KDa ultrafiltration concentration tube to remove the uncoupled payload, and the antibody 2-drug conjugate sample was obtained.

### Example 55: Preparation of ADC-1

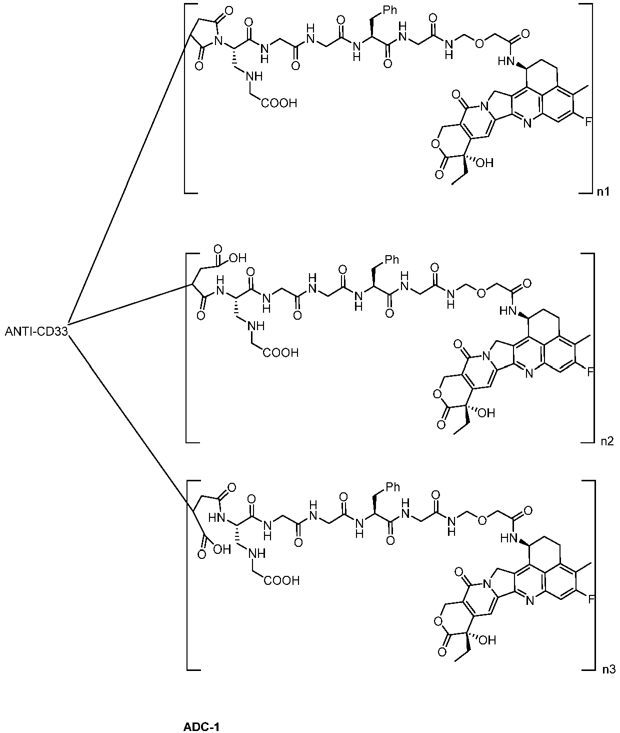

**ADC-1** was prepared according to the generic coupling method of the antibody-drug conjugate in Example 54.

### Example 56: Preparation of ADC-2

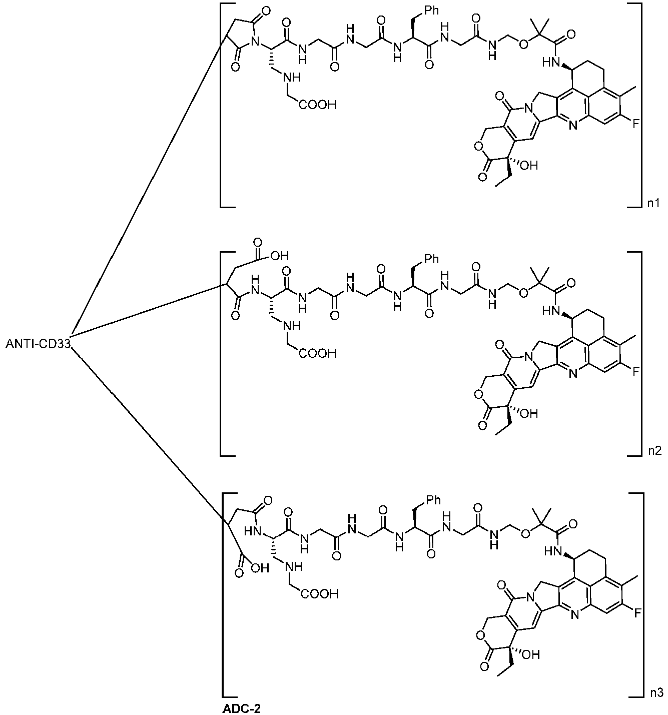

**ADC-2** was prepared according to the generic coupling method of the antibody-drug conjugate in Example 54.

### Example 57: Preparation of ADC-3

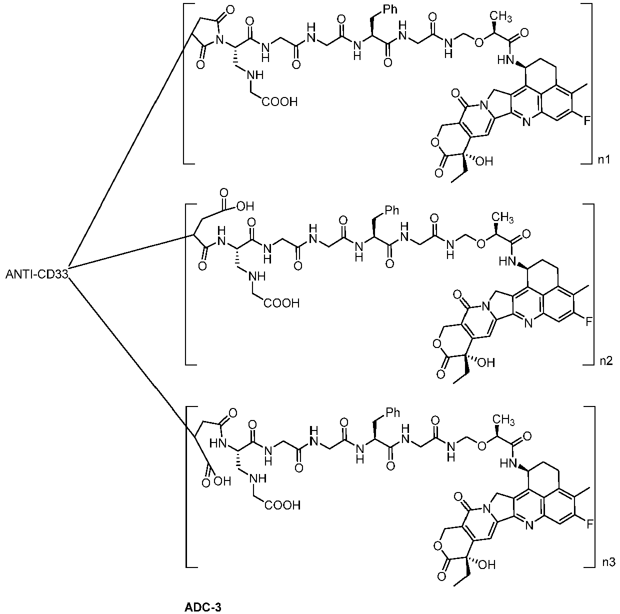

**ADC-3** was prepared according to the generic coupling method of the antibody-drug conjugate in Example 54.

### Example 58: Preparation of ADC-4

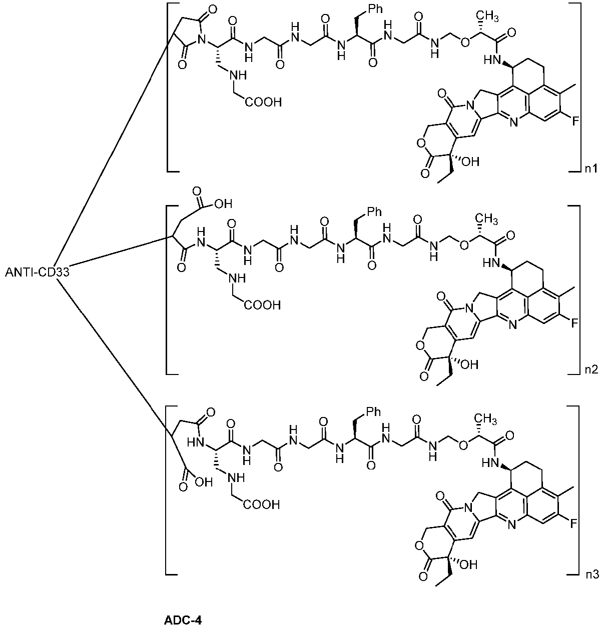

**ADC-4** was prepared according to the generic coupling method of the antibody-drug conjugate in Example 54.

### Example 59: Preparation of ADC-5

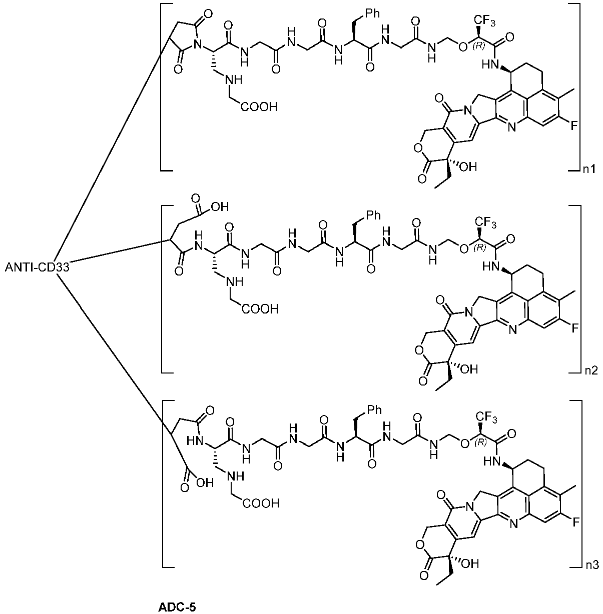

**ADC-5** was prepared according to the generic coupling method of the antibody-drug conjugate in Example 54.

### Example 60: Preparation of ADC-6

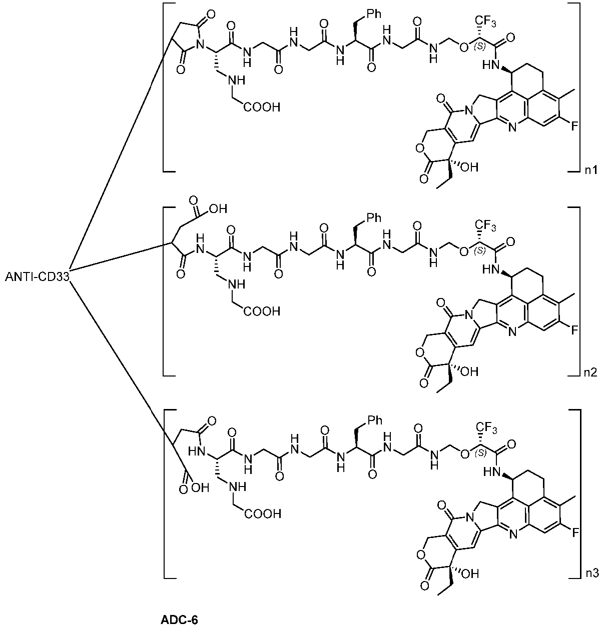

**ADC-6** was prepared according to the generic coupling method of the antibody-drug conjugate in Example 54.

### Example 61: Preparation of ADC-7

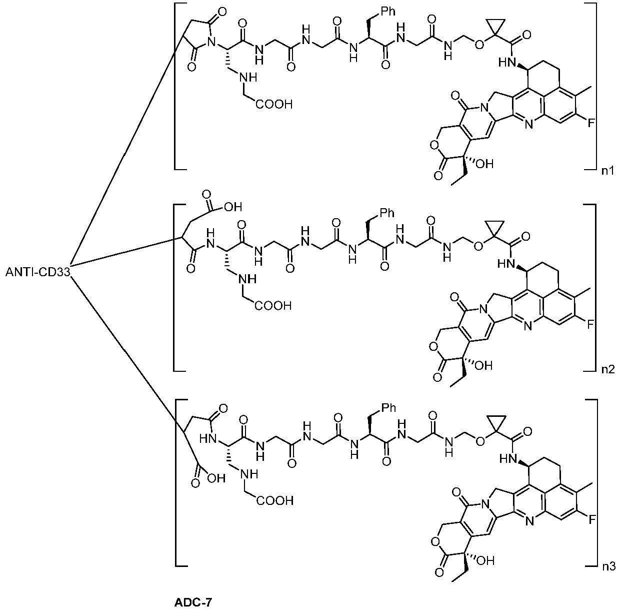

**ADC-7** was prepared according to the generic coupling method of the antibody-drug conjugate in Example 54.

### Example 62: Preparation of ADC-8

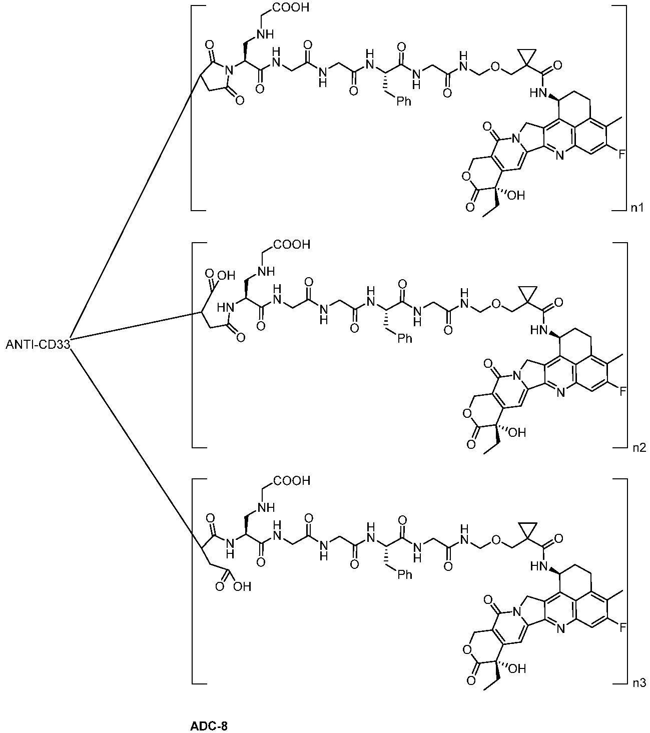

**ADC-8** was prepared according to the generic coupling method of the antibody-drug conjugate in Example 54.

### Example 63: Preparation of ADC-9

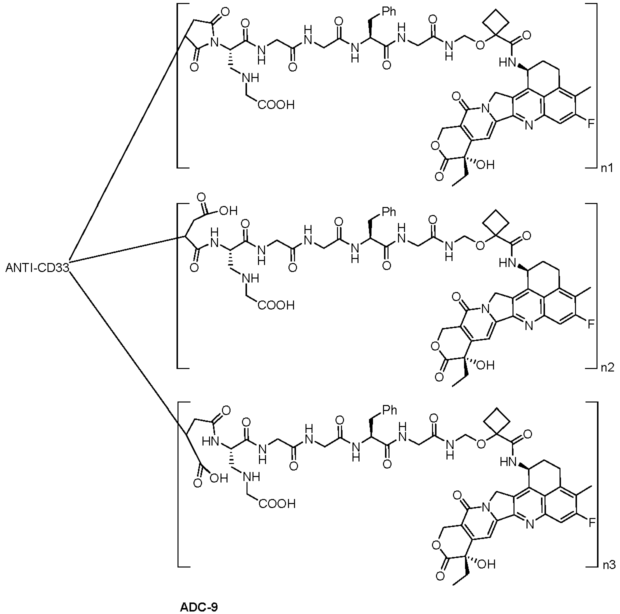

**ADC-9** was prepared according to the generic coupling method of the antibody-drug conjugate in Example 54.

### Example 64: Preparation of ADC-10

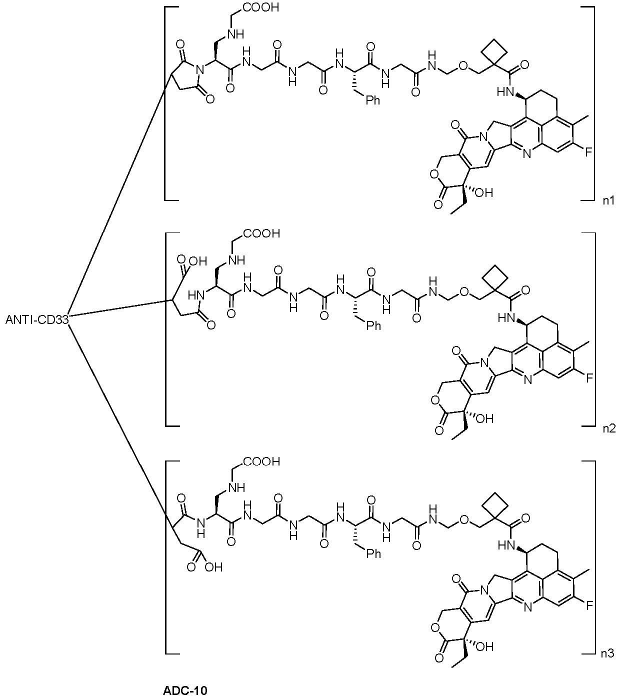

**ADC-10** was prepared according to the generic coupling method of the antibody-drug conjugate in Example 54.

### Example 65: Preparation of ADC-11

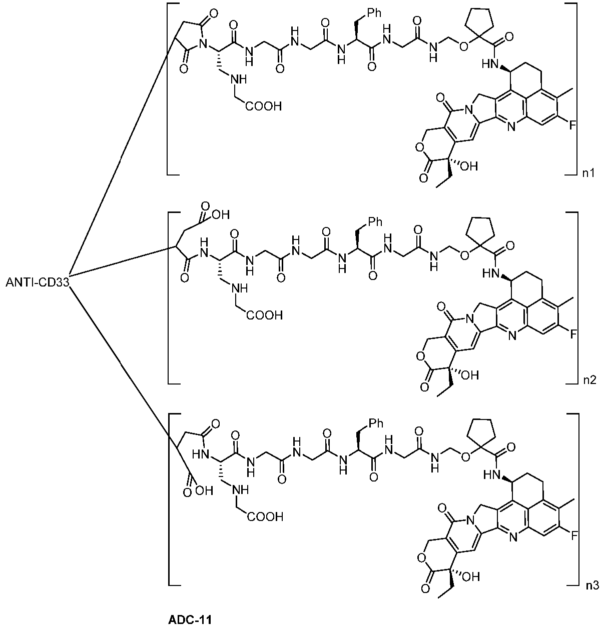

**ADC-11** was prepared according to the generic coupling method of the antibody-drug conjugate in Example 54.

### Example 66: Preparation of ADC-12

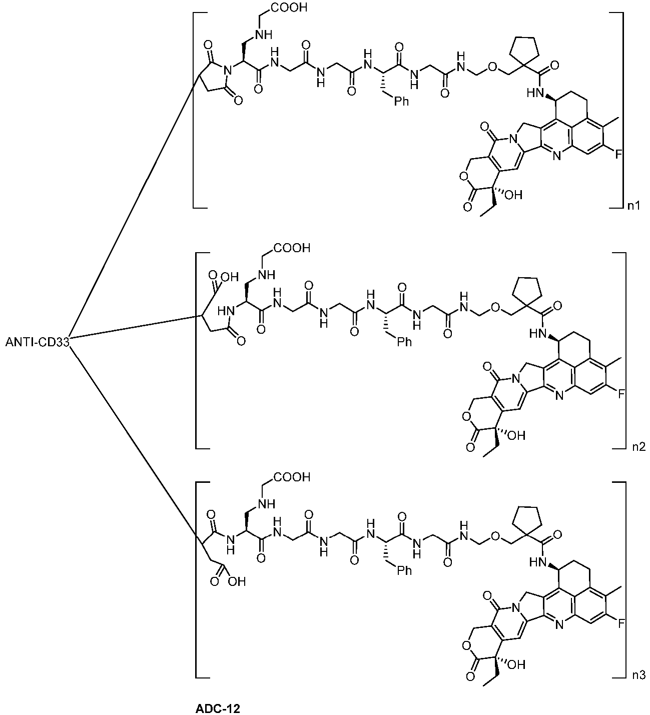

**ADC-12** was prepared according to the generic coupling method of the antibody-drug conjugate in Example 54.

### Example 67: Preparation of ADC-13

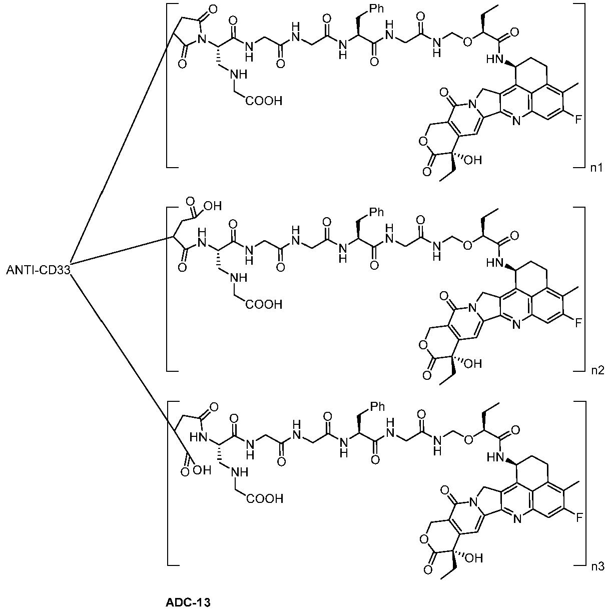

**ADC-13** was prepared according to the generic coupling method of the antibody-drug conjugate in Example 54.

### Example 68: Preparation of ADC-14

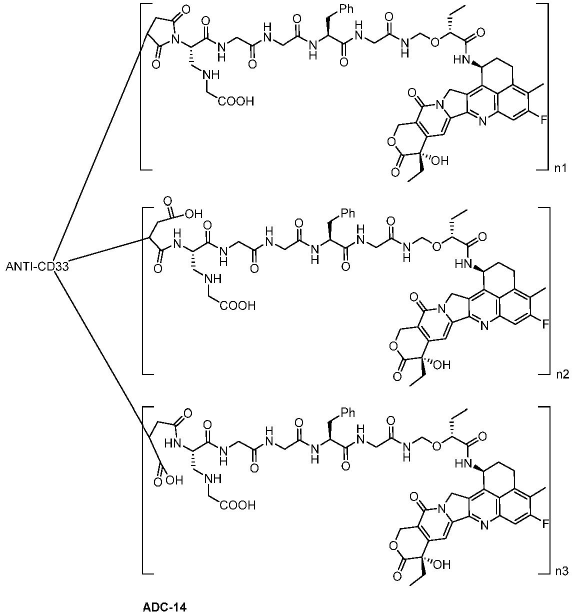

**ADC-14** was prepared according to the generic coupling method of the antibody-drug conjugate in Example 54.

### Example 69: Preparation of ADC-15

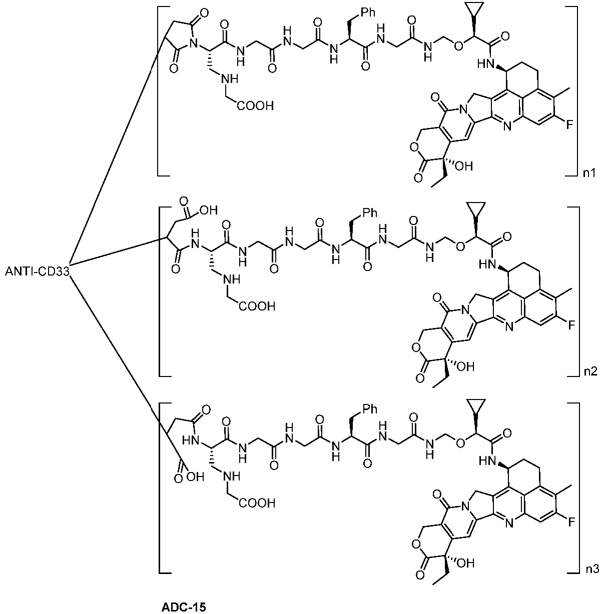

**ADC-15** was prepared according to the generic coupling method of the antibody-drug conjugate in Example 54.

### Example 70: Preparation of ADC-16

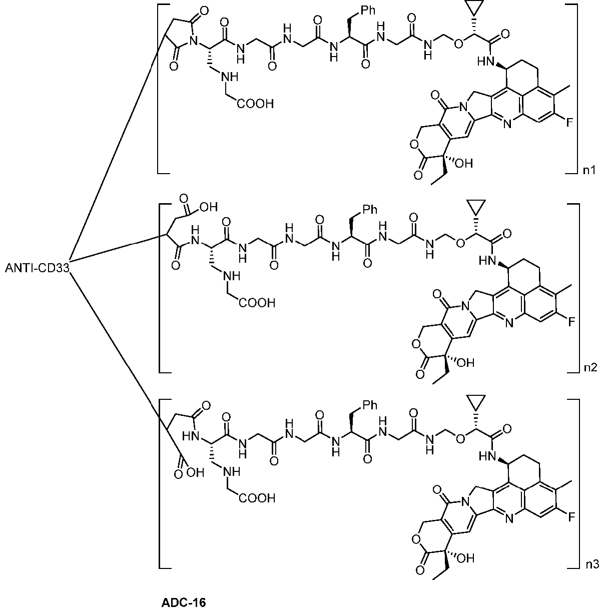

**ADC-16** was prepared according to the generic coupling method of the antibody-drug conjugate in Example 54.

### Example 71: Preparation of ADC-17

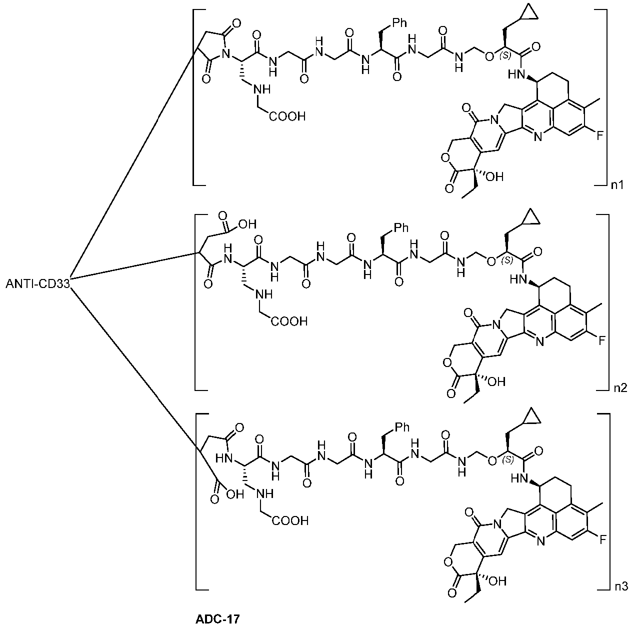

**ADC-17** was prepared according to the generic coupling method of the antibody-drug conjugate in Example 54.

### Example 72: Preparation of ADC-18

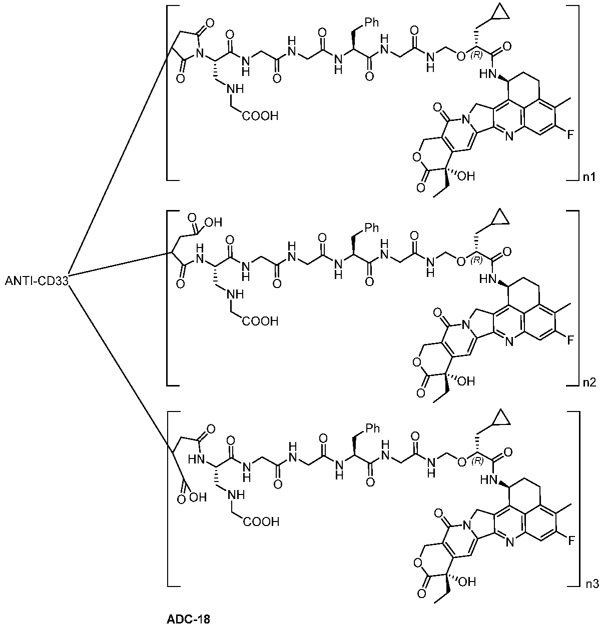

**ADC-18** was prepared according to the generic coupling method of the antibody-drug conjugate in Example 54.

### Example 73: Preparation of ADC-19

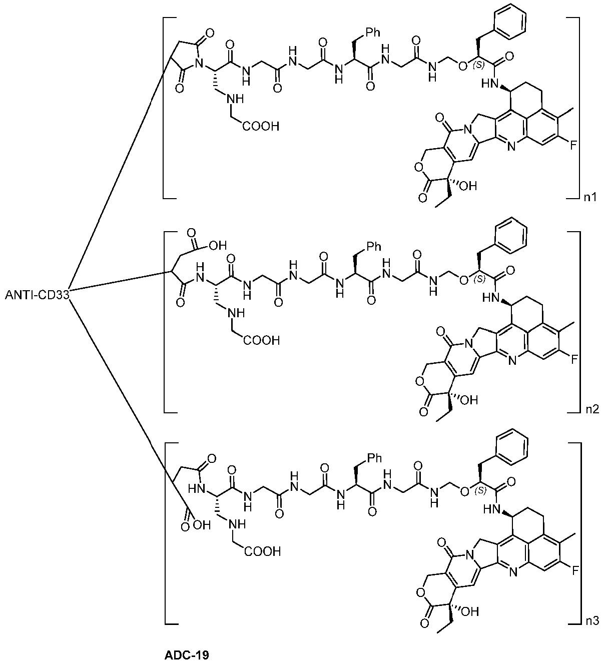

**ADC-19** was prepared according to the generic coupling method of the antibody-drug conjugate in Example 54.

### Example 74: Preparation of ADC-20

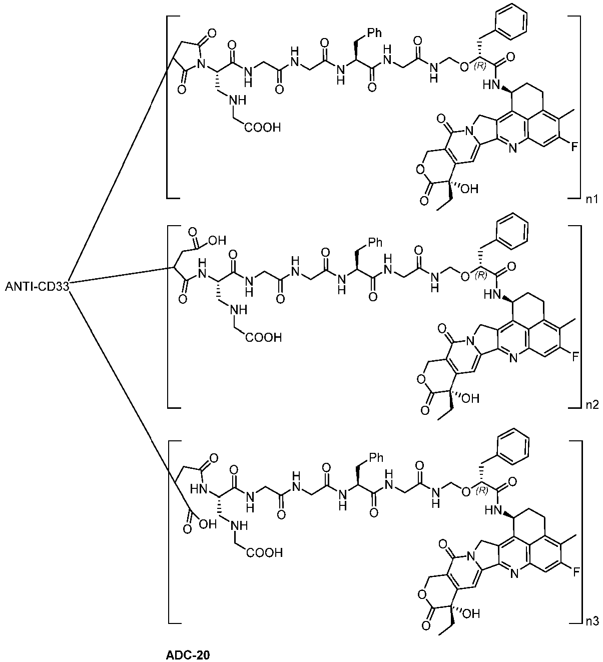

**ADC-20** was prepared according to the generic coupling method of the antibody-drug conjugate in Example 54.

### Example 75: Preparation of ADC-21

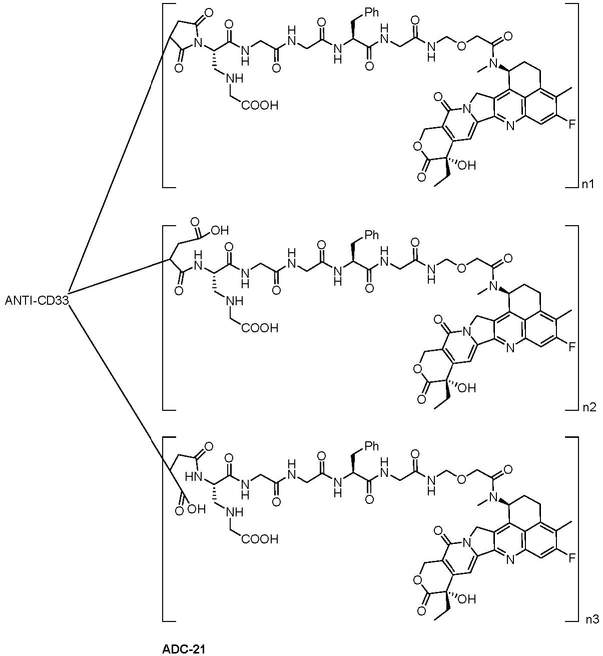

**ADC-21** was prepared according to the generic coupling method of the antibody-drug conjugate in Example 54.

### Example 76: Preparation of ADC-22

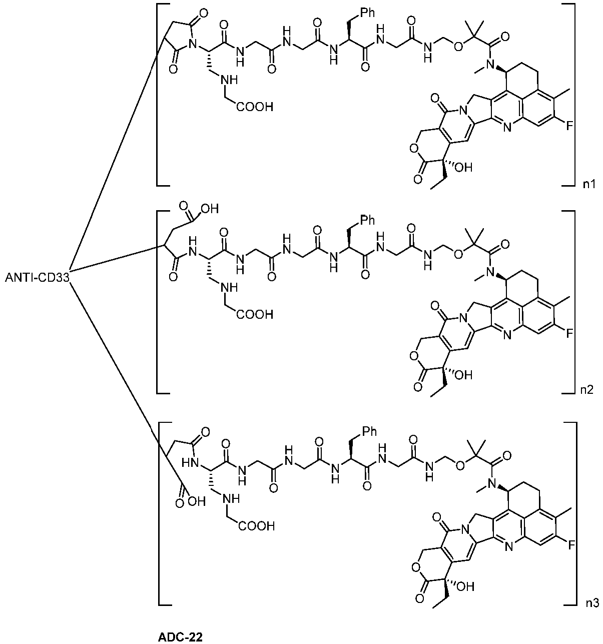

**ADC-22** was prepared according to the generic coupling method of the antibody-drug conjugate in Example 54.

### Example 77: Preparation of ADC-23

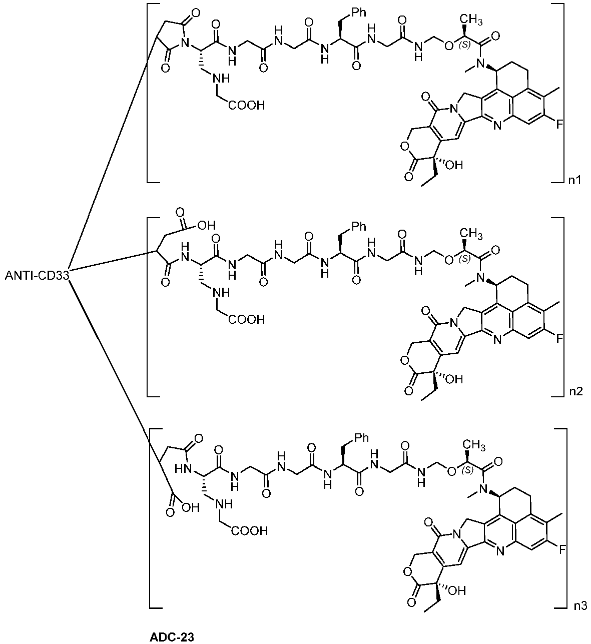

**ADC-23** was prepared according to the generic coupling method of the antibody-drug conjugate in Example 54.

### Example 78: Preparation of ADC-24

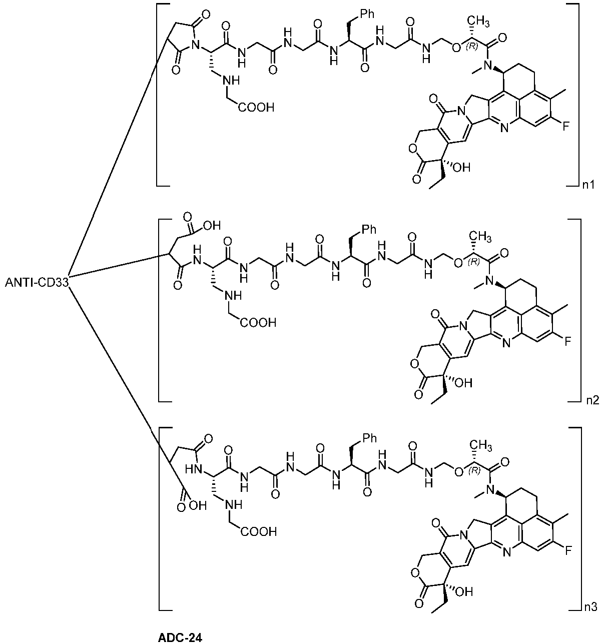

**ADC-24** was prepared according to the generic coupling method of the antibody-drug conjugate in Example 54.

### Example 79: Preparation of ADC-25

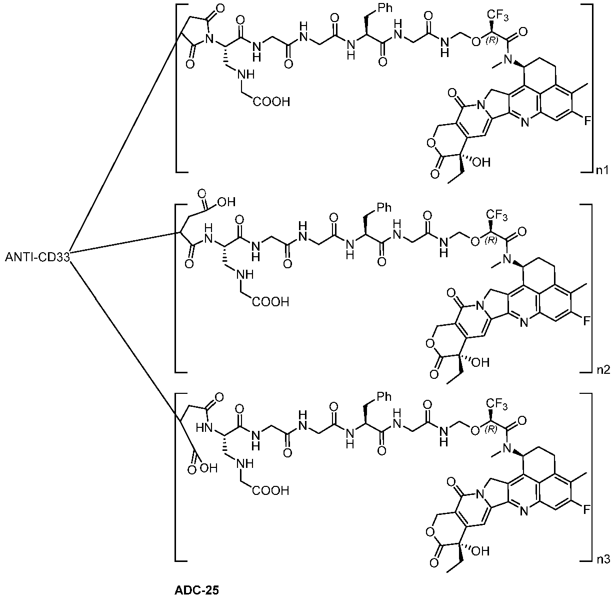

**ADC-25** was prepared according to the generic coupling method of the antibody-drug conjugate in Example 54.

### Example 80: Preparation of ADC-26

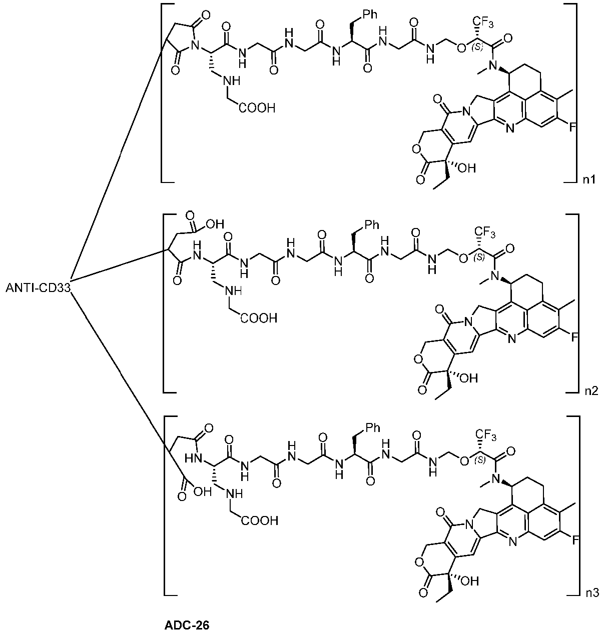

**ADC-26** was prepared according to the generic coupling method of the antibody-drug conjugate in Example 54.

### Example 81: Preparation of ADC-27

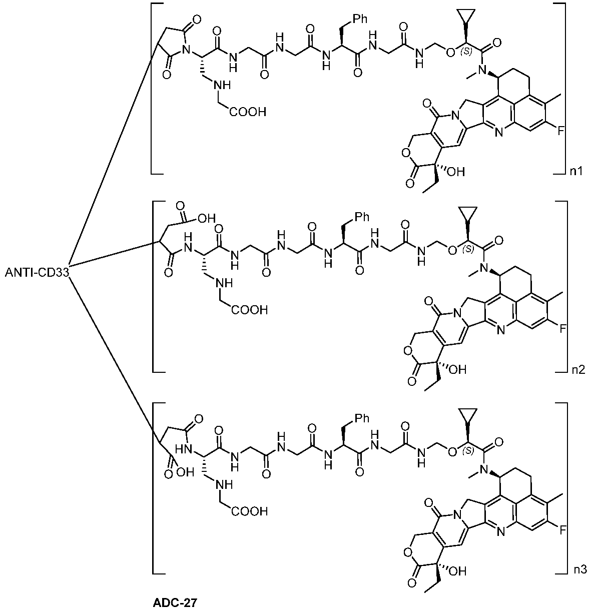

**ADC-27** was prepared according to the generic coupling method of the antibody-drug conjugate in Example 54.

### Example 82: Preparation of ADC-28

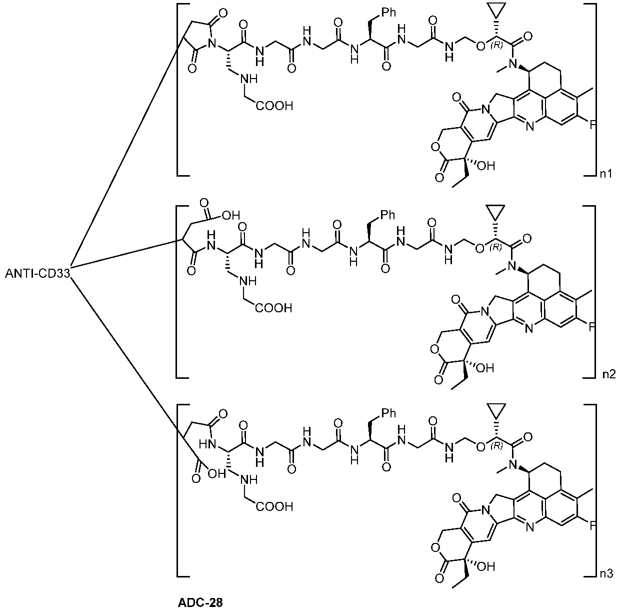

**ADC-28** was prepared according to the generic coupling method of the antibody-drug conjugate in Example 54.

### Example 83: Preparation of ADC-29

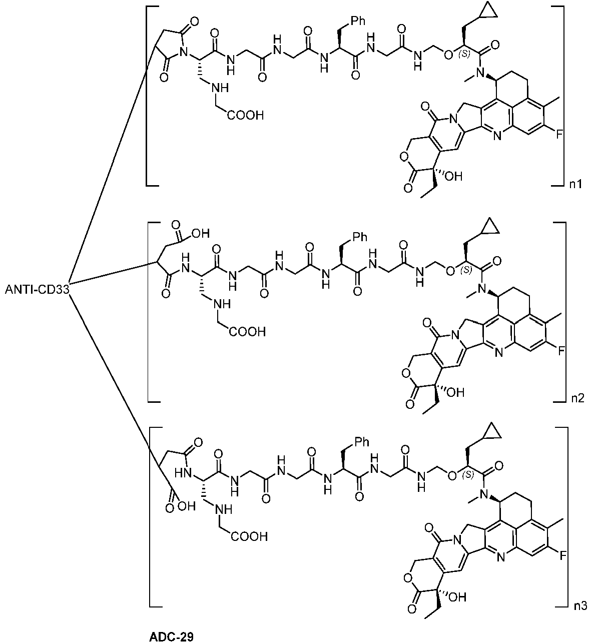

**ADC-29** was prepared according to the generic coupling method of the antibody-drug conjugate in Example 54.

### Example 84: Preparation of ADC-30

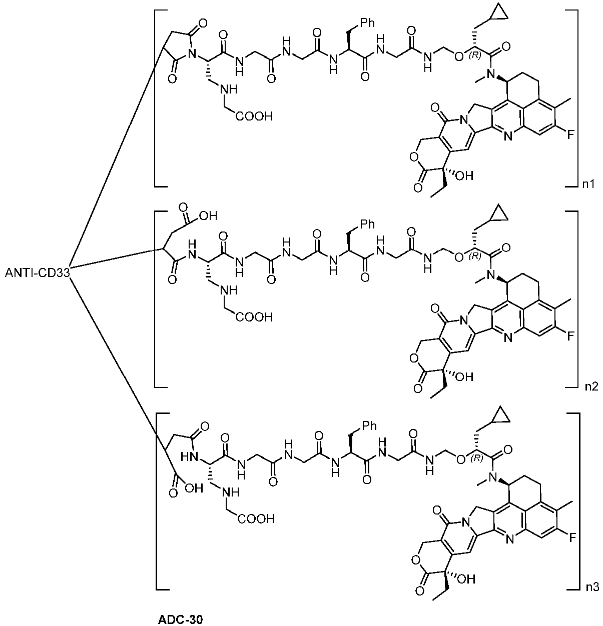

**ADC-30** was prepared according to the generic coupling method of the antibody-drug conjugate in Example 54.

### Example 85: Preparation of ADC-31

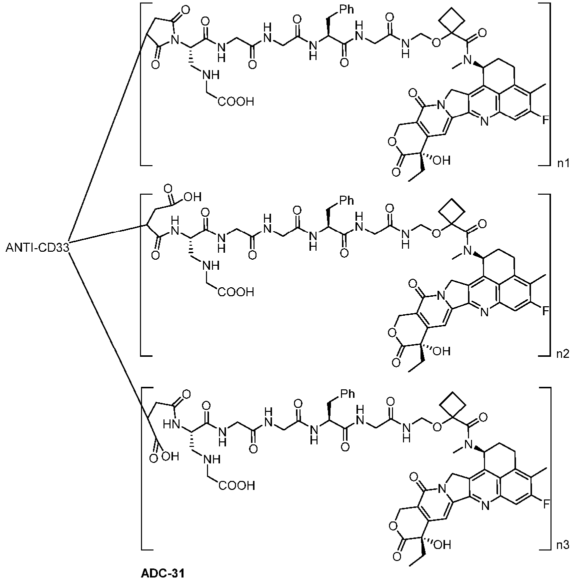

**ADC-31** was prepared according to the generic coupling method of the antibody-drug conjugate in Example 54.

### Example 86: Preparation of ADC-32

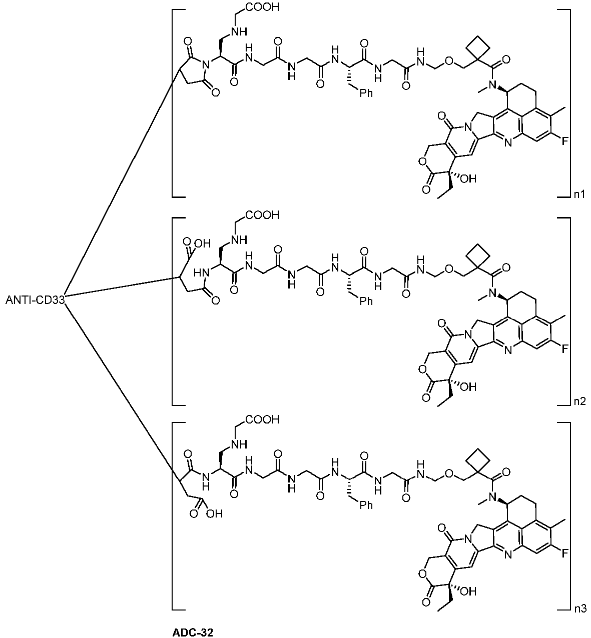

**ADC-32** was prepared according to the generic coupling method of the antibody-drug conjugate in Example 54.

### Example 87: Preparation of ADC-33

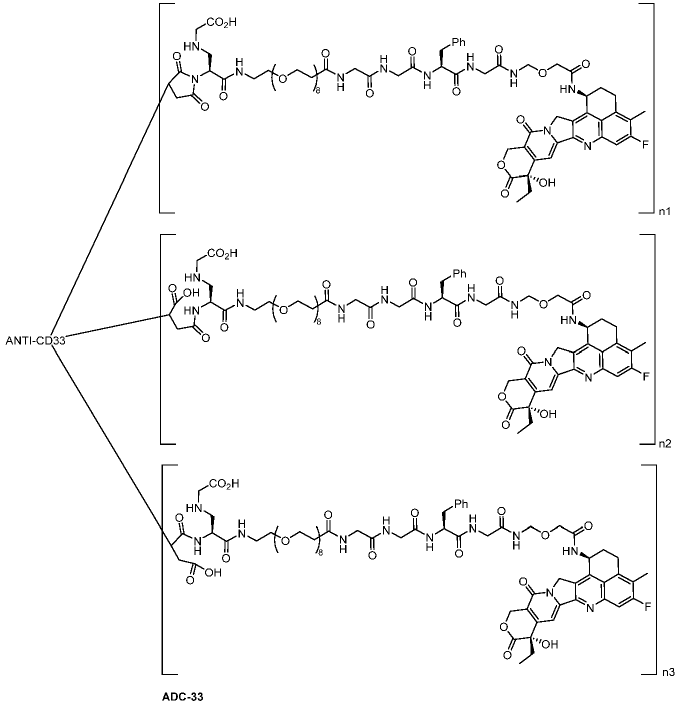

**ADC-33** was prepared according to the generic coupling method of the antibody-drug conjugate in Example 54.

### Example 88: Preparation of ADC-34

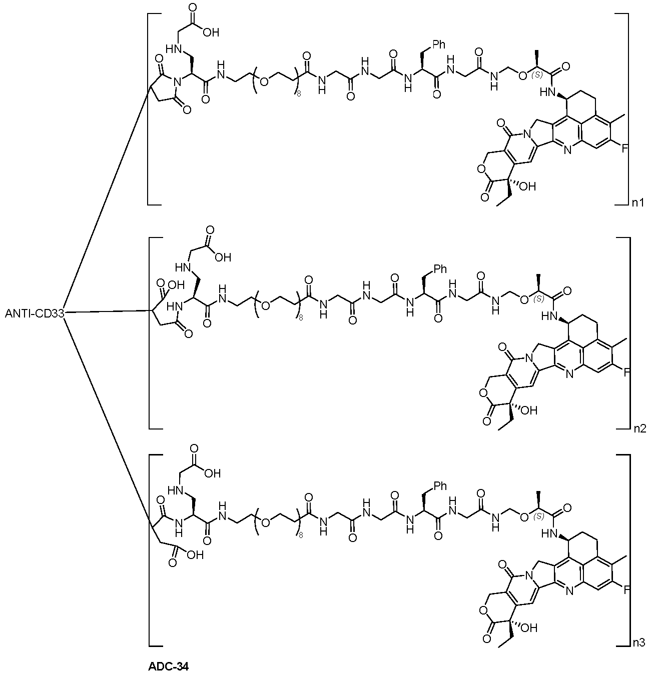

**ADC-34** was prepared according to the generic coupling method of the antibody-drug conjugate in Example 54.

### Example 89: Preparation of ADC-35

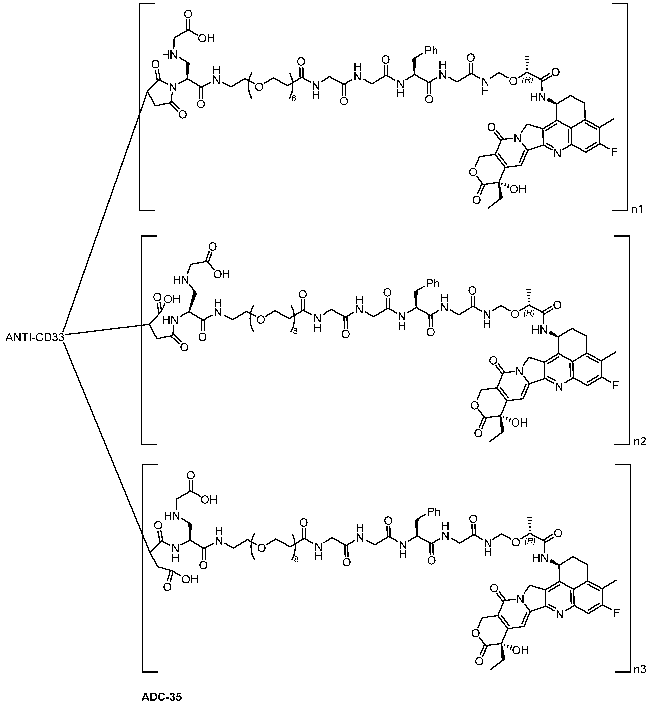

**ADC-35** was prepared according to the generic coupling method of the antibody-drug conjugate in Example 54.

### Example 90: Preparation of ADC-36

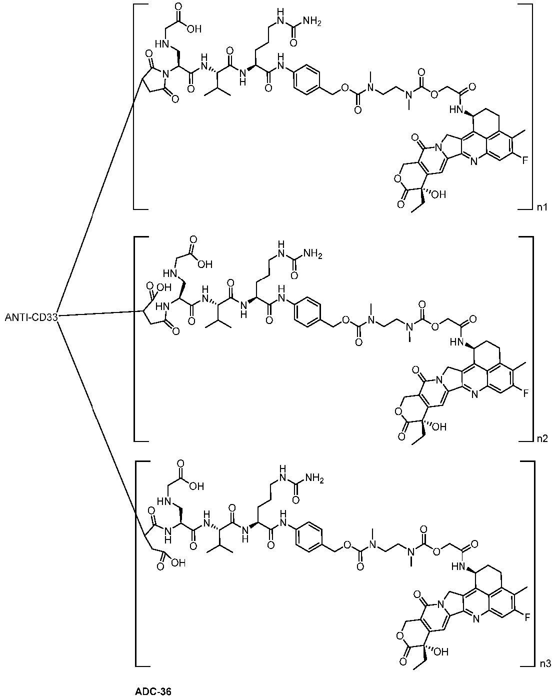

**ADC-36** was prepared according to the generic coupling method of the antibody-drug conjugate in Example 54.

### Example 91: Preparation of ADC-37

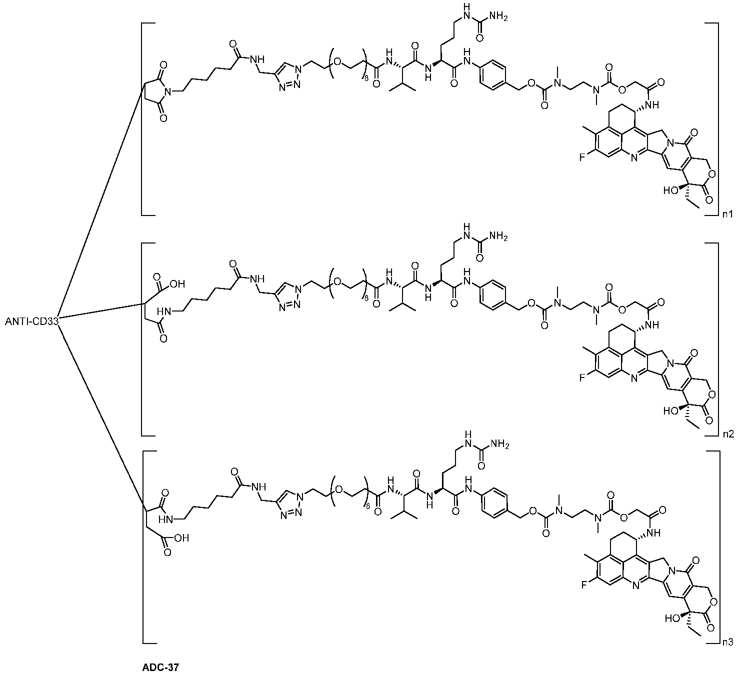

**ADC-37** was prepared according to the generic coupling method of the antibody-drug conjugate in Example 54.

### Example 92: Preparation of ADC-38

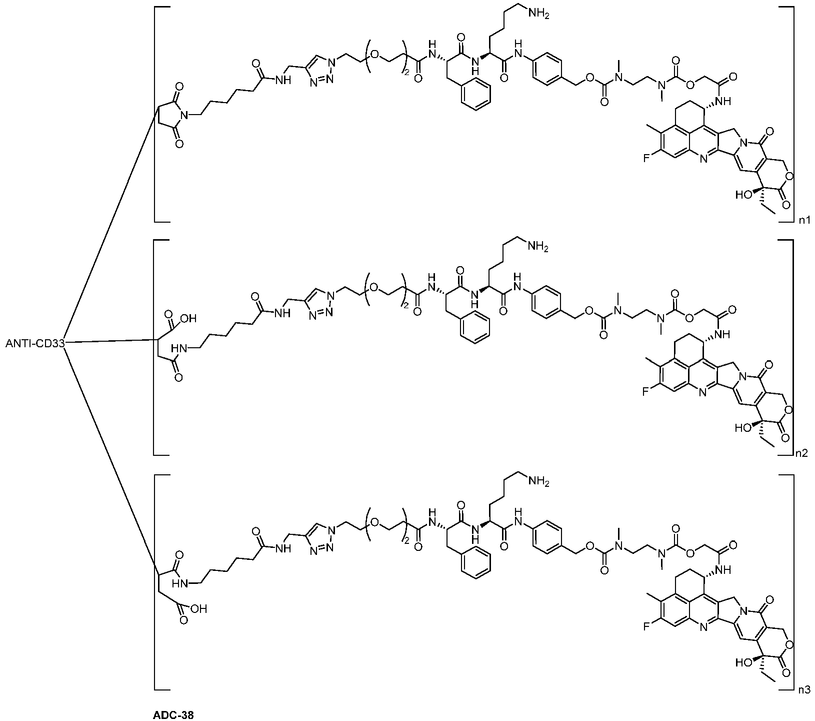

**ADC-38** was prepared according to the generic coupling method of the antibody-drug conjugate in Example 54.

### Example 93: Preparation of ADC-39

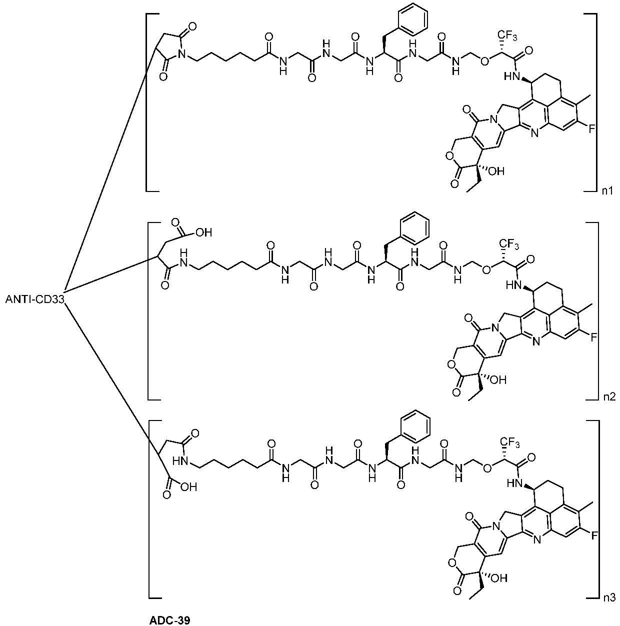

**ADC-39** was prepared according to the generic coupling method of the antibody-drug conjugate in Example 54.

### Example 94: Preparation of ADC-40

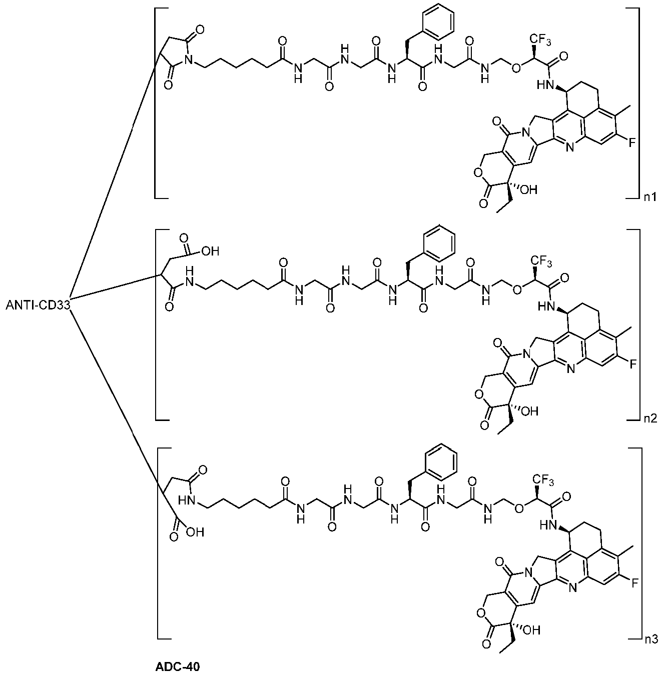

**ADC-40** was prepared according to the generic coupling method of the antibody-drug conjugate in Example 54.

### Example 95: Preparation of ADC-41

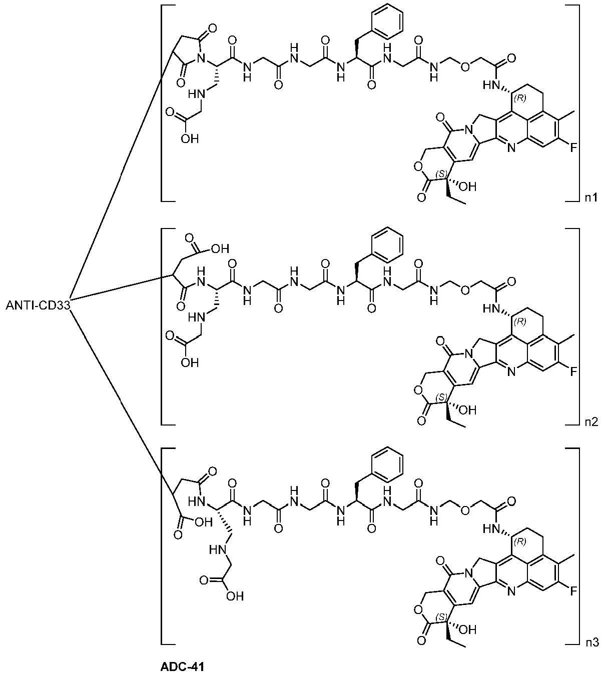

**ADC-41** was prepared according to the generic coupling method of the antibody-drug conjugate in Example 54.

### Example 96: Preparation of ADC-42

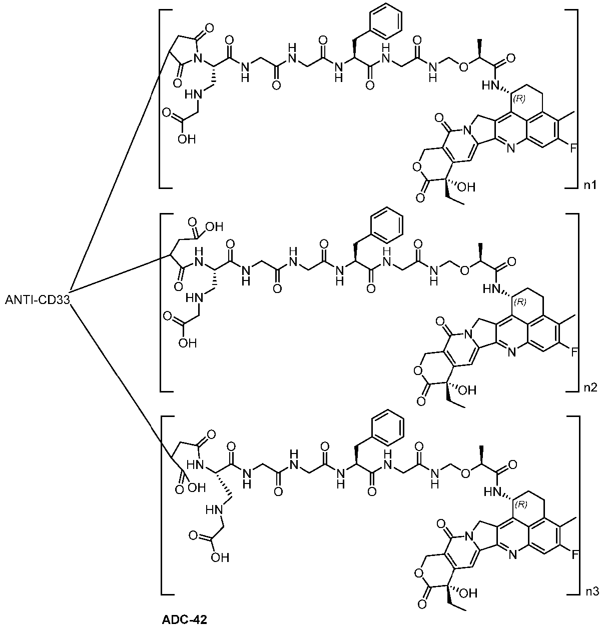

**ADC-42** was prepared according to the generic coupling method of the antibody-drug conjugate in Example 54.

### Example 97: Preparation of ADC-43

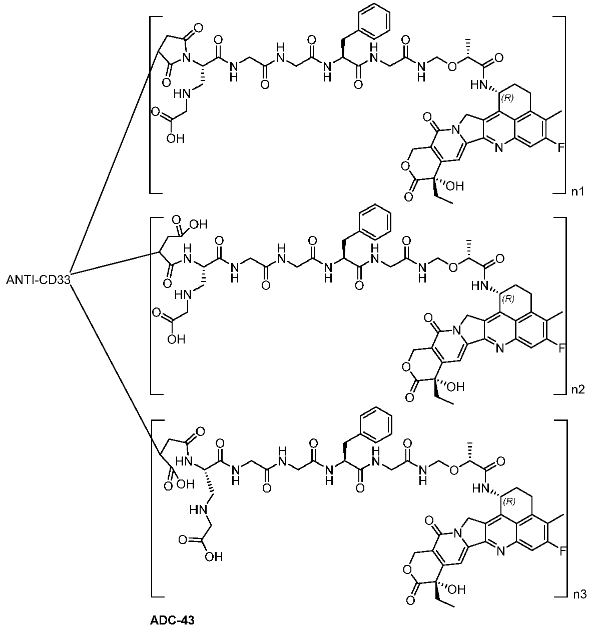

**ADC-43** was prepared according to the generic coupling method of the antibody-drug conjugate in Example 54.

### Example 98: Preparation of ADC-44

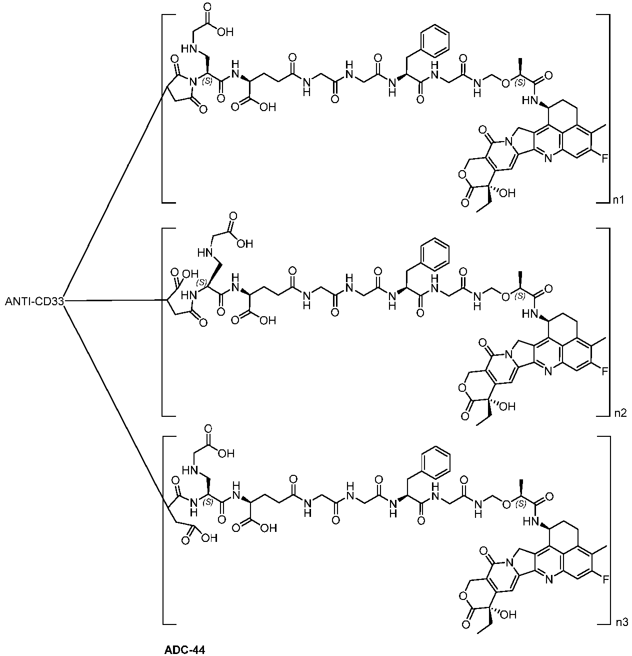

**ADC-44** was prepared according to the generic coupling method of the antibody-drug conjugate in Example 54.

### Example 99: Preparation of ADC-45

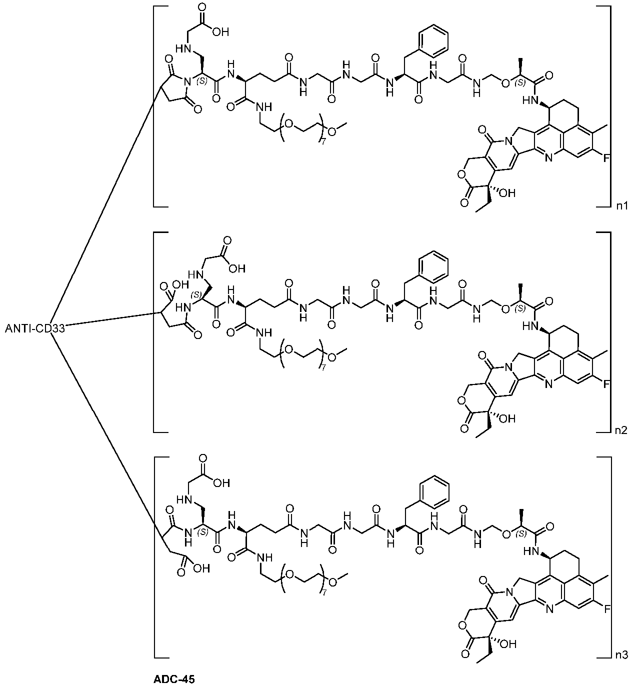

**ADC-45** was prepared according to the generic coupling method of the antibody-drug conjugate in Example 54.

### Example 100: Preparation of ADC-46

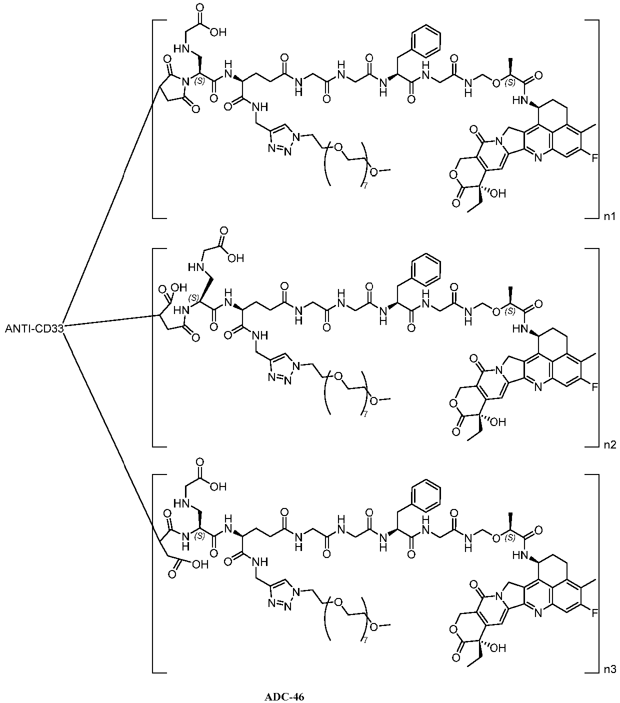

**ADC-46** was prepared according to the generic coupling method of the antibody-drug conjugate in Example 54.

### Example 101: Preparation of ADC-47

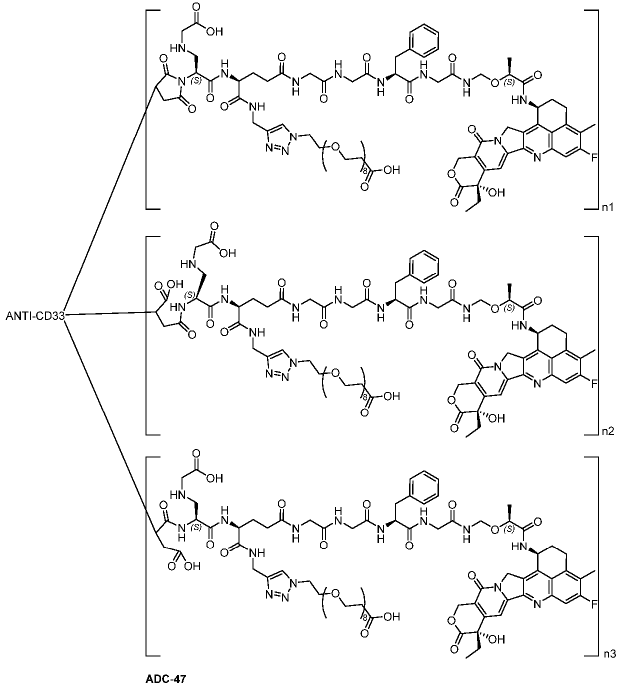

**ADC-47** was prepared according to the generic coupling method of the antibody-drug conjugate in Example 54.

### Example 102: Preparation of ADC-48

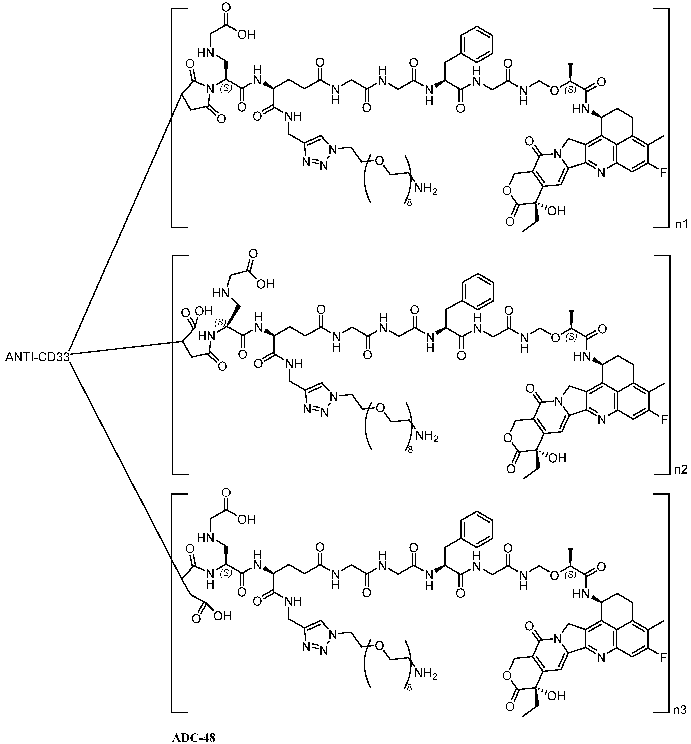

The **ADC-48** was prepared according to the generic coupling method of the antibody-drug conjugate in Example 54.

### Example 103: Preparation of ADC-49(control)

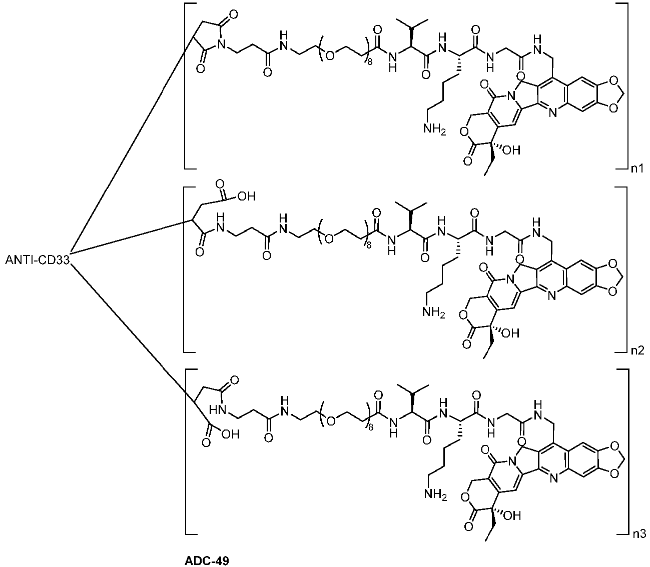

The payload of **ADC-49** was prepared according to the synthetic route of Example 4-3 on page 148 of WO2019195665A1, and **ADC-49** was prepared according to the coupling method of the generic antibody-drug conjugate in Example 54.

### Example 104: Preparation of ADC-50 (control)

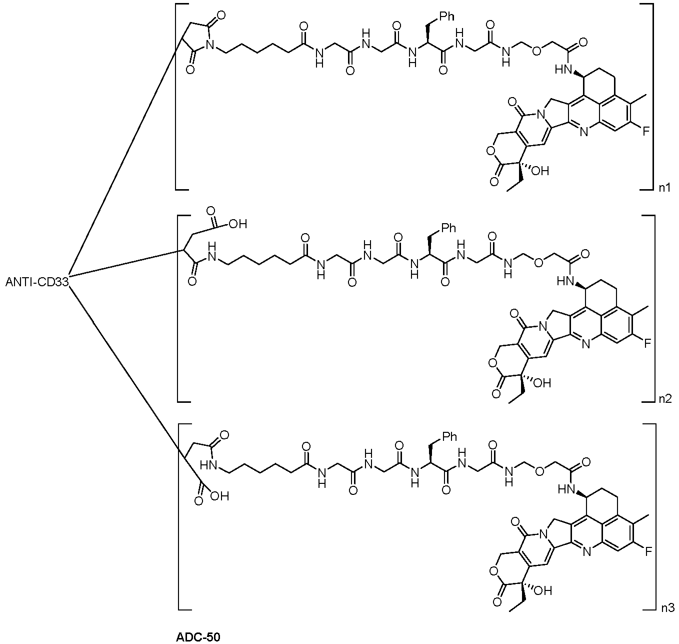

The payload of **ADC-50** was prepared according to the synthetic route of the compound represented by formula (14) on page 47 of CN111065621A, and **ADC-50** was prepared according to the coupling method of the antibody-drug conjugate in Example 54.

### Example 105: Preparation of ADC-51(control)

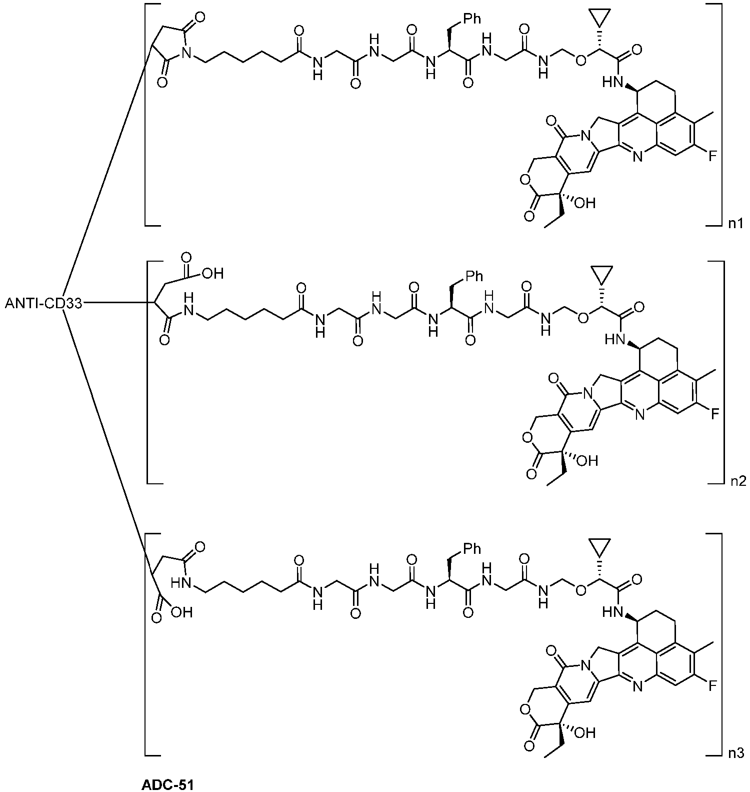

The payload of **ADC-51** was prepared according to the synthetic route of the compound shown in Example 9 on page 58 of WO2020063676A1, and **ADC-51** was prepared according to the coupling method of the general antibody-drug conjugate in Example 54.

### Example 106: Determination of monomer rate by method SEC-HPLC

Determination of monomer rate method: using SEC-HPLC
Column: Biocore SEC-300 5 µm, 4.6×300 mm
Manufacturer: NanoChrom, Item No.: B213-050030-04630S
Mobile phase: 50 mM PB+300 mM NaCl+200 mM Arg+5% IPA, pH=6.5

**Table 1: Methodological parameters**

| Parameters | Set Up |
|---|---|
| Flow Rates | 0.3 mL/min |
| Wavelength | 214 nm and 280 nm |
| Column Temperature | 30 °C |
| Sample Plate Temperature | Indoor Temperature |
| Injection Volume | 20 ug |
| Maximum Pressure | 150 bar/15 MPa/2175 PSI |
| Gradient | Equal Degree |
| Running Time | 20 minutes. |

**Table 2. The data of antibody-drug conjugate (ADC) monomer rate disclosed.**

| Molecule name | Degradation % | Aggregate % | Monomer rate % |
|---|---|---|---|
| **ADC-3** | 0.000 | 0.793 | 99.207 |
| **ADC-34** | 0.000 | 2.804 | 97.196 |
| **ADC-44** | 0.000 | 1.061 | 98.939 |
| **ADC-46** | 0.000 | 7.705 | 92.295 |
| **ADC-47** | 0.000 | 8.448 | 91.552 |
| **ADC-48** | 0.000 | 5.215 | 94.785 |

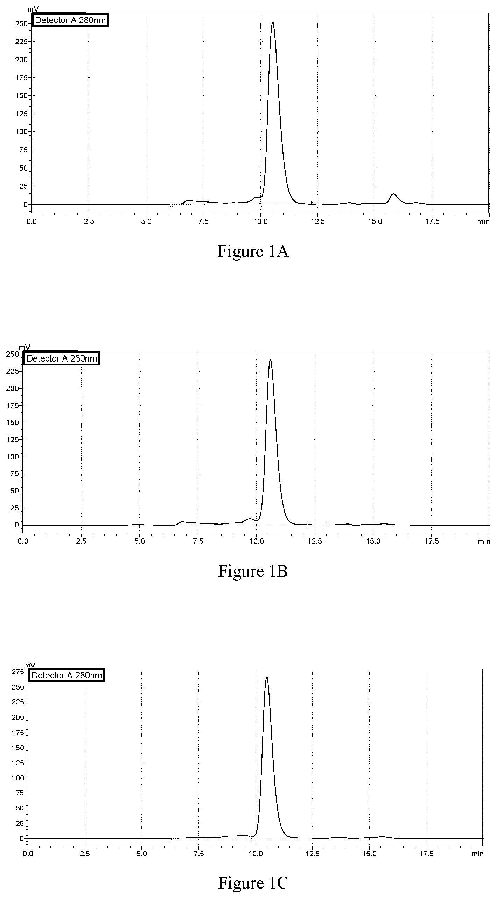

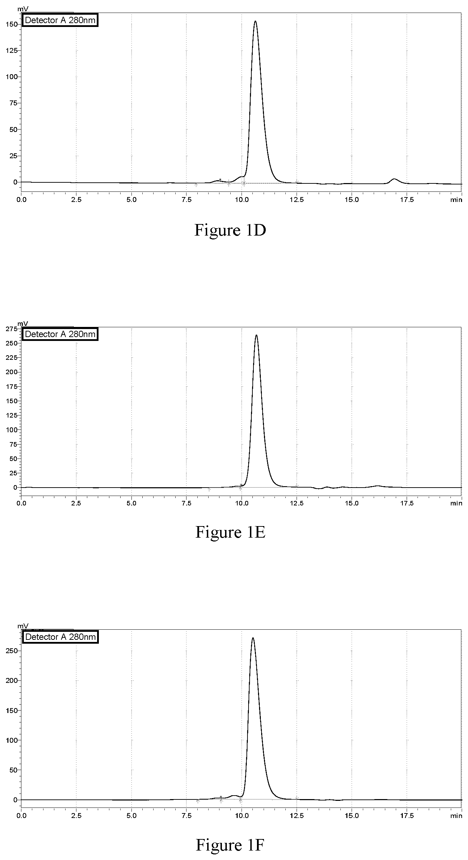

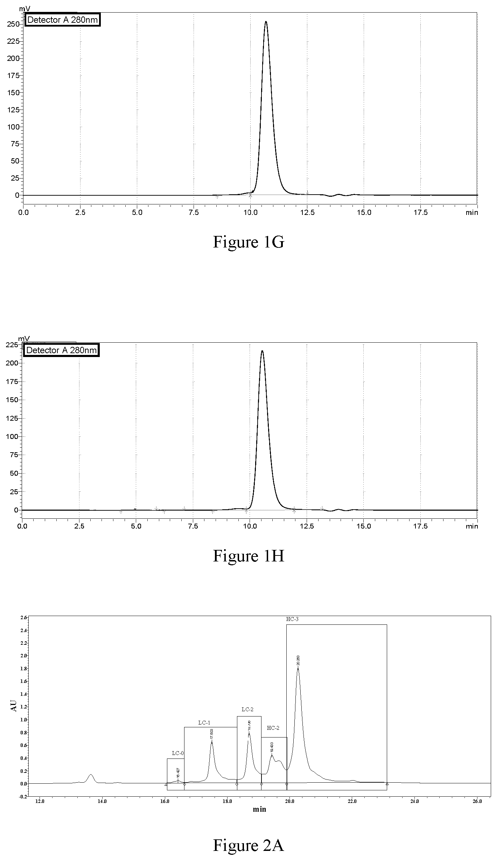

The results are shown by Table 2 and Figures 1A~1H

### CONCLUSION:

The ADC disclosed in the present invention has the characteristics of low degradation rate and low aggregation rate and has the excellent property of high monomer rate.

### Example 107: Determination of Drug-Antibody Ratio (DAR)

Drug-Antibody Ratio (DAR) was determined using RP-HPLC method:
Column name: Proteomix RP-1000 4.6×100mm 5µm 1000A
Manufacturer: Sepax Item No.: 465950-4610

**Table 3: Methodological parameters**

| Parameters | Set Up | | | |
|---|---|---|---|---|
| Mobile Phase | A: 0.1% TFAAqueous Solution; | | | |
| | B: 0.1% TFAAcetonitrile Solution | | | |
| Flow Rates | 0.5 ml/min | | | |
| Wavelength | 214 nm and 280 nm | | | |
| Column Temperature | 65 °C | | | |
| Sample Plate Temperature | Room Temperature | | | |
| Injection Volume | 25 µg | | | |
| Maximum Pressure | 100bar/10MPa/1450PSI | | | |
| Gradient | Time (min) | Flow Rate (ml/min) | Mobile Phase A (%) | Mobile Phase B (%) |
| | 0.0 | 0.5 | 75 | 25 |
| | 3 | 0.5 | 75 | 25 |
| | 28 | 0.5 | 50 | 50 |
| | 30 | 0.5 | 5 | 95 |
| | 32 | 0.5 | 5 | 95 |
| | 33 | 0.5 | 75 | 25 |
| | 40 | 0.5 | 75 | 25 |

**Table 4: Detailed DAR Data of Antibody-Drug Conjugate (ADC)**

| Sample Name | RP-DAR |
|---|---|
| **ADC-3** | 8.66 |
| **ADC-34** | 9.40 |
| **ADC-44** | 8.63 |
| **ADC-46** | 8.56 |
| **ADC-47** | 8.32 |
| **ADC-48** | 8.01 |

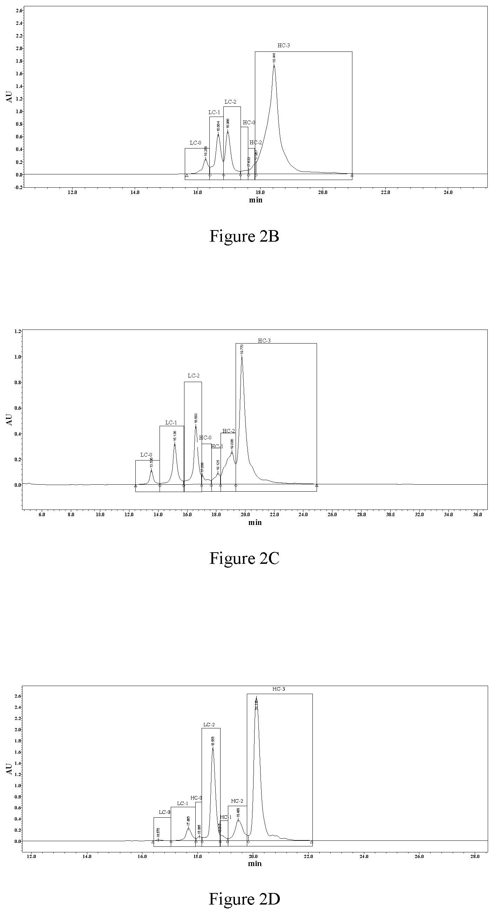

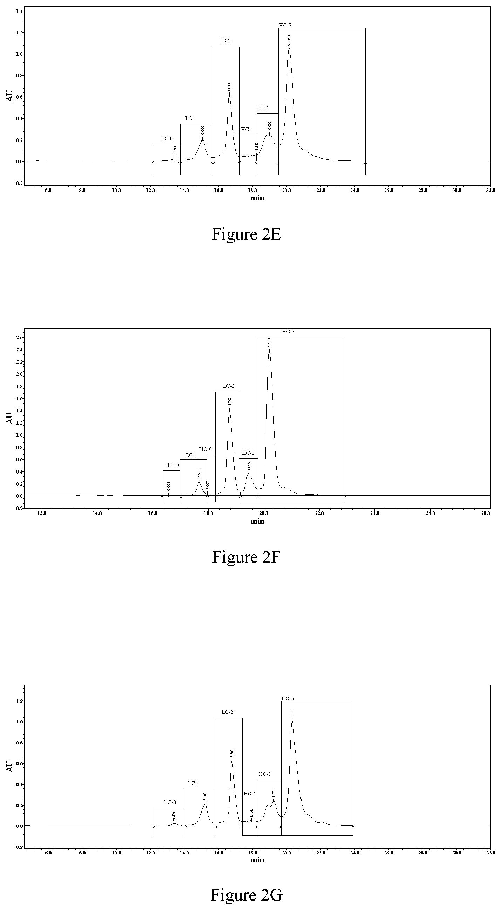

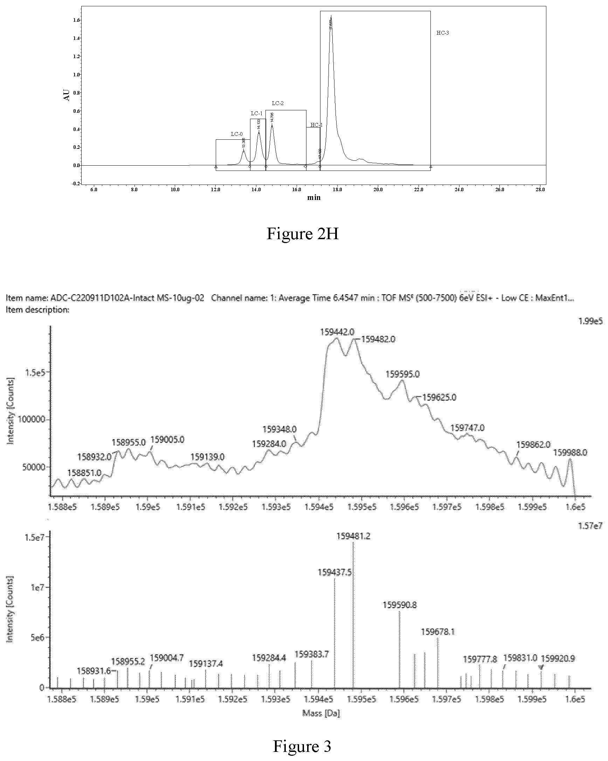

The results are shown in Table 4 and Figures 2A to 2H.
Conclusion: The ADC disclosed in the present invention has the excellent property of high DAR value and can significantly increase the drug concentration at the target site when using the same dosage of ADC drug.

### Example 108: Hydrolysis Analysis of Antibody-Drug Conjugates

The present invention uses molecular weight analysis to characterize the hydrolysis of antibody-drug conjugates.

### 1) Purpose and principle of the experiment:

The purpose of this experiment was to confirm the intact molecular weight of ADC by comparing its LC-MS measured molecular weight and theoretical molecular weight. Liquid chromatography-mass spectrometry (LC-MS) analysis of intact proteins is an important technical means for comparing the theoretical average molecular weight of antibodies with the measured average molecular weight. Any difference between the theoretical value and the measured value of the molecular weight indicates a change in the structure of the antibody-drug conjugate. The payload in the ADC of the present invention easily interacts with water molecules, undergoes a ring-opening reaction, and the molecular weight increases by 18Da. By comparing the theoretical molecular weight of ADC with the measured molecular weight, the degree of hydrolysis and ring-opening of the ADC drug can be confirmed.

### 2) Experimental test conditions:

**Table 5-1 List of Instruments and Equipment**

| Instrumentation | Company | Item Number |
|---|---|---|
| Acquity UPLC H-Class | Waters | INS-Q-147 (equipment code) |
| Xevo G2-XS QTof with UNIFI | Waters | |
| ACQUITY UPLC Protein BEH SEC Column, | Waters | 186005225 |
| 200Å, 1.7µm, 4.6mm×150mm | | |
| Zeba^{™} Desalting Centrifuge Column, 7K MWCO | Thermo Scientific | 89882 |

**Table 5-2 List of Reagents**

| Reagents | Company | Item Number |
|---|---|---|
| Mass spectrometry grade water | Fisher Chemical | W6-4 |
| Ammonium acetate, mass spectrometry grade | Sigma | 73594-25G-F |

### A. Sample preparation

Use Zeba desalting centrifugal columns to desalt and replace the sample with ultrapure water and take 10 µg of each sample and load it onto LC-MS for analysis.

### B. UPLC separation method settinga

Mobile phase: 50 mM ammonium acetate aqueous solution
Column: ACQUITY UPLC Protein BEH SEC Column, 200 Å, 1.7 µm, 4.6 mm×150 mm;
Flow rate: 0.3 mL/min, column temperature: 30°C, detection wavelength: 280 nm

### B. UPLC separation method settings

**Table 5-3 Gradient parameters**

| Time (min) | Flow Rate (mL/min) | Mobile Phase (%) |
|---|---|---|
| 0 | 0.3 | 100 |
| 3.5 | 0.3 | |
| 3.6 | 0.065 | |
| 7.5 | 0.065 | |
| 7.8 | 0.3 | |
| 10.0 | 0.3 | |

### C. Mass spectrometry parameterization

| | |
|---|---|
| Data acquisition: | positive ion mode |
| Capillary voltage: | 3 kV; cone bore voltage: 200V; |
| Ion source temperature: | 150°C; desolvent gas temperature: 500°C; |
| Cone hole gas flow rate: | 50 L/Hr; desolventized gas flow rate: 600 L/Hr; |
| Scan settings: | 3.6-7.8 min for MS analysis; |
| Low mass end (m/z): | 500; high mass end (m/z): 7500; |
| Scan time: 1.000 s; | collision energy: 6 eV; |
| Mass lock: | Leucine enkephalin (m/z 556.2766) |

### D. Data processing: UNIFI software settings

Data analysis was accomplished using Waters Biopharmaceutical Platform Solutions and UNIFI software (version number: V.1.9.4 ).

Data analysis was performed using Waters Biopharmaceutical Platform Solution and UNIFI software (version number: V 1.9.4).
- MaxEnt1
   o Input mass-to-charge ratio range:Antibody: 4800-6300 m/z; ADC: 5100-6500 m/z
   o Output quality range:
      ▪ Antibody: 148000~149000 Da; ADC: 158000~162000 Da
- MaxEnt1 Parameters
   o Output resolution: 1.0 Da
   o Minimum strength ratio (left): 30%; Minimum strength ratio (right): 30%
   o Auto Noise Cancellation on
   o Peak width mode: manual
      - Starting m/z half peak width: 1.0 (antibody or ADC)
      - End m/z half peak width: 1.0 (antibody or ADC)
   o Iterations: 20 (antibody); 15 (ADC)
During data processing, the structure of the theoretical sequence is set as follows:
For the antibody: the disulfide bonds are all bonded according to the theoretical linkage; however, the light chain cysteine fixed insertion sites are free;
For ADC samples: the intra-chain disulfide bonds are all bonded according to the theoretical linkage; inter-chain disulfide bonds corresponding to Cys and light chain cysteine fixed-point insertion sites are free to link the payload;

### 3) Experimental results:

**Table 5: Detailed data on hydrolysis of ADC**

| Name | Observed RT min | Expected Mass (Da) | Observed Mass (Da) | Misalignment (Da) | Hydrolysis |
|---|---|---|---|---|---|
| Ab | 6.30 | 148600.1889 | 148610.174 | +9.99 | / |
| **ADC-3** | 6.45 | 159434.778 | 159437.5384 | +2.76 | 20% |
| | | 159488.8239 | 159481.1591 | -7.66 | 50% |
| | | 159578.9003 | 159590.7599 | +11.86 | 100% |

The data disclosed in Table 5 show that the theoretical molecular weight of naked antibody is 148600.1889 Da, the measured molecular weight is 148610.174 Da, and the deviation is +9.99 Da, which does not exceed the instrument deviation range (20 Da).

When the antibody is conjugated with 10 payloads, its theoretical molecular weight is 159398.757 Da. As shown in Figure 3, the measured molecular weights include 159437.5384 Da, 159481.1591 Da, and 159590.7599 Da. When the succinimide linker of the payload in ADC-3 is hydrolyzed and 2, 5, and 10 succinimides are hydrolyzed, the theoretical molecular weights are 159434.778 Da, 159488.8239 Da, and 159578.9003 Da, respectively (as shown in Table 5), which are close to the measured values, with deviations of +2.76 Da, -7.66 Da, and +11.86 Da, respectively. The deviations are within the error range of the instrument. Therefore, the antibody-drug conjugate is partially hydrolyzed.

### Example 109: Plasma Stability Assay

A mixture of ADC and IgG-free human plasma was prepared to a final concentration of ADC of 0.6 mg/mL and placed in a water bath in a 37°C incubator for incubation. The incubation time was set to 0 days, 3 days, and 7 days. At the same time, unincubated and unextracted controls and unincubated and extracted controls were set. The incubated samples were purified and extracted, and the drug-antibody ratio DAR was measured to reflect the stability of ADC in human plasma.

**Table 6. Plasma stability DAR data of ADCs**

| Sample Name | Unincubated No Extract | Unincubated Extract | Incubate Day 0 | Incubate Day 3 | Incubate Day 7 |
|---|---|---|---|---|---|
| **ADC-3** | 8.66 | 8.81 | 8.66 | 8.54 | 8.42 |
| **ADC-34** | 9.40 | 9.00 | 9.10 | 8.91 | 8.55 |
| **ADC-44** | 8.63 | 8.72 | 8.75 | 8.48 | 8.22 |
| **ADC-46** | 8.56 | 8.46 | 8.49 | 8.46 | 8.32 |
| **ADC-47** | 8.32 | 8.17 | 8.24 | 8.16 | 8.2 |
| **ADC-48** | 8.01 | 7.91 | 7.88 | 7.65 | 7.29 |
| **ADC-50 (control group)** | 9.25 | 8.99 | 8.71 | 8.25 | 7.32 |
| **ADC-51 (control group)** | 8.91 | 9.03 | 8.82 | 7.88 | 7.37 |

| | | | | | |
|---|---|---|---|---|---|
| Note: No incubation and no extraction is the DAR value of the ADC's stability at 4°C for 0 days; No incubation and extraction is the DAR value of the ADC after purification and extraction without incubation with plasma, mainly to see whether extraction has an effect on DAR; Incubation for 0 days refers to the DAR value measured after the purification and extraction steps start after the addition of ADC to plasma; Incubation for 3 days refers to the DAR value measured after the addition of ADC to plasma at 37°C for 3 days followed by the purification and extraction steps; Incubation for 7 days refers to the DAR value measured after the addition of ADC to plasma at 37°C for 7 days followed by the purification and extraction steps; | | | | | |

Conclusion: The ADC disclosed in the present invention has good plasma stability, and there is no significant change in the DAR value. The plasma stability is better than that of the control ADC-50 and ADC-51.

Example 110: ELISA to detect the binding of antibody-drug conjugates to recombinant human CD33 protein

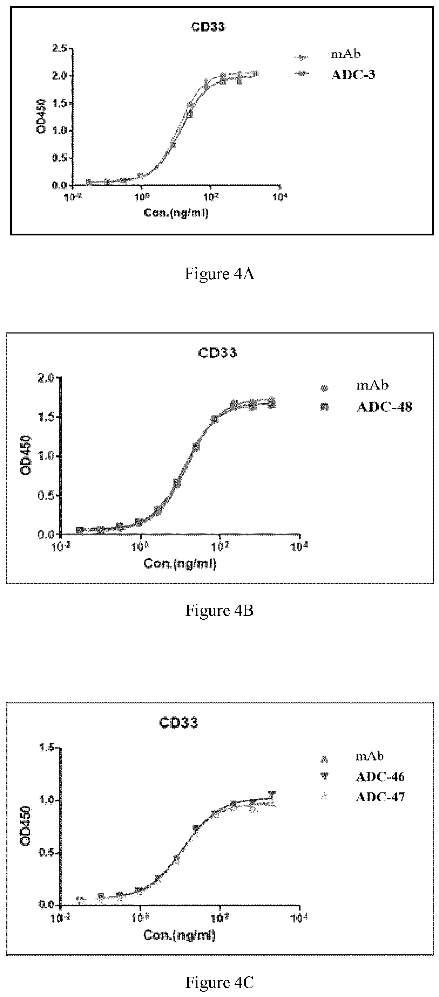

The binding ability of antibodies to antigens can be preliminarily determined by ELISA. In the present invention, the recombinant human CD33 protein was fixed on the enzyme strip, and a series of CD33 antibodies with a concentration gradient were added for detection.
1. Coating: Dilute CD33 to 0.5 µg/mL with 1×PBS, 100 µL per well, and coat overnight at 4°C.
2. Washing: Wash the plate 3 times with washing buffer (1×PBS+0.05%Tween20), 200 µL per well, and let stand for 1 min each time.
3. Blocking: After patting the washing buffer dry for the third time, add blocking buffer (1×PBS+0.05%Tween20+1% BSA), 200 µL per well, and block at 37°C for 1 hour.
4. Washing: Wash the plate 3 times with washing buffer, 200 µL per well, and let stand for 1 min each time.
5. Loading of primary antibody: dilute the antibody-drug conjugate from 2 µg/mL in a 3-fold gradient with sample diluent (1×PBS+0.05%Tween20+1% BSA); wash the plate 3 times with plate washing buffer, 200 µL per well, and let stand for 1 min each time.
6. Loading of secondary antibody: dilute Solarbio's rabbit anti-human antibody 1:10000 to 100 µL per well with analysis buffer (1×PBS+0.05%Tween20+1% BSA), and let stand for 1 hour at 37°C.
7. Washing: Wash the plate 3 times with plate washing buffer, 200 µL per well, and let stand for 1 min each time.
8. Reading: Add TMB colorimetric solution (50 µL per well) and stop the color development after 7 min by adding 2 M H2SO4 (50 µL per well). Read the plate at 450 nm using an ELISA reader.
Conclusion: As shown in Figures 4A, 4B, and 4C, the conjugated **ADC-3, ADC-46, ADC-47, and ADC-48** respectively maintained similar affinity to the naked antibody, and there was no significant difference in EC50 values, indicating that the naked antibody of the present invention does not affect its affinity with the antigen after being conjugated to the toxin. Example 111: *In vitro* efficacy detection of antibody-drug conjugates.

In the present invention, various human tumor cell lines (HL-60, HEL92.1.7, TF-1, MV4-11, MOLM-13, U937 and CMK) were used as experimental models to evaluate the *in vitro* efficacy of ADC drugs. A certain number of tumor cell lines were inoculated in a 96-well plate, and gradient dilutions of the test antibody and the corresponding ADC drug were added to the cells. After treatment for 5 days, Alamar Blue or MTS was used to detect cell viability, and the inhibitory effect of the test antibody and ADC on the tumor cell line was evaluated by calculating IC50. The initial concentration of the antibody drug was 500 nM, the dilution multiple was 7 times, a total of 8 concentration points, and the treatment lasted for 5 days. The final algorithm was based on the survival rate = (experimental group-blank)/(control group-blank group) × 100%, and then the half-maximal inhibitory concentration (IC50) was calculated using Graph Pad Prism to fit the curve.

**Table 7: In vitro efficacy results of the anti-CD33 naked antibody, ADC-3, ADC-47 and ADC-48 in human tumor cell lines HL-60, HEL92.1.7, TF-1, MV4-11, MOLM-13, U937 and CMK:**

| **Group** | **Name** | **IC50 (nM)** |
|---|---|---|
| HL-60 | anti-CD33 | > 500 |
| | ADC-3 | 2.01 |
| | ADC-47 | 2.76 |
| | ADC-48 | 20.48 |
| HEL92.1.7 | anti-CD33 | > 500 |
| | ADC-3 | 1.30 |
| | ADC-47 | 1.31 |
| | ADC-48 | 20.20 |
| TF-1 | anti-CD33 | > 500 |
| | ADC-3 | 0.11 |
| | ADC-47 | 0.13 |
| | ADC-48 | 0.75 |
| MV4-11 | anti-CD33 | > 500 |
| | ADC-3 | 0.25 |
| | ADC-47 | 0.49 |
| | ADC-48 | 0.84 |
| MOLM-13 | anti-CD33 | > 500 |
| | ADC-3 | 0.03 |
| | ADC-47 | 0.13 |
| | ADC-48 | 0.06 |
| U937 | anti-CD33 | > 500 |
| | ADC-3 | 4.16 |
| | ADC-47 | 7.64 |
| | ADC-48 | 176.88 |
| CMK | anti-CD33 | > 500 |
| | ADC-3 | 3.51 |
| | ADC-47 | 7.05 |
| | ADC-48 | 60.12 |

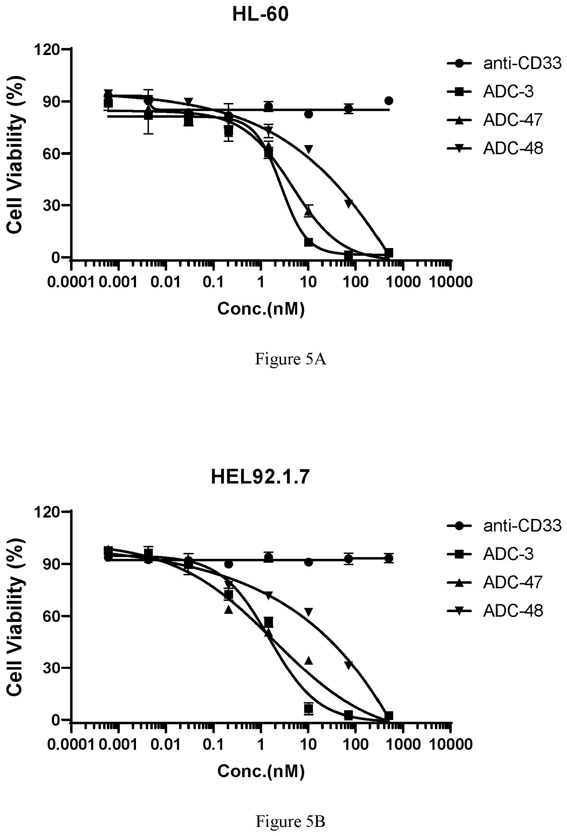

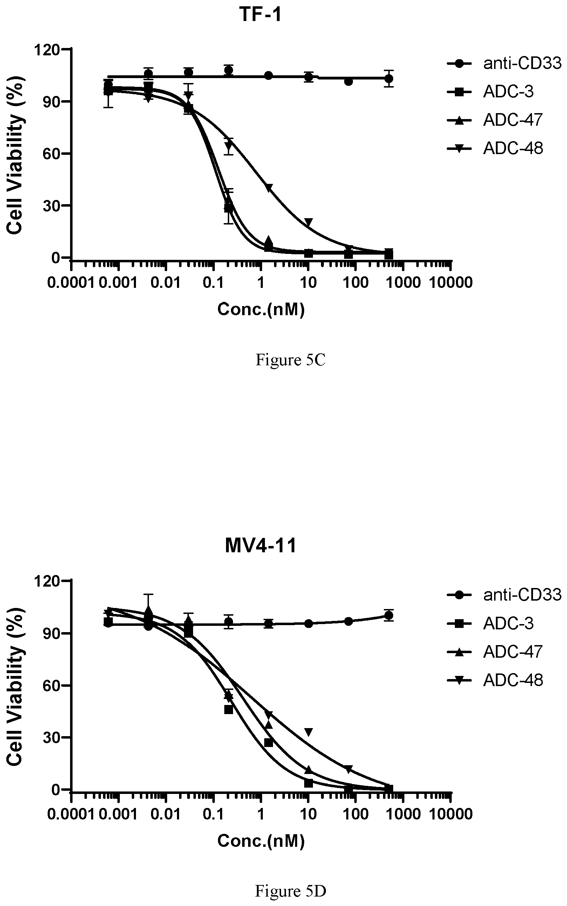

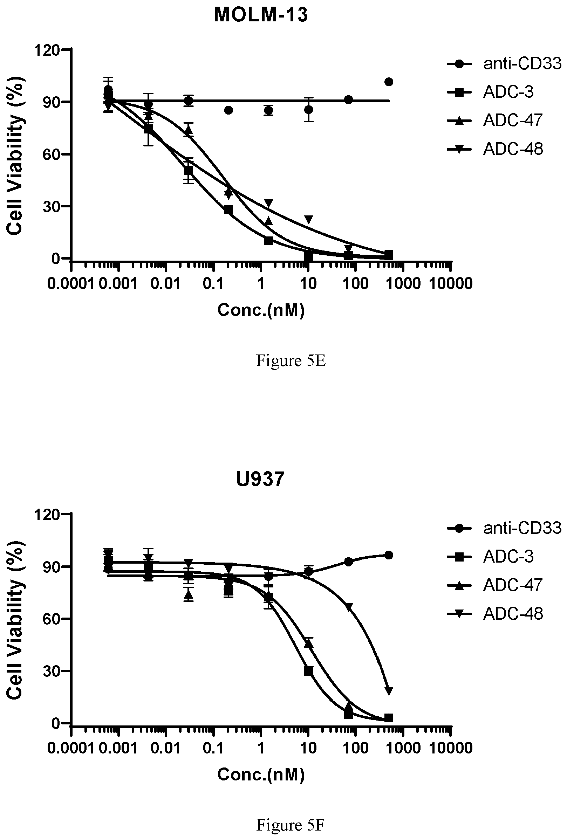

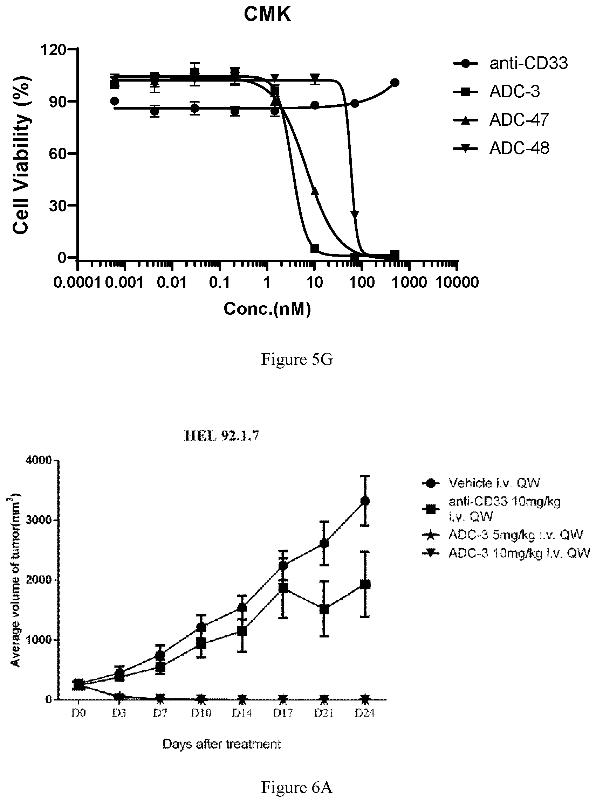

The results are shown in Table 7 and Figures 5A to 5G.

Conclusion: In HL-60, HEL92.1.7, TF-1, MV4-11, MOLM-13, U937 and CMK cell models, naked anti-CD33 had no tumor cell killing activity; ADC-3 had the best effect in multiple myeloid tumor cells.

### Example 112: In vivo efficacy test of antibody-drug conjugates

The present invention established a subcutaneous transplant tumor model of human erythroleukemia cells HEL92.1.7 in BALB/c Nude mice to evaluate the *in vivo* efficacy of ADC drugs, anti-CD33 mab and ADC-3. 5×106 HEL92.1.7 cells (0.1mL/mouse) were subcutaneously injected into the right shoulder blade of BALB/c Nude mice aged 5 to 6 weeks. When the average tumor size of the mice grew to about 230 to 270 mm3, they were randomly divided into a vehicle control group (Vehicle), an anti-CD33 mab treatment group (10 mg/kg), and an ADC-3 treatment group (5 mg/kg, 10 mg/kg), with 5 mice in each group, and drug administration began (D0). Each group was administered with a tail vein injection of 10 mL/kg body weight, once a week, for 4 consecutive weeks (QWx4). All groups observed that on the 24th day (D24) after group administration (Table 8, Figure 6A), the data were shown as the average tumor volume ± SE at the time of measurement. Anti-CD33 was administered at a dose of 10 mg/kg, and ADC-3 was administered at a dose of 5 mg/kg and 10 mg/kg respectively by tail vein administration (QWx4), and the tumor inhibition effect was significant.

**Table 8 Efficacy analysis of each group in the subcutaneous transplant tumor model of human erythroleukemia cells HEL92.1.7 in BALB/c-Nude mice**

| Experimental Group | Day 0 after administration | Day 24 after administration | | | | |
|---|---|---|---|---|---|---|
| | Mean tumor volume (x ±S) mm³ | Mean tumor volume (x ±S) mm³ | Relative tumor volume (x ±S) | TGI (%) | T/C (%) | P value |
| Group 1 Vehicle | 269.92 ± 68.00 | 3326.407 ± 375.15 | 19.25 ± 5.33 | - | - | - |
| Group 2 anti-CD33 (10 mg/kg) | 242.24 ± 48.81 | 1934.62 ± 483.76 | 10.12 ± 2.22 | 35.19 | 64.81 | 0.034 |
| Group 3 **ADC-3** (5 mg/kg) | 244.91 ± 61.35 | 0.00 ± 0.00 | 0.00 ± 0.00 | 100.00 | 0.00 | <0.001 |
| Group 4 **ADC-3** (10 mg/kg) | 256.14 ± 61.73 | 0.00 ± 0.00 | 0.00 ± 0.00 | 100.00 | 0.00 | <0.001 |

| | | | | | | |
|---|---|---|---|---|---|---|
| Note: 1. Data are expressed as "mean ± standard error"; | | | | | | |

2. T/C % = T_{RTV} / C_{RTV} × 100%; TGI% = (1-T/C) × 100
*3. P* value is obtained by comparing the tumor volume of each treatment group with the vehicle control group on day 24 (24 days after administration). (Analyzed by Graphpad prism 6, when the *P* value of one-way ANOVA analysis is less than 0.05, Tukey's multiple tests is used for pairwise comparisons between all groups, and the *P* value less than 0.05 is considered statistically significant). The comparison results between the groups are as follows:

| **Test** | ***P* value** | **Significance** | **Test** | ***P* values** | **Significance** |
|---|---|---|---|---|---|
| G1 vs. G2 | 0.034 | * | G1 vs. G3 | <0.001 | *** |
| G1 vs. G4 | <0.001 | *** | G2 vs. G3 | 0.002 | ** |
| G2 vs. G4 | 0.002 | ** | G3 vs. G4 | >0.999 | ns |

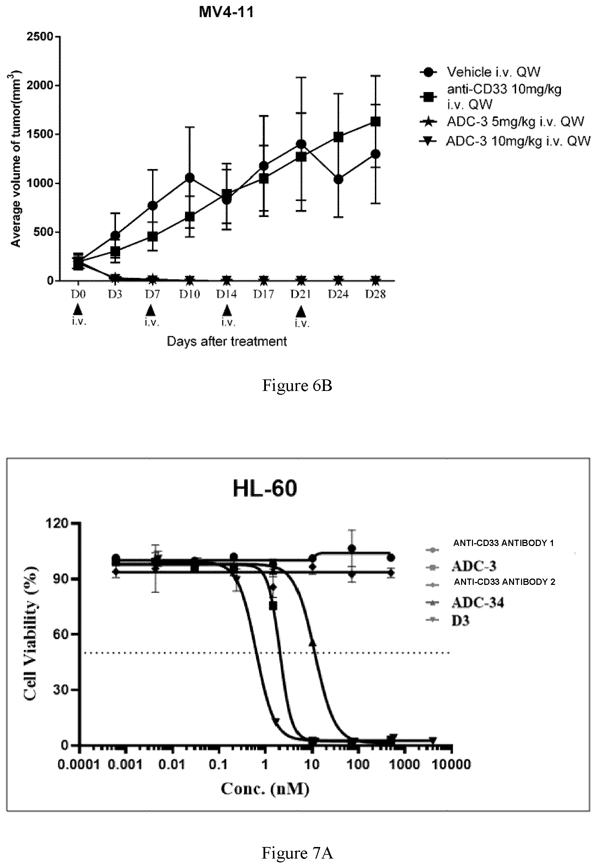

The present invention establishes a human myeloid monocytic leukemia cell MV-4-11 subcutaneous transplant tumor model in BALB/c Nude mice to evaluate the *in vivo* efficacy of ADC drugs, anti-CD33 mAb and ADC-3. 3.08×106 MV4-11 cells (0.1mL/mouse) were subcutaneously injected into the right shoulder of BALB/c Nude mice aged 5 to 6 weeks. When the average tumor size of the mice grew to about 180~200 mm3, they were randomly divided into a vehicle control group (Vehicle), an anti-CD33 mab treatment group (10 mg/kg), and an ADC-3 treatment group (5 mg/kg, 10 mg/kg), 5 mice in each group, and medication was started (D0). Each group was administered by tail vein injection at 10 mL/kg body weight, once a week, for 4 consecutive weeks (QWx4). All groups observed that on the 28th day (D28) after group administration (Table 9, Figure 6B), the data were shown as the average tumor volume ± SE at the time of measurement. Anti-CD33 was administered at a dose of 10 mg/kg, and ADC-3 was administered at a dose of 5 mg/kg and 10 mg/kg respectively through the tail vein (QWx4), and the tumor inhibition effect was significant.

**Table 9 Efficacy analysis of each group in the subcutaneous transplant tumor model of human myelomonocytic leukemia cell MV4-11 in BALB/c-Nude mice**

| Experimental Group | Day 0 after administration | Day 28 after administration | | | | |
|---|---|---|---|---|---|---|
| | Mean tumor volume (*x̅* ±S) mm³ | Mean tumor volume (*x̅* ±S) mm³ | Relative tumor volume (*x̅* ±S) | TGI (%) | T/C (%) | P value |
| Group 1 Vehicle | 199.77 ± 81.86 | 1299.85 ± 390.87 | 13.94 ± 5.90 | - | - | - |
| Group 2 anti-CD33 mab (10 mg/kg) | 197.05 ± 81.40 | 1630.90 ± 469.04 | 10.70 ± 3.62 | -27.20 | 127.20 | 0.896 |
| Group 3 **ADC-3** (5 mg/kg) | 181.36 ± 53.04 | 0.00 ± 0.00 | 0.00 ± 0.00 | 100.00 | 0.00 | 0.068 |
| Group 4 **ADC-3** (10 mg/kg) | 200.25 ± 65.51 | 0.00 ± 0.00 | 0.00 ± 0.00 | 100.00 | 0.00 | 0.068 |

| | | | | | | |
|---|---|---|---|---|---|---|
| Note: 1. Data are expressed as "mean ± standard error"; | | | | | | |

2. T/C % = T_{RTV} / C_{RTV} × 100%; TGI% = (1-T/C) × 100
*3. P* value is obtained by comparing the tumor volume of each treatment group with the vehicle control group on day 24 (24 days after administration). (Analyzed by Graphpad prism 6, when the *P* value of one-way ANOVA analysis is less than 0.05, Tukey's multiple tests is used for pairwise comparisons between all groups, and the *P* value less than 0.05 is considered statistically significant). The comparison results between the groups are as follows:

| **Test** | ***P* value** | **Significance** | **Test** | ***P* value** | **Significance** |
|---|---|---|---|---|---|
| G1 vs. G2 | 0.896 | ns | G1 vs. G3 | 0.068 | ns |
| G1 vs. G4 | 0.068 | ns | G2 vs. G3 | 0.006 | ** |
| G2 vs. G4 | 0.006 | ** | G3 vs. G4 | >0.999 | ns |

### Example 113: In vitro efficacy of compound P3

### 1) Experimental materials:

Cells: The test cells were from the cell bank of the Chinese Academy of Sciences;
Cell culture medium DMEM: Gibco;
FBS: BIOWEST.

### 2) Preparation of culture medium:

Growth medium (with 10% FBS, Penicillin/streptomycin (100U/mL);
Detection medium (with 1% FBS, Penicillin/streptomycin (100U/mL).

### 3) Operation:

Turn on the UV light of the biosafety cabinet 30 minutes in advance and then open the ventilation for 3 minutes. Preheat the growth medium, detection medium, D-PBS and trypsin in a 37 °C constant temperature water bath, then disinfect the surface with alcohol and put it in the biosafety cabinet. Place cells with a confluence rate of about 80% in the biosafety cabinet, remove the old culture medium, rinse with D-PBS, discard, digest with trypsin for 2 to 3 minutes, then add growth medium to neutralize, and centrifuge at 1200 rpm for 3 minutes. Remove the supernatant from the centrifugation, mix with 4 mL detection medium, take 100 uL for counting (take 50 uL of cell fluid and add 50 uL Trypan Blue Stain and mix well, count after mixing). Plate the cells according to the pre-optimized cell plating density, 80ul/well in a 96-well plate, add only 80 uL of detection medium to wells E11 and F11, and add 150 uL of DPBS to the edge wells. Add diluted antibodies 24 hours after plating, 20 uL per well and set controls, only 20 uL of detection medium is added to the 11th column, and 2 replicates are set for each concentration. After adding, mix on a cell vortex shaker at 550 rpm for 3 min.

Dilution of solution: Use detection medium to prepare a 5 uM starting concentration, 300 uL of test solution in the first column of a V-shaped 96-well plate, and add 240 uL of detection medium to the second to tenth columns respectively. Take 60 uL from the first column and add it to the second column. Mix up and down 10 times with a spray gun, discard the gun tip, and operate the next 7 concentrations in sequence

### 4) Detection:

After 4 days, take out the MTS reagent, thaw at room temperature and avoid light, vortex thoroughly to mix, and then add 20 µL of CellTiter One Solution Reagen MTS reagent for every 100 µL of cell culture volume along the side wall of the well in the biosafety cabinet. Gently tap the plate surface to mix the MTS solution evenly and then place it in a cell culture incubator and incubate for 2 hours in the dark. After the reaction is completed, take out the 96-well plate, detect the OD490 nm absorbance value in the microplate reader, and record, organize, analyze and store the data.

### 5) Results:

Table 10 *In vitro* inhibitory effects of compound **P3** on human non-small cell lung adenocarcinoma cells H1975, human non-small cell lung cancer cells HCC827, human epidermal carcinoma cells A431, human gastric cancer cells NCI-N87, human pancreatic adenocarcinoma cells BXPC-3, human epidermal carcinoma cells A431+human colon cancer cells SW620, human breast cancer cells ZR-75-1, human plasma cell leukemia cells H929, human multiple myeloma cells RPMI8226, human leukemia cells JJN-3, human breast cancer cells MDA-MB-361, and human breast cancer cells SK-BR-3.

| **Group** | **IC50 (nM)** | **Efficacy%** |
|---|---|---|
| H1975 | 970.33 | 76.78 |
| HCC827 | 655.60 | 80.57 |
| A431 | 438.77 | 84.97 |
| NCI-N87 | 1023.92 | 63.93 |
| BxPC-3 | 320.09 | 64.4 |
| A431+SW620 | 604.87 | 68.26 |
| ZR-75-1 | 557.95 | 54.79 |
| H929 | 23.94 | 99.03 |
| RPMI8226 | 147.00 | 93.61 |
| JJN-3 | 36.11 | 80.59 |
| MDA-MB-361 | 572.13 | 62.34 |
| SK-BR-3 | 732.43 | 70.9 |

Table 10 shows that compound **P3** has a good inhibitory effect on both solid tumor cells and hematological tumor cells.

### Example 114: In vitro efficacy detection of naked antibodies, small molecule drugs and antibody-drug conjugates (ADCs)

The *in vitro* efficacy of naked antibodies, small molecule drugs and ADC conjugates was evaluated using three human tumor cell lines (HL-60, JVM-3, Raji) experimental models.

A certain number of tumor cells were inoculated in a 96-well plate, and gradient dilutions of small molecule drugs, test antibodies and corresponding ADC drugs were added to the cells for 5 days. Cell viability was detected using Alamar Blue or MTS, and the inhibitory effect of the test drugs on tumor cell lines was evaluated by calculating IC50. The initial concentration of the antibody drug was 500 nM, the dilution multiple was 7 times, a total of 8 concentration points, and the treatment lasted for 5 days. The final algorithm was based on the survival rate = (experimental group-blank)/(control group-blank group) × 100%, and then the half-maximal inhibitory concentration (IC50) was calculated using Graph Pad Prism to fit the curve.

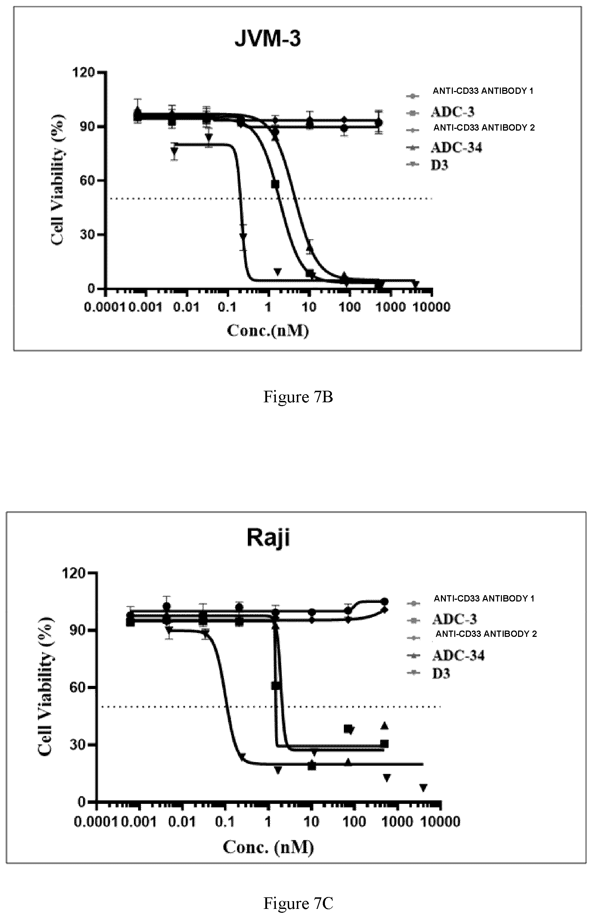

The results are shown in Table 11 and Figures 7A-7C.

**Table 11 In vitro efficacy testing of naked antibodies, small molecule drugs and antibody-drug conjugates (ADCs):**

| **Groups** | HL-60 | | JVM-3 | | Raji | |
|---|---|---|---|---|---|---|
| | IC₅₀ (nM) | Efficacy% | IC₅₀ (nM) | Efficacy% | IC₅₀ (nM) | Efficacy% |
| Anti-CD33 antibody1 | >500 | N/A | >500 | N/A | >500 | N/A |
| **ADC-3** | 2.056 | 95.34 | 1.876 | 91.13 | 1.456 | 65.44 |
| Anti-CD33 antibody2 | >500 | N/A | >500 | N/A | >500 | N/A |
| **ADC-34** | 11.480 | 96.79 | 4.358 | 92.07 | 1.933 | 70.24 |
| D3 | 0.630 | 96.65 | 0.215 | 75.47 | 0.100 | 69.86 |

Conclusion: The small molecule drugs and antibody-drug conjugates (ADCs) prepared in the present invention showed excellent tumor inhibitory activity in the experimental models of three human tumor cell lines (HL-60, JVM-3, and Raji).

## Claims

1. An antibody-drug conjugate of general formula I or its stereoisomer, pharmaceutically acceptable salt or solvate thereof, 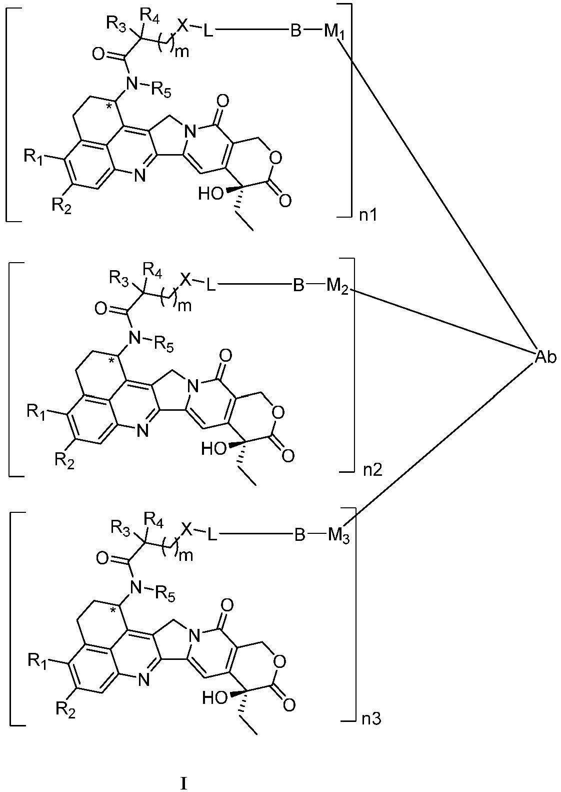 wherein:
the drug in the antibody-drug conjugate is a camptothecin antitumor drug or a stereoisomer thereof, as shown in formula **II** below 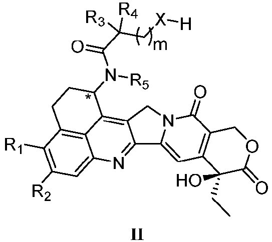
Ab comprises an anti-CD33 antibody or an antigen-binding fragment thereof;
M₁, M₂, and M₃ are each independently selected from 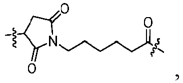 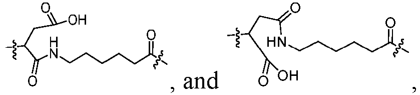
and M₁, M₂, and M₃ are not identical to each other; or M₁, M₂, and M₃ are each independently selected from the structure represented by formula A₁, formula A₂, and formula A₃, and M₁, M₂, and M₃ are different from each other; 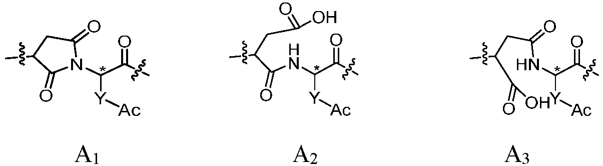
wherein Y is a scaffold selected from C1-C6 alkyl, substituted C1-C6 alkyl, or C3-C8 cycloalkyl; preferably Y is C1-C6 alkyl; Ac is a hydrophilic structural unit; and the position indicated by the wavy line on the left is connected to Ab, and the position indicated by the wavy line on the right is connected to B or L;
B is present or absent, and, when present, B is a modifier unit;
L is a linker unit, preferably a peptide-containing linker unit;
X is selected from -NH-, -O- and -S-, preferably -O-;
R₁ and R₂ are the same or different and are each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, 5-10-membered heteroaryl, and substituted 5-10-membered heteroaryl;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, C6- C10 aryl C1-C6 alkyl, 5-10-membered heteroaryl, and substituted 5-10-membered heteroaryl; preferably R₃, R₄ are each independently selected from hydrogen, C1-C6 alkyl, substituted C1-C6 alkyl, C3-C8 cycloalkyl, C3-C8 cycloalkyl C1-C6 alkyl, and C6-C10 aryl C1-C6 alkyl;
alternatively, R₃, R₄ and the carbon atoms to which they are attached constitute a C3-C8 cycloalkyl group, a 3-7-membered heterocyclic group, a substituted 3-7-membered heterocyclic group; preferably R₃, R₄ and the carbon atoms to which they are attached constitute a C3-C8 cycloalkyl group;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl; preferably, R₅ is selected from hydrogen, and C1-C6 alkyl;
m is an integer from 0-5;
n1, n2, and n3 are each independently an integer or a decimal from 0 to 10, n1, n2, and n3 are not simultaneously 0, and 1 ≤ n1 + n2 + n3 ≤ 10;
the chiral carbon atom indicated at position * has an R or S absolute configuration; and the following conditions are met, when M₁, M₂, and M₃ are each independently selected from 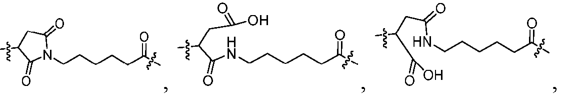 M₁, M₂, and M₃ are not identical to each other; B is present and is a modifier unit.

2. The antibody-drug conjugate or its stereoisomer, pharmaceutically acceptable salt or solvate thereof according to claim 1, wherein M₁, M₂, M₃ are each independently selected from 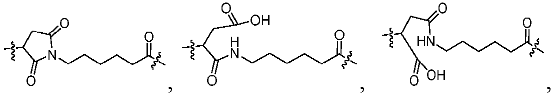 and M₁, M₂, M₃ are not identical to each other;
alternatively, M₁, M₂, M₃ are each independently selected from the structure represented by formula A₁, formula A₂, formula A₃, and M₁, M₂, and M₃ are not identical to each other;
preferably, M₁, M₂, M₃ are each independently selected from the structures shown in formula A₁, formula A₂, formula A₃, and M₁, M₂, M₃ are not identical to each other; 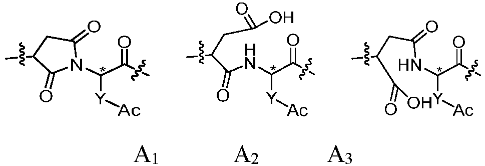
wherein Y is a scaffold selected from C1-C6 alkyl, substituted C1-C6 alkyl, and C3-C8 cycloalkyl; preferably Y is C1-C6 alkyl, more preferably C1-C3 alkyl, and most preferably methylene;
the chiral carbon atom indicated at position * has an R or S absolute configuration;
the position indicated by the left wavy line is connected to Ab, and the position shown by the right wavy line is connected to B or L;
the hydrophilic structural unit Ac comprises a natural or non-natural amino acid, 1-20 polyethylene glycol, phosphate group, carboxylic acid group, sulfonic acid group, sulfinic acid group, or the following structures: 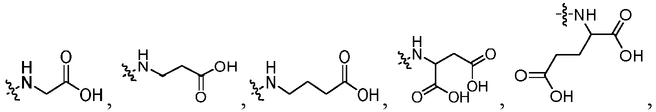 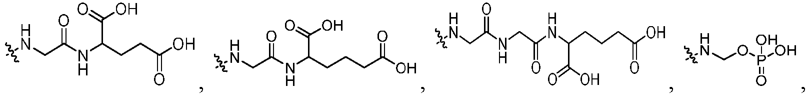 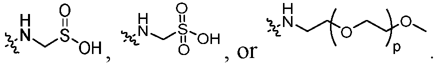
wherein p is an integer from 0 to 10; the position indicated by the wavy line is connected to the bracket Y;
preferably, the hydrophilic structural unit Ac is selected from 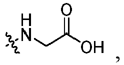 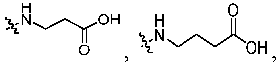
and is connected to the bracket Y at the position indicated by the wavy line;
more preferably, the hydrophilic structural unit Ac is 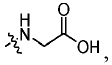 connected to the bracket Y at the position indicated by the wavy line.

3. The antibody-drug conjugate or its stereoisomer, pharmaceutically acceptable salt or solvate thereof according to Claim 1 or 2, wherein M₁, M₂, M₃ are selected from any one of the following combinations (1)-(7), and M₁, M₂, M₃ are not identical to each other:
(1) 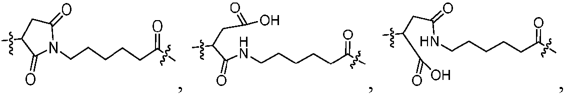
(2) 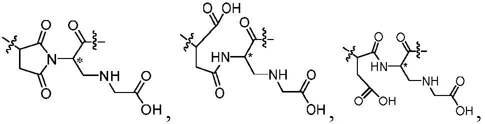
(3) 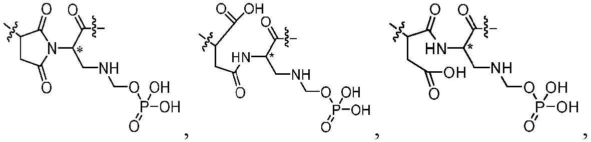
(4) 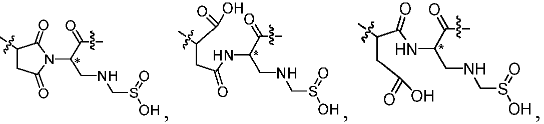
(5) 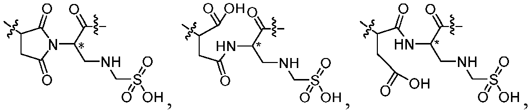
(6) 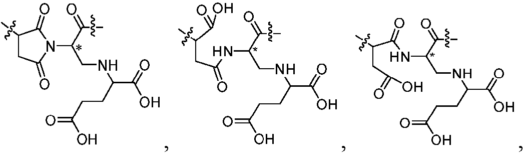 or
(7) 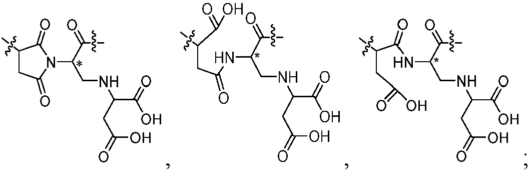
preferably, M₁, M₂, M₃ are selected from any one of the following combinations (1)-(2), and M₁, M₂, M₃ are not identical to each other,
(1) 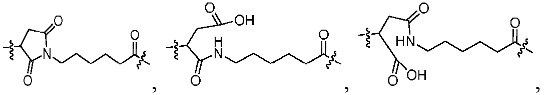 or
(2) 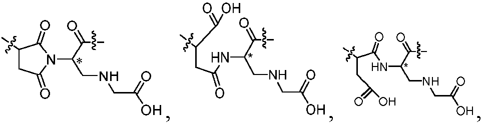
more preferably, M₁, M₂, M₃ are each independently selected from 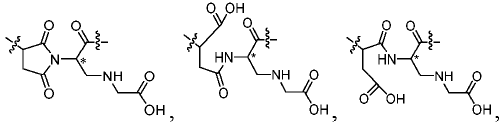 and M₁, M₂, M₃ are not identical to each other;
wherein:
the chiral carbon atom indicated at position * has an R or S absolute configuration;
the position indicated by the left wavy line is connected to Ab; the position indicated by the right wavy line is connected to the modifier unit B or the linker unit L.

4. The antibody-drug conjugate or its stereoisomer, pharmaceutically acceptable salt or solvate thereof according to any one of Claims 1 to 3, wherein the modifier unit B is present or absent, and when present, B comprises a hydrophilicity-enhancing structure or a stereoisomer thereof.

5. The antibody-drug conjugate or its stereoisomer, pharmaceutically acceptable salt or solvate thereof according to any one of Claims 1 to 4, wherein the modifier unit B is present or absent, and when present, B comprises a hydrophilicity-enhancing structure or a stereoisomer thereof as shown in the following formula: 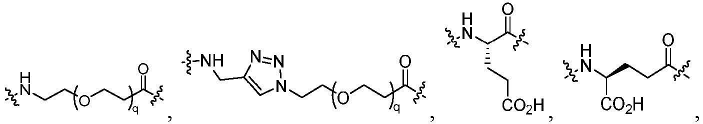 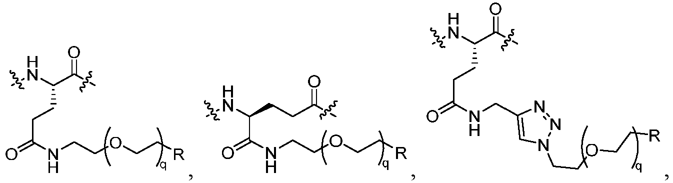 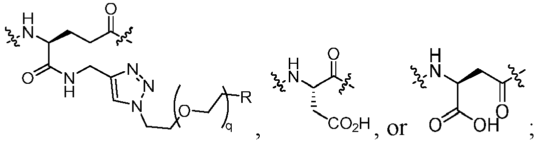
preferably, the modifier unit B is present or absent, and when present, the modifier unit B comprises a hydrophilicity-enhancing structure or a stereoisomer thereof as shown in the following formula . 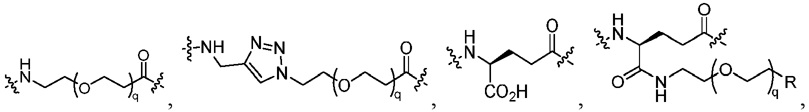 and 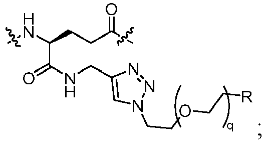
wherein:
R comprises hydroxyl, amino, polyethylene glycol, carboxylic acid group, sulfonic acid group, sulfinic acid group, phosphoric acid group, C1-C6 alkyl, C1-C6 alkoxy, natural or non-natural amino acid residue, sugar or a derivative thereof, or a combination thereof;
preferably, R is selected from C1-C6 alkoxy, carboxylic acid group, and amino group;
more preferably, R is selected from methoxy, carboxylic acid group, and amino group;
the position indicated by the wavy line on the left is connected to M₁, M₂, or M₃;
the position indicated by the wavy line on the right is connected to L;
q is an integer from 1-10, preferably 2, 7, or 8;
most preferably, the modifier unit B is selected from a hydrophilicity-enhancing structure or a stereoisomer thereof as shown in the following formula: 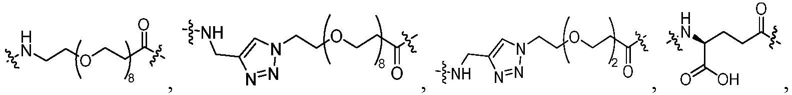 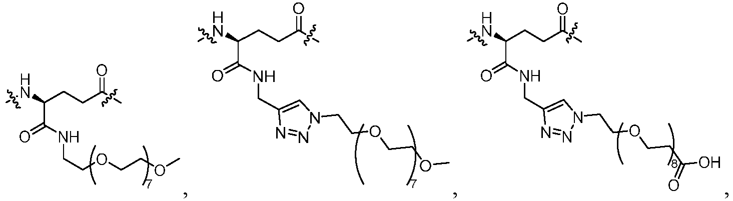 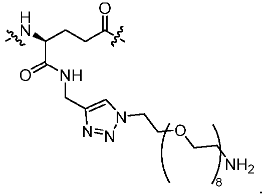
the position indicated by the left wavy line is connected to M₁, M₂, or M₃, and the position indicated by the right wavy line is connected to the linker unit L.

6. The antibody-drug conjugate or its stereoisomer, pharmaceutically acceptable salt or solvate thereof according to any one of Claims 1 to 5, wherein the linker unit L is L₁-L₂,
L₁ is a peptide residue comprising 2-10 amino acid residues, the peptide residue having an amino terminus connected to the modifier unit B or to M₁, M₂, M₃ and a carbonyl terminus connected to L₂;
preferably, L₁ is a peptide residue comprising 2-4 amino acid residues, the peptide residue having the amino terminus connected to B or to M₁, M₂, M₃, and the carbonyl terminus connected to L₂; preferably, the amino acid is selected from valine, alanine, phenylalanine, glycine, lysine, citrulline, serine, glutamic acid, and aspartic acid; more preferably, the amino acid is selected from valine, alanine, phenylalanine, glycine, lysine, and citrulline; most preferably, the amino acid is selected from valine, phenylalanine, glycine, lysine, and citrulline;
more preferably, L₁ is selected from the following peptide residues: glycine-glycine-phenylalanine-glycine, valine-citrulline, phenylalanine-lysine, valine-alanine, and alanine-alanine-alanine (preferably selected from glycine-glycine-phenylalanine-glycine, valine-citrulline, and phenylalanine-lysine), the amino terminus of the peptide residues is connected to B or to M₁, M₂, M₃, and the carbonyl terminus is connected to L₂;
most preferably, L₁ is selected from the following peptide residues: 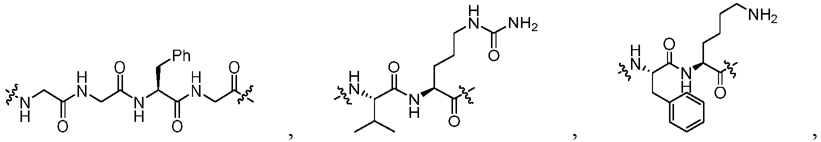 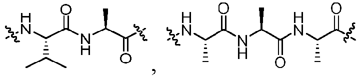
(preferably selected from 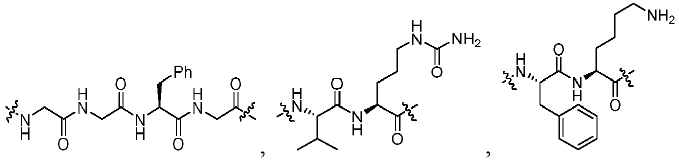 ), the peptide residues having an amino terminus connected to B or to M₁, M₂, M₃ and a carbonyl terminus to L₂;
L₂ is selected from 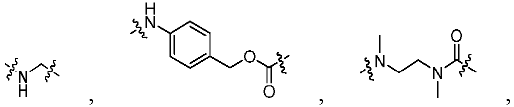 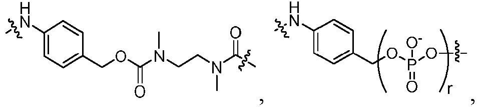 and is connected to L₁ at the position indicated by the left wavy line, and to X at the position indicated by the right wavy line;
preferably, L₂ is selected from 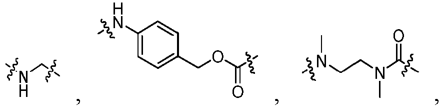 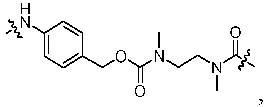 and is connected to L₁ at the position indicated by the left wavy line, and to X at the position indicated by the right wavy line;
more preferably, L₂ is selected from 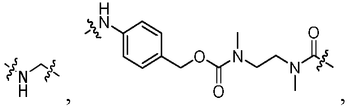
and is connected to L₁ at the position indicated by the left wavy line, and to X at the position indicated by the right wavy line;
preferably, the linker unit L comprises the following structures or stereoisomers thereof. 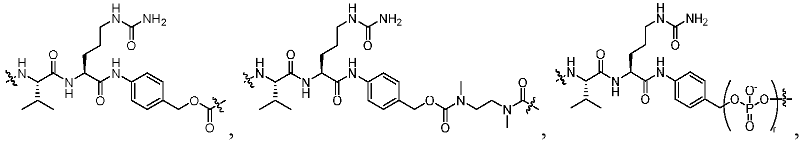 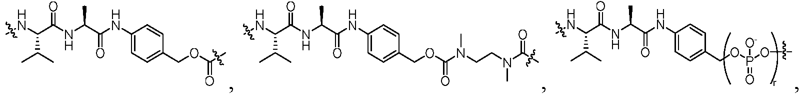 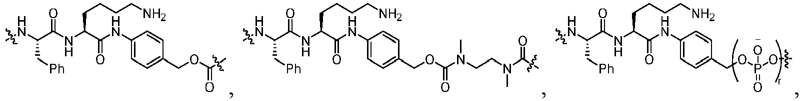 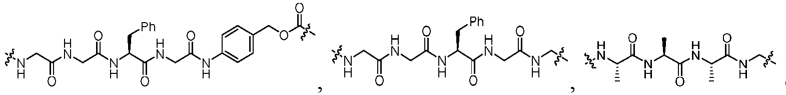
or 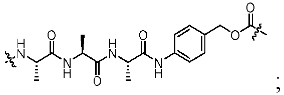
wherein:
r is an integer from 1-10;
more preferably, the linker unit L is selected from the following structures or stereoisomers thereof: 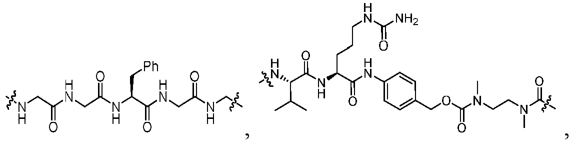 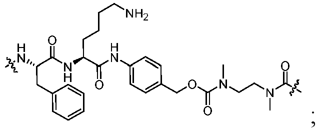
the linker unit L is connected to the modifier unit B or to M₁, M₂, M₃ at the position indicated by the wavy line on the left side;
the linker unit L is connected to X at the position indicated by the wavy line on the right side.

7. The antibody-drug conjugate or its stereoisomer, pharmaceutically acceptable salt or solvate thereof according to any one of claims 1 to 6, wherein R₁ and R₂ are the same or different and are each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, 5- 10 heteroaryl, and substituted 5-10-membered heteroaryl;
preferably, R₁ and R₂ are the same or different and are each independently selected from halogen, and C1-C6 alkyl;
more preferably, Rₗ is selected from C1-C6 alkyl and R₂ is selected from halogen;
most preferably, R₁ is methyl and R₂ is fluoride;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclic, substituted 3-7-membered heterocyclic, C6-C10 aryl, substituted C6-C10 aryl, C6- C10 aryl C1-C6 alkyl, 5-10-membered heteroaryl, and substituted 5-10-membered heteroaryl;
preferably, R₃, R₄ are the same or different, each independently selected from hydrogen, C1-C6 alkyl, substituted C1-C6 alkyl, C3-C8 cycloalkyl, C3-C8 cycloalkyl C1-C6 alkyl and C6-C10 aryl C1-C6 alkyl;
further preferably, R₃, R₄ are the same or different, each independently selected from hydrogen, C1-C6 alkyl, halo-C1-C6 alkyl, C3-C6 cycloalkyl, C3-C6 cycloalkyl C1-C6 alkyl, and phenyl C1-C6 alkyl;
more preferably, R₃, R₄ are the same or different, each independently selected from hydrogen, methyl, ethyl, trifluoromethyl, cyclopropyl, cyclopropylmethyl, and benzyl;
most preferably, of R₃, R₄, any one is selected from hydrogen, methyl, and the other is selected from hydrogen, methyl, ethyl, trifluoromethyl, cyclopropyl, cyclopropylmethyl, benzyl;
alternatively, R₃, R₄ and the carbon atoms to which they are attached constitute a C3-C8 cycloalkyl group, a 3-7-membered heterocyclic group, a substituted 3-7-membered heterocyclic group;
preferably, R₃, R₄ and the carbon atoms to which they are attached constitute a C3-C8 cycloalkyl group;
more preferably, R₃, R₄ and the carbon atoms to which they are attached constitute a C3-C6 cycloalkyl group;
most preferably, R₃, R₄ and the carbon atom to which it is attached constitute a cyclopropyl, cyclobutyl or cyclopentyl group;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl, R₅ is preferably selected from hydrogen, and C1-C6 alkyl, R₅ is more preferably selected from hydrogen, and methyl;
X is selected from -NH-, -O- and -S-, preferably -O-;
m is an integer from 0 to 5, preferably 0, or 1.

8. The antibody-drug conjugate or the stereoisomer, pharmaceutically acceptable salt or solvate thereof according to any one of Claims 1 to 7, wherein the camptothecin antitumor drug comprises the following compounds or their stereoisomers :

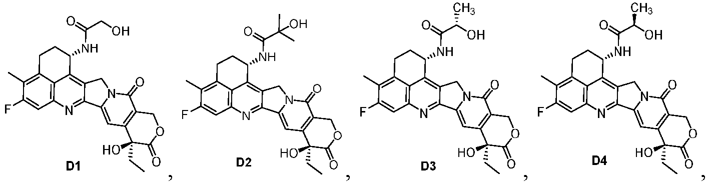

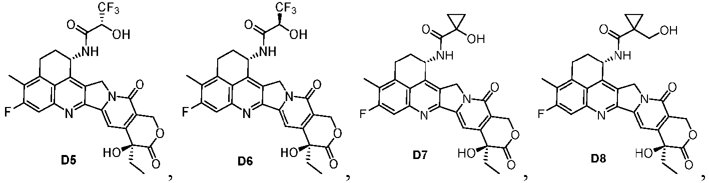

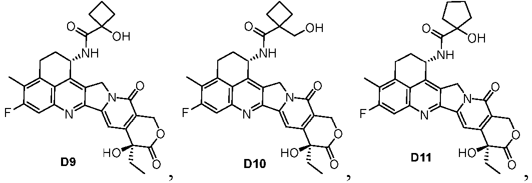

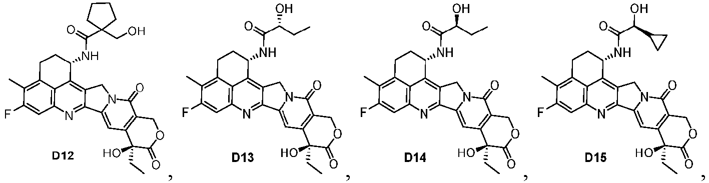

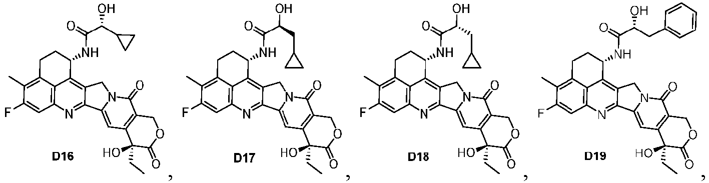

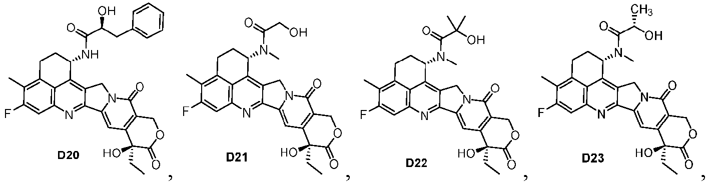

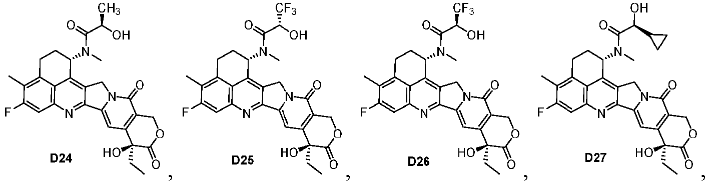

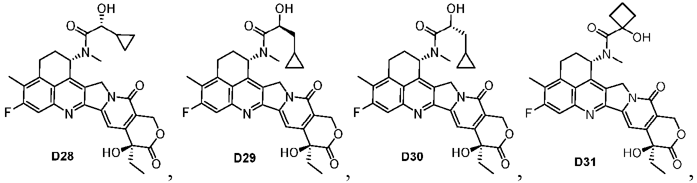

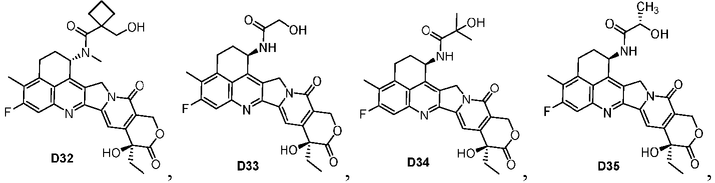

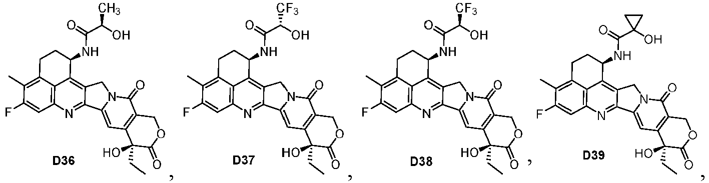

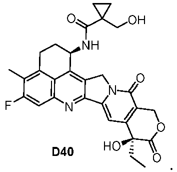

9. The antibody-drug conjugate or its stereoisomer, pharmaceutically acceptable salt or solvate thereof according to any one of Claims 1 to 8, wherein the antibody-drug conjugate comprises the following structures: 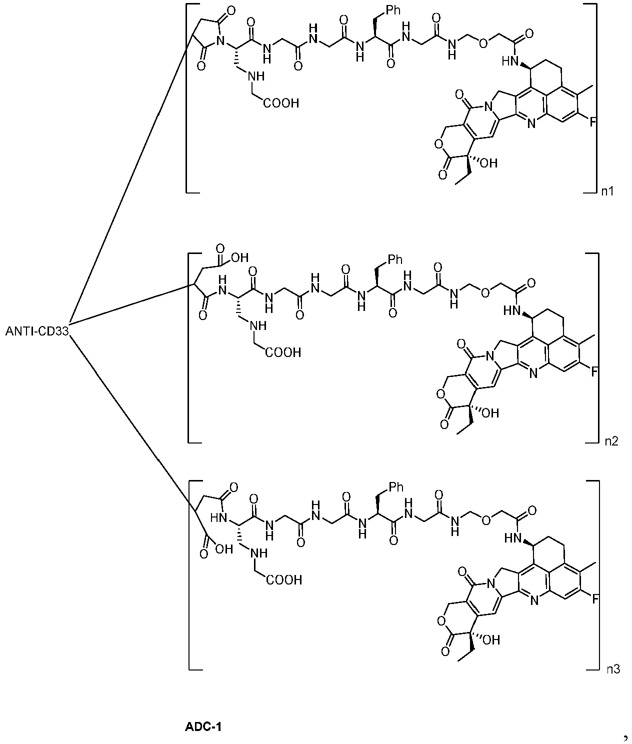 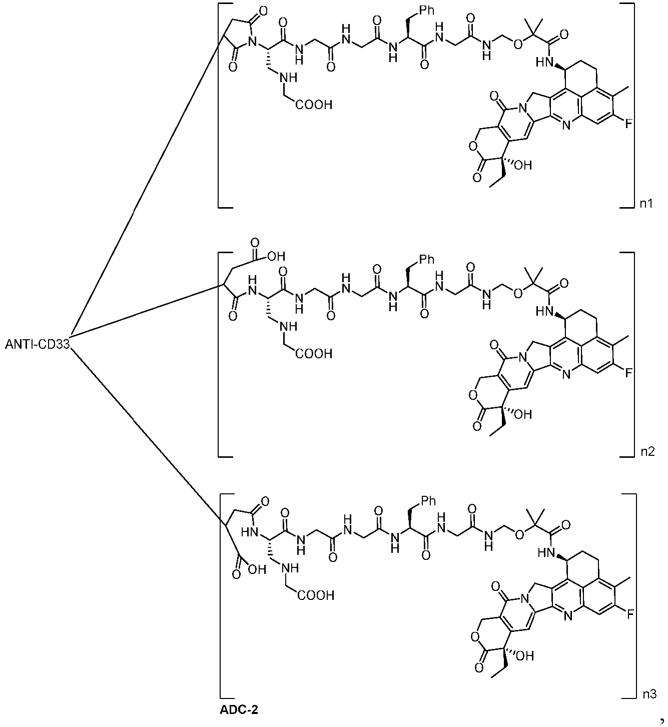 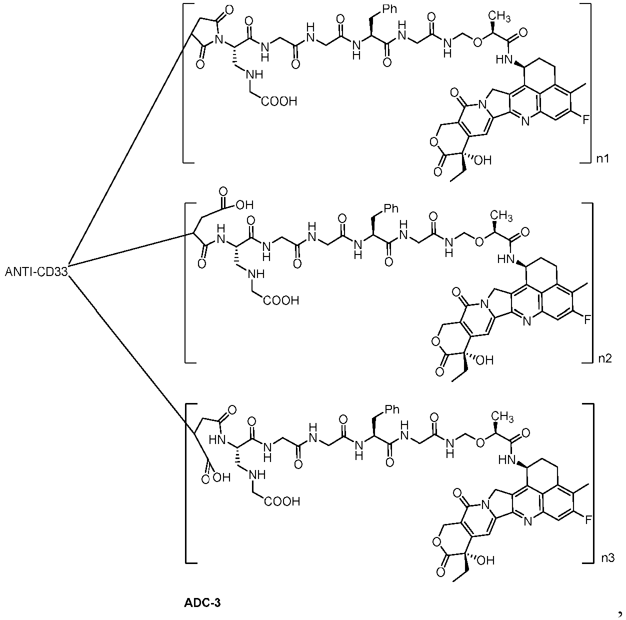 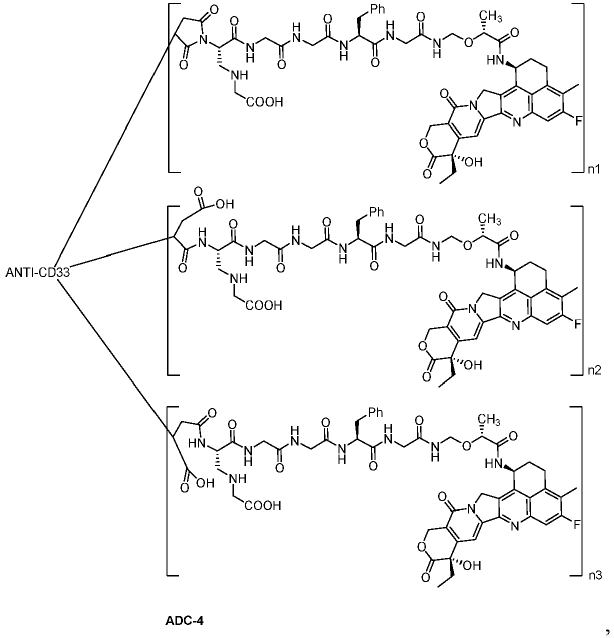 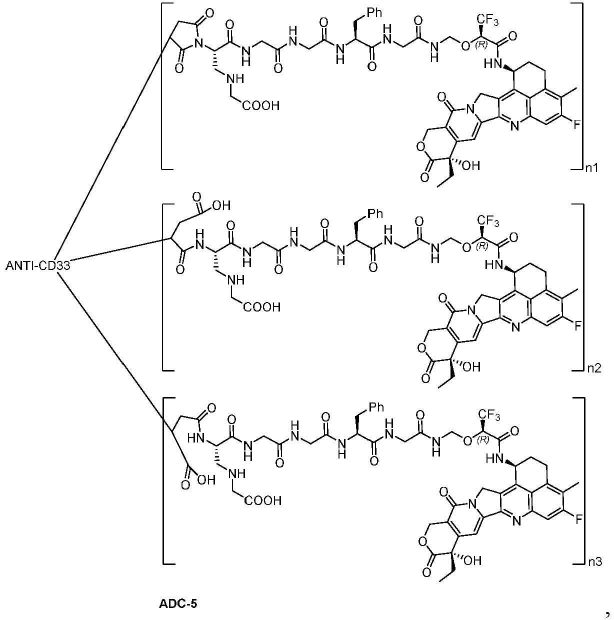 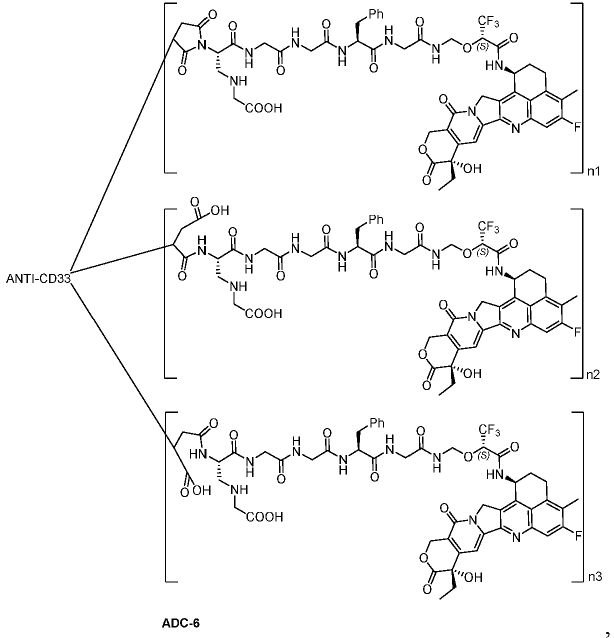 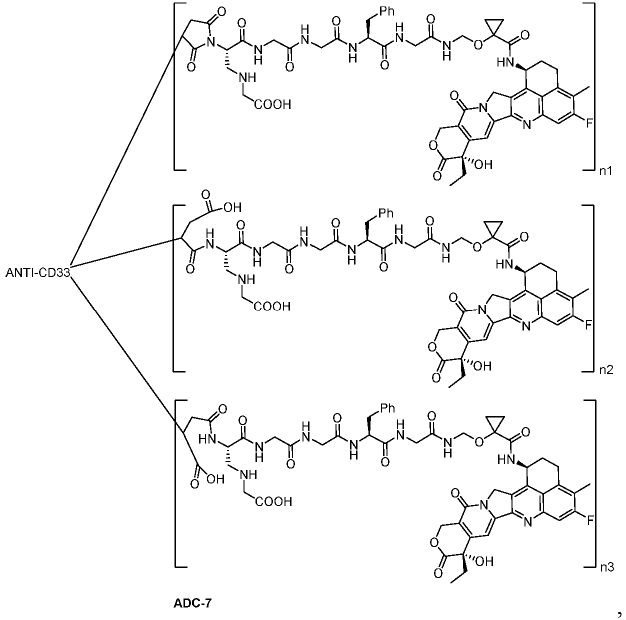 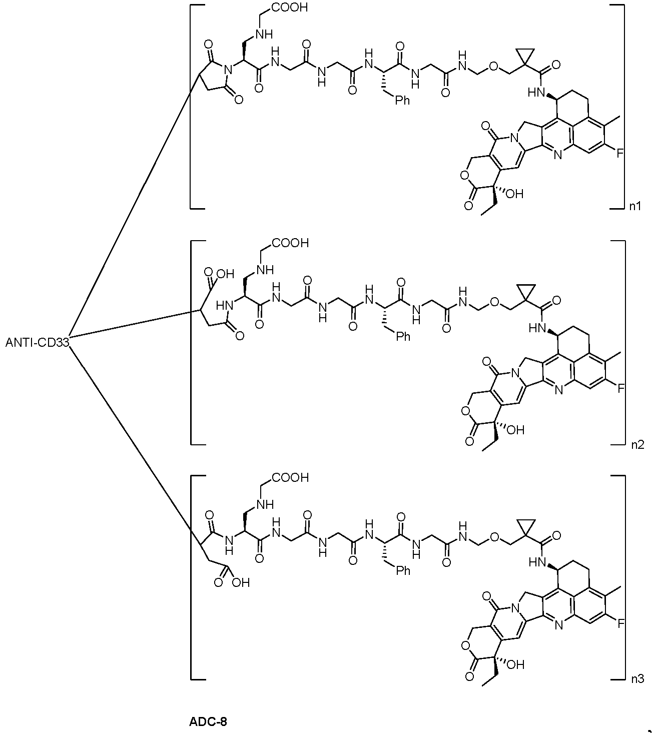 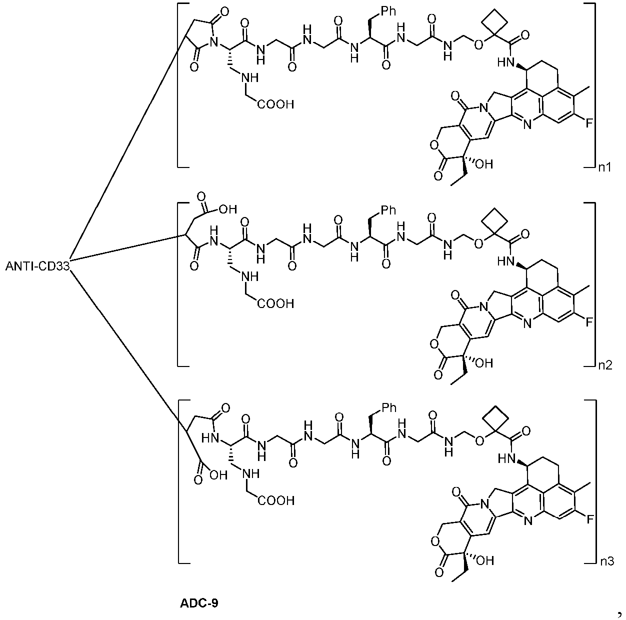 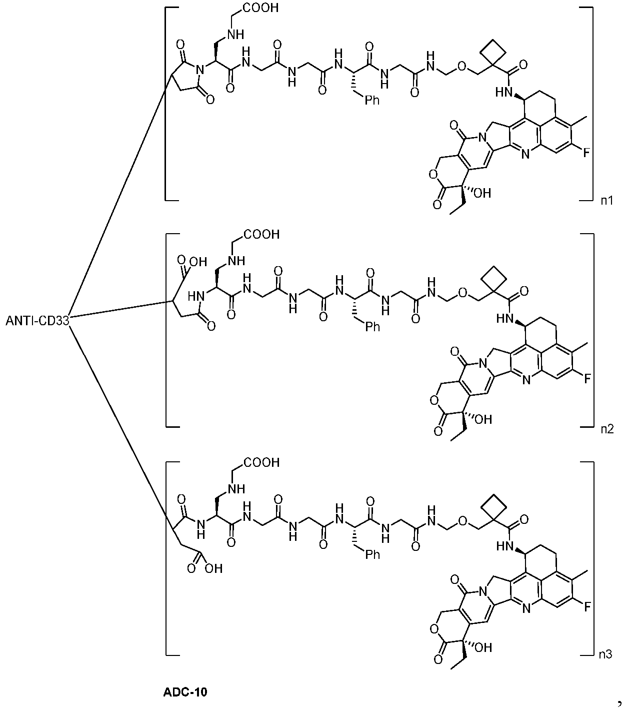 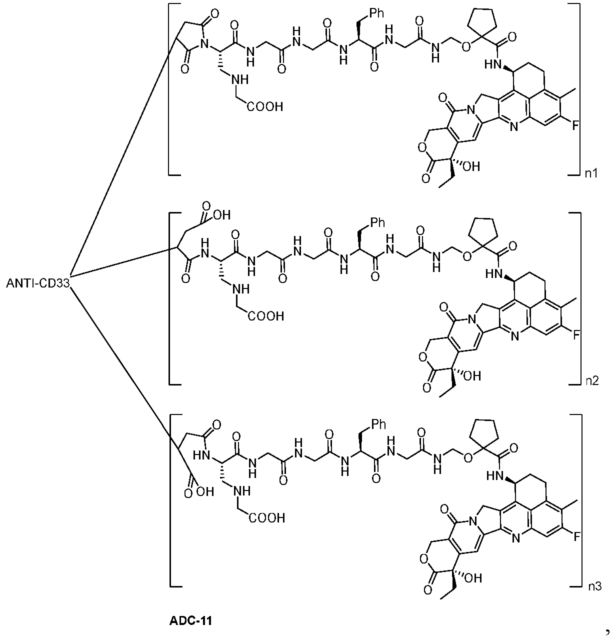 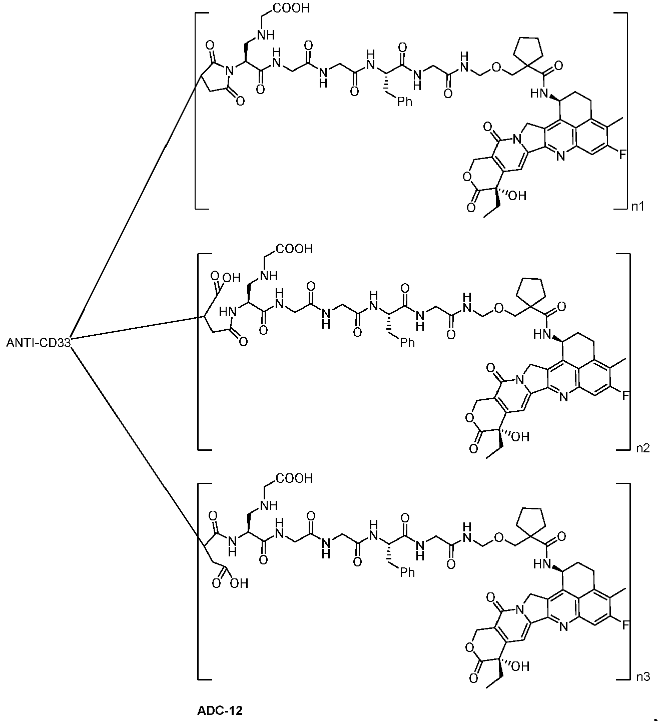 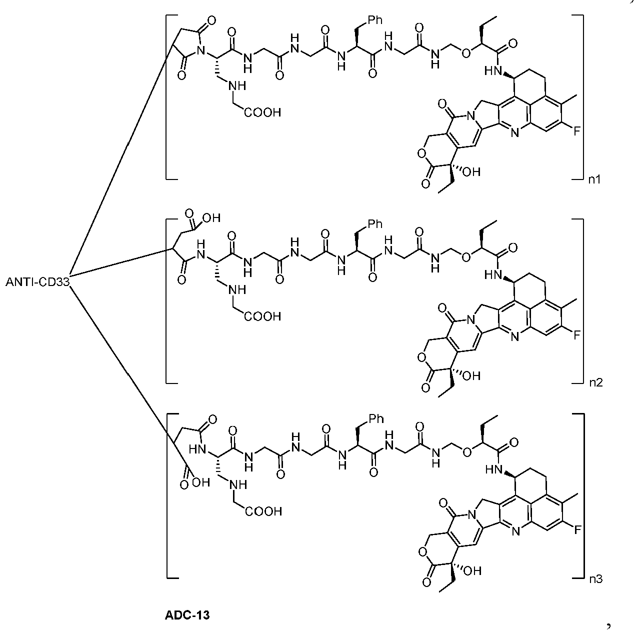 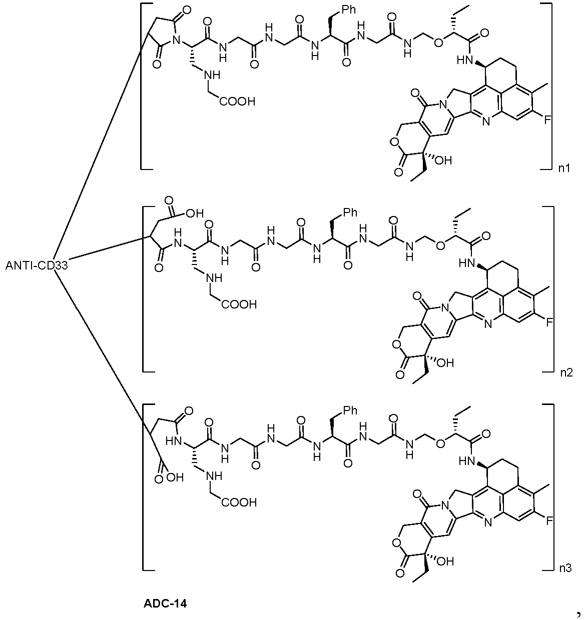 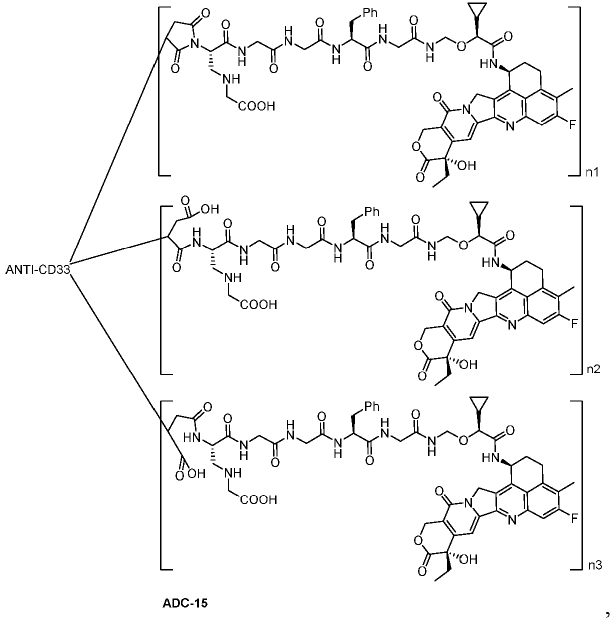 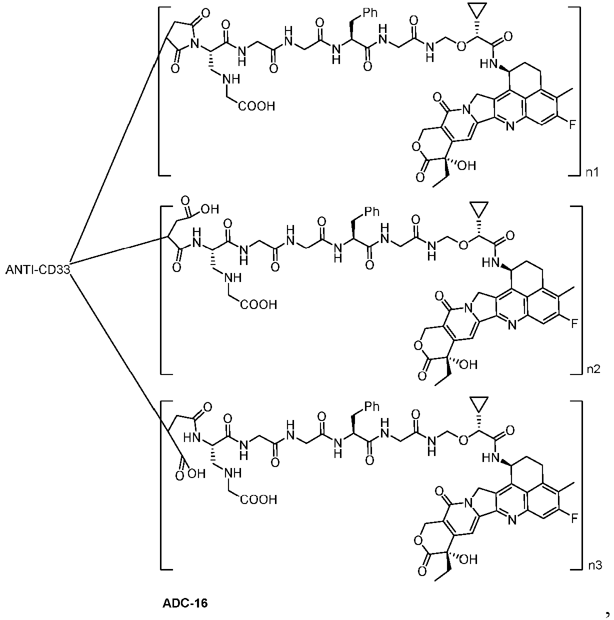 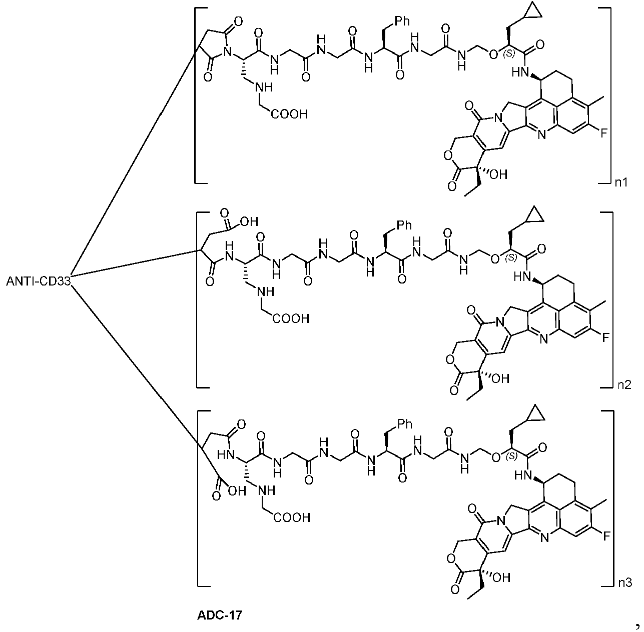 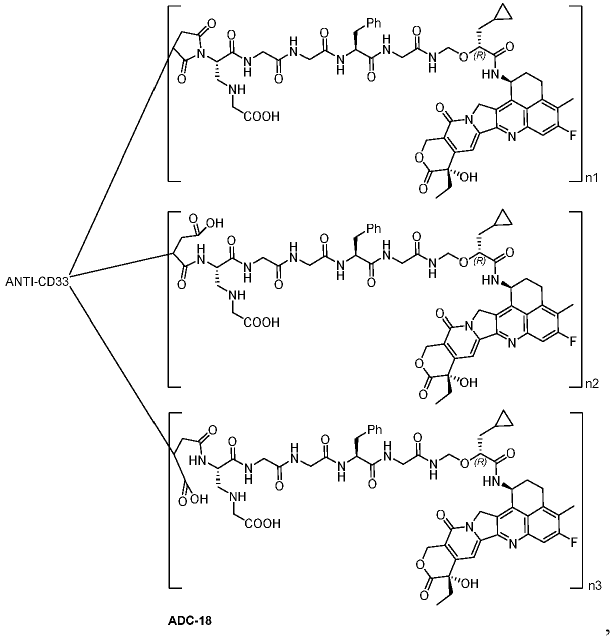 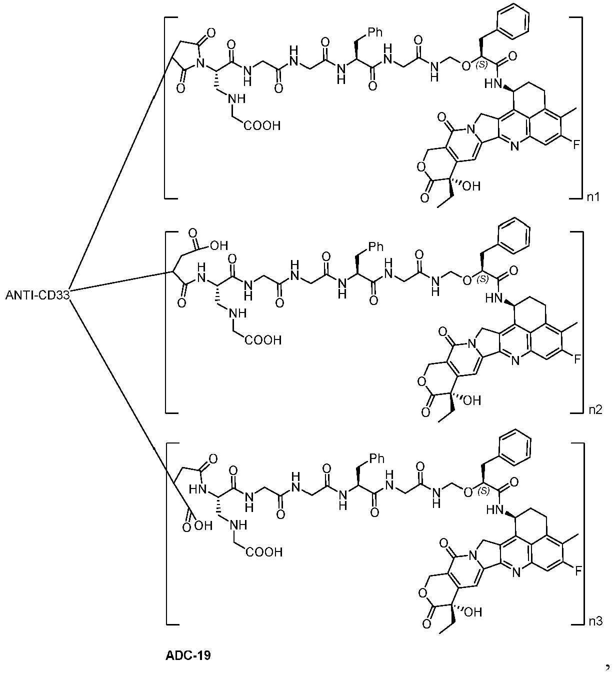 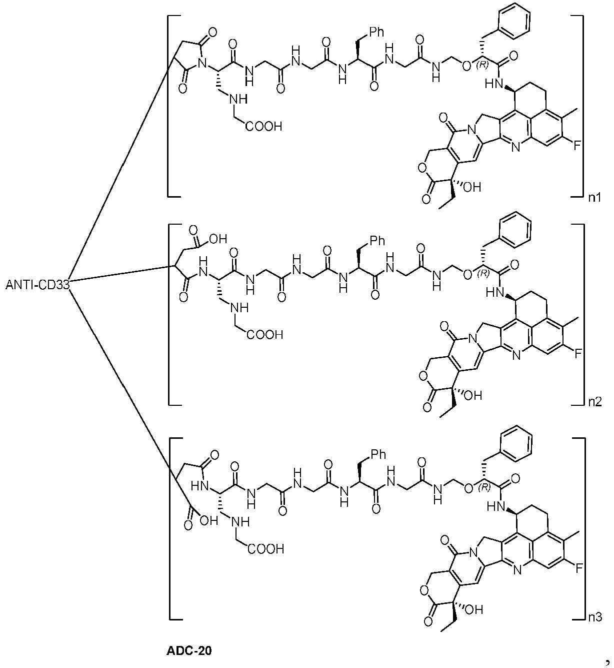 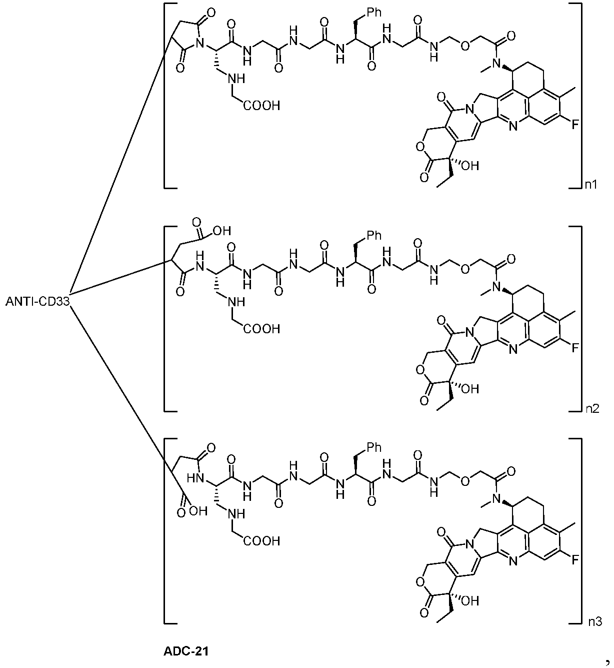 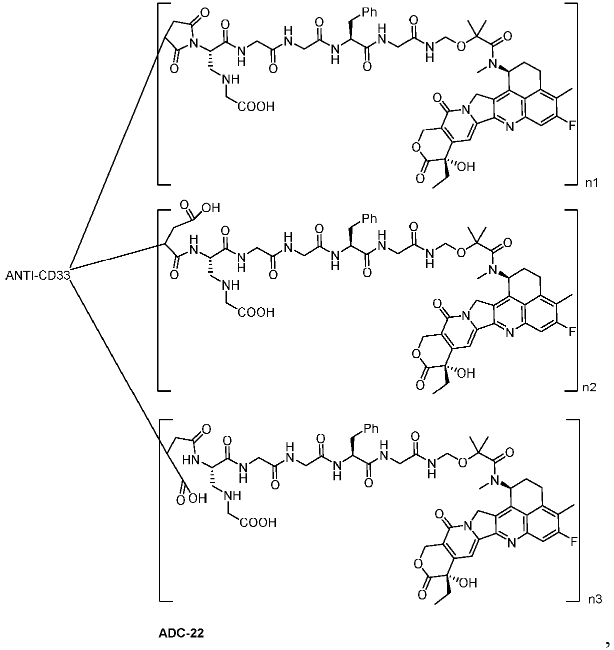 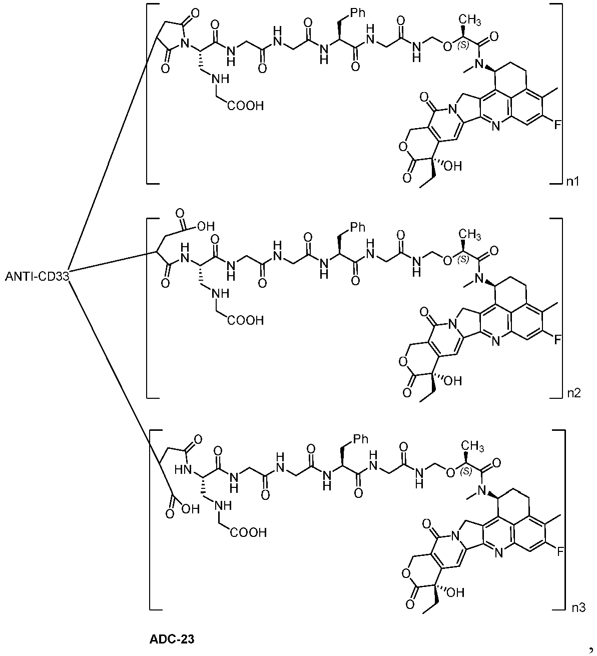 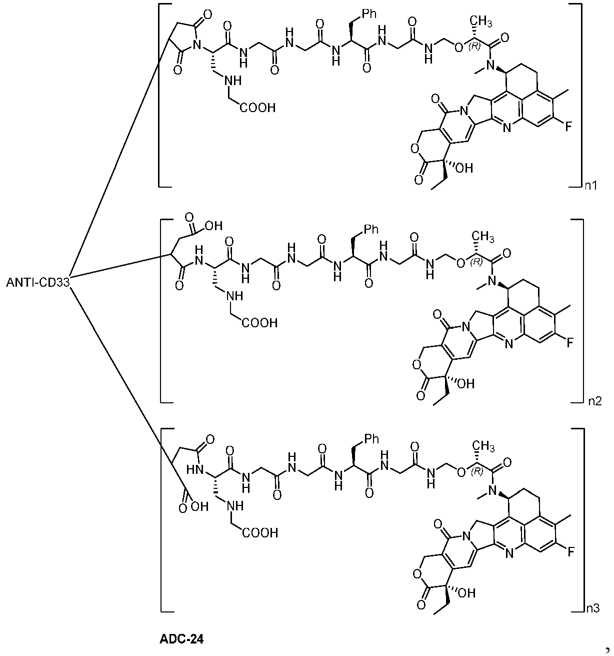 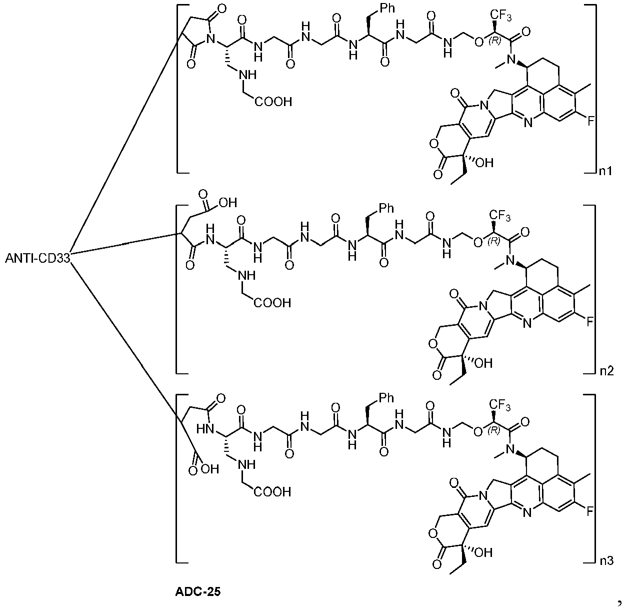 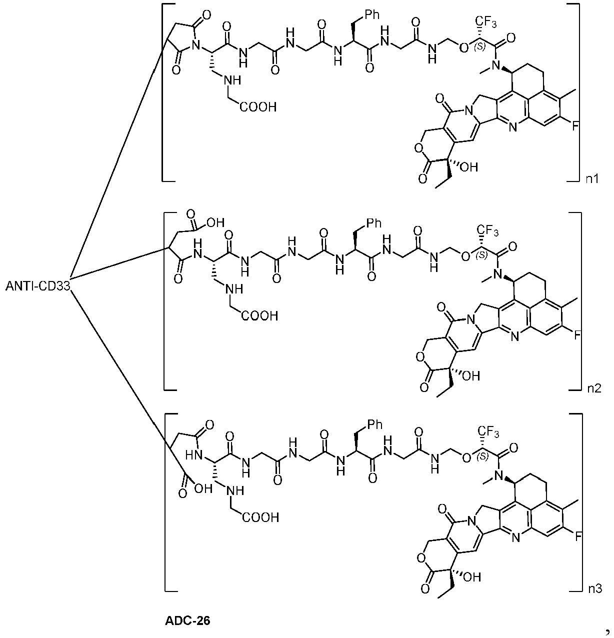 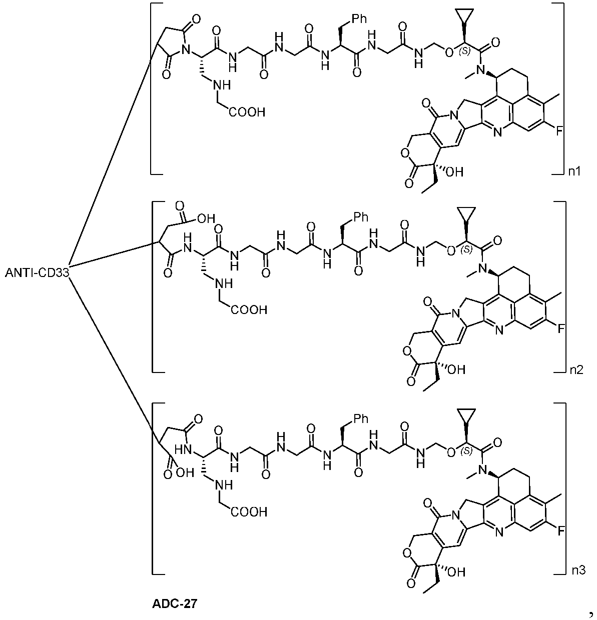 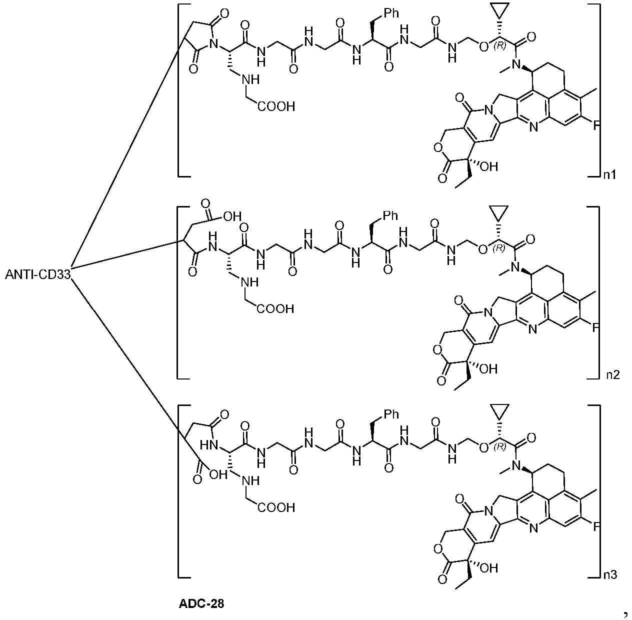 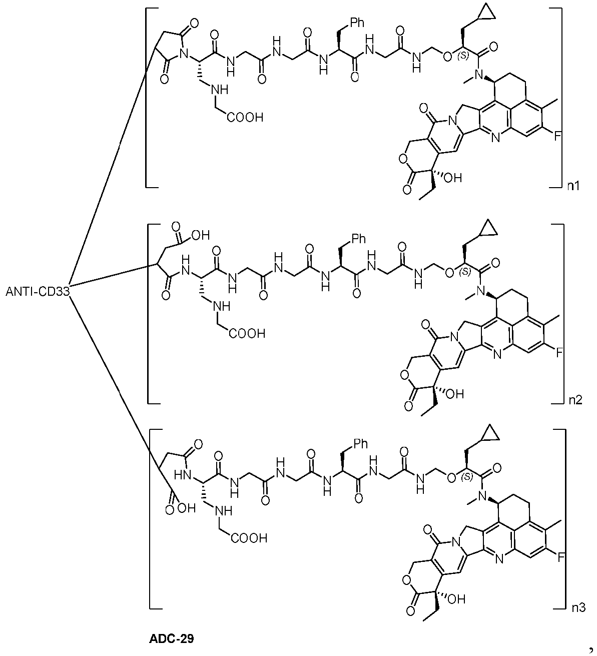 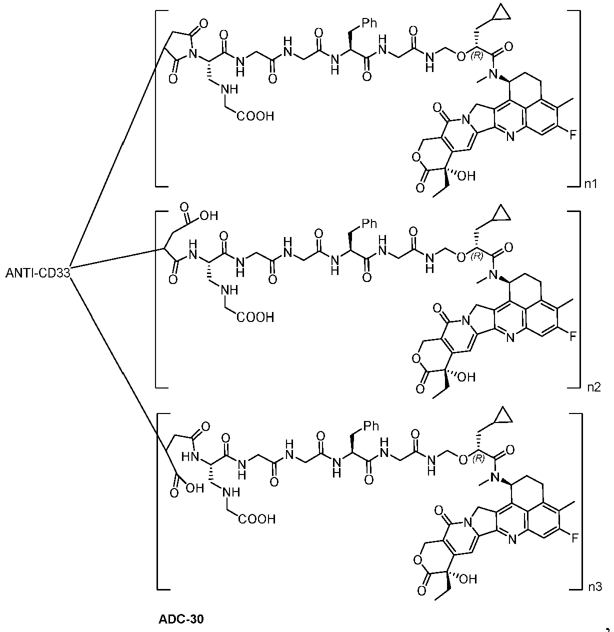 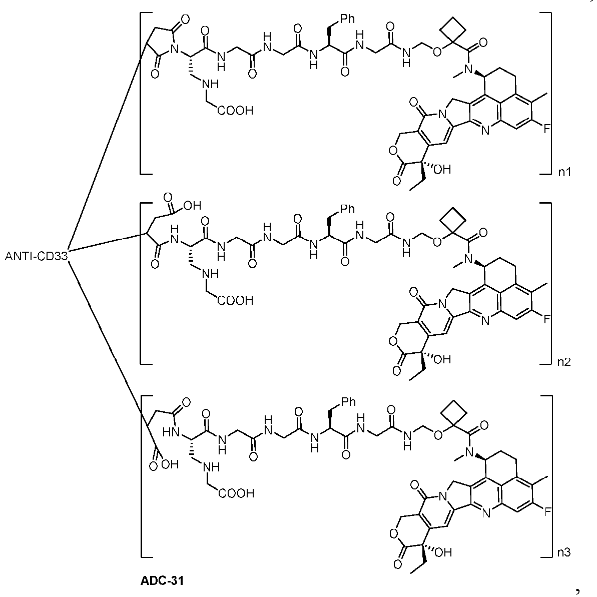 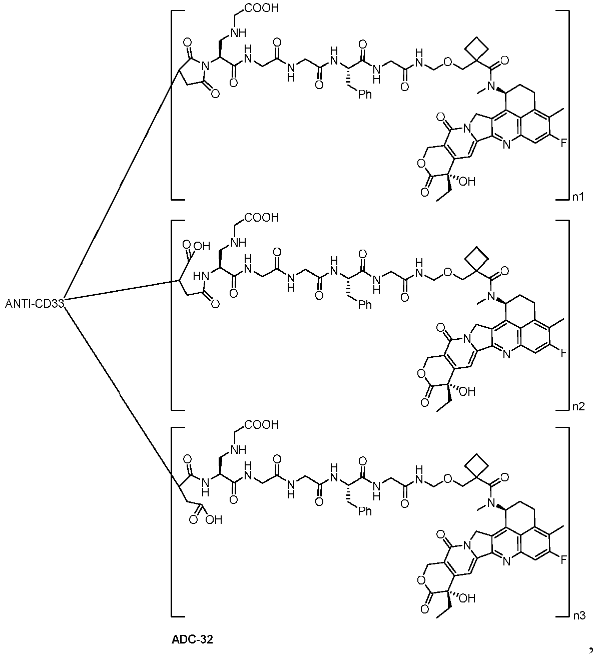 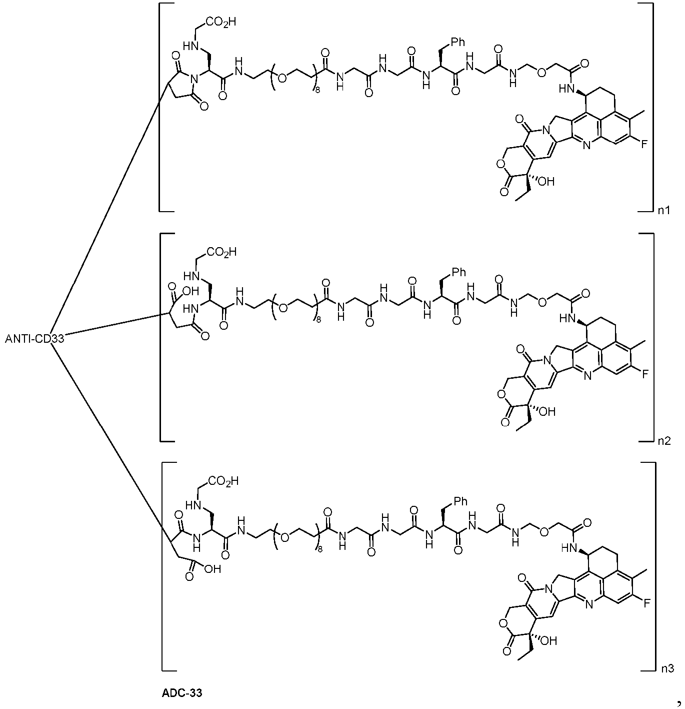 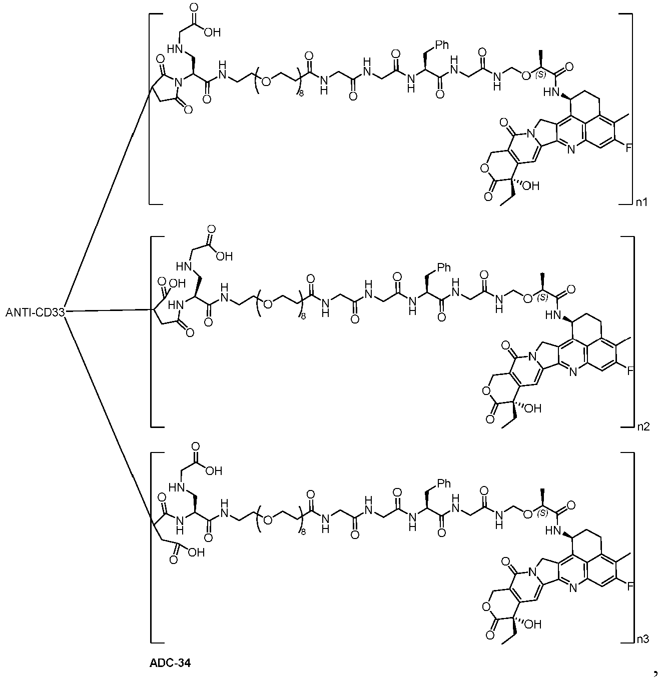 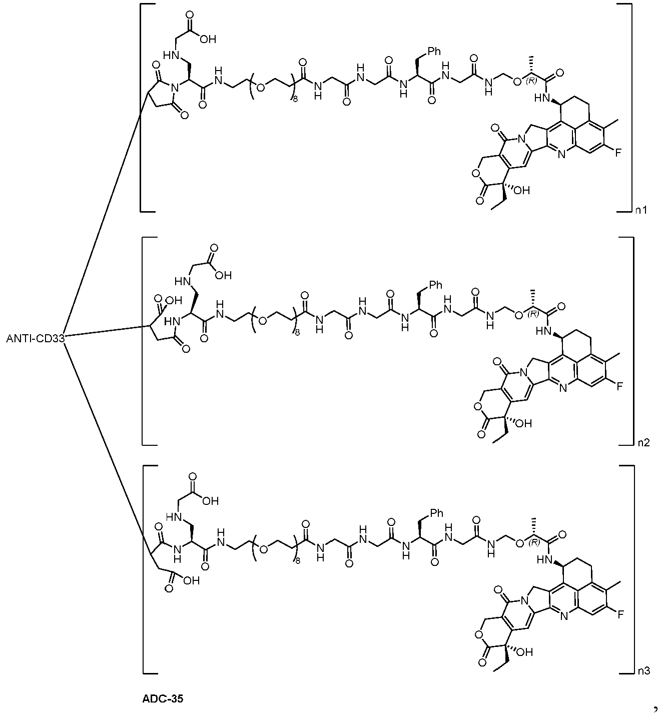 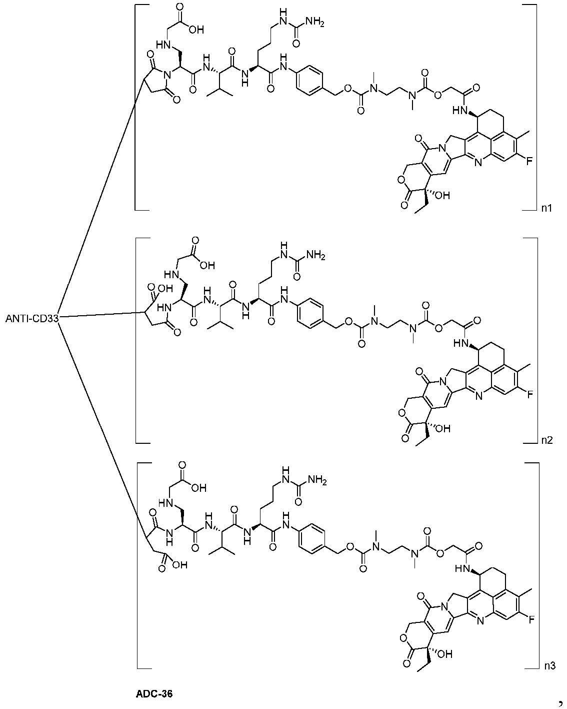 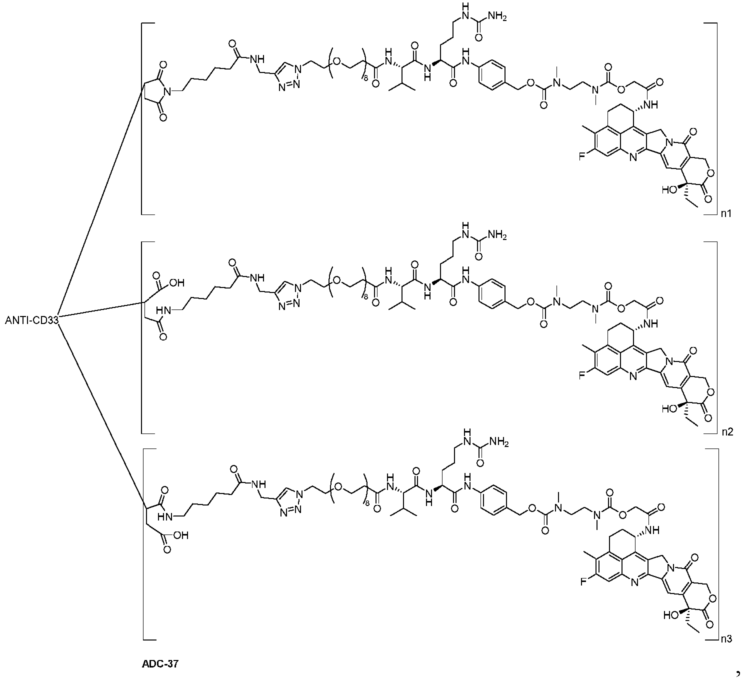 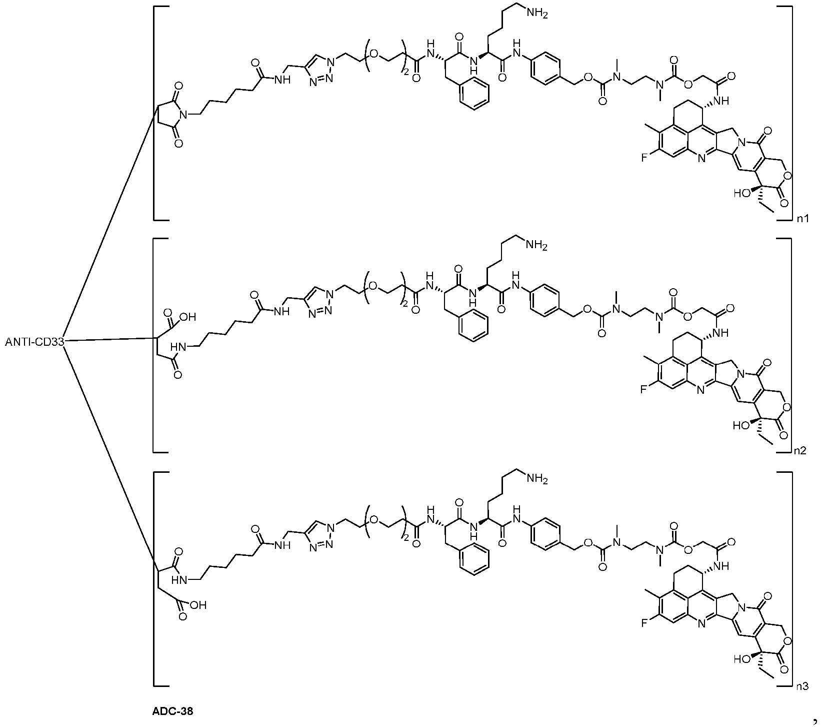 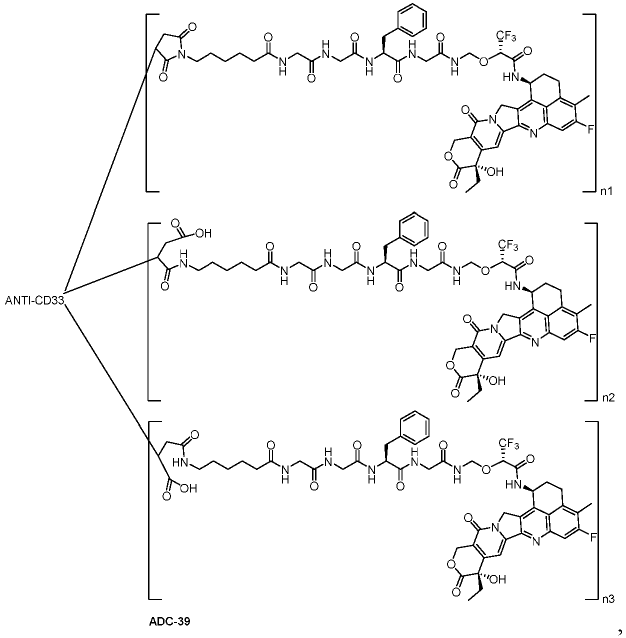 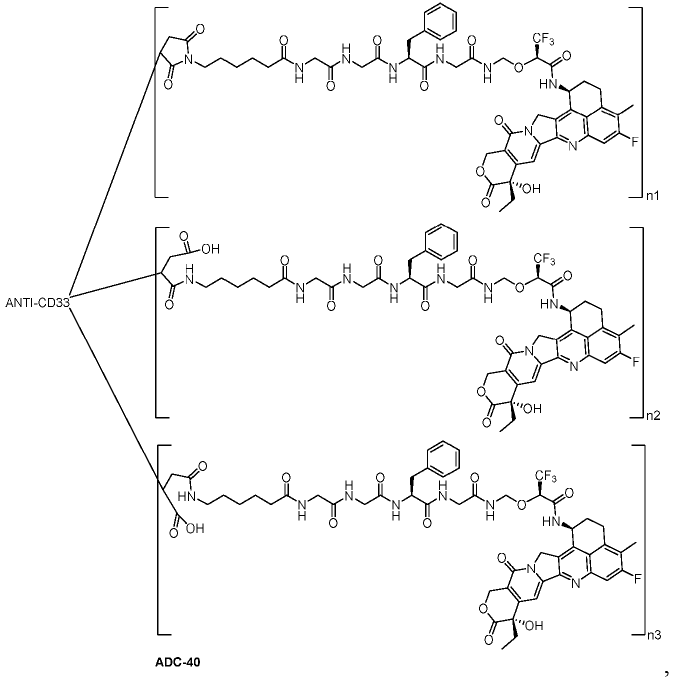 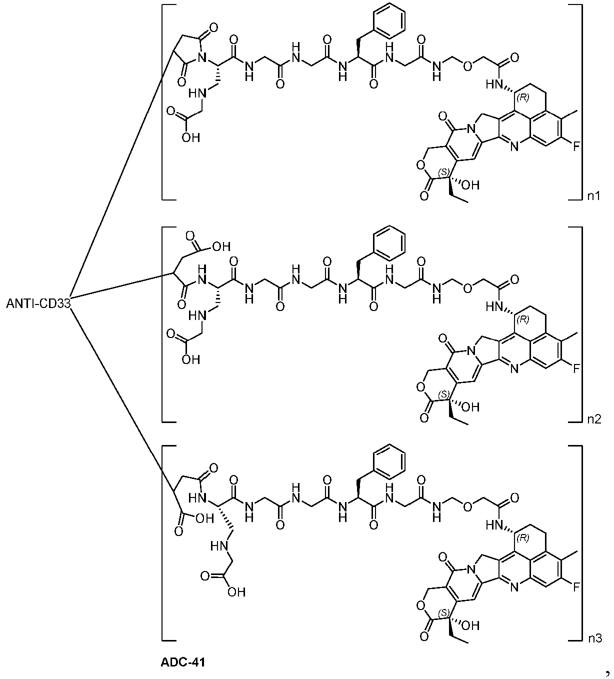 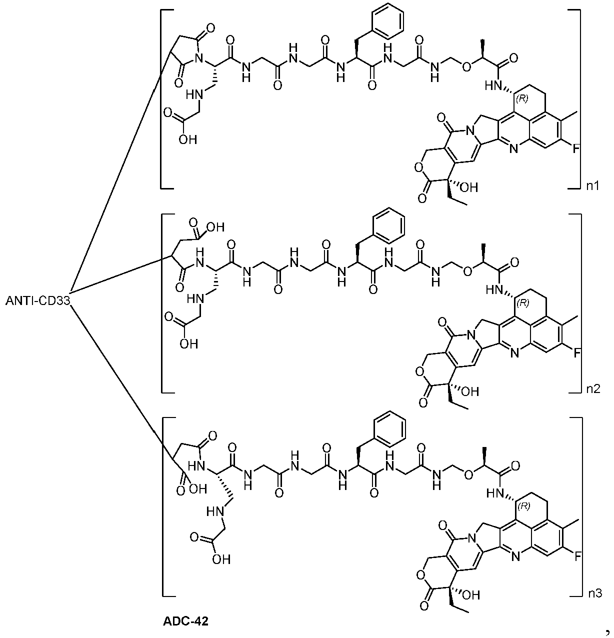 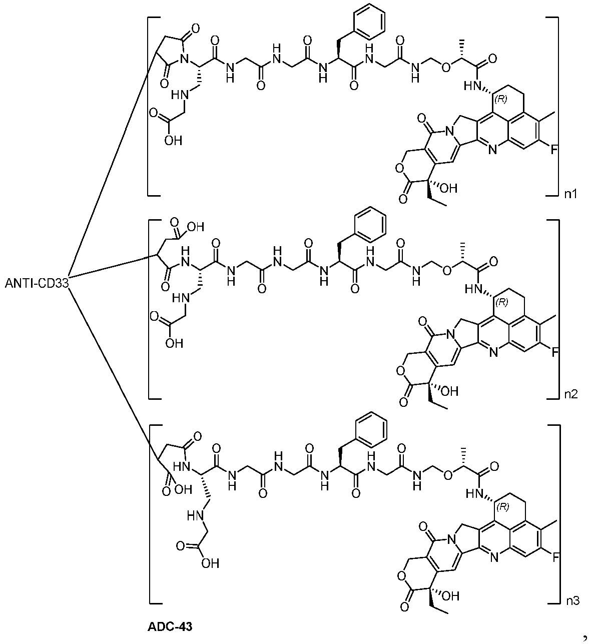 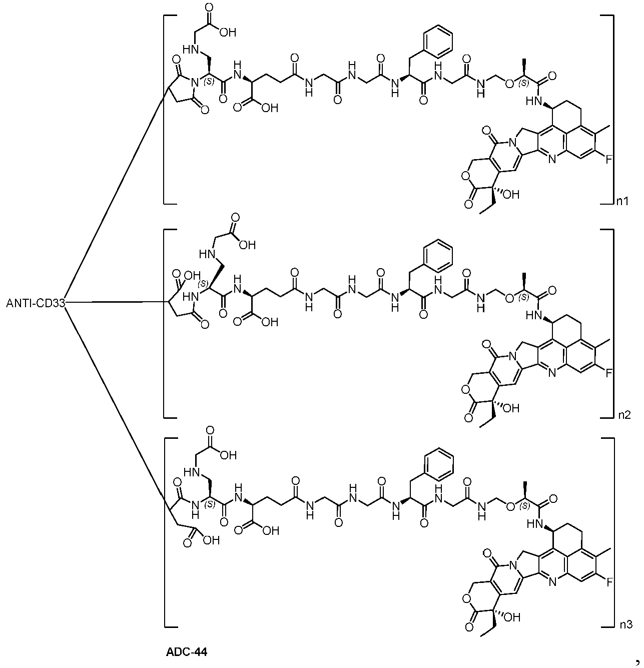 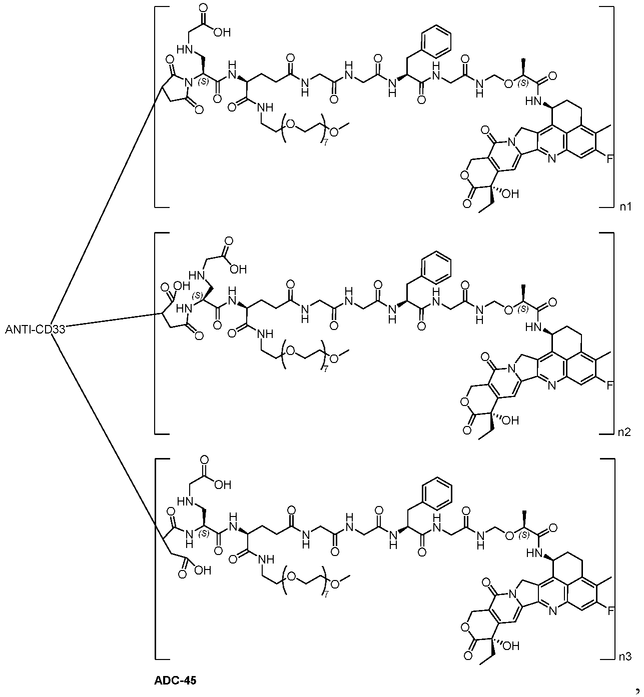 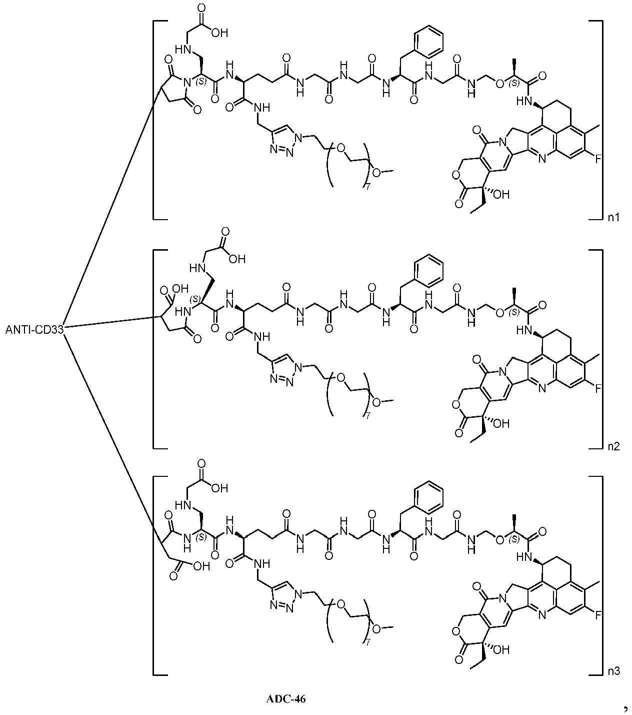 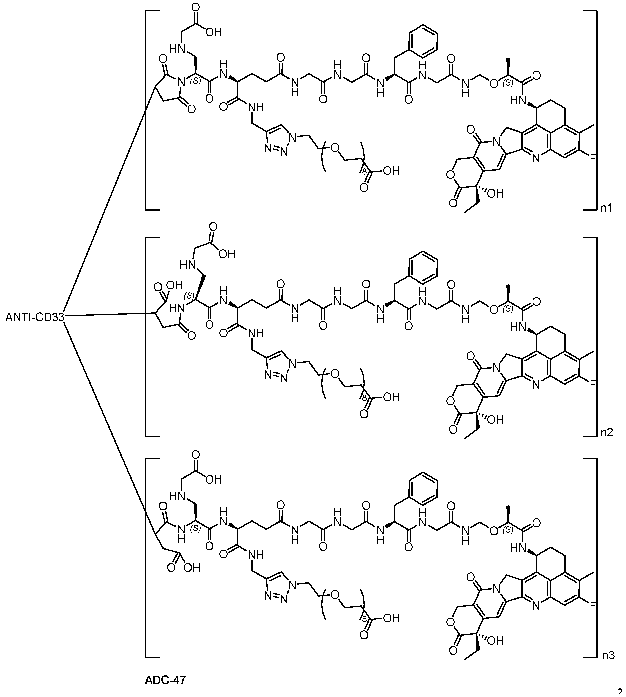 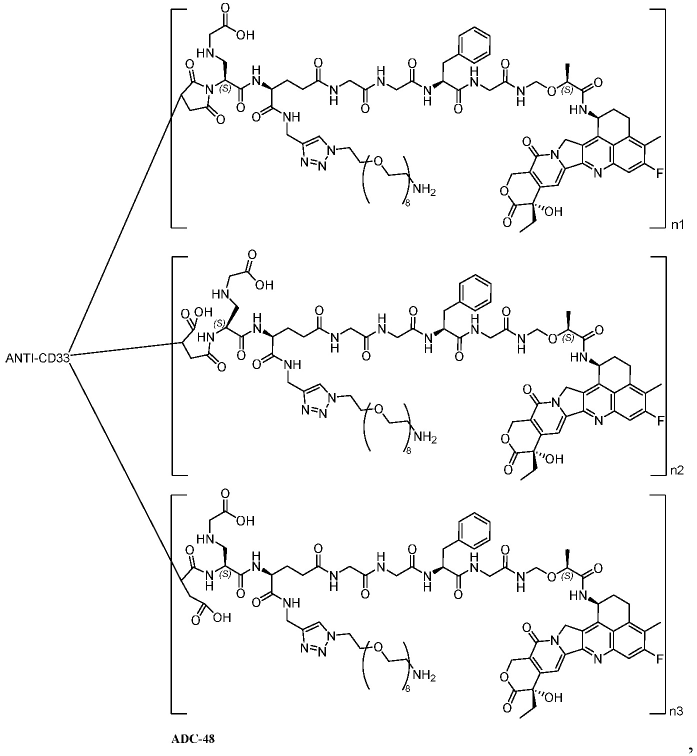 wherein:
ANTI-CD33 is an anti CD33 antibody or an antigen-binding fragment thereof;
n1, n2, and n3 are each independently any integer or any decimal from 0 to 10, n1, n2, and n3 are not simultaneously 0, and 1≤ n1+n2+n3≤ 10;
preferably, n1, n2, and n3 each is independently an integer or a decimal number from 0 to 10, n1, n2, and n3 are not simultaneously 0, and 8≤ n1+n2+n3≤ 10.

10. The antibody-drug conjugate or its stereoisomer, pharmaceutically acceptable salt or solvate thereof according to any of Claims 1 to 9, wherein the light chain variable region of the anti-CD33 antibody or antigen-binding fragment thereof comprises CDR1, CDR2, CDR3 as shown in SEQ ID NO: 8, SEQ ID NO: 9, SEQ ID NO: 10, respectively, and the heavy chain variable region comprises CDR1, CDR2, CDR3 as shown in SEQ ID NO: 2, SEQ ID NO: 3, SEQ ID NO: 4, respectively.

11. The antibody-drug conjugate or its stereoisomer, pharmaceutically acceptable salt or solvate thereof according to any one of Claims 1 to 10, wherein the anti-CD33 antibody or antigen-binding fragment thereof has a light-chain variable region as shown in SEQ ID NO: 7 and a heavy-chain variable region as shown in SEQ ID NO: 1.

12. The antibody-drug conjugate or its stereoisomer, pharmaceutically acceptable salt or solvate thereof according to any one of claims 1 to 11, wherein the anti-CD33 antibody comprises a constant region derived from human immunoglobulin;
preferably, the light chain of the anti-CD33 antibody comprises a light chain constant region derived from a human immunoglobulin (e.g., kappa or lambda); and the heavy chain of the antibody comprises a heavy chain constant region derived from a human immunoglobulin (e.g., IgG1, IgG2, IgG3, or IgG4),
preferably, the amino acid sequence of the light chain of the anti-CD33 antibody is SEQ ID NO: 11 and the amino acid sequence of the heavy chain is SEQ ID NO: 5;
preferably, the nucleic acid coding sequence for the light chain of the anti-CD33 antibody is SEQ ID NO: 23 and the nucleic acid coding sequence for the heavy chain is SEQ ID NO: 17.

13. The antibody-drug conjugate or its stereoisomer, pharmaceutically acceptable salt or solvate thereof according to any one of claims 10 to 12, wherein the anti-CD33 antibody further comprises a site-specific cysteine insertion site;
preferably, the cysteine insertion site is in the light chain constant region;
preferably, the cysteine insertion site is position 206 (Kabat numbering) of the kappa light chain constant region;
preferably, the anti-CD33 antibody has a light chain amino acid sequence of SEQ ID NO: 25 and a heavy chain amino acid sequence of SEQ ID NO: 5;
preferably, the nucleic acid coding sequence for the light chain of the anti-CD33 antibody is SEQ ID NO: 27 and the nucleic acid coding sequence for the heavy chain is SEQ ID NO: 17.

14. The antibody-drug conjugate or its stereoisomer, pharmaceutically acceptable salt or solvate thereof according to any one of Claims 1 to 13, wherein the pharmaceutically acceptable salt comprises sodium, potassium, calcium or magnesium salts formed with acidic functional groups in the structural formula, and acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate, or p-toluenesulfonate formed with basic functional groups in the structure.

15. A linker-payload as shown in formula III or its stereoisomer, pharmaceutically acceptable salt or solvate thereof, 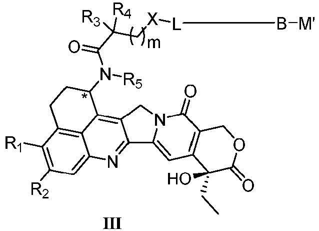 wherein:
M' is selected from 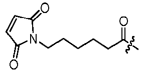 or a connector unit shown in formula A' below; 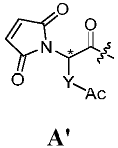
wherein Y is a scaffold selected from C1-C6 alkyl, substituted C1-C6 alkyl and C3-C8 cycloalkyl; preferably Y is C1-C6 alkyl; Ac is a hydrophilic structural unit; and the position indicated by the wavy line on the right side is connected to B or L;
B is present or absent, and when present, B is a modifier unit;
L is a linker unit, preferably a peptide-containing linker unit;
X is selected from -NH-, -O- or -S-, preferably -O-;
R₁ and R₂ are the same or different and are each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclyl, substituted 3-7-membered heterocyclyl, C6-C10 aryl, substituted C6-C10 aryl, 5-10-membered heteroaryl, and substituted 5-10-membered heteroaryl;
preferably, R₁ and R₂ are the same or different and are each independently selected from halogen, C1-C6 alkyl;
more preferably, R₁ is selected from C1-C6 alkyl and R₂ is selected from halogen;
most preferably, R₁ is methyl and R₂ is fluoride;
R₃ and R₄ are the same or different, each independently selected from hydrogen, deuterium, halogen, C1-C6 alkyl, substituted C1-C6 alkyl, C3-C8 cycloalkyl, C3-C8 cycloalkyl C1-C6 alkyl, C1-C6 alkoxy C1-C6 alkyl, 3-7-membered heterocyclic, substituted 3-7-membered heterocyclic, C6-C10 aryl, substituted C6-C10 aryl, C6- C10 aryl C1-C6 alkyl, 5-10-membered heteroaryl, and substituted 5-10-membered heteroaryl;
preferably, R₃, R₄ are the same or different, each independently selected from hydrogen, C1-C6 alkyl, substituted C1-C6 alkyl, C3-C8 cycloalkyl, C3-C8 cycloalkyl C1-C6 alkyl and C6-C10 aryl C1-C6 alkyl;
further preferably, R₃, R₄ are the same or different, each independently selected from hydrogen, C1-C6 alkyl, halo-C1-C6 alkyl, C3-C6 cycloalkyl, C3-C6 cycloalkyl C1-C6 alkyl, and phenyl C1-C6 alkyl;
more preferably, R₃, R₄ are the same or different, each independently selected from hydrogen, methyl, ethyl, trifluoromethyl, cyclopropyl, cyclopropylmethyl, and benzyl;
most preferably, of R₃, R₄, one is selected from hydrogen, methyl, and the other is selected from hydrogen, methyl, ethyl, trifluoromethyl, cyclopropyl, cyclopropylmethyl, benzyl;
alternatively, R₃, R₄ and the carbon atoms to which they are attached constitute a C3-C8 cycloalkyl group, a 3-7-membered heterocyclic group, a substituted 3-7-membered heterocyclic group;
preferably, R₃, R₄ and the carbon atoms to which they are attached constitute a C3-C8 cycloalkyl group;
more preferably, R₃, R₄ and the carbon atoms to which they are attached constitute a C3-C6 cycloalkyl group;
most preferably, R₃, R₄ and the carbon atoms to which they are attached constitute cyclopropyl, cyclobutyl or cyclopentyl;
R₅ is selected from hydrogen, deuterium, halogen, C1-C6 alkyl, substituted C1-C6 alkyl, C3-C8 cycloalkyl C1-C6 alkyl, and C1-C6 alkoxy C1-C6 alkyl, R₅ is preferably selected from hydrogen, C1-C6 alkyl, R₅ is more preferably selected from hydrogen, methyl;
m is an integer from 0 to 5, preferably 0, or 1;
the chiral carbon atom indicated at position * has an R or S absolute configuration;
and the following conditions is met, when M' is 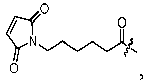 B is present and is a modifier unit.

16. The linker-payload, or its stereoisomer, pharmaceutically acceptable salt or solvate thereof according to Claim 15, wherein the M' is selected from 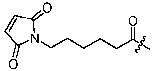 or a connector unit as shown in formula A' below, preferably M' is a connector unit as shown in formula A' below; 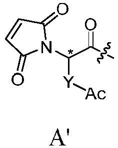
wherein Y is a scaffold selected from C1-C6 alkyl, substituted C1-C6 alkyl, and C3-C8 cycloalkyl; preferably Y is C1-C6 alkyl, more preferably C1-C3 alkyl, and most preferably methylene;
the chiral carbon atom indicated at position * has an R or S absolute configuration;
the position indicated by the wavy line on the right is connected to B or L;
the hydrophilic structural unit Ac comprises a natural or non-natural amino acid, 1-20 polyethylene glycol, phosphate group, carboxylic acid group, sulfonic acid group, sulfinic acid group, or the following structures. 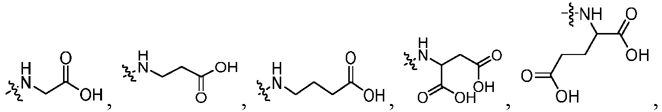 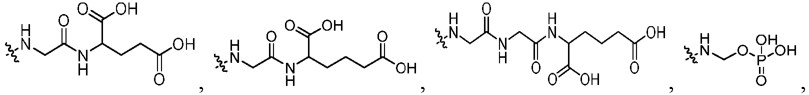 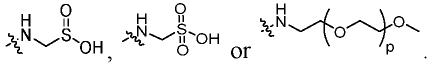
wherein p is an integer from 0 to 10; the position indicated by the wavy line is connected to the bracket Y;
preferably, the hydrophilic structural unit Ac is selected from 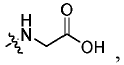 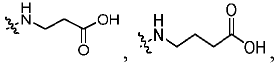 and is connected to the bracket Y at the position indicated by the wavy line;
more preferably, the hydrophilic structural unit Ac is 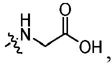 connected to the bracket Y at the position indicated by the wavy line.

17. The linker-payload, or its stereoisomer, pharmaceutically acceptable salt or solvate thereof according to Claim 15 to 16, wherein the connector unit M' comprises the structure shown in the following formula: 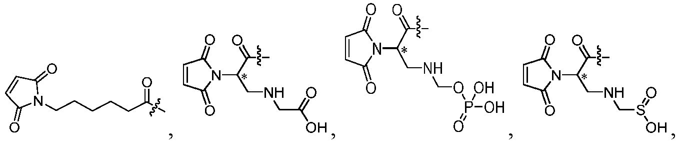 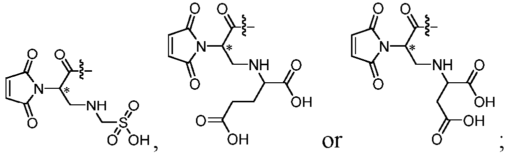
preferably, the connector unit M' is selected from 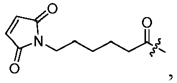 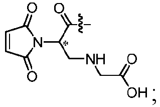
more preferably, the connector unit M' is 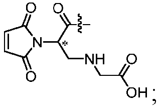
wherein:
the chiral carbon atom indicated at position * has an R or S absolute configuration;
the position indicated by the wavy line on the right is connected to B or L.

18. The linker-payload, or its stereoisomer, pharmaceutically acceptable salt or solvate thereof according to any one of Claims 15 to 17, wherein the modifier unit B is present or absent, and when present, B comprises a hydrophilicity-enhancing structure or a stereoisomer thereof.

19. The linker-payload, or its stereoisomer, pharmaceutically acceptable salt or solvate thereof according to any one of Claims 15 to 18, wherein the modifier unit B is present or absent, and when present, B comprises a hydrophilicity-enhancing structure or a stereoisomer thereof as shown in the following formula: 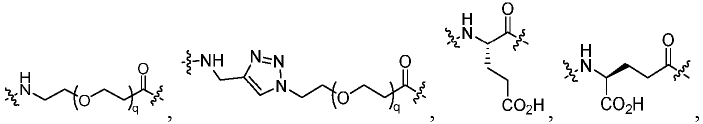 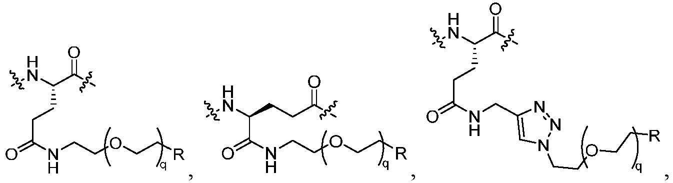 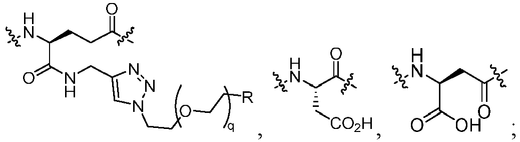
preferably, the modifier unit B is present or absent, and when present, B comprises a hydrophilicity-enhancing structure or a stereoisomer thereof as shown in the following formula: 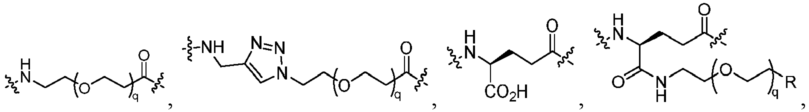 and 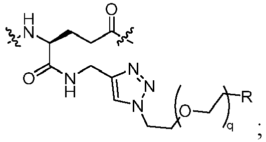
wherein:
R comprises hydroxyl, amino, polyethylene glycol, carboxylic acid group, sulfonic acid group, sulfinic acid group, phosphoric acid group, C1-C6 alkyl, C1-C6 alkoxy, natural or non-natural amino acid residue, sugar, or a derivative thereof, or a combination thereof;
preferably, R is selected from C1-C6 alkoxy, carboxylic acid group, and amino group;
more preferably, R is selected from methoxy, carboxylic acid group, and amino group;
the position indicated by the wavy line on the left is connected to M';
the position indicated by the wavy line on the right is connected to L;
q is an integer from 1-10, preferably 2, 7, or 8;
most preferably, the modifier unit B is selected from a hydrophilicity-enhancing structure or a stereoisomer thereof as shown in the following formula: 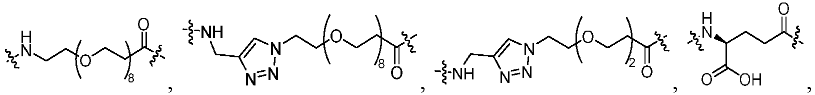 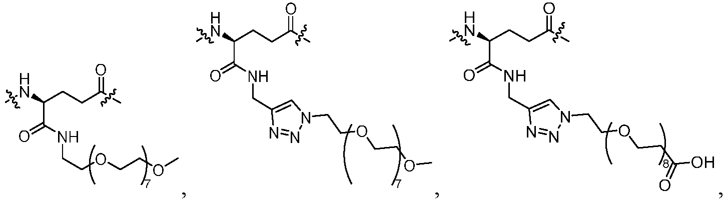 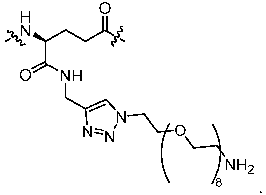
the position indicated by the left wavy line is connected to M', and the position indicated by the right wavy line is connected to L.

20. The linker-payload of or its stereoisomer, pharmaceutically acceptable salt or solvate thereof according to any one of Claims 15 to 19, wherein the linker unit L is L₁-L₂.
L₁ is a peptide residue comprising 2-10 amino acid residues, the peptide residue having the amino terminus connected to M' or B and the carbonyl terminus connected to L₂;
preferably, L₁ is a peptide residue comprising 2-4 amino acid residues, the peptide residue having an amino terminus connected to M' or B and a carbonyl terminus connected to L₂; preferably, the amino acid is selected from valine, alanine, phenylalanine, glycine, lysine, citrulline, serine, glutamic acid, and aspartic acid; more preferably, the amino acid is selected from valine, alanine, phenylalanine, glycine, lysine, and citrulline; most preferably, the amino acid is selected from valine, phenylalanine, glycine, lysine, and citrulline;
more preferably, L₁ is selected from the following peptide residues: glycine-glycine-phenylalanine-glycine, valine-citrulline, phenylalanine-lysine, valine-alanine, alanine-alanine-alanine (preferably selected from glycine-glycine-phenylalanine-glycine, valine-citrulline, phenylalanine-lysine), the amino terminus of the peptide residues is connected to M' or B and the carbonyl terminus is connected to L₂;
most preferably, L₁ is selected from the following peptide residues: 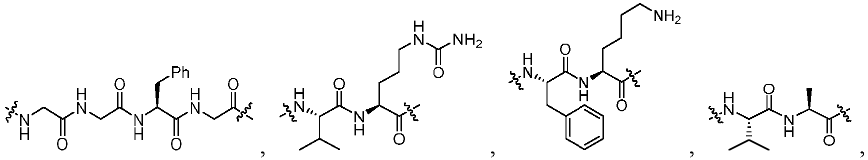 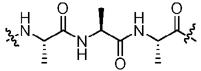
(preferably from 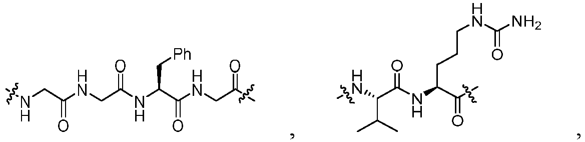 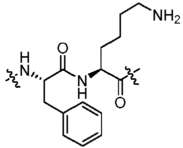 ), the peptide residues having an amino terminus connected to M' or B and a carbonyl terminus connected to L₂;
L₂ is selected from 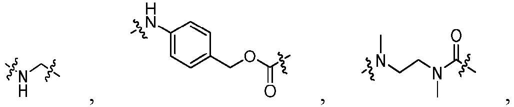 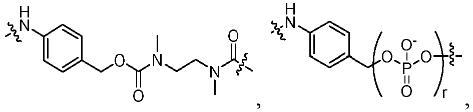 and is connected to L₁ at the position indicated by the left wavy line, and to X at the position indicated by the right wavy line;
preferably, L₂ is selected from 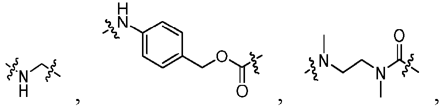 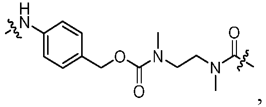 and is connected to L₁ at the position indicated by the left wavy line, and to X at the position indicated by the right wavy line;
more preferably, L₂ is selected from 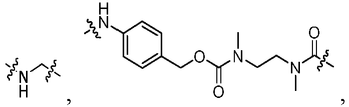 and is connected to L₁ at the position indicated by the left wavy line, and to X at the position indicated by the right wavy line;
preferably, linker unit L comprises the following structures or stereoisomers thereof. 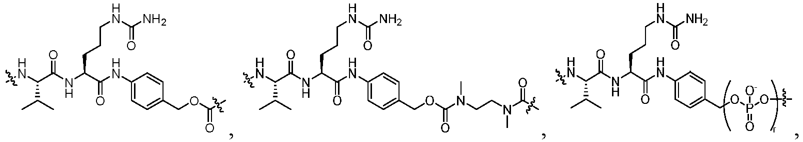 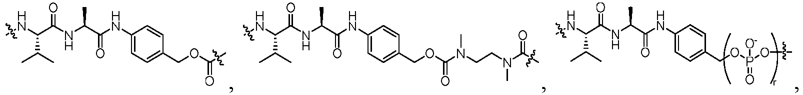 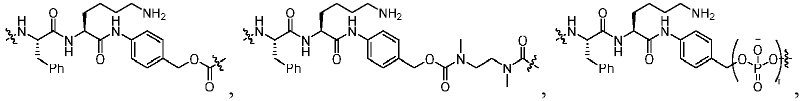 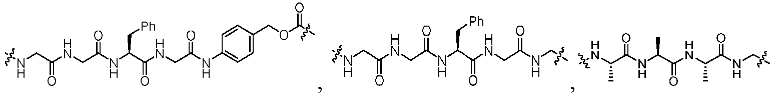
or 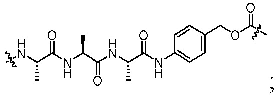
wherein:
r is an integer between 1-10;
more preferably, the linker unit L is selected from the following structures or stereoisomers thereof: 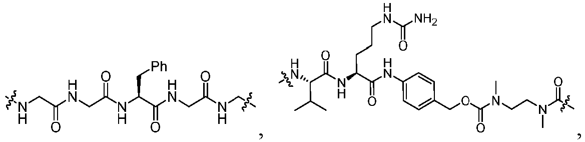 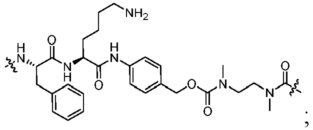
the linker unit L is connected to the connector unit M' or the modifier unit B at the position indicated by the wavy line on the left side;
the linker unit L is connected to X at the position indicated by the wavy line on the right side.

21. The linker-payload or its stereoisomer, pharmaceutically acceptable salt or solvate thereof according to any one of Claims 15 to 20, wherein the linker-payload comprises the following structures or stereoisomers thereof: 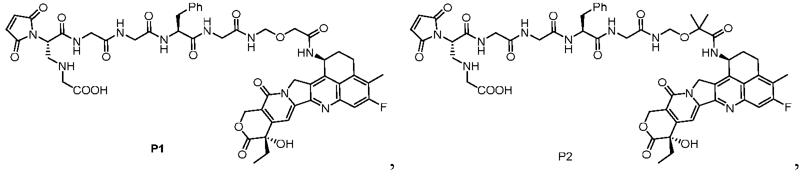 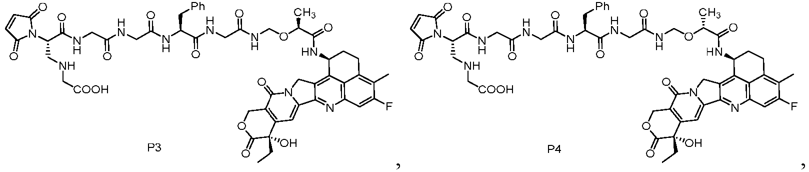 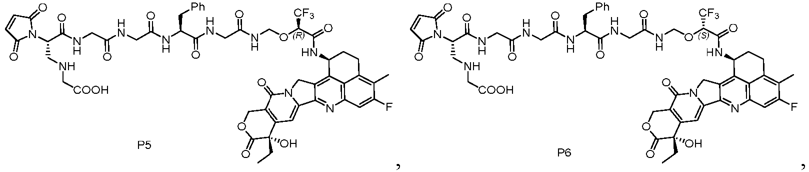 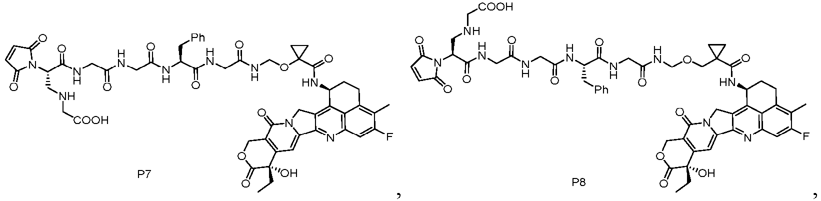 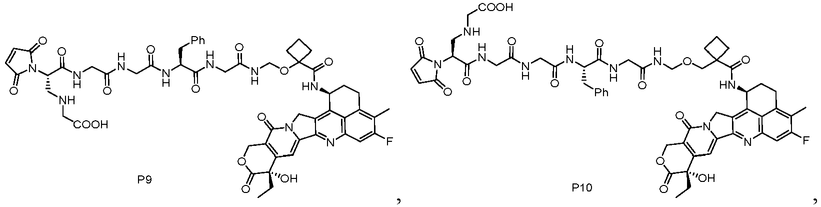 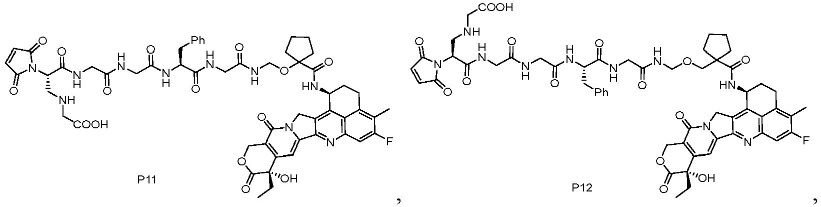 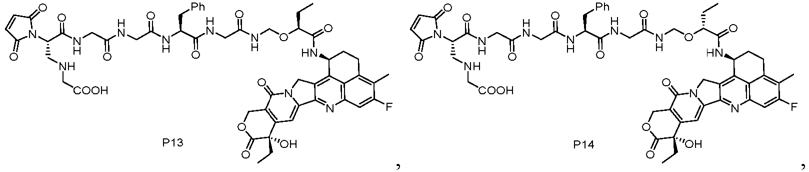 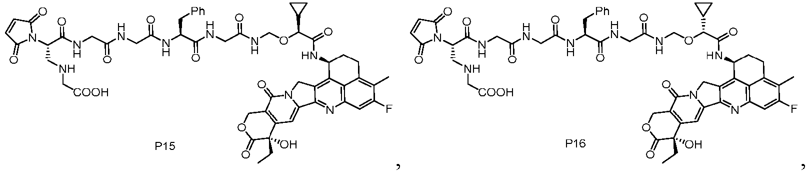 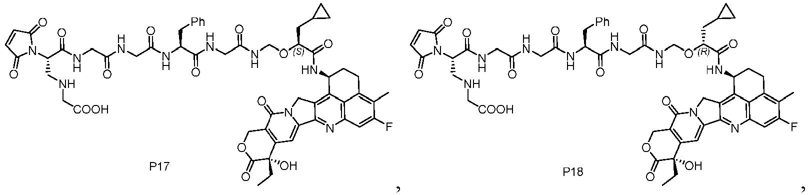 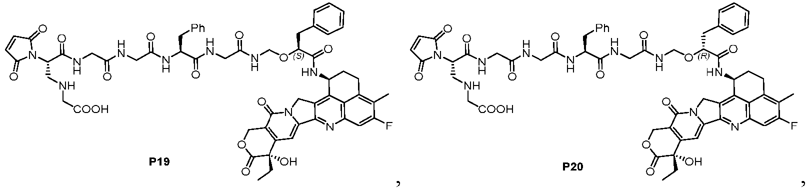 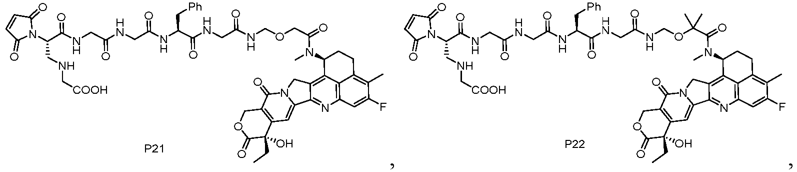 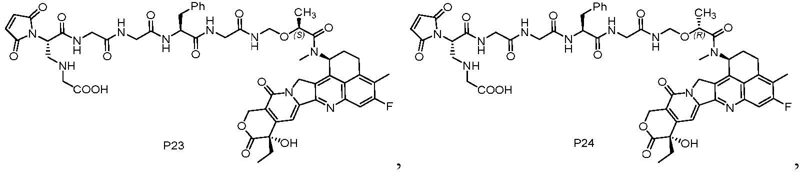 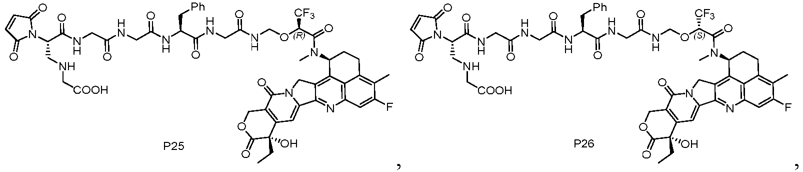 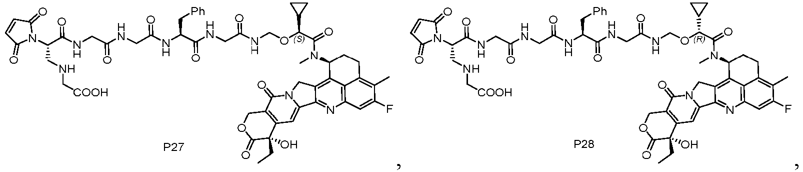 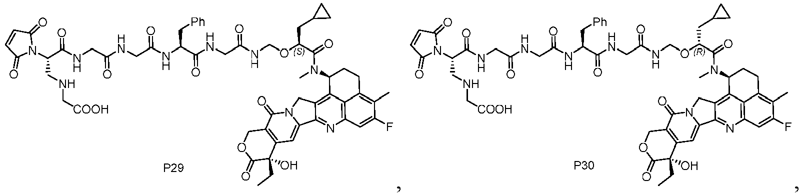 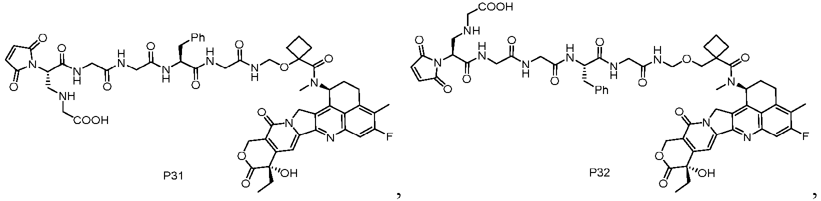 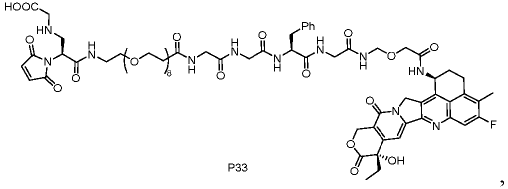 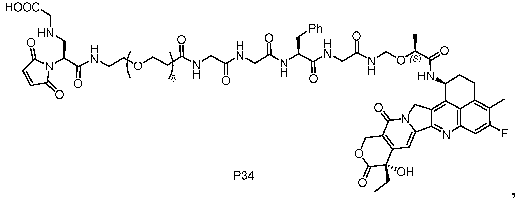 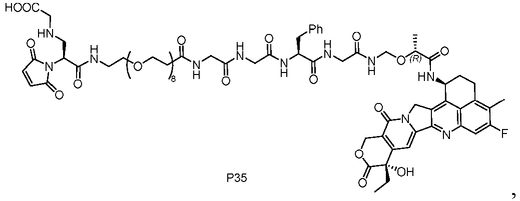 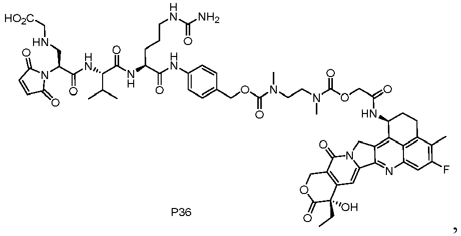 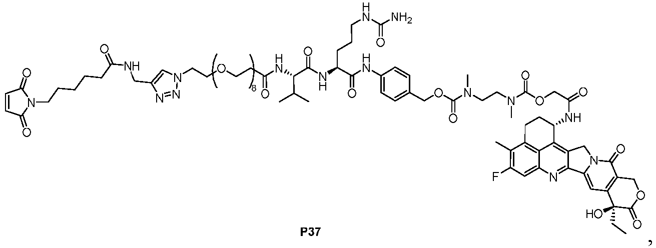 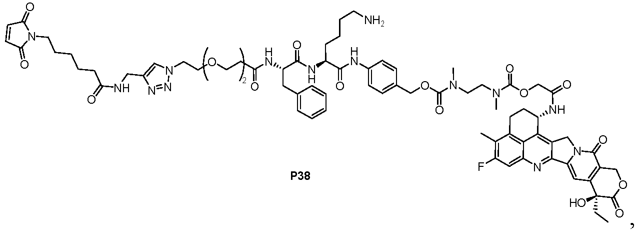 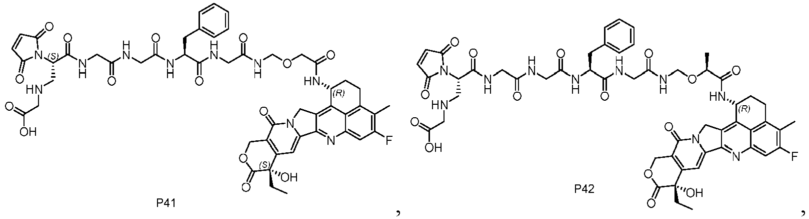 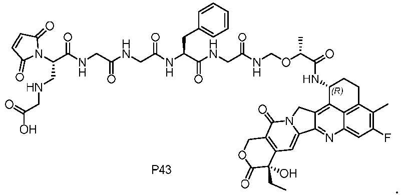 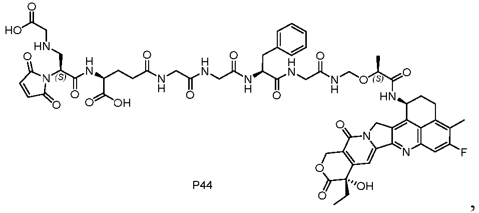 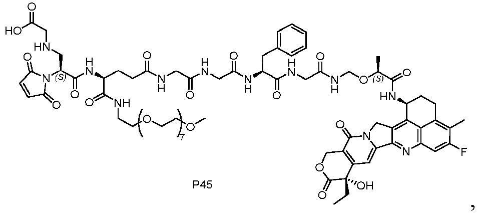 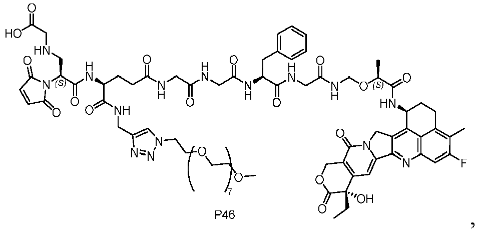 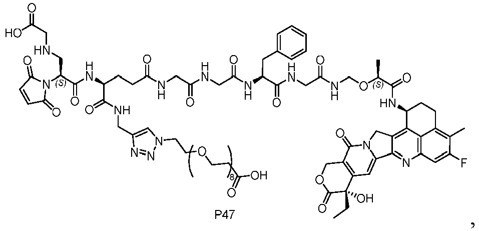 or

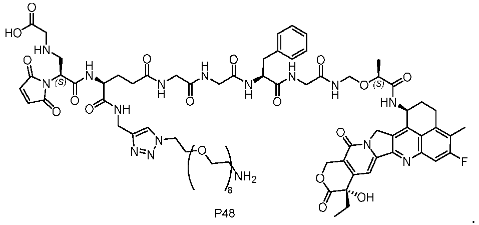

22. A method of preparing the antibody-drug conjugate or its stereoisomer, pharmaceutically acceptable salt or solvate thereof according to any one of Claims 1 to 14, comprising: 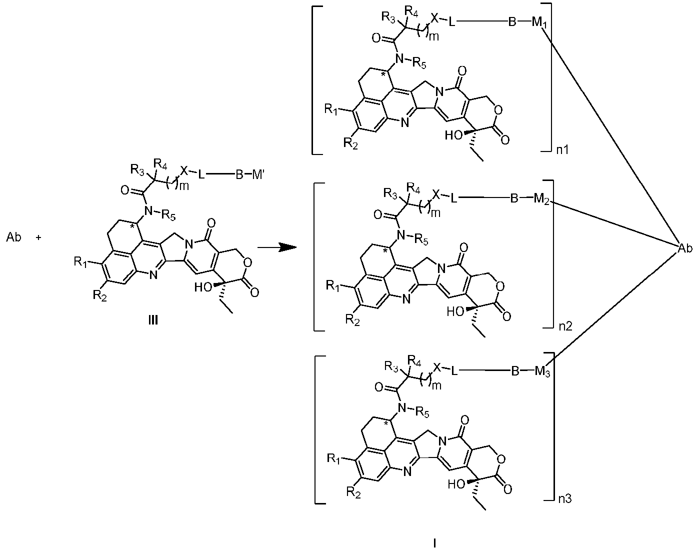
conjugating Ab with a linker-payload shown in general formula **III** to obtain the antibody-drug conjugate or its stereoisomer, pharmaceutically acceptable salt or solvate thereof shown in general formula **I** of any one of Claims 1 to 14,
wherein:
Ab is selected from anti-CD33 antibodies and antigen-binding fragments thereof;
preferably, the anti-CD33 antibody is as defined in any one of claims 10 to 13;
M₁, M₂, M₃, B, L, X, R₁, R₂, R₃, R₄, R₅, m, n1, n2, n3 are as defined in any one of Claims 1 to 8 and M' is as defined in any one of Claims 15 to 17;
the chiral carbon atom indicated at position * has an R or S absolute configuration.

23. A pharmaceutical composition comprising the antibody-drug conjugate or its stereoisomer, pharmaceutically acceptable salt or solvate thereof according to any one of Claim 1 to 14, or the linker-payload or the stereoisomer, pharmaceutically acceptable salt or solvate thereof according to any one of Claims 15 to 21, and optionally a pharmaceutically acceptable carrier.

24. A pharmaceutical formulation comprising the antibody-drug conjugate or its stereoisomer, pharmaceutically acceptable salt or solvate thereof according to any one of Claim 1 to 14, or the linker-payload or the stereoisomer, pharmaceutically acceptable salt or solvate thereof according to any one of Claims 15 to 21.

25. Use of the antibody-drug conjugate or the stereoisomer, pharmaceutically acceptable salt or solvate thereof according to any one of Claim 1 to 14, or the linker-payload or the stereoisomer, pharmaceutically acceptable salt or solvate thereof according to any one of claims 15 to 21, the pharmaceutical composition of Claim 23, or the pharmaceutical formulation of Claim 24, in the preparation of a drug for treating or prevention of cancer or tumors;
preferably, the cancer or tumor expresses CD33;
more preferably, the cancer or tumor is selected from solid tumors or hematologic tumors such as adenocarcinoma, ovarian cancer, cervical cancer, uterine cancer, prostate cancer, renal cancer, urethral cancer, bladder cancer, liver cancer, gastric cancer, endometrial cancer, salivary gland cancer, esophageal cancer, lung cancer (e.g., nonsmall cell lung cancer), colon cancer, breast cancer (e.g., triple-negative breast cancer), rectal cancer, colorectal cancer, bone cancer, skin cancer (e.g., epidermal cancer), thyroid cancer, pancreatic cancer, melanoma, glioma, neuroblastoma, glioblastoma multiforme, sarcoma, lymphoma, myeloma, and leukemia.

26. Use of the antibody-drug conjugate or the stereoisomer, pharmaceutically acceptable salt or solvate thereof according to any one of Claim 1 to 14, or the linker-payload or the stereoisomer, pharmaceutically acceptable salt or solvate thereof according to any one of Claims 15 to 21, the pharmaceutical composition of Claim 23, or the pharmaceutical formulation of Claim 24, in treatment or prevention of cancer or tumors;
preferably, the cancer or tumor expresses CD33;
more preferably, the cancer or tumor is selected from solid tumors or hematologic tumors such as adenocarcinoma, ovarian cancer, cervical cancer, uterine cancer, prostate cancer, renal cancer, urethral cancer, bladder cancer, liver cancer, gastric cancer, endometrial cancer, salivary gland cancer, esophageal cancer, lung cancer (e.g., nonsmall cell lung cancer), colon cancer, breast cancer (e.g., triple-negative breast cancer), rectal cancer, colorectal cancer, bone cancer, skin cancer (e.g., epidermal cancer), thyroid cancer, pancreatic cancer, melanoma, glioma, neuroblastoma, glioblastoma multiforme, sarcoma, lymphoma, myeloma, and leukemia.
